(19) Europäisches Patentamt
European Patent Office
Office européen des brevets

(11) EP 2 271 770 B1

(12) **EUROPEAN PATENT SPECIFICATION**

(45) Date of publication and mention of the grant of the patent:
**22.08.2018 Bulletin 2018/34**

(21) Application number: **09726775.1**

(22) Date of filing: **31.03.2009**

(51) Int Cl.:
***C12Q 1/68*** *(2018.01)*

(86) International application number:
**PCT/US2009/039033**

(87) International publication number:
**WO 2009/124090 (08.10.2009 Gazette 2009/41)**

(54) **COMPOSITIONS AND METHODS FOR TREATING AND DIAGNOSING ASTHMA**

ZUSAMMENSETZUNGEN UND VERFAHREN ZUR BEHANDLUNG UND DIAGNOSE VON ASTHMA

COMPOSITIONS ET PROCÉDÉS POUR TRAITER ET DIAGNOSTIQUER L'ASTHME

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **31.03.2008 US 72572 P**
**01.04.2008 US 41480 P**
**20.05.2008 US 128383 P**
**16.01.2009 US 205392 P**

(43) Date of publication of application:
**12.01.2011 Bulletin 2011/02**

(60) Divisional application:
**13162396.9 / 2 631 302**

(73) Proprietors:
- **Genentech, Inc.**
  **South San Francisco, CA 94080 (US)**
- **The Regents of the University of California**
  **Oakland, CA 94607 (US)**

(72) Inventors:
- **ARRON, Joseph, R.**
  **San Mateo, CA 94402 (US)**
- **FAHY, John, V.**
  **San Francisco, CA 94116 (US)**
- **MODREK, Barmak**
  **Durham, NC 27707 (US)**
- **WOODRUFF, Prescott**
  **San Francisco, CA 94114 (US)**

(74) Representative: **Weber, Martin**
**Jones Day**
**Prinzregentenstraße 11**
**80538 München (DE)**

(56) References cited:
**WO-A-03/021261**

- **SIMPSON JODIE L ET AL: "Inflammatory subtypes in asthma: assessment and identification using induced sputum." RESPIROLOGY (CARLTON, VIC.) JAN 2006, vol. 11, no. 1, January 2006 (2006-01), pages 54-61, XP002532411 ISSN: 1323-7799**
- **WENZEL SALLY E: "Asthma: defining of the persistent adult phenotypes." LANCET 26 AUG 2006, vol. 368, no. 9537, 26 August 2006 (2006-08-26), pages 804-813, XP002532409 ISSN: 1474-547X**
- **WOODRUFF PRESCOTT G ET AL: "Genome-wide profiling identifies elpithelial cell genes associated with asthma and with treatment response to corticosteroids" PROCEEDINGS OF THE NATIONAL ACADEMY OF SCIENCES OF USA, NATIONAL ACADEMY OF SCIENCE, WASHINGTON, DC, US, vol. 104, no. 40, 1 October 2007 (2007-10-01), pages 15858-15863, XP002532408 ISSN: 0027-8424**
- **IZUHARA KENJI ET AL: "Microarray-based identification of novel biomarkers in asthma." ALLERGOLOGY INTERNATIONAL : OFFICIAL JOURNAL OF THE JAPANESE SOCIETY OF ALLERGOLOGY DEC 2006, vol. 55, no. 4, December 2006 (2006-12), pages 361-367, XP002532410 ISSN: 1323-8930**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **TAKAYAMA ET AL: "Periostin: A novel component of subepithelial fibrosis of bronchial asthma downstream of IL-4 and IL-13 signals" JOURNAL OF ALLERGY AND CLINICAL IMMUNOLOGY, MOSBY - YEARLY BOOK, INC, US, vol. 118, no. 1, 1 July 2006 (2006-07-01), pages 98-104, XP005610109 ISSN: 0091-6749**
- **STANKOVIC KONSTANTINA M ET AL: "Gene expression profiling of nasal polyps associated with chronic sinusitis and aspirin-sensitive asthma" LARYNGOSCOPE, TRIOLOGICAL FOUNDATION, ST LOUIS, US, vol. 118, no. 5, 1 May 2008 (2008-05-01), pages 881-889, XP009113976 ISSN: 0023-852X**
- **TILMAN GAÃ LLE ET AL: "Human periostin gene expression in normal tissues, tumors and melanoma: evidences for periostin production by both stromal and melanoma cells" MOLECULAR CANCER, BIOMED CENTRAL, LONDON, GB, vol. 6, no. 1, 17 December 2007 (2007-12-17), page 80, XP021036924 ISSN: 1476-4598**
- **LIAO QUAN ET AL: "Combined detection of serum tumor markers for differential diagnosis of solid lesions located at the pancreatic head." HEPATOBILIARY & PANCREATIC DISEASES INTERNATIONAL : HBPD INT DEC 2007, vol. 6, no. 6, December 2007 (2007-12), pages 641-645, XP002541522 ISSN: 1499-3872**
- **SASAKI HIDEFUMI ET AL: "Elevated serum periostin levels in patients with bone metastases from breast but not lung cancer." BREAST CANCER RESEARCH AND TREATMENT FEB 2003, vol. 77, no. 3, February 2003 (2003-02), pages 245-252, XP002541523 ISSN: 0167-6806**
- **SANZ M L ET AL: "Serum eosinophil peroxidase (EPO) levels in asthmatic patients." ALLERGY APR 1997, vol. 52, no. 4, April 1997 (1997-04), pages 417-422, XP002541524 ISSN: 0105-4538**
- **WENZEL S E ET AL: "Bronchoscopic evaluation of severe asthma. Persistent inflammation associated with high dose glucocorticoids." AMERICAN JOURNAL OF RESPIRATORY AND CRITICAL CARE MEDICINE SEP 1997, vol. 156, no. 3 Pt 1, September 1997 (1997-09), pages 737-743, XP002541525 ISSN: 1073-449X**
- **VIGNOLA A M ET AL: "Airway inflammation in mild intermittent and in persistent asthma." AMERICAN JOURNAL OF RESPIRATORY AND CRITICAL CARE MEDICINE FEB 1998, vol. 157, no. 2, February 1998 (1998-02), pages 403-409, XP002541526 ISSN: 1073-449X**
- **WENZEL ET AL: "Effect of an interleukin-4 variant on late phase asthmatic response to allergen challenge in asthmatic patients: results of two phase 2a studies", THE LANCET, THE LANCET PUBLISHING GROUP, GB, vol. 370, no. 9596, 18 October 2007 (2007-10-18), pages 1422-1431, XP022303966, ISSN: 0140-6736, DOI: 10.1016/S0140-6736(07)61600-6**

## Description

FIELD

Compositions and methods for diagnosing subtypes of asthma patients are provided. Also disclosed are methods for identifying effective asthma therapeutic agents and predicting responsiveness to asthma therapeutic agents.

BACKGROUND

Asthma is traditionally thought to result from aeroallergen-induced inflammation driven by T-helper type 2 (Th2) processes and mediated by cytokines including interleukin (IL)-4, IL-5 and IL-13. IL-13 is a pleiotropic Th2 cytokine produced by activated T cells, basophils, eosinophils, and mast cells, and it has been strongly implicated in the pathogenesis of asthma in preclinical models [2]. Elevated levels of IL-13 have been detected in the airways of human asthma patients; however, this elevation is only observed in a subset of asthmatics [3-6]. Recent research has been directed at understanding how Th2 cytokines cause asthma-like pathology and physiology [49, 50].

While asthma is often characterized by eosinophilic infiltration of the airways, there is increasing evidence that there are other subtypes of the disease driven by alternative forms of inflammation [1, 39, 48]. For example, studies of the cellular components of airway inflammation in asthma provide evidence for distinct eosinophilic and non-eosinophilic phenotypes of asthma [1, 39, 48]. Whether the molecular mechanisms underlying these clinical and cellular phenotypes of asthma differ is unknown. The identification of and development of biomarkers for distinct molecular phenotypes of asthma would guide the direction of basic research and the clinical application of emerging asthma therapies that specifically target Th2 responses in the lung.

Periostin is a secreted protein associated with fibrosis whose expression is upregulated by recombinant IL-4 and IL-13 in bronchial epithelial cells [7, 8] and bronchial fibroblasts [9]. It is expressed at elevated levels in vivo in bronchial epithelial cells [8] and in the subepithelial bronchial layer [9] of human asthmatics as well as in a mouse model of asthma [10]. It is also expressed at elevated levels in the esophageal epithelium of patients with eosinophilic esophagitis in an IL-13 dependent manner [11]. Elevated periostin expression has been observed in several types of epithelial derived cancers [64-67], and elevated levels of soluble periostin have been observed in the serum of some cancer patients [64, 68-70].

Genome-wide expression microarray analyses of bronchial epithelial cells from 42 mild-to-moderate, steroid-naive asthmatics and 28 healthy control subjects have been performed [8]. In those studies, three of the most differentially expressed epithelial genes between all asthmatics and all healthy controls were periostin, CLCA1, and serpinB2 [8]. Furthermore, those genes were significantly downregulated in bronchial epithelial cells of asthmatics after 7 days of inhaled corticosteroid (ICS) treatment [8]. All three of those genes are induced in bronchial epithelial cells by recombinant IL-13 treatment in vitro and their expression is markedly attenuated by addition of corticosteroids to the cell culture medium [8].

To date, such genome-wide expression analyses have not identified genetic biomarkers that are prognostic or predictive of therapeutic response to treatment for individual asthma patients, nor have they identified genetic biomarkers that distinguish subtypes of asthmatic patients. In addition, no reliable nongenetic biomarkers with broad clinical applicability for prognostic or predictive responses to therapeutic treatment, or diagnostic of subtypes of asthma, have been identified. Thus, as asthma patients seek treatment, there is considerable trial and error involved in the search for therapeutic agent(s) effective for a particular patient. Such trial and error often involves considerable risk and discomfort to the patient in order to find the most effective therapy.

Thus, there is a need for more effective means for determining which patients will respond to which treatment and for incorporating such determinations into more effective treatment regimens for asthma patients.

The invention described herein meets the above-described needs and provides other benefits.

SUMMARY

Using gene expression signatures in bronchial epithelium, we have defined distinct molecular subtypes of asthma. Surprisingly, supervised clustering of the data based on a set of genes whose expression was highly correlated to genes known to be upregulated by IL-4 or IL-13 stimulation revealed not one but two distinct clusters of asthma patients. Furthermore, analysis of these dichotomous subsets of asthmatics revealed significant associations between "IL-4/13 signature" status and serum total IgE levels, serum CEA levels, serum periostin levels, peripheral blood eosinophilia, (bronchoalveolar lavage) BAL eosinophilia, and responsiveness to inhaled corticosteroids (each $p<0.05$ by Wilcoxon rank sum test).

Accordingly, the present invention relates to methods of diagnosing a subpopulation of asthma patients comprising measuring the gene expression of POSTN, wherein elevated expression levels of POSTN as compared to healthy controls, are indicative of the asthma patients subpopulation. In one embodiment, the asthma patients additionally express elevated levels of any one or combination of genes selected from CST1, CCL26, CLCA1, CST2, PRR4, SERPINB2, CEACAM5, iNOS, SERPINB4, CST4, PRB4, TPSD1, TPSG1, MFSD2, CPA3, GPR105, CDH26, GSN, C2ORF32, TRACH2000196 (TMEM71), DNAJC12, RGS13, SLC18A2, SERPINB10, SH3RF2, FCER1B, RUNX2, PTGS1, and ALOX15. In one embodiment, the gene expression is measured of POSTN and any one or combination of genes selected from the group of consisting of CST1, CST2, CCL26, CLCA1, PRR4, PRB4, SERPINB2, CEACAM5, iNOS, SERPINB4, CST4, and SERPINB10. According to one embodiment, the gene expression is measured by microarray. According to another embodiment, the gene expression is measured by observing protein expression levels of an aforementioned gene. According to another embodiment, the gene expression is considered elevated when compared to a healthy control if the relative mRNA level of the gene of interest is greater than 2.5 of the level of a control gene mRNA. According to another embodiment, the relative mRNA level of the gene of interest is greater than 3 fold, 5 fold, 10 fold, 15 fold 25 fold or 30 fold compared to a healthy control gene expression level. According to one embodiment, the gene expression is measured by a method selected from the group consisting of a PCR method, a microarray method or a immunoassay method. In one embodiment, the microarray method comprises the use of a microarray chip having one or more nucleic acid molecules that can hybridize under stringent conditions to a nucleic acid molecule encoding a gene mentioned above or having one or more polypeptides (such as peptides or antibodies) that can bind to one or more of the proteins encoded by the genes mentioned above. In one embodiment, the PCR method is qPCR. According to one embodiment, the immunoassay method comprises the steps of binding an antibody to protein expressed from a gene mentioned above in a patient sample mentioned above and determining if the protein level from the patient sample is elevated. According to one embodiment, a control gene is a housekeeping gene selected from the group consisting of actin, GAPDH, GASB and GUSB.

The present application discloses a microarray chip comprising nucleic acid sequences encoding the following genes: POSTN, CST1, CST2, CCL26, CLCA1, PRR4, SERPINB2, CEACAM5, iNOS, SERPINB4, CST4, and SERPINB10 or fragments there of. The present application discloses a microarray chip comprising nucleic acid sequences encoding the following genes: POSTN, CST1, CCL26, CLCA1, CST2, PRR4, SERPINB2, CEACAM5, iNOS, SERPINB4, CST4, PRB4, TPSD1, TPSG1, MFSD2, CPA3, GPR105, CDH26, GSN, C2ORF32, TRACH2000196 (TMEM71), DNAJC12, RGS13, SLC18A2, SERPINB10, SH3RF2, FCER1B, RUNX2, PTGS1, and ALOX1, or fragments thereof.

The present application discloses a subpopulation of asthma patients to be treated with the therapeutic agents of this invention, wherein the ratio of Muc5AC:MUC5B protein or mRNA levels in the airway epithelial cells of asthma patients is greater than 25.

The present application also discloses to methods of diagnosing a subpopulation of asthma patients by taking single or combinations of measurements of systemic biomarkers selected from serum CEA levels, serum IgE levels, serum periostin levels, peripheral blood eosinophil counts and eosinophil percentages in bronchoalveolar lavage fluid (BAL). Systemic biomarkers typically are nongenetic biomarkers and are typically measured in samples obtained by noninvasive procedures, for example, but not limited to, collection of blood or blood components, e.g., serum or plasma. According to one embodiment, greater than 100IU/ml IgE levels and/or 0.14 x 10e9/L eosinophils is predictive of a patient population to be treated with the therapeutic agents of this invention.

The present application further discloses to methods of treating asthma comprising administering a therapeutic agent to a patient expressing elevated levels of any one or combination of the genes selected from POSTN, CST1, CCL26, CLCA1, CST2, PRR4, SERPINB2, CEACAM5, iNOS, SERPINB4, CST4, PRB4, TPSD1, TPSG1, MFSD2, CPA3, GPR105, CDH26, GSN, C2ORF32, TRACH2000196 (TMEM71), DNAJC12, RGS13, SLC18A2, SERPINB10, SH3RF2, FCER1B, RUNX2, PTGS1, ALOX15. The patient may express elevated levels of any one or combination of genes selected from the group consisting of periostin, CST1, CST2, CCL26, CLCA1, PRR4, SerpinB2, CEACAM5, iNOS, PRB4, SerpinB4, SerpinB10 and CST4. The may be to be treated may be a mild-to-moderate, steroid-naive (never treated with steroids) asthma patient. The patient to be treated may be a moderate-to-severe, steroid-resistant (non-responsive to steroids) asthma patient. Such patients are treated with a therapeutically effective amount of a therapeutic agent. The patient may have asthma induced by the TH2 pathway.

The therapeutic agent may be an anti-IL13/IL4 pathway inhibitor. The therapeutic agent may target the TH2 induced asthma pathway. Exemplary targets include, but are not limited to, cytokines or ligands such as: IL-9, IL-5, IL-13, IL-4, OX40L, TSLP, IL-25, IL-33 and IgE; and receptors such as: IL-9 receptor, IL-5 receptor, IL-4receptor alpha, IL-13receptoralpha1 and IL-13receptoralpha2, OX40, TSLP-R, IL-7Ralpha (a co-receptor for TSLP), IL17RB (receptor for IL-25), ST2 (receptor for IL-33), CCR3, CCR4, CRTH2, FcepsilonRI and FcepsilonRII/CD23 (receptors for IgE). Accordingly, a therapeutic agent according to this invention includes an agent that can bind to the target above, such as a polypeptide(s) (e.g., an antibody, an immunoadhesin or a peptibody), an aptamer or a small molecule.

For example, the therapeutic agent may be an anti-IL 13 antibody. The anti-IL-13 antibody may comprise a VH sequence comprising SEQ ID NO: 193 and a VL sequence comprising SEQ ID NO:194. According to another embodiment, the anti-IL13 antibody comprises: (a) an HVR-L1 comprising amino acid sequence RASKSVDSYGNSFMH (SEQ ID NO:195); (b) an HVR-L2 comprising amino acid sequence LASNLES (SEQ ID NO:196); (c) an HVR-L3 comprising amino acid sequence QQNNEDPRT (SEQ ID NO: 197); (d) an HVR-H1 comprising amino acid sequence AYSVN (SEQ ID NO:198); (e) an HVR-H2 comprising amino acid sequence MIWGDGKIVYNSALKS (SEQ ID NO: 199); and (f) an HVR-H3 comprising amino acid sequence DGYYPYAMDN (SEQ ID NO: 200). Alternatively, the therapeutic agent is an anti-OX40 ligand (OX40L) antibody, or an anti-IL13/anti-IL4 bispecific antibody, or an anti-IgE antibody. Specifically, the therapeutic agent is an antibody directed against the membrane proximal M1' region of surface expressed IgE on B cells. The therapeutic agent may be an inhaled corticosteroid for example, the inhaled corticosteroid selected from beclomethasone diproprionate, budesonide, flunisolide, fluticasone propionate, mometasone, and triamcinolone acetonide.

The anti-OX40L antibody may comprise: (a) an HVR-L1 comprising sequence RSSQSPVHSNGNTYLH (SEQ ID NO:201); (b) an HVR-L2 comprising sequence KVSNRFS (SEQ ID NO: 202); (c) an HVR-L3 comprising sequence SQSTHIPWT (SEQ ID NO: 203); (d) an HVR-H1 comprising sequence SYWMH (SEQ ID NO: 204); (e) an HVR-H2 comprising sequence EIDPSNGRTNYNEKFKS (SEQ ID NO: 205); and (f) an HVR-H3 comprising sequence ERSPRYFDV (SEQ ID NO:206). According to another embodiment, the anti-OX40L antibody comprises: (a) an HVR-L1 comprising sequence RSSQSIVHGNGNTYLE (SEQ ID NO:207); (b) an HVR-L2 comprising sequence RVSNRFS (SEQ ID NO:208); (c) an HVR-L3 comprising sequence FQGSHVPYT (SEQ ID NO:209); (d) an HVR-H1 comprising sequence SYWLN (SEQ ID NO:210); (e) an HVR-H2 comprising sequence MIDPSDSETHYNQVFKD (SEQ ID NO:211); and (f) an HVR-H3 comprising sequence GRGNFYGGSHAMEY (SEQ ID NO:212). According to another embodiment, the anti-OX40L antibody comprises (a) an HVR-H1 comprising sequence SYTMH (SEQ ID NO:215), SYAMS (SEQ ID NO:216), NFGMH (SEQ ID NO:217), or NYGMH (SEQ ID NO:218), (b) an HVR- H2 comprising sequence IISGSGGFTYYADSVKG (SEQ ID NO:219), AIWYDGHDKYYSYYVKG (SEQ ID NO:220), AIWYDGHDKYYAYYVKG (SEQ ID NO:221), VIWYDGSNKYYVDSVKG (SEQ ID NO:222), or VIWNDGSNKYYVDSVKG (SEQ ID NO:223), (c) an HVR-H3 comprising sequence DSSSWYRYFDY (SEQ ID NO:224), DRLVAPGTFDY (SEQ ID NO:225), KNWSFDF (SEQ ID NO:226), or DRMGIYYYGMDV (SEQ ID NO:227), (d) an HVR-L1 comprising sequence RASQGISSWLA (SEQ ID NO:228), RASQSVSSSYLA (SEQ ID NO:229), RASQSVSSNYLA (SEQ ID NO:230), RASQGVSRYLA (SEQ ID NO:231), or RASQSVSSYLA (SEQ ID NO:232), (e) an HVR- L2 comprising sequence GASSRAT (SEQ ID NO:233), AASSLQS (SEQ ID NO:234), MPPVWKV (SEQ ID NO:235), DASNRAT (SEQ ID NO:236), or LHPLCKV (SEQ ID NO:237); and (f) an HVR-L3 comprising sequence NSLIVTLT (SEQ ID NO:238), QQYNSYPYT (SEQ ID NO:239), QQYGSSFT (SEQ ID NO:240),QQRSNWQYT (SEQ ID NO:241), QQRSNWT (SEQ ID NO:242), or NSIIVSLT (SEQ ID NO:243), wherein the anti-OX40L antibody binds OX40L. The anti-IgE antibody may comprise a VL sequence comprising SEQ ID NO:213 and a VH sequence comprising SEQ ID NO:214. The anti-IgE antibody may comprise: (a) an HVR-L1 comprising sequence RSSQSLVHNNANTYLH (SEQ ID NO:244) (b) an HVR-L2 comprising sequence KVSNRFS (SEQ ID NO: 245); (c) an HVR-L3 comprising sequence SQNTLVPWT (SEQ ID NO: 246); (d) an HVR-H1 comprising sequence GFTFSDYGIA (SEQ ID NO: 247); (e) an HVR-H2 comprising sequence AFISDLAYTIYYADTVTG (SEQ ID NO: 248); and (f) an HVR-H3 comprising sequence ARDNWDAMDY (SEQ ID NO:249). The anti-IgE antibody may comprise a VH sequence comprising SEQ ID NO:250 and a VL sequence comprising SEQ ID NO:251, or the anti-IgE antibody may comprise a VH sequence comprising SEQ ID NO:252 and a VL sequence comprising SEQ ID NO:253. The anti-IgE antibody may comprises: (a) an HVR-L1 comprising sequence RSSQDISNSLN (SEQ ID NO:254) (b) an HVR-L2 comprising sequence STSRLHS (SEQ ID NO: 255); (c) an HVR-L3 comprising sequence QQGHTLPWT (SEQ ID NO: 256); (d) an HVR-H1 comprising sequence GYTFTDYYMM (SEQ ID NO: 257); (e) an HVR-H2 comprising sequence GDNIDPNNYDTSYNQKFKG (SEQ ID NO: 258); and (f) an HVR-H3 comprising sequence ASKAY (SEQ ID NO:259). The anti-IgE antibody may comprise: (a) an HVR-L1 comprising sequence RSSQDISNALN (SEQ ID NO:260) (b) an HVR-L2 comprising sequence STSRLHS (SEQ ID NO: 255); (c) an HVR-L3 comprising sequence QQGHTLPWT (SEQ ID NO: 256); (d) an HVR-H1 comprising sequence GYTFTDYYMM (SEQ ID NO: 257); (e) an HVR-H2 comprising sequence GDNIDPNNYDTSYNQKFKG (SEQ ID NO: 258); and (f) an HVR-H3 comprising sequence ASKAY (SEQ ID NO:259). The anti-IgE antibody may comprise: (a) an HVR-L1 comprising sequence RSSQDISNALN (SEQ ID NO:260) (b) an HVR-L2 comprising sequence STSRLHS (SEQ ID NO: 255); (c) an HVR-L3 comprising sequence QQGHTLPWT (SEQ ID NO: 256); (d) an HVR-H1 comprising sequence GYTFTDYYIM (SEQ ID NO: 261); (e) an HVR-H2 comprising sequence GDNIDPNNYDTSYNQKFKG (SEQ ID NO: 258); and (f) an HVR-H3 comprising sequence ASKAY (SEQ ID NO:259).

The patient may have asthma that does not involve the TH2 pathway (non-TH2 asthma), and the therapeutic agent may target non-TH2 asthma. For example, the therapeutic agent is an IL-17 pathway inhibitor, for example anti-IL-17 antibody, or an antibody cross-reactive with both IL-17A and IL-17F, or a bispecific antibody capable of binding both IL-17A and IL-17F, or an anti-IL-17A/F antibody.

The present application further discloses a kit for diagnosing an asthma subtype in a patient comprising (1) one or more nucleic acid molecules that hybridize with a gene, wherein the gene is selected from the group of consisting of POSTN, CST1, CST2, CCL26, CLCA1, PRR4, PRB4, SERPINB2, CEACAM5, iNOS, SERPINB4, CST4, and SERPINB10 and (2) instructions for measuring the expression levels of the gene from an asthma patient sample, wherein the elevated expression levels of any one, combination or all of said genes is indicative of the asthma subtype. The kit may further comprise a gene selected from the group consisting of: PRB4, TPSD1, TPSG1, MFSD2, CPA3, GPR105, CDH26, GSN, C2ORF32, TRACH2000196 (TMEM71), DNAJC12, RGS13, SLC18A2, SH3RF2, FCER1B, RUNX2, PTGS1, and ALOX15. The gene expression level may be measured by assaying for mRNA levels. The assay may comprise a PCR method or the use of a microarray chip. For example The PCR method may be qPCR. The mRNA levels of the gene of interest relative to a control gene mRNA level greater than 2.5 fold is indicative of the asthma subtype.

The application discloses a kit for diagnosing an asthma subtype in a patient comprising (1) one or more protein molecules that bind to a protein selected from the group of consisting of POSTN, CST1, CST2, CCL26, CLCA1, PRR4, PRB4, SERPINB2, CEACAM5, iNOS, SERPINB4, CST4, and SERPINB10 and (2) instructions for measuring the expression levels of the protein from a patient sample, wherein the elevated expression levels of any one, combination or all of said proteins is indicative of the asthma subtype. The kit may further comprise a protein molecule that binds to a protein selected from the group consisting of: PRB4, TPSD1, TPSG1, MFSD2, CPA3, GPR105, CDH26, GSN, C2ORF32 , TRACH2000196 (TMEM71), DNAJC12, RGS13, SLC18A2, SH3RF2. FCER1B, RUNX2, PTGS1, and ALOX15. The protein molecule may be a antibody, a peptide or a peptibody. The kit may comprise a microarray chip comprising the protein molecule(s).

The present application discloses a kit for diagnosing an asthma subtype in a patient comprising instructions for measuring any one of the biomarkers from a patient sample selected from the group consisting of: serum total IgE levels, serum CEA levels, serum periostin levels, peripheral blood eosinophils and bronchoalveolar lavage (BAL) eosinophils, wherein elevated levels of CEA, serum periostin, peripheral blood eosinophils and bronchoalveolar lavage (BAL) eosinophils. The kit may further include instructions wherein an IgE level greater than 100IU/ml is indicative of the asthma subtype. The kit may further include instruction, wherein a peripheral blood eosinophil level greater than 0.14 x 10e9/L is indicative of the asthma subtype.

The present application discloses a kit for diagnosing an asthma subtype in a patient comprising instructions for measuring the ratio of Muc5AC:MUC5B mRNA or protein from a sample of an asthma patient, wherein a ratio greater than 25 is indicative of the asthma subtype. The sample may be obtained from an epithelial brushing. The sample may comprise airway epithelial cells. The kit may include a nucleic acid molecule that hybridizes under stringent conditions with Muc5AC and a nucleic acid molecule that hybridizes under stringent conditions with MUC5B. Alternatively the kit may include a protein molecule that binds to Muc5AC and a protein molecule that binds to MUC5B. The protein molecule may be an antibody.

BRIEF DESCRIPTION OF THE DRAWINGS

Figure 1 shows gene expression levels in airway epithelium as described in Examples 1 and 2. (A) Relative expression levels of periostin (left panel), CLCA1 (middle panel), and serpinB2 (right panel) in healthy controls (N=27) and in asthmatics (N= 42) are shown. Normalized fluorescence units are indicated on the left axis of each plot. (B) Two-way comparisons of expression levels of periostin and CLCA1 (left panel), periostin and serpinB2 (middle panel), and CLCA1 and serpinB2 (right panel) in 42 asthmatics are shown. Spearman's rank order correlation (p) and p-values are indicated in each panel. (C) Gene expression microarray analysis for healthy controls and asthmatics identifying expression levels of periostin and co-regulated genes; IL-4/13 signature high cluster (cluster 1); IL-4/13 signature low cluster (cluster 2); healthy controls. (D) Heatmap depicting unsupervised hierarchical clustering (Euclidean complete) of periostin, CLCA1, and serpinB2 expression levels in bronchial epithelium across all subjects at baseline. (E) Mean (± SEM) expression levels of IL-4, IL-5, and IL-13 in bronchial biopsy homogenates obtained contemporaneously with bronchial brushings from a subset of subjects depicted in Figures 1A-D (cluster 1:18 "IL-13 high" asthmatics; cluster 2: 16 healthy controls and 14 "IL-13 low" asthmatics). Two-way correlations across all subjects between IL-4, IL-5, and IL-13 indicated at right (Spearman's rank order correlation, ρ, and p-values).

Figure 2 shows gene families for serpins, cystatins, and PRRs, and expression levels of those genes as described in Example 3. (A) Serpins (top), cystatins (middle), and PRRs (bottom) genomic loci and organization as viewed at the University of California Santa Cruz genome browser available at http://genome.ucsc.edu. (B) Hierarchical clustering of all probes encoding cystatin and serpin genes as depicted in panel A. (C) Relative gene expression levels in airway epithelium of PRR4 (left panel), PRB4 (middle panel), and CEACAM5 (right panel) in healthy controls (N=27) and in asthamatics (N= 42) are shown. Normalized fluorescence units are indicated on the left axis of each plot.

Figure 3 shows microarray analysis of bronchial epithelial brushings at baseline and after one week of inhaled fluticasone propionate (ICS) treatment as described in Example 6. (A) Periostin expression; (B) PRR4 expression; (C) RUNX2 expression.

Figure 4 shows a composite graph of serum IgE and peripheral blood eosinophils in asthmatic patients as described in Examples 7 and 9.

Figure 5 shows various clinical features of IL-13 high and IL-13 low subphenotypes of asthma as described in Example 8. (A) Volume of air exhaled in the first second of a forced expiration ($FEV_1$), a measure of airway obstruction.

(B) Improvement in $FEV_1$ after 4 puffs (360 μg) of albuterol (bronchodilator reversibility testing). (C) Provocative concentration of methacholine required to induce a 20% decline in $FEV_1$ ($PC_{20}$), a measure of airway hyper-reponsiveness.

Figure 6 shows various markers of allergy, eosinophilic inflammation and airway remodeling of IL-13-high and IL-13 low subphenotypes of asthma as described in Example 8. (A) Allergen skin prick test (SPT) results using a panel of 12 aeroallergens. (B) Serum IgE concentration. (C) Peripheral blood eosinophil count. (D) Eosinophils as a percentage of total bronchoalveolar lavage fluid (BAL) cells. (E) Stereo logic measurement of reticular basement membrane (RBM) thickness on endobronchial biopsy, a measure of sub-epithelial fibrosis. (F) Ratio of MUC5AC to MUC5B expression in epithelial brushings as determined by qPCR.

Figure 7 shows various clinical features of IL-13 high and IL-13 low subphenotypes of asthma as described in Example 8. (A) Percentage of subjects responding to specific aeroallergens as indicated along the bottom axis. "IL-13 low" asthma subphenotype; "IL-13 high" asthma subphenotype (*, $p<0.05$). (B) Number of positive SPT reactions vs. BAL eosinophil percentage; IL-13 asthma subphentoype as indicated (high, open squares; low, closed circles). (C) Number of positive SPT reactions vs. serum IgE; IL-13 asthma subphentoype as indicated (high, open squares; low, closed circles). (D) Number of positive SPT reactions vs. peripheral blood eosinophil count; IL-13 asthma subphentoype as indicated (high, open squares; low, closed circles). Spearman's rank order correlation (p) and p-values are indicated in each plot for B-D.

Figure 8 shows airway epithelial mucin content and composition in subjects with IL-13 high and IL-13 low asthma subphenotypes and healthy controls as described in Example 8. (A) Volume of mucin per volume of epithelium, a measure of airway epithelial mucin content. (B) Expression of mucin MUC2 as determined by qPCR. (C) Expression of mucin MUC5AC as determined by qPCR. (D) Expression of mucin MUC5B as determined by qPCR.

Figure 9 shows responses of subjects with IL-13 high and IL-13 low asthma subphenotypes to inhaled corticosteroids. (A) $FEV_1$ measured at baseline (week 0), after 4 and 8 weeks on daily fluticasone, and one week after the cessation of fluticasone (week 9). (*): *see* Table 5 for number of subjects in each group and p-values. (B) Heatmap depicting unsupervised hierarchical clustering of periostin, CLCA1, and serpinB2 (as in Fig. ID) in bronchial epithelium of asthmatics one week after the initiation of either fluticasone (N=19) or placebo treatment (N=13). Cluster identification at baseline for individual subjects and treatment are indicated below heatmap. (cluster 1: "IL-13 high" asthmatics; cluster 2: "IL-13 low" asthmatics).

Figure 10 shows alveolar macrophage gene expression in subjects with IL-13 high and IL-13 low subphenotypes of asthma as described in Example 8. Healthy controls (N=15); IL-13 low subphenotype of asthma (N=5); IL-13 high subphenotype of asthma (N=9) are indicated. The figure shows the mean (+SEM) expression levels of 15-lipoxygenase (ALOX15) and tumor necrosis factor-α (TNF-α) as determined by qPCR. (*): $p < 0.03$.

Figure 11 shows gene expression microarray analysis using 35 probes covering 28 genes of samples from healthy controls and asthmatics as described in Example 9.

Figure 12 shows gene expression microarray analysis and qPCR analysis for periostin and CEACAM5 as described in Example 9. (A) Periostin expression in healthy controls, cluster 2 asthmatics ("IL-13 LOW"), and cluster 1 asthmatics ("IL-13 high"); (B) CEACAM5 expression in healthy controls, cluster 2 asthmatics ("IL-13 LOW"), and cluster 1 asthmatics ("IL-13 HIGH"); (C) a composite graph of CEACAM5 and periostin in "IL-13 high" asthmatics (squares) and "IL-13 low" asthmatics (circles); (D) Receiver operating characteristic (ROC) analysis of an optimized algorithm for qPCR-based expression levels of periostin and CEACAM5 showing sensitivity and specificity for healthy controls, "IL-13 high" asthmatics, and "IL-13 low" asthmatics.

Figure 13 shows serum levels of serum proteins in asthmatics and in healthy controls as described in Example 9. (A) serum levels of IgE; (B) serum levels of periostin; (C) serum levels of CEA; (D) serum levels of YKL-40; (E) serum levels of IgE in asthmatics treated with inhaled corticosteroids (ICS) (+) or not (-); (F) serum levels of periostin in asthmatics treated with inhaled corticosteroids (ICS) (+) or not (-); (G) serum levels of CEA in asthmatics treated with inhaled corticosteroids (ICS) (+) or not (-); (H) serum levels of YKL-40 in asthmatics treated with inhaled corticosteroids (ICS) (+) or not (-); (I) composite graph of serum levels of periostin in asthmatics having < 100 IU/ml serum IgE (<100) and asthmatics having ≥ 100 IU/ml serum IgE (≥100); (J) composite graph of serum levels of CEA in asthmatics having < 100 IU/ml serum IgE (<100) and asthmatics having ≥ 100 IU/ml serum IgE (≥100); (K) composite graph of serum levels of YKL-40 in asthmatics having < 100 IU/ml serum IgE (<100) and asthmatics having ≥ 100 IU/ml serum IgE (≥100); (L) composite graph of serum levels of periostin and CEA in asthmatics having < 100 IU/ml serum IgE (circles) and asthmatics having ≥ 100 IU/ml serum IgE (squares).

## DETAILED DESCRIPTION

## DEFINITIONS

Unless otherwise defined, all terms of art, notations and other scientific terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. The techniques and procedures described or referenced herein are generally well understood and commonly employed using conventional methodology by those skilled in the art, such as, for example, the widely utilized molecular cloning methodologies described in Sambrook et al., Molecular Cloning: A Laboratory Manual 2nd. edition (1989) Cold Spring Harbor Laboratory Press, Cold Spring Harbor, N.Y. As appropriate, procedures involving the use of commercially available kits and reagents are generally carried out in accordance with manufacturer defined protocols and/or parameters unless otherwise noted.

"IL-4/IL-13 gene signature," "IL-4/IL-13 signature," "IL-13 gene signature," and "IL-13 signature" are used interchangeably herein and refer to a combination of 30 genes as set forth in Table 4, or a subcombination of these 30 genes as set forth in Table 9, the gene expression pattern of which correlates with certain asthma patients. The 30 genes include POSTN, CST1, CCL26, CLCA1, CST2, PRR4, SERPINB2, CEACAM5, iNOS, SERPINB4, CST4, PRB4, TPSD1, TPSG1, MFSD2, CPA3, GPR105, CDH26, GSN, C2ORF32, TRACH2000196 (TMEM71), DNAJC12, RGS13, SLC18A2, SERPINB10, SH3RF2, FCER1B, RUNX2, PTGS1, ALOX15. The polypeptides of the IL-4/IL13 gene signature are "targeted polypeptides" of this invention.

The term "targeted polypeptide" when used herein refers to "native sequence" polypeptides and variants (which are further defined herein).

A "native sequence" polypeptide comprises a polypeptide having the same amino acid sequence as the corresponding polypeptide derived from nature. Thus, the term "native sequence polypeptide" includes naturally-occurring truncated, augmented, and frameshifted forms of a polypeptide, including but not limited to alternatively spliced forms, isoforms and polymorphisms.

"Naturally occurring variant" means a polypeptide having at least about 60% amino acid sequence identity with a reference polypeptide and retains at least one biological activity of the naturally occurring reference polypeptide. Naturally occurring variants can include variant polypeptides having at least about 65% amino acid sequence identity, at least about 70% amino acid sequence identity, at least about 75% amino acid sequence identity, at least about 80% amino acid sequence identity, at least about 80% amino acid sequence identity, at least about 85% amino acid sequence identity, at least about 90% amino acid sequence identity, at least about 95% amino acid sequence identity, at least about 98% amino acid sequence identity or at least about 99% amino acid sequence identity to a reference polypeptide.

Examples of POSTN include a polypeptide comprising SEQ ID NO:1 and other POSTN native sequence polypeptides, such as naturally occurring variants and native sequence polypeptides encoded by a nucleic acid sequence that can hybridize under stringent conditions to SEQ ID NOs:31 and/or 32.

Examples of CST1 include a polypeptide comprising SEQ ID NO:2 and other CST1 native sequence polypeptides, such as naturally occurring variants and native sequence polypeptides encoded by a nucleic acid sequence that can hybridize under stringent conditions to SEQ ID NO:33.

Examples of CCL26 include a polypeptide comprising SEQ ID NO:3 and other CCL26 native sequence polypeptides, such as naturally occurring variants and native sequence polypeptides encoded by a nucleic acid sequence that can hybridize under stringent conditions to SEQ ID NO:34.

Examples of CLCA1 include a polypeptide comprising SEQ ID NO:4 and other CLCA1 native sequence polypeptides, such as naturally occurring variants and native sequence polypeptides encoded by a nucleic acid sequence that can hybridize under stringent conditions to SEQ ID NO:35.

Examples of CST2 include a polypeptide comprising SEQ ID NO:5 and other CST native sequence polypeptides, such as naturally occurring variants and native sequence polypeptides encoded by a nucleic acid sequence that can hybridize under stringent conditions to SEQ ID NO:36.

Examples of PRR4 include a polypeptide comprising SEQ ID NO:6 and other PRR4 native sequence polypeptides, such as naturally occurring variants and native sequence polypeptides encoded by a nucleic acid sequence that can hybridize under stringent conditions to SEQ ID NO:37.

Examples of SERPINB2 include a polypeptide comprising SEQ ID NO:7 and other SERPINB2 native sequence polypeptides, such as naturally occurring variants and native sequence polypeptides encoded by a nucleic acid sequence that can hybridize under stringent conditions to SEQ ID NO:38.

Examples of CEACAM5 include a polypeptide comprising SEQ ID NO:8 and other CEACAM5 native sequence polypeptides, such as naturally occurring variants and native sequence polypeptides encoded by a nucleic acid sequence that can hybridize under stringent conditions to SEQ ID NO:39.

Examples of iNOS include a polypeptide comprising SEQ ID NO:9 and other iNOS native sequence polypeptides, such as naturally occurring variants and native sequence polypeptides encoded by a nucleic acid sequence that can hybridize under stringent conditions to SEQ ID NO:40.

Examples of SERPINB4 include a polypeptide comprising SEQ ID NO:10 and other SERPINB4 native sequence polypeptides, such as naturally occurring variants and native sequence polypeptides encoded by a nucleic acid sequence that can hybridize under stringent conditions to SEQ ID NOs:41 and/or 42.

Examples of CST4 include a polypeptide comprising SEQ ID NO: 11 and other CST4 native sequence polypeptides, such as naturally occurring variants and native sequence polypeptides encoded by a nucleic acid sequence that can hybridize under stringent conditions to SEQ ID NO:43.

Examples of PRB4 include a polypeptide comprising SEQ ID NO:12 and other PRB4 native sequence polypeptides, such as naturally occurring variants and native sequence polypeptides encoded by a nucleic acid sequence that can hybridize under stringent conditions to SEQ ID NO:44.

Examples of TPSD1 include a polypeptide comprising SEQ ID NO:13 and other TPSD1 native sequence polypeptides, such as naturally occurring variants and native sequence polypeptides encoded by a nucleic acid sequence that can hybridize under stringent conditions to a sequence selected from the group consisting of SEQ ID NO:45-51.

Examples of TPSG1 include a polypeptide comprising SEQ ID NO:14 and other TPSG1 native sequence polypeptides, such as naturally occurring variants and native sequence polypeptides encoded by a nucleic acid sequence that can hybridize under stringent conditions a sequence selected from the group consisting of SEQ ID NO:52-55.

Examples of MFSD2 include a polypeptide comprising SEQ ID NO:15 and other MFSD2 native sequence polypeptides, such as naturally occurring variants and native sequence polypeptides encoded by a nucleic acid sequence that can hybridize under stringent conditions to SEQ ID NO:56.

Examples of CPA3 include a polypeptide comprising SEQ ID NO:16 and other CPA3 native sequence polypeptides, such as naturally occurring variants and native sequence polypeptides encoded by a nucleic acid sequence that can hybridize under stringent conditions to SEQ ID NO:57.

Examples of GPR105 include a polypeptide comprising SEQ ID NO:17 and other GPR105 native sequence polypeptides, such as naturally occurring variants and native sequence polypeptides encoded by a nucleic acid sequence that can hybridize under stringent conditions to SEQ ID NO:58.

Examples of CDH26 include a polypeptide comprising SEQ ID NO:18 and other CDH26 native sequence polypeptides, such as naturally occurring variants and native sequence polypeptides encoded by a nucleic acid sequence that can hybridize under stringent conditions to SEQ ID NO:59.

Examples of GSN include a polypeptide comprising SEQ ID NO:19 and other GSN native sequence polypeptides, such as naturally occurring variants and native sequence polypeptides encoded by a nucleic acid sequence that can hybridize under stringent conditions to SEQ ID NO:60.

Examples of C2ORF32 include a polypeptide comprising SEQ ID NO:20 and other C2ORF32 native sequence polypeptides, such as naturally occurring variants and native sequence polypeptides encoded by a nucleic acid sequence that can hybridize under stringent conditions to SEQ ID NO:61.

Examples of TRACH2000196 (TMEM71) include a polypeptide comprising SEQ ID NO:21 and other TRACH2000196 (TMEM71) native sequence polypeptides, such as naturally occurring variants and native sequence polypeptides encoded by a nucleic acid sequence that can hybridize under stringent conditions to SEQ ID NO:62.

Examples of DNAJC12 include a polypeptide comprising SEQ ID NO:22 and other DNAJC12 native sequence polypeptides, such as naturally occurring variants and native sequence polypeptides encoded by a nucleic acid sequence that can hybridize under stringent conditions to SEQ ID NO:63.

Examples of RGS13 include a polypeptide comprising SEQ ID NO:23 and other RGS13 native sequence polypeptides, such as naturally occurring variants and native sequence polypeptides encoded by a nucleic acid sequence that can hybridize under stringent conditions to SEQ ID NO:64.

Examples of SLC18A2 include a polypeptide comprising SEQ ID NO:24 and other SLC18A2 native sequence polypeptides, such as naturally occurring variants and native sequence polypeptides encoded by a nucleic acid sequence that can hybridize under stringent conditions to SEQ ID NO:65.

Examples of SERPINB10 include a polypeptide comprising SEQ ID NO:25 and other SERPINB10 native sequence polypeptides, such as naturally occurring variants and native sequence polypeptides encoded by a nucleic acid sequence that can hybridize under stringent conditions to SEQ ID NO:66.

Examples of SH3RF2 include a polypeptide comprising SEQ ID NO:26 and other SH3RF2 native sequence polypeptides, such as naturally occurring variants and native sequence polypeptides encoded by a nucleic acid sequence that can hybridize under stringent conditions to SEQ ID NO:67.

Examples of FCER1B include a polypeptide comprising SEQ ID NO:27 and other FCER1B native sequence polypeptides, such as naturally occurring variants and native sequence polypeptides encoded by a nucleic acid sequence that can hybridize under stringent conditions to SEQ ID NO:68.

Examples of RUNX2 include a polypeptide comprising SEQ ID NO:28 and other RUNX2 native sequence polypeptides, such as naturally occurring variants and native sequence polypeptides encoded by a nucleic acid sequence that can hybridize under stringent conditions to SEQ ID NO:69.

Examples of PTGS1 include a polypeptide comprising SEQ ID NO:29 and other PTGS1 native sequence polypeptides, such as naturally occurring variants and native sequence polypeptides encoded by a nucleic acid sequence that can hybridize under stringent conditions to SEQ ID NO:70.

Examples of ALOX15 include a polypeptide comprising SEQ ID NO:30 and other ALOX15 native sequence polypeptides, such as naturally occurring variants and native sequence polypeptides encoded by a nucleic acid sequence that can hybridize under stringent conditions to SEQ ID NO:71.

"An anti-IL13/IL4 pathway inhibitor" refers to an agent that blocks the IL-13 and/or IL-4 signalling. Examples of an anti-IL13, anti-IL4 or anti-IL13/IL4 inhibitors include, but are not limited to, anti-IL13 binding agents, anti-IL4 binding agents, anti-IL4receptoralpha binding agents, anti-IL13receptoralpha1 binding agents and anti-IL13 receptoralpha2 binding agents. Single domain antibodies that can bind IL-13, IL-4, IL-13Ralpha1, IL-13Ralpha2 or IL-4Ralpha are specifically included as inhibitors. It should be understood that molecules that can bind more than one target are included.

"Anti-IL4 binding agents" refers to agent that specifically binds to human IL-4. Such binding agents can include a small molecule, an apatmer or a polypeptide. Such polypeptide can include, but is not limited to, a polypeptide(s) selected from the group consisting of an immunoadhesin, an antibody, a peptibody and a peptide. The binding agent may bind to a human IL-4 sequence with an affinity between 1 uM - 1 pM. Specific examples of anti-IL4 binding agents can include soluble IL4Receptor alpha (e.g., extracellular domain of IL4Receptor fused to a human Fc region), anti-IL4 antibody, and soluble IL13receptoralpha1 (e.g., extracellular domain of IL13receptoralpha1 fused to a human Fc region).

"Anti-IL4receptoralpha binding agents" refers to an agent that specifically binds to human IL4 receptoralpha. Such binding agents can include a small molecule, an aptamer or a polypeptide. Such polypeptide can include, but is not limited to, a polypeptide(s) selected from the group consisting of an immunoadhesin, an antibody, a peptibody and a peptide. The binding agent may bind to a human IL-4 receptor alpha sequence with an affinity between 1 uM - 1 pM. Specific examples of anti-IL4 receptoralpha binding agents can include anti-IL4 receptor alpha antibodies.

"Anti-IL13 binding agent" refers to agent that specifically binds to human IL-13. Such binding agents can include a small molecule, aptamer or a polypeptide. Such polypeptide can include, but is not limited to, a polypeptide(s) selected from the group consisting of an immunoadhesin, an antibody, a peptibody and a peptide. The binding agent may bind to a human IL-13 sequence with an affinity between 1 uM - 1 pM. Specific examples of anti-IL13 binding agents can include anti-IL13 antibodies, soluble IL13receptoralpha2 fused to a human Fc, soluble IL4receptoralpha fused to a human Fc, soluble IL13 receptoralpha fused to a human Fc. The anti-IL13 antibody may comprise the variable domains of the TNX-650 antibody (WO2005/062972). The variable domains of the TNX-650 antibody comprise (1) a VH comprising QVTLRESGPALVKPTQTLTLTCTVSGFSLSAYSVNWIRQPPGKALEWLAMIWGDGKI VYNSALKSRLTISKDTSKNQVVLTMTNMDPVDTATYYCAGDGYYPYAMDNWGQG SLVTVSS (SEQ ID NO:193) and (2) a VL comprising: DIVMTQSPDSLSVSLGERATINCRASKSVDSYGNSFMHWYQQKPGQPPKLLIYLASN LESGVPDRFSGSGSGTDFTLTISSLQAEDVAVYYCQQNNEDPRTFGGGTKVEIK (SEQ ID NO:194). Other examples of anti-IL13 antibodies are described in WO2008/083695 (e.g., IMA-638 and IMA-026), US2008/0267959, US2008/0044420 and US2008/0248048.

Anti-IL13receptoralpha1 binding agents" refers to an agent that specifically binds to human IL13 receptoralpha1. Such binding agents can include a small molecule, aptamer or a polypeptide. Such polypeptide can include, but is not limited to, a polypeptide(s) selected from the group consisting of an immunoadhesin, an antibody, a peptibody and a peptide. The binding agent may bind to a human IL-13 receptor alpha 1 sequence with an affinity between 1 uM - 1 pM. Specific examples of anti-IL13 receptoralpha1 binding agents can include anti-IL13 receptor alpha1 antibodies.

"Anti-IL13receptoralpha2 binding agents" refers to an agent that specifically binds to human IL13 receptoralpha2. Such binding agents can include a small molecule, an aptamer or a polypeptide. Such polypeptide can include, but is not limited to, a polypeptide(s) selected from the group consisting of an immunoadhesin, an antibody, a peptibody and a peptide. the binding agent may bind to a human IL-13 receptor alpha2 sequence with an affinity between 1 uM - 1 pM. Specific examples of anti-IL13 receptoralpha2 binding agents can include anti-IL13 receptor alpha2 antibodies.

"Anti IgE binding agents" refers to an agent that specifically binds to human IgE. Such binding agents can include a small molecule, an aptamer or a polypeptide. Such polypeptide can include, but is not limited to, a polypeptide(s) selected from the group consisting of an immunoadhesin, an antibody, a peptibody and a peptide. The anti-IgE antibody may comprise a VL sequence comprising Asp Ile Gln Leu Thr Gln Ser Pro Ser Ser Leu Ser Ala Ser Val Gly Asp Arg Val Thr Ile Thr Cys Arg Ala Ser Gln Ser Val Asp Tyr Asp Gly Asp Ser Tyr Met Asn Trp Tyr Gln Gln Lys Pro Gly Lys Ala Pro Lys Leu Leu Ile Tyr Ala Ala Ser Tyr Leu Glu Ser Gly Val Pro Ser Arg Phe Ser Gly Ser Gly Ser Gly Thr Asp Phe Thr Leu Thr Ile Ser Ser Leu Gln Pro Glu Asp Phe Ala Thr Tyr Tyr Cys Gln Gln Ser His Glu Asp Pro Tyr Thr Phe Gly Gln Gly Thr Lys Val Glu Ile Lys Arg Thr Val (SEQ ID NO:213) and a VH sequence comprising Glu Val Gln Leu Val Glu Ser Gly Gly Gly Leu Val Gln Pro Gly Gly Ser Leu Arg Leu Ser Cys Ala Val Ser Gly Tyr Ser Ile Thr Ser Gly Tyr Ser Trp Asn Trp Ile Arg Gln Ala Pro Gly Lys Gly Leu Glu Trp Val Ala Ser Ile Thr Tyr Asp Gly Ser Thr Asn Tyr Asn Pro Ser Val Lys Gly Arg Ile Thr Ile Ser Arg Asp Asp Ser Lys Asn Thr Phe Tyr Leu Gln Met Asn Ser Leu Arg Ala Glu Asp Thr Ala Val Tyr Tyr Cys Ala Arg Gly Ser His Tyr Phe Gly His Trp His Phe Ala Val Trp Gly Gln Gly (SEQ ID NO:214).

"Anti-M1' binding agents" refers to an agent that specifically binds to the membrane proximal M1' region of surface expressed IgE on B cells. Such binding agents can include a small molecule, an aptamer or a polypeptide. Such polypeptide can include, but is not limited to, a polypeptide(s) selected from the group consisting of an immunoadhesin, an antibody, a peptibody and a peptide. The anti-IgE antibody may comprise an antibody described in WO2008/116149 or a variant thereof.

The term "small molecule" refers to an organic molecule having a molecular weight between 50 Daltons to 2500 Daltons.

The term "antibody" is used in the broadest sense and specifically covers, for example, monoclonal antibodies, polyclonal antibodies, antibodies with polyepitopic specificity, single chain antibodies, multi-specific antibodies and fragments of antibodies. Such antibodies can be chimeric, humanized, human and synthetic. Such antibodies and methods of generating them are described in more detail below.

The term "variable" refers to the fact that certain segments of the variable domains differ extensively in sequence among antibodies. The V regions mediate antigen binding and define specificity of a particular antibody for its particular antigen. However, the variability is not evenly distributed across the 110-amino acid span of the variable domains. Instead, the V domains consist of relatively invariant stretches called framework regions (FRs) of 15-30 amino acids separated by shorter regions of extreme variability called "hypervariable regions" that are each 9-12 amino acids long. The variable domains of native heavy and light chains each comprise four FRs, largely adopting a beta-sheet configuration, connected by three hypervariable regions, which form loops connecting, and in some cases forming part of, the beta-sheet structure. The hypervariable regions in each chain are held together in close proximity by the FRs and, with the hypervariable regions from the other chain, contribute to the formation of the antigen-binding site of antibodies (see Kabat et al., Sequences of Proteins of Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

The term "hypervariable region" (or "HVR") when used herein refers to the amino acid residues of an antibody which are responsible for antigen-binding. The hypervariable region generally comprises amino acid residues from a "complementarity determining region" or "CDR" (e.g. around about residues 24-34 (L1), 50-56 (L2) and 89-97 (L3) in the VL, and around about 31-35B (HI), 50-65 (H2) and 95-102 (H3) in the VH (Kabat et al., Sequences of Proteins of Immunological Interest, 5th Ed. Public Health Service, National Institutes of Health, Bethesda, MD. (1991)) and/or those residues from a "hypervariable loop" (e.g. residues 26-32 (L1), 50-52 (L2) and 91-96 (L3) in the VL, and 26-32 (HI), 52A-55 (H2) and 96-101 (H3) in the VH (Chothia and Lesk J. Mol. Biol. 196:901-917 (1987)).

Hypervariable regions may comprise "extended hypervariable regions" as follows: 24-36 (L1), 46-56 (L2) and 89-97 (L3) in the VL and 26-35B (HI), 47-65 (H2) and 93-102 (H3) in the VH. The variable domain residues are numbered according to Kabat et al., supra for each of these definitions.

"Framework" or "FR" residues are those variable domain residues other than the hypervariable region residues as herein defined. For example, light chain framework 1 (LC-FR1), framework 2 (LC-FR2), framework 3 (LC-FR3) and framework 4 (LC-FR4) region may comprise residues numbered 1-23, 35-49, 57-88 and 98-107 of an antibody (Kabat numbering system), respectively. In another example, heavy chain framework 1 (HC-FR1), heavy chain framework 2 (HC-FR2), heavy chain framework 3 (HC-FR3) and heavy chain framework 4 (HC-FR4) may comprise residues 1-25, 36-48, 66-92 and 103-113, respectively, of an antibody (Kabat numbering system).

As referred to herein, the "consensus sequence" or consensus V domain sequence is an artificial sequence derived from a comparison of the amino acid sequences of known human immunoglobulin variable region sequences.

The term "monoclonal antibody" as used herein refers to an antibody from a population of substantially homogeneous antibodies, *i.e.*, the individual antibodies comprising the population are identical and/or bind the same epitope(s), except for possible variants that may arise during production of the monoclonal antibody, such variants generally being present in minor amounts. Such monoclonal antibody typically includes an antibody comprising a polypeptide sequence that binds a target, wherein the target-binding polypeptide sequence was obtained by a process that includes the selection of a single target binding polypeptide sequence from a plurality of polypeptide sequences. For example, the selection process can be the selection of a unique clone from a plurality of clones, such as a pool of hybridoma clones, phage clones or recombinant DNA clones. It should be understood that the selected target binding sequence can be further altered, for example, to improve affinity for the target, to humanize the target binding sequence, to improve its production in cell culture, to reduce its immunogenicity *in vivo*, to create a multispecific antibody, *etc*., and that an antibody comprising the altered target binding sequence is also a monoclonal antibody of this invention. In contrast to polyclonal antibody preparations which typically include different antibodies directed against different determinants (epitopes), each monoclonal antibody of a monoclonal antibody preparationis directed against a single determinant on an antigen. In addition to their specificity, the monoclonal antibody preparations are advantageous in that they are typically uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including the hybridoma method (*e.g.*, Kohler et al., Nature, 256:495 (1975); Harlow et al., Antibodies: A Laboratory Manual, (Cold Spring Harbor Laboratory Press, 2nd ed. 1988); Hammerling et al., in: Monoclonal Antibodies and T-Cell Hybridomas 563-681, (Elsevier, N.Y., 1981), recombinant DNA methods (see, *e.g.*, U.S. Patent No. 4,816,567), phage display technologies (see, *e.g.*, Clackson et al., Nature, 352:624-628 (1991); Marks et al., J. Mol. Biol., 222:581-597 (1991); Sidhu et al., J. Mol. Biol. 338(2):299-310 (2004); Lee et al., J.Mol.Biol.340(5): 1073-1093 (2004); Fellouse, Proc. Nat. Acad. Sci. USA 101(34):12467-12472 (2004); and Lee et al. J. Immunol. Methods 284(1-2):119-132 (2004) and technologies for producing human or human-like antibodies from animals that have parts or all of the human immunoglobulin loci or genes encoding human immunoglobulin sequences (see, e.g., WO98/24893, WO/9634096, WO/9633735, and WO/91 10741, Jakobovits et al., Proc. Natl. Acad. Sci. USA, 90:2551 (1993); Jakobovits et al., Nature, 362:255-258 (1993); Bruggemann et al., Year in Immuno., 7:33 (1993); U.S. Patent Nos. 5,545,806, 5,569,825, 5,591,669 (all of GenPharm); 5,545,807; WO 97/17852, U.S. Patent Nos. 5,545,807; 5,545,806; 5,569,825; 5,625,126; 5,633,425; and 5,661,016, and Marks et al., Bio/Technology, 10: 779-783 (1992); Lonberg et al., Nature, 368: 856-859 (1994); Morrison, Nature, 368: 812-813 (1994); Fishwild et al., Nature Biotechnology, 14: 845-851 (1996); Neuberger, Nature Biotechnology, 14: 826 (1996); and Lonberg and Huszar, Intern. Rev. Immunol., 13: 65-93 (1995).

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while portions of the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; Morrison et al., Proc. Natl. Acad. Sci. USA, 81:6851-6855 (1984)). Methods of making chimeric antibodies are known in the art.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Humanized antibodies may be human immunoglobulins (recipient antibody) in which residues from a complementarity-determining region (CDR) of the recipient are replaced by residues from a CDR of a non-human species (donor antibody) such as mouse, rat or rabbit the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are generally made to further refine and maximize antibody performance. Typically, the humanized antibody will comprise substantially all of at least one variable domain, in which all or substantially all of the hypervariable loops derived from a non-human immunoglobulin and all or substantially all of the FR regions are derived from a human immunoglobulin sequence although the FR regions may include one or more amino acid substitutions to, e.g., improve binding affinity. Preferably, the humanized antibody will also comprise at least a portion of an immunoglobulin constant region (Fc), typically that of a human immunoglobulin or a human consensus constant sequence. For further details, see Jones et al., Nature, 321:522-525 (1986); Reichmann et al., Nature, 332:323-329 (1988); and Presta, Curr. Op. Struct. Biol., 2:593-596 (1992). The humanized antibody includes a PRIMATIZED® antibody wherein the antigen-binding region of the antibody is derived from an antibody produced by, e.g., immunizing macaque monkeys with the antigen of interest. Methods of making humanized antibodies are known in the art.

Human antibodies can also be produced using various techniques known in the art, including phage-display libraries. Hoogenboom and Winter, J. Mol. Biol., 227:381 (1991); Marks et al., J. Mol. Biol., 222:581 (1991). The techniques of Cole et al. and Boerner et al. are also available for the preparation of human monoclonal antibodies. Cole et al., Monoclonal Antibodies and Cancer Therapy, Alan R. Liss, p. 77 (1985); Boerner et al., J. Immunol., 147(1):86-95 (1991). See also, Lonberg and Huszar, Int. Rev. Immunol. 13:65-93 (1995). PCT publications WO 98/24893; WO 92/01047; WO 96/34096; WO 96/33735; European Patent No. 0 598 877; U.S. Pat. Nos. 5,413,923; 5,625,126; 5,633,425; 5,569,825; 5,661,016; 5,545,806; 5,814,318; 5,885,793; 5,916,771; and 5,939,598.

"Antibody fragments" comprise a portion of a full length antibody, generally the antigen binding or variable region thereof. Examples of antibody fragments include Fab, Fab', F(ab')2, and Fv fragments; diabodies; linear antibodies; single-chain antibody molecules; and multispecific antibodies formed from antibody fragments.

"Fv" is the minimum antibody fragment which contains a complete antigen-recognition and -binding site. This fragment consists of a dimer of one heavy- and one light-chain variable region domain in tight, non-covalent association. From the folding of these two domains emanate six hypervariable loops (3 loops each from the H and L chain) that contribute the amino acid residues for antigen binding and confer antigen binding specificity to the antibody. However, even a single variable domain (or half of an Fv comprising only three CDRs specific for an antigen) may have the ability to recognize and bind antigen, although at a lower affinity than the entire binding site.

"Functional fragments" of the antibodies of the invention are those fragments that retain binding to polypeptide with substantially the same affinity as the intact full chain molecule from which they are derived and are active in at least one assay (e.g., inhibition of TH2-induced asthma pathway such as in mouse models or inhibition of a biological activity of the antigen that binds to the antibody fragment in vitro).

Antibody "effector functions" refer to those biological activities attributable to the Fc region (a native sequence Fc region or amino acid sequence variant Fc region) of an antibody, and vary with the antibody isotype. Examples of antibody effector functions include: C1q binding and complement dependent cytotoxicity; Fc receptor binding; antibody-dependent cell-mediated cytotoxicity (ADCC); phagocytosis; down regulation of cell surface receptors (*e.g.* B cell receptor); and B cell activation. A "native sequence Fc region" comprises an amino acid sequence identical to the amino acid sequence of an Fc region found in nature.

"Percent (%) amino acid sequence identity" or "homology" with respect to the polypeptide and antibody sequences identified herein is defined as the percentage of amino acid residues in a candidate sequence that are identical with the amino acid residues in the polypeptide being compared, after aligning the sequences considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for measuring alignment, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc. and the source code has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California. The ALIGN-2 program should be compiled for use on a UNIX operating system, preferably digital UNIX V4.0D. All sequence comparison parameters are set by the ALIGN-2 program and do not vary.

The term "Fc region-comprising polypeptide" refers to a polypeptide, such as an antibody or immunoadhesin (see definitions below), which comprises an Fc region. The C-terminal lysine (residue 447 according to the EU numbering system) of the Fc region may be removed, for example, during purification of the polypeptide or by recombinantly engineering the nucleic acid encoding the polypeptide. Accordingly, a composition comprising polypeptides, including antibodies, having an Fc region according to this invention can comprise polypeptides populations with all K447 residues removed, polypeptide populations with no K447 residues removed or polypeptide populations having a mixture of polypeptides with and without the K447 residue.

Throughout the present specification and claims, the Kabat numbering system is generally used when referring to a residue in the variable domain (approximately, residues 1-107 of the light chain and residues 1-113 of the heavy chain) (e.g, Kabat et al., Sequences of Immunological Interest. 5th Ed. Public Health Service, National Institutes of Health, Bethesda, Md. (1991)). The "EU numbering system" or "EU index" is generally used when referring to a residue in an immunoglobulin heavy chain constant region (e.g., the EU index reported in Kabat et al., Sequences of Proteins of Immunological Interest, 5th Ed. Public Health Service, National Institutes of Health, Bethesda, MD (1991) expressly incorporated herein by reference). Unless stated otherwise herein, references to residues numbers in the variable domain of antibodies means residue numbering by the Kabat numbering system. Unless stated otherwise herein, references to residue numbers in the constant domain of antibodies means residue numbering by the EU numbering system (e.g., see United States Provisional Application No. 60/640,323, Figures for EU numbering).

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 μg/ml), 0.1% SDS, and 10% dextran sulfate at 42C, with a 10 minute wash at 42C in 0.2 x SSC (sodium chloride/sodium citrate) followed by a 10 minute high-stringency wash consisting of 0.1 x SSC containing EDTA at 55C.

"Moderately stringent conditions" can be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50C. The skilled artisan will recognize how to adjust the temperature, ionic strength, etc. as necessary to accommodate factors such as probe length and the like.

As used herein, a subject to be treated is a mammal (e.g., human, non-human primate, rat, mouse, cow, horse, pig, sheep, goat, dog, cat, etc.). The subject may be a clinical patient, a clinical trial volunteer, an experimental animal, etc. The subject may be suspected of having or at risk for having asthma or be diagnosed with asthma. Preferably the subject to be treated is a human.

"Treating" or "treatment" or "alleviation" refers to measures, wherein the object is to prevent or slow down (lessen) the targeted pathologic condition or disorder or relieve some of the symptoms of the disorder. Those in need of treatment include can include those already with the disorder as well as those prone to have the disorder or those in whom the disorder is to be prevented. A subject or mammal is successfully "treated" for asthma if, after receiving a therapeutic agent of the present invention, the patient shows observable and/or measurable reduction in or absence of one or more of the following: recurrent wheezing, coughing, trouble breathing, chest tightness, symptoms that occur or worsen at night, symptoms that are triggered by cold air, exercise or exposure to allergens.

The term "therapeutically effective amount" refers to an amount of a polypeptide of this invention effective to "alleviate" or "treat" a disease or disorder in a subject.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Forced expiratory volume (FEV1)" refers to a standard test that measures the volume of air expelled in the first second of a forced expiration. FEV1 is measured by a spirometer, which consists of a mouthpiece and disposable tubing connected to a machine that records the results and displays them on a graph. To perform spirometry, a person inhales deeply, closes the mouth tightly around the tube and then exhales through the tubing while measurements are taken. The volume of air exhaled, and the length of time each breath takes is recorded and analyzed. Spirometry results are expressed as a percentage. Examples of normal spirometry results include a FEV1 of 75 percent of vital capacity after one second. An example of abnormal spirometry results include a reading of less than 80 percent of the normal predicted value. An abnormal result usually indicates the presence of some degree of obstructive lung disease such as asthma, emphysema or chronic bronchitis, or restrictive lung disease such as pulmonary fibrosis. For example, FEV1 values (percentage of predicted) can be used to classify the obstruction that may occur with asthma and other obstructive lung diseases like emphysema or chronic bronchitis: FEV1 65 percent to 79 percent predicted = mild obstruction, FEV1 40 percent to 59 percent predicted = moderate obstruction, and FEV1 less than 40 percent predicted = severe obstruction.

Examples of nucleic acid probes that may be used to identify the proteins described herein (e.g., by microarray analysis), include, but are not limited to the probes described in Table 4.

"Elevated expression level" or "elevated levels" refers to an increased expression of a mRNA or a protein in a patient relative to a control, such as an individual or individuals who are not suffering from asthma.

All publications (including patents and patent applications) cited herein are hereby incorporated in their entirety by reference.

Throughout this specification and claims, the word "comprise," or variations such as "comprises" or "comprising," will be understood to imply the inclusion of a stated integer or group of integers but not the exclusion of any other integer or group of integers.

The foregoing written description is considered to be sufficient to enable one skilled in the art to practice the invention. The following Examples are offered for illustrative purposes only, and are not intended to limit the scope of the present invention in any way.

Partial list of references:


1. Haldar, P. and I.D. Pavord, Noneosinophilic asthma: a distinct clinical and pathologic phenotype. J Allergy Clin Immunol, 2007. 119(5): p. 1043-52; quiz 1053-4.
2. Hershey, G.K., IL-13 receptors and signaling pathways: an evolving web. J Allergy Clin Immunol, 2003. 111(4): p. 677-90; quiz 691.
3. Berry, M.A., et al., Sputum and bronchial submucosal IL-13 expression in asthma and eosinophilic bronchitis. J Allergy Clin Immunol, 2004. 114(5): p. 1106-9.
4. Humbert, M., et al., Elevated expression of messenger ribonucleic acid encoding IL-13 in the bronchial mucosa of atopic and nonatopic subjects with asthma. J Allergy Clin Immunol, 1997. 99(5): p. 657-65.
5. Truyen, E., et al., Evaluation of airway inflammation by quantitative Th1/Th2 cytokine mRNA measurement in sputum of asthma patients. Thorax, 2006. 61(3): p. 202-8.
6. Saha, S.K., et al., Increased sputum and bronchial biopsy IL-13 expression in severe asthma. J Allergy Clin Immunol, 2008. 121(3): p. 685-91.

7. Yuyama, N., et al., Analysis of novel disease-related genes in bronchial asthma. Cytokine, 2002. 19(6): p. 287-96.

8. Woodruff, P.G., et al., Genome-wide profiling identifies epithelial cell genes associated with asthma and with treatment response to corticosteroids. Proc Natl Acad Sci U S A, 2007. 104(40): p. 15858-63.

9. Takayama, G., et al., Periostin: a novel component of subepithelial fibrosis of bronchial asthma downstream of IL-4 and IL-13 signals. J Allergy Clin Immunol, 2006. 118(1): p. 98-104.

10. Hayashi, N., et al., T helper 1 cells stimulated with ovalbumin and IL-18 induce airway hyperresponsiveness and lung fibrosis by IFN-gamma and IL-13 production. Proc Natl Acad Sci USA, 2007. 104(37): p. 14765-70.

11. Blanchard, C., et al., IL-13 involvement in eosinophilic esophagitis: transcriptome analysis and reversibility with glucocorticoids. J Allergy Clin Immunol, 2007. 120(6): p. 1292-300.

12. Nakanishi, A., et al., Role of gob-5 in mucus overproduction and airway hyperresponsiveness in asthma. Proc Natl Acad Sci USA, 2001. 98(9): p. 5175-80.

13. Zhou, Y., et al., Characterization of a calcium-activated chloride channel as a shared target of Th2 cytokine pathways and its potential involvement in asthma. Am J Respir Cell Mol Biol, 2001. 25(4): p. 486-91.

14. Kuperman, D.A., et al., Dissecting asthma using focused transgenic modeling and functional genomics. J Allergy Clin Immunol, 2005. 116(2): p. 305-11.

15. Ray, R., et al., Uteroglobin suppresses SCCA gene expression associated with allergic asthma. J Biol Chem, 2005. 280(11): p. 9761-4.

16. Sabatini, L.M., et al., Tissue distribution of RNAs for cystatins, histatins, statherin, and proline-rich salivary proteins in humans and macaques. J Dent Res, 1989. 68(7): p. 1138-45.

17. Lindahl, M., B. Stahlbom, and C. Tagesson, Newly identified proteins in human nasal and bronchoalveolar lavage fluids: potential biomedical and clinical applications. Electrophoresis, 1999. 20(18): p. 3670-6.

18. Cimerman, N., et al., Serum cystatin C, a potent inhibitor of cysteine proteinases, is elevated in asthmatic patients. Clin Chim Acta, 2000. 300(1-2): p. 83-95.

19. Finkelman, F.D., et al., Suppressive effect of IL-4 on IL-13-induced genes in mouse lung. J Immunol, 2005. 174(8): p. 4630-8.

20. Zimmermann, N., et al., Expression and regulation of small proline-rich protein 2 in allergic inflammation. Am J Respir Cell Mol Biol, 2005. 32(5): p. 428-35.

21. Warner, T.F. and E.A. Azen, Proline-rich proteins are present in serous cells of submucosal glands in the respiratory tract. Am Rev Respir Dis, 1984. 130(1): p. 115-8.

22. Maeda, Y., et al., [Concentrations of carcinoembryonic antigen in serum and bronchoalveolar lavage fluid of asthmatic patients with mucoid impaction]. Nihon Kokyuki Gakkai Zasshi, 2004. 42(12): p. 988-93.

23. Suresh, V., J.D. Mih, and S.C. George, Measurement of IL-13-induced iNOS-derived gas phase nitric oxide in human bronchial epithelial cells. Am J Respir Cell Mol Biol, 2007. 37(1): p. 97-104.

24. Storey, J.D. and R. Tibshirani, Statistical significance for genomewide studies. Proc Natl Acad Sci USA, 2003. 100(16): p. 9440-5.

25. Eisen, M.B., et al., Cluster analysis and display of genome-wide expression patterns. Proc Natl Acad Sci USA, 1998. 95(25): p. 14863-8.

26. Saldanha, A.J., Java Treeview--extensible visualization of microarray data. Bioinformatics, 2004. 20(17): p. 3246-8.

27. Kelly-Welch, A. E., Hanson, E. M., Boothby, M. R. & Keegan, A. D. Interleukin-4 and interleukin-13 signaling connections maps. Science 300, 1527-8 (2003).

28. Fixman, E. D., Stewart, A. & Martin, J. G. Basic mechanisms of development of airway structural changes in asthma. Eur Respir J 29, 379-89 (2007).

29. Holgate, S. T. Pathogenesis of asthma. Clin Exp Allergy 38, 872-97 (2008).

30. Roche, W. R., Beasley, R., Williams, J. H. & Holgate, S. T. Subepithelial fibrosis in the bronchi of asthmatics. Lancet 1, 520-4 (1989).

31. Ferrando, R. E., Nyengaard, J. R., Hays, S. R., Fahy, J. V. & Woodruff, P. G. Applying stereology to measure thickness of the basement membrane zone in bronchial biopsy specimens. J Allergy Clin Immunol 112, 1243-5 (2003).

32. Ordonez, C. L. et al. Mild and moderate asthma is associated with airway goblet cell hyperplasia and abnormalities in mucin gene expression. Am J Respir Crit Care Med 163, 517-23 (2001).

33. Martin, R. J. et al. The Predicting Response to Inhaled Corticosteroid Efficacy (PRICE) trial. J Allergy Clin Immunol 119, 73-80 (2007).

34. Wenzel, S., Wilbraham, D., Fuller, R., Getz, E. B. & Longphre, M. Effect of an interleukin-4 variant on late phase asthmatic response to allergen challenge in asthmatic patients: results of two phase 2a studies. Lancet 370, 1422-31 (2007).

35. Grunig, G. et al. Requirement for IL-13 independently of IL-4 in experimental asthma. Science 282, 2261-3 (1998).

36. Wills-Karp, M. et al. Interleukin-13: central mediator of allergic asthma. Science 282, 2258-61 (1998).

37. Simpson, J. L., Scott, R., Boyle, M. J. & Gibson, P. G. Inflammatory subtypes in asthma: assessment and identification using induced sputum. Respirology 11, 54-61 (2006).
38. Wenzel, S. E. et al. Evidence that severe asthma can be divided pathologically into two inflammatory subtypes with distinct physiologic and clinical characteristics. Am J Respir Crit Care Med 160, 1001-8 (1999).
39. Wenzel, S. E. Asthma: defining of the persistent adult phenotypes. Lancet 368, 804-13 (2006).
40. Berry, M. et al. Pathological features and inhaled corticosteroid response of eosinophilic and non-eosinophilic asthma. Thorax 62, 1043-9 (2007).
41. Pavord, I. D., Brightling, C. E., Woltmann, G. & Wardlaw, A. J. Non-eosinophilic corticosteroid unresponsive asthma. Lancet 353, 2213-4 (1999).
42. McKinley, L. et al. TH17 cells mediate steroid-resistant airway inflammation and airway hyperresponsiveness in mice. J Immunol 181, 4089-97 (2008).
43. Holgate, S. T. Epithelium dysfunction in asthma. J Allergy Clin Immunol 120, 1233-44; quiz 1245-6 (2007).
44. Martin, R. J., Kraft, M., Chu, H. W., Berns, E. A. & Cassell, G. H. A link between chronic asthma and chronic infection. J Allergy Clin Immunol 107, 595-601 (2001).
45. Dolganov, G. M. et al. A novel method of gene transcript profiling in airway biopsy homogenates reveals increased expression of a Na+-K+-Cl- cotransporter (NKCC1) in asthmatic subjects. Genome Res 11, 1473-83 (2001).
46. Innes, A. L. et al. Epithelial mucin stores are increased in the large airways of smokers with airflow obstruction. Chest 130, 1102-8 (2006).
47. Gentleman, R. C. et al. Bioconductor: open software development for computational biology and bioinformatics. Genome Biol 5, R80 (2004).
48. Douwes J, Gibson P, Pekkanen J, Pearce N. Non-eosinophilic asthma: importance and possible mechanisms. Thorax 2002;57(7):643-8.
49. Galli SJ, Tsai M, Piliponsky AM. The development of allergic inflammation. Nature 2008;454(7203):445-54.
50. Barnes PJ. The cytokine network in asthma and chronic obstructive pulmonary disease. J Clin Invest 2008;118(11):3546-56.
51. Woodruff PG, Koth LL, Yang YH, et al. A distinctive alveolar macrophage activation state induced by cigarette smoking. Am J Respir Crit Care Med 2005;172(11):1383-92.
52. Weibel ER, Hsia CC, Ochs M. How much is there really? Why stereology is essential in lung morphometry. J Appl Physiol 2007;102(1):459-67.
53. Seibold MA, Donnelly S, Solon M, et al. Chitotriosidase is the primary active chitinase in the human lung and is modulated by genotype and smoking habit. J Allergy Clin Immunol 2008;122(5):944-50 e3.
54. Kim EY, Battaile JT, Patel AC, et al. Persistent activation of an innate immune response translates respiratory viral infection into chronic lung disease. Nat Med 2008;14(6):633-40.
55. Peters-Golden M. The alveolar macrophage: the forgotten cell in asthma. Am J Respir Cell Mol Biol 2004;31(1):3-7.
56. Chu HW, Balzar S, Westcott JY, et al. Expression and activation of 15-lipoxygenase pathway in severe asthma: relationship to eosinophilic phenotype and collagen deposition. Clin Exp Allergy 2002;32(11): 1558-65.
57. Gordon S. Alternative activation of macrophages. Nat Rev Immunol 2003;3(1):23-35.
58. Anderson GP. Endotyping asthma: new insights into key pathogenic mechanisms in a complex, heterogeneous disease. Lancet 2008;372(9643):1107-19.
59. Berry MA, Hargadon B, Shelley M, et al. Evidence of a role of tumor necrosis factor alpha in refractory asthma. N Engl J Med 2006;354(7):697-708.
60. Takatsu K, Nakajima H. IL-5 and eosinophilia. Curr Opin Immunol 2008;20(3):288-94.
61. Zimmermann N, Hershey GK, Foster PS, Rothenberg ME. Chemokines in asthma: cooperative interaction between chemokines and IL-13. J Allergy Clin Immunol 2003;111(2):227-42; quiz 43.
62. Flood-Page PT, Menzies-Gow AN, Kay AB, Robinson DS. Eosinophil's role remains uncertain as anti-interleukin-5 only partially depletes numbers in asthmatic airway. Am J Respir Crit Care Med 2003;167(2):199-204.
63. Flood-Page P, Swenson C, Faiferman I, et al. A study to evaluate safety and efficacy of mepolizumab in patients with moderate persistent asthma. Am J Respir Crit Care Med 2007; 176(11): 1062-71.
64. Baril, P., et al., Periostin promotes invasiveness and resistance of pancreatic cancer cells to hypoxia-induced cell death: role of the beta4 integrin and the PI3k pathway. Oncogene, 2007: 26(14): p. 2082-94.
65. Kudo, Y., et al., Periostin promotes invasion and anchorage-independent growth in the metastatic process of head and neck cancer. Cancer Res, 2006:66(14): p. 6928-35
66. Puglisi, F., et al., Expression of periostin in human breast cancer. J Clin Pathol, 2008:61(4): p. 494-8. 15.
67. Siriwardena, B.S., et al., Periostin is frequently overexpressed and enhances invasion and angiogenesis in oral cancer. Br J Cancer, 2006:95(10): p. 1396-403.
68. Sasaki, H., et al., Serum level of the periostin, a homologue of an insect cell adhesion molecule, as a prognostic marker in nonsmall cell lung carcinomas. Cancer, 2001:92(4): p. 843-8.
69. Sasaki, H., et al., Expression of Periostin, homologous with an insect cell adhesion molecule, as a prognostic marker in non-small cell lung cancers. Jpn J Cancer Res, 2001:92(8): p. 869-73.
70. Sasaki, H., et al., Elevated serum periostin levels in patients with bone metastases from breast but not lung cancer. Breast Cancer Res Treat, 2003:77(3): p. 245-52.
71. Komiya, A., et al., Concerted expression of eotaxin-1, eotaxin-2, and eotaxin-3 in human bronchial epithelial cells. Cell Immunol, 2003:225(2): p. 91-100.
72. Chupp, G.L., et al., A chitinase-like protein in the lung and circulation of patients with severe asthma. N Engl J Med, 2007:357(20): p. 2016-27.
73. Liu, S.M., et al., Immune cell transcriptome datasets reveal novel leukocyte subset-specific genes and genes associated with allergic processes. J Allergy Clin Immunol, 2006:118(2): p. 496-503.
74. Nakajima, T., et al., Identification of granulocyte subtype-selective receptors and ion channels by using a high-density oligonucleotide probe array. J Allergy Clin Immunol, 2004:113(3): p. 528-35.
75. De Filippis, D., A. D'Amico, and T. Iuvone, Cannabinomimetic control of mast cell mediator release: new perspective in chronic inflammation. J Neuroendocrinol, 2008:20 Suppl 1: p. 20-5.
76. Reed, C.E. and H. Kita, The role of protease activation of inflammation in allergic respiratory diseases. J Allergy Clin Immunol, 2004:114(5): p. 997-1008; quiz 1009.
77. Blanchard, C., et al., Periostin facilitates eosinophil tissue infiltration in allergic lung and esophageal responses. Mucosal Immunol, 2008:1(4): p. 289-96.
78. Wenzel, S.E., et al., Bronchoscopic evaluation of severe asthma. Persistent inflammation associated with high dose glucocorticoids. Am J Respir Crit Care Med, 1997:156(3 Pt 1): p. 737-43.
79. Leung, D.Y., et al., Dysregulation of interleukin 4, interleukin 5, and interferon gamma gene expression in steroid-resistant asthma. J Exp Med, 1995:181(1): p. 33-40.
80. Walsh, G.M., Emerging drugs for asthma. Expert Opin Emerg Drugs, 2008:13(4): p. 643-53.
81. Ballesta, A.M., et al., Carcinoembryonic antigen in staging and follow-up of patients with solid tumors. Tumour Biol, 1995:16(1): p. 32-41.

## EXAMPLES

### Example 1 - Methods

<u>Airway Tissue Bank</u>

We studied biological samples stored in the Airway Tissue Bank at the University of California, San Francisco (UCSF) that had been collected during bronchoscopy performed for research purposes in healthy and asthmatic volunteers. Research bronchoscopy had included collection of epithelial brushings, bronchoalveolar lavage (BAL) and bronchial biopsies using specific methods previously described [8, 46]. BAL cell counts and differentials had been performed and databased, and macrophages had been sorted from BAL fluid using flow cytometry [51]. Four to six bronchial biopsies had been obtained from 2nd-through 5th-order carinae (contralateral to the brushing site), formalin-fixed, and then paraffin-embedded in isotropic uniform random orientation [31] to enable quantitative measures of inflammation and remodeling using methods of design-based stereology [52]. An additional 2 bronchial biopsies had been homogenized and processed for RNA using the Qiagen RNeasy minikit (Qiagen Inc., Valencia, CA). RNA extracted from epithelial brushings, homogenates of bronchial biopsies, and lavage macrophages had been quality assured and aliquoted for future microrray- and PCR-based gene profiling. All research bronchoscopy studies had been approved by the UCSF Committee on Human Research (CHR), written informed consent had been obtained from all subjects, and all studies had been performed in accordance with the principles expressed in the Declaration of Helsinki. The Airway Tissue Bank procedures were also reviewed and approved by UCSF's CHR. Samples of epithelial brushings and macrophages from this tissue bank have been used in previously reported studies [8, 14, 46, 51, 53]. Most recently, microarray analyses of differentially expressed genes in epithelial brushings in asthmatic subjects have been reported by us [8].

For the purposes of identifying subsets of patients with asthma who differ with respect to the molecular mechanism underlying their airway inflammation and the distinct inflammatory, pathological and clinical phenotypes characteristic of these subsets, we first conducted new analyses on our previously generated epithelial cell microarray data, and we then supplemented these new analyses with review of additional and detailed clinical characterization data (including data on bronchodilator reversibility and allergen skin test reactivity) from these same subjects and newly generated data, including: (i) gene expression profiles in homogenates of bronchial biopsies and alveolar macrophages; (ii) quantitative measures of subepithelial collagen and airway epithelial mucin in bronchial biopsies; (iii) total and differential cell counts in BAL.

Human Subjects and Samples

Subjects with asthma (N=42) had a prior physician diagnosis of asthma, symptoms consistent with asthma confirmed by a study physician, airway hyper-responsiveness (defined as a drop in forced expiratory volume in the first second ($FEV_1$) of 20% or greater with inhalation of <8 mg/mL of methacholine [$PC_{20}$ methacholine] and *either*: 1) symptoms on 2 or more days per week, 2) β-agonist use on 2 or more days per week, or 3) an $FEV_1$<85% predicted. They did not take inhaled or oral corticosteroids for 4 weeks prior to enrollment. Healthy controls (N=27) had no history of lung disease and lacked airway hyper-responsiveness ($PC_{20}$ methacholine >16 mg/mL). Certain studies included current smokers without asthma (N=16). Exclusion criteria for all subjects included upper respiratory tract infection in the previous 4 weeks, asthma exacerbation within 6 weeks and current use of salmeterol, astemizole, nedocromil sodium, sodium cromoglycate, methlyxanthines, montelukast or zafirlukast. Subjects underwent baseline evaluation by study physicians (including spirometry and methacholine challenge testing as described previously [8]). Subjects also underwent allergen skin prick testing (ASPT) with a panel of 12 aeroallergens, a positive control and a negative control (Table 6).

Thirty-two of the subjects with asthma had also been enrolled in a double-blind randomized controlled clinical trial of inhaled fluticasone (500 μg, twice daily, N=19) or matched placebo (N=13) (ClinicalTrials.gov Identifier: NCT00187499). The trial was designed to determine the effects of inhaled steroid (fluticasone) on airway gene expression and to relate gene expression changes to improvements in lung function. The asthma subjects in the clinical trial had undergone baseline bronchoscopy and had been randomized to receive study medication before undergoing repeat bronchoscopy one week later after starting study drug. Asthma subjects continued study medication for a total of 8 weeks. Healthy control subjects and smokers were enrolled in one of three cross-sectional studies, which comprised two visits each, the first for characterization and the second for bronchoscopy 1 week later. Thirty-five subjects had adequate baseline bronchoscopy, and 32 had RNA available from epithelial brushings at both bronchoscopies. Lung function was measured (by spirometry) after 4 weeks and 8 weeks on study medication, and a final spirometry was completed after a one week run-out. Methods for bronchoscopy, epithelial brushing, bronchoalveaolar lavage, spirometry, and sample handling were identical across all studies.

Bronchoalveolar lavage (BAL) was performed by instilling 4 aliquots of 50ml of sterile saline into either the lingula or right middle lobe, with recovery by suction. Cell counts were performed using a hemocytometer and Turks solution (1% glacial acetic acid and 0.01% gentian violet in distilled $H_2O$). Then BAL cell differentials were performed on cytocentrifuged preparations using the Shandon Kwik-Diff stain kit (Thermo Fisher Scientific, Waltham MA). Thirty-two of the subjects with asthma were also enrolled in a double-blind randomized controlled clinical trial of inhaled fluticasone (500 mcg BID) or matched placebo. In addition to the inclusion criteria above, these subjects were also required to have *either* asthma symptoms on 2 or more days per week, or β-agonist use on 2 or more days per week, or $FEV_1$<85% predicted. Subjects in the clinical trial underwent a baseline visit and baseline bronchoscopy as described above, were randomized to receive study medication and underwent repeat bronchoscopy one week later. Then, they continued study medication for a total of 8 weeks with scheduled re-assessment of spirometry and methacholine challenge testing. All clinical studies were approved by the University of California at San Francisco Committee on Human Research, written informed consent was obtained from all subjects, and all studies were performed in accordance with the principles expressed in the Declaration of Helsinki.

Microarray Analyses and Morphometry

Microarray data from mild-moderate non-smoking asthma patients and healthy non-smoking subjects were obtained from a previous study as described [8]. Methodological detail and microarray data are also available from the Gene Expression Omnibus public database, which can be accessed online at the National Center for Biotechnology Information, accession number GSE4302. Microarray data was analysed in the present study to determine whether genes were differentially regulated within the asthmatic group. Also, the microarray data was analyzed to determine whether other genes were co-regulated with top asthma-related, IL-13 induced genes. Two step real-time PCR (qPCR) was performed as described previously [45] using the primers and probes in Table 1 (i.e., multiplex PCR followed by real time PCR on cDNA generated products).

Morphometric analyses were performed by applying design-based stereology to 4-6 endobronchial biopsies from each subject as described previously. Specifically, analysis of reticular basement membrane thickness was measured in trichrome 3μm sections using the orthogonal intercept method [31]. Airway mucin content was measured in Alcian blue/Periodic acid Schiff 3 μm sections using point and line intersect counting methods [46].

Statistical Methods

Microarray preprocessing was performed using RMA with Bioconductor open source software [47] in the R statistical environment. Unsupervised hierarchical clustering was performed using the Euclidean metric with complete linkage. All other statistical analyses including were performed using the JMP statistical analysis software package (SAS Institute, Cary, NC). Values are presented as mean±standard deviation or median (range) unless otherwise specified. Correlation was performed using Spearman's rank order correlation. For significance testing of $PC_{20}$ and serum IgE levels, data were log transformed for normality. A $p<0.05$ was taken as statistically significant and sidak correction for multiple comparisons was employed after initial three-group comparisons by ANOVA.

**Table** 1. Primer and probe sequences for qPCR

| Gene | Type | Sequence |
|---|---|---|
| IL-13 | RT-forward | GGATGCTGAGCGGATTCTG [SEQ ID NO:73] |
| | RT-reverse | CCCTCGCGAAAAAGTTTCTT [SEQ ID NO:74] |
| | Taqman-forward | AAGGTCTCAGCTGGGCAGTTT [SEQ ID NO:75] |
| | Taqman-reverse | AAACTGGGCCACCTCGATT [SEQ ID NO:76] |
| | probe | CCAGCTTGCATGTCCGAGACACCA [SEQ ID NO:77] |
| IL-4 | RT-forward | GGGTCTCACCTCCCAACTGC [SEQ ID NO:78] |
| | RT-reverse | TGTCTGTTACGGTCAACTCGGT [SEQ ID NO:79] |
| | Taqman-forward | GCTTCCCCCTCTGTTCTTCCT [SEQ ID NO:80] |
| | Taqman-reverse | GCTCTGTGAGGCTGTTCAAAGTT [SEQ ID NO:81] |
| | probe | TCCACGGACACAAGTGCGATATCACC [SEQ ID NO:82] |
| IL-5 | RT-forward | GCCATGAGGATGCTTCTGCA [SEQ ID NO:83] |
| | RT-reverse | GAATCCTCAGAGTCTCATTGGCTATC [SEQ ID NO:84] |
| | Taqman-forward | AGCTGCCTACGTGTATGCCA [SEQ ID NO:85] |
| | Taqman-reverse | GTGCCAAGGTCTCTTTCACCA [SEQ ID NO:86] |
| | probe | CCCCACAGAAATTCCCACAAGTGCA [SEQ ID NO:87] |
| MUC2 | RT-forward | ACTCCTCTACCTCCATCAATAACTCC [SEQ ID NO:88] |
| | RT-reverse | TGGCTCTGCAAGAGATGTTAGCT [SEQ ID NO:89] |
| | Taqman-forward | GCTGGCTGGATTCTGGAAAA [SEQ ID NO:90] |
| | Taqman-reverse | TGGCTCTGCAAGAGATGTTAGC [SEQ ID NO:91] |
| | probe | TCTCCAATCAATTCTGTGTCTCCACCTGG [SEQ ID NO:92] |
| MUC5ac2 | RT-forward | TGTGGCGGGAAAGACAGC [SEQ ID NO:93] |
| | RT-reverse | CCTTCCTATGGCTTAGCTTCAGC [SEQ ID NO:94] |
| | Taqman-forward | CGTGTTGTCACCGAGAACGT [SEQ ID NO:95] |
| | Taqman-reverse | ATCTTGATGGCCTTGGAGCA [SEQ ID NO:96] |
| | probe | CTGCGGCACCACAGGGACCA [SEQ ID NO:97] |
| MUC5b | RT-forward | TTGAGGACCCCTGCTCCCT [SEQ ID NO:98] |
| | RT-reverse | AGGCGTGCACATAGGAGGAC [SEQ ID NO:99] |
| | T aqman-forward | CGATCCCAACAGTGCCTTCT [SEQ ID NO:100] |
| | Taqman-reverse | CCTCGCTCCGCTCACAGT [SEQ ID NO:101] |
| | probe | CAACCCCAAGCCCTTCCACTCGA [SEQ ID NO:102] |
| ALOX15 | RT-forward | CCAACCACCAAGGATGCAA [SEQ ID NO:103] |
| | RT-reverse | TCTGCCCAGCTGCCAAGT [SEQ ID NO:104] |
| | Taqman-forward | CCAACCACCAAGGATGCAA [SEQ ID NO:105] |
| | Taqman-reverse | GGAGAGAAGCCTGGTGGAAGT [SEQ ID NO:106] |
| | probe | CAGTGTCGCCATCACTGTCTCCAGC [SEQ ID NO:107] |
| ALOX5 | RT-forward | ACGTCCACCAGACCATCACC [SEQ ID NO:108] |
| | RT-reverse | GAATCTCACGTGTGCCACCA [SEQ ID NO:109] |
| | Taqman-forward | ATTGCAATGTACCGCCAGC [SEQ ID NO:110] |
| | Taqman-reverse | GAATCTCACGTGTGCCACCA [SEQ ID NO:111] |
| | probe | CTGCTGTGCACCCCATTTTCAAGCTG [SEQ ID NO:112] |
| ALOX5AP | RT-forward | CATAAAGTGGAGCACGAAAGCA [SEQ ID NO:113] |
| | RT-reverse | GGTACGCATCTACACAGTTCTGGTT [SEQ ID NO:114] |

(continued)

| Gene | Type | Sequence |
|---|---|---|
| | Taqman-forward | CAGAATGGGAGGAGCTTCCA [SEQ ID NO:115] |
| | Taqman-reverse | CACAGTTCTGGTTGGCAGTGTAG [SEQ ID NO:116] |
| | probe | CCGGAACACTTGCCTTTGAGCGG [SEQ ID NO:117] |
| ARG1 | RT-forward | CAAGGTCTGTGGGAAAAGCAA [SEQ ID NO:118] |
| | RT-reverse | TGGCCAGAGATGCTTCCAAT [SEQ ID NO:119] |
| | Taqman-forward | GCAGAAGTCAAGAAGAACGGAAGA [SEQ ID NO:120] |
| | Taqman-reverse | TGCTTCCAATTGCCAAACTG [SEQ ID NO:121] |
| | probe | TCTCCGCCCAGCACCAGGCT [SEQ ID NO:122] |
| IL1B | RT-forward | ACTTAAAGCCCGCCTGACAGA [SEQ ID NO:123] |
| | RT-reverse | GCTACTTCTTGCCCCCTTTGAA [SEQ ID NO:124] |
| | Taqman-forward | CCACGGCCACATTTGGTT [SEQ ID NO:125] |
| | Taqman-reverse | AGGGAAGCGGTTGCTCATC [SEQ ID NO:126] |
| | probe | AGAAACCCTCTGTCATTCGCTCCCACAT [SEQ ID NO:127] |
| IL1rn | RT-forward | CTCCGCAGTCACCTAATCACTCT [SEQ ID NO:128] |
| | RT-reverse | GGCTCAATGGGTACCACATCTATCT [SEQ ID NO:129] |
| | Taqman-forward | TTCCTGTTCCATTCAGAGACGAT [SEQ ID NO:130] |
| | Taqman-reverse | AGATTCTGAAGGCTTGCATCTTG [SEQ ID NO:131] |
| | probe | TGCCGACCCTCTGGGAGAAAATCC [SEQ ID NO:132] |
| LTA4H | RT-forward | ATTCAAGGATCTTGCTGCCTTT [SEQ ID NO:133] |
| | RT-reverse | TGCAGTCACGGGATGCAT [SEQ ID NO:134] |
| | Taqman-forward | CAAGGATCTTGCTGCCTTTGA [SEQ ID NO:135] |
| | Taqman-reverse | TGCTTGCTTTGTGCTCTTGGT [SEQ ID NO:136] |
| | probe | AAATCCCATGATCAAGCTGTCCGAACC [SEQ ID NO:137] |
| LTC4S | RT-forward | CACCACACCGACGGTACCA [SEQ ID NO:138] |
| | RT-reverse | TGCGCGCCGAGATCA [SEQ ID NO: 139] |
| | Taqman-forward | CCATGAAGGACGAGGTAGCTCTA [SEQ ID NO:140] |
| | Taqman-reverse | TGCGCGCCGAGATCA [SEQ ID NO:141] |
| | probe | CCTGGGAGTCCTGCTGCAAGCCTACT [SEQ ID NO:142] |
| MRC1 | RT-forward | CGCTACTAGGCAATGCCAATG [SEQ ID NO:143] |
| | RT-reverse | GCAATCTGCGTACCACTTGTTTT [SEQ ID NO:144] |
| | Taqman-forward | CGCTACTAGGCAATGCCAATG [SEQ ID NO:145] |
| | Taqman-reverse | GCAATCTGCGTACCACTTGTTTT [SEQ ID NO:146] |
| | probe | AGCAACCTGTGCATTCCCGTTCAAGT [SEQ ID NO:147] |
| MRC2 | RT-forward | GGGAGCACTGCTATTCTTTCCA [SEQ ID NO:148] |
| | RT-reverse | CAAACACATTCTCCATCTCATCCA [SEQ ID NO:149] |
| | Taqman-forward | GAGCACTGCTATTCTTTCCACATG [SEQ ID NO:150] |
| | Taqman-reverse | TCTCCATCTCATCCAGGATAGACA [SEQ ID NO:151] |
| | probe | CCACCCGCTCTCTGGCAGCG [SEQ ID NO:152] |
| SCYA22 | RT-forward | GCATGGCTCGCCTACAGACT [SEQ ID NO:153] |
| | RT-reverse | CAGACGGTAACGGACGTAATCAC [SEQ ID NO:154] |
| | Taqman-forward | TGGCGCTTCAAGCAACTG [SEQ ID NO:155] |
| | Taqman-reverse | CAGACGGTAACGGACGTAATCA [SEQ ID NO:156] |
| | probe | AGGCCCCTACGGCGCCAACAT [SEQ ID NO:157] |
| TNFa | RT-forward | CTGGTATGAGCCCATCTATCTGG [SEQ ID NO:158] |
| | RT-reverse | TTGGATGTTCGTCCTCCTCAC [SEQ ID NO:159] |
| | Taqman-forward | GGAGAAGGGTGACCGACTCA [SEQ ID NO:160] |
| | Taqman-reverse | TGCCCAGACTCGGCAAAG [SEQ ID NO:161] |

(continued)

| Gene | Type | Sequence |
|---|---|---|
| | probe | CGCTGAGATCAATCGGCCCGACTA [SEQ ID NO:162] |
| SCYA20 | RT-forward | GGCTGTGACATCAATGCTATCATC [SEQ ID NO:163] |
| | RT-reverse | GTCCAGTGAGGCACAAATTAGATAAG [SEQ ID NO:164] |
| | Taqman-forward | TCTGGAATGGAATTGGACATAGCCCAAG [SEQ ID NO:165] |
| | Taqman-reverse | CCAACCCCAGCAAGGTTCTTTCTG [SEQ ID NO:166] |
| | probe | ACCCTCCATGATGTGCAAGTGAAACC [SEQ ID NO:167] |
| SCYA17 | RT-forward | GGATGCCATCGTTTTTGTAACTG [SEQ ID NO:168] |
| | RT-reverse | CCTCTCAAGGCTTTGCAGGTA [SEQ ID NO:169] |
| | Taqman-forward | GGGCAGGGCCATCTGTTC [SEQ ID NO:170] |
| | Taqman-reverse | TCTCAAGGCTTTGCAGGTATTTAA [SEQ ID NO:171] |
| | probe | ACCCCAACAACAAGAGAGTGAAGAATGCA [SEQ ID NO:172] |
| IL12A | RT-forward | CCTCCTCCTTGTGGCTACCC [SEQ ID:173] |
| | RT-reverse | CAATCTCTTCAGAAGTGCAAGGG [SEQ ID:174] |
| | Taqman-forward | TCCTCCTGGACCACCTCAGT[SEQ ID:175] |
| | Taqman-reverse | GAACATTCCTGGGTCTGGAGTG [SEQ ID:176] |
| | Probe | TGGCCAGAAACCTCCCCGTGG [SEQ ID:177] |
| IFNγ | RT-forward | GTAACTGACTTGAATGTCCAACGC [SEQ ID:178] |
| | RT-reverse | GACAACCATTACTGGGATGCTC [SEQ ID:179] |
| | Taqman-forward | CCAACGCAAAGCAATACATGA [SEQ ID:180] |
| | Taqman-reverse | TTTTCGCTTCCCTGTTTTAGCT [SEQ ID:181] |
| | Probe | TCCAAGTGATGGCTGAACTGTCGCC [SEQ ID:182] |
| IL-10 | RT-forward | GTTGCCTGGTCCTCCTGACT [SEQ ID:183] |
| | RT-reverse | TGTCCAGCTGATCCTTCATTTG [SEQ ID:184] |
| | Taqman-forward | TGAGAACAGCTGCACCCACTT [SEQ ID:185] |
| | Taqman-reverse | GCTGAAGGCATCTCGGAGAT [SEQ ID:186] |
| | Probe | CAGGCAACCTGCCTAACATGCTTCG [SEQ ID:187] |
| IL-17A | RT-forward | ACTGCTACTGCTGCTGAGCCT [SEQ ID:188] |
| | RT-reverse | GGTGAGGTGGATCGGTTGTAGT [SEQ ID:189] |
| | Taqman-forward | CAATCCCACGAAATCCAGGA [SEQ ID:190] |
| | Taqman-reverse | TTCAGGTTGACCATCACAGTCC [SEQ ID:191] |
| | Probe | CCCAAATTCTGAGGACAAGAACTTCCCC [SEQ ID:192] |

For qPCR for periostin and CEACAM5, relative copy number for periostin and CEACAM5 expression in baseline bronchial epithelial brushing samples were obtained according to a previously described method [45] and $\log_{10}$ transformed. The 35-probe IL13 signature described in Example 9 (*see also* Fig. 11) was used as a response metric. All models were derived iteratively using the Fit Model platform in JMP 7.0. Ordinal logistic regression was performed to predict response (35 probe IL13 status) having levels (Healthy control; HC) < (IL13 Low) < (IL13 High). The generalized predicitive model for probability for each level is described as follows:

$$p_{HC} = \frac{1}{(1+e^{(-\beta_{HC}-\beta_{S})})}$$

$$p_{IL13low} = \frac{1}{(1+e^{(-\beta_{IL13Low}-\beta_{S})})} - p_{HC}$$

$$p_{IL13high} = 1-(p_{HC}+p_{IL13Low})$$

$$\beta_0 = \sum_{qPCR_i}^{k} A_i \times X_i \text{ (Linear sum)}$$

$$\beta_0 = \prod_{qPCR_i}^{k} A_i \times X_i \text{ (Product for cross terms)}$$

$$\beta_x = \text{Intercept estimate of qPCR parameter x}$$

Ordinal logistic regression was performed for the following model: (35 probe IL13 status) ~ (POSTN) + (CEACAM5). A whole model p-value of <0.0001 was derived from the dataset based on an iterative fit.

IL13 responsive genes

The relationship between periostin (also known as osteoblast specific factor) (POSTN: 210809_s_at), CLCA1 (also known as chloride channel, calcium activated, family member 1) (CLCA1: 210107_at), and SERPINB2 (also known as serpin peptidase inhibitor, clade B (ovalbumin), member 2) (SERPINB2: 204614_at) expression level was confirmed using the Wilcoxon Rank Sum test. POSTN expression level was used to categorize baseline asthma samples. A cutoff of 800 units was used, resulting in 21 asthma baseline asthma samples being classified as "IL13 low" (POSTN < 800 units) and the remaining 21 samples as "IL13 high" (POSTN > 800). Wilcoxon Rank Sum test followed by false discovery rate analysis (qvalue < 0.05) [24] identified 35 probes differentially expressed among the two groups. Hierarchical clustering using these probes was undertaken. Due to the presence of many cystatin and serpin family genes in the list differentially regulated probes, additional cystatin and serpin family probes were identified and used in an additional cluster analysis. All statistical analyses were performed using R. Microarray cluster analysis was performed using Cluster and visualized using Java Treeview [25, 26].

Serum analyte assays

Serum IgE was measured by UCSF clinical laboratories or by ELISA using a human serum IgE ELISA kit according to manufacturer's instructions (Bethyl Laboratories). Serum CEA was measured using a human serum CEA ELISA kit according to manufacturer's instructions (Alpco Diagnostics). We developed an electrochemiluminescent assay (ECLA) to measure serum periostin using anti-periostin antibodies (R&D systems). Briefly, monoclonal anti-periostin was coated onto plates at 1.5 micrograms/ml in sodium carbonate buffer, pH 9.6 overnight at 4°C. Plates were blocked in assay buffer (IX PBS pH 7.4, 0.35 M NaCl, 0.5% BSA, 0.05% Tween 20, 0.25% CHAPS, 5mM EDTA, 15PPM Proclin) + 3% BSA for 2 hours at room temperature, then washed 4x with TBST (Tris-buffered saline + 0.1% Tween-20). Serum was diluted 1:5 in assay buffer and incubated with agitation at room temperature for 2 h, then washed 4x with TBST. Recombinant periostin (R&D Systems) was used to establish a standard range. Biotinylated polyclonal anti-human periostin (1.5 microgram/ml) (R&D Systems; biotinylated *in vitro* according to standard methods known in the art) and Ruthenium-streptavidin (0.75 microgram/ml) (Meso Scale Devices) were added in assay buffer + 5% goat serum and incubated for 90 minutes at room temperature. Reading buffer (Meso Scale Devices) was added and electrochemiluminescence was read (Meso Scale Devices). Dynamic range was 5-2000 ng/ml.

Example 2 - IL-4/13 signature and subsets of asthmatics

To determine if three IL-13 induced genes (periostin, CLCA1, and serpinB2) reflect a broader pattern of gene expression in asthmatic airway epithelium, we examined whether their expression was co-regulated at baseline within individual subjects among the 42 asthmatics studied. In pairwise comparisons, the expression levels of periostin, CLCA1, and serpinB2 were significantly correlated within individual asthmatics. Furthermore, these genes were highly expressed in some, but not all, of the asthmatic subjects (Figs. 1A and 1B). In addition, expression levels of these three genes were highly correlated within individual subjects with asthma (Fig. 1B). These data suggest that certain IL-13 markers are over-expressed in a specific subset of patients with asthma. In further experiments, we sought to identify additional genes or markers that might be directly or indirectly regulated by IL-13 and we sought to characterize subsets of asthma patients based on expression of IL-13 markers.

To identify other genes or markers that could potentially be regulated directly or indirectly by IL-13 in asthmatic airway epithelium, we examined the entire microarray dataset across the 42 asthmatic subjects for genes whose expression was significantly correlated with that of periostin. We identified a cluster of 653 probes whose expression was coregulated with periostin in individual subjects below a threshold q-value of 0.05. Unsupervised clustering of all subjects including healthy controls and asthmatics based on expression levels of those 653 probes revealed two major clusters: a cluster with high expression levels of periostin and co-regulated genes and a cluster with low expression levels of periostin and co-regulated genes. The core of this gene cluster (Fig. 1C, right panel) comprises a subset of 35 probes representing the genes shown in Figure 13, which we refer to herein as "IL-4/13 signature," "IL-4/13 gene signature," "IL-13 signature," or "IL-13 gene signature." As indicated previously, those terms are used synonymously herein. The cluster with high expression of periostin and co-regulated genes comprised 21 asthmatic subjects and no healthy controls (Fig. 1C, right panel, labeled "Il-4/13 signature high") whereas the cluster with low expression of periostin and co-regulated genes comprised the remaining 21 asthmatics (Fig. 1C, right panel, labeled "IL-4/13 signature low") interspersed with all 27 of the healthy controls (Fig. 1C, right panel).

Cluster 1 ("IL-4/13 signature high") is characterized by high expression levels of the genes corresponding to probes for periostin, CST1, CST2, CST4, CCL26, CLCA1, CDH26, PRR4, serpinB2, serpinB10, CEACAM5, iNOS, C2ORF32, PTGS1, P2RY14, RUNX2, SH3RF2, WLRW300, DNAJC12, ALOX15, GSN, RGS13, TGSAB1, PTSG1, FCER1B, and CPA3 and consists of approximately half the asthmatics in the study (N=23 out of 42 asthmatics) and one healthy control out of 27 total healthy controls. Cluster 2 (Healthy controls and "IL-4/13 signature low") is characterized by low expression levels of the genes corresponding to the indicated probes and consists of the remaining 19 asthmatics and 26/27 healthy controls. Probes corresponding to genes predominantly expressed in mast cells, including RGS13, TPSG1, TPSAB1, FCER1B, CPA3, and SLC18A2 are indicated in blue in Table 2 and probes corresponding to genes predominantly expressed in eosinophils, including P2RY14 and ALOX15 are indicated in orange. Although the epithelial brushings consisted of predominantly epithelial cells and goblet cells (mean 97%, median 98%, minimum 91%), small numbers of infiltrating mast cells and eosinophils were observed in the brushings from cluster 1 asthmatics, and the presence of mast cell and eosinophil genes in the signature likely reflects this infiltration.

To characterize subsets of subjects with asthma based on expression of IL-13 markers, we performed unsupervised hierarchical clustering of all 70 subjects (42 asthmatics and 27 healthy controls) based on the microarray expression levels of periostin, CLCA1, and serpinB2 (Figure 1D). In this analysis, approximately half of subjects with asthma (N=22) showed consistently high expression levels of IL-13-induced genes and grouped together in one major branch of the cluster dendrogram (cluster 1, the "IL-13 high" subset). Remarkably, although periostin, CLCA1, and serpinB2 were significantly over-expressed when comparing all 42 asthmatics to all 27 healthy controls [8], nearly half of the asthmatics examined in this study (N=20) were indistinguishable from healthy controls on the basis of expression of these three genes. This subset of asthmatics (the "IL-13 low" subset) and all the healthy controls grouped together in the second major branch of the dendrogram (Figure 1D, cluster 2). Thus, hierarchical clustering based on epithelial gene expression identified two distinct subsets of patients with asthma, referred to herein as "IL-13 high" subset and "IL-13 low" subset.

To confirm the validity of these asthma patient subsets, identified using IL-13 inducible marker expression in epithelial cells, we measured the expression level of IL-13 and certain other Th2 cytokines (i.e. IL-4 and IL-5) in bronchial biopsies obtained contemporaneously from 48 of the subjects (14 healthy controls, 18 cluster 1 asthmatics, and 16 cluster 2 asthmatics). Using qPCR, we found that IL-13, IL-5 and IL-4 expression was detectable in homogenates of bronchial biopsies. Notably, IL-13 and IL-5 expression, but not IL-4 expression, were significantly higher (Fig. IE, *, $p < 0.002$) in cluster 1 asthmatics compared to cluster 2 asthmatics or healthy controls. There were no significant differences, however, in IL-4, IL-5, or IL-13 expression between asthmatics in cluster 2 and healthy controls (Fig. IE). In addition, we found that expression levels of IL-13 and IL-5 were highly correlated across all of the subjects with asthma (Spearman's rank order correlation $\rho$=0.58, $p<0.0001$; Fig. IE). IL-4 shares a dominant signaling pathway with IL-13 and has been shown to induce periostin [7, 9] and CLCA1 [12] expression similarly to IL-13. As elevated levels of IL-4 expressing T cells have been reported in bronchoalveolar lavage (BAL) fluid [79] from asthmatics and we did not specifically examine cytokine gene expression in BAL T cells or cytokine protein levels in BAL or bronchial tissue in this study, we cannot rule out the possibility that the observed induction of periostin, CLCA1, and serpinB2 is due in part to IL-4 as well as to IL-13. Based on the data shown herein, we can confidently discern a correlation between bronchial IL-13 expression and epithelial periostin, CLCA1, and serpinB2 expression. Thus, we use the terms "IL-4/13 high" and "IL-13 high" synonymously to refer to cluster 1 asthmatics and we use the terms "IL-4/13 low" and "IL-13 low" synonymously to refer to cluster 2 asthmatics. It is understood that when the terms "IL-13 high" and "IL-13 low" are used, IL-4 and/or other as yet unidentified factors may also contribute in part to the observed gene expression patterns.

Example 3 - Constituent Genes of IL-4/13 signature

Within the IL-4/13 signature, there are two major groups of genes: epithelial or goblet cell expressed genes and mast cell expressed genes. Greater than 90% of cells in each bronchial brushing sample were bronchial epithelial cells or goblet cells (mean 97%, median 98%, minimum 91%). Expression levels of probes corresponding to the following epithelial or goblet cell genes were most significantly co-regulated with those of periostin: CST1, CST2, CCL26, CLCA1, PRR4, serpinB2, CEACAM5, and iNOS (Table 2, indicated with asterisks; > 3-fold higher expression in IL-4/13 signature high vs. IL-4/13 signature low subjects). The mouse orthologue of CLCA1, mCLCA3 (also known as gob-5) has been previously identified as a gene associated with goblet cell metaplasia of airway epithelium and mucus production; both are induced by Th2 cytokines including IL-9 and IL-13 [12-14]

Table 2.

| Probe | Gene Name | Fold change, High vs. Low | p-value, Low High vs. Low | q-value, High vs. Low | Healthy Mean | IL-4/13 signature Low mean | IL-4/13 signature High mean | Fold change, High vs. Control | Fold change, Low vs. Control |
|---|---|---|---|---|---|---|---|---|---|
| 1555778 _a_at | **POSTN*** | **11.35** | 2.60E-11 | 7.11E-07 | 14.93 | 15.73 | 178.51 | 11.96 | 1.05 |
| 206224_at | **CST1*** | **11.12** | 9.09E-06 | 0.021609818 | 8.76 | 32.37 | 360.02 | 41.12 | 3.70 |
| 223710_at | **CCL26*** | **10.22** | 2.88E-05 | 0.045024394 | 6.33 | 3.87 | 39.57 | 6.25 | 0.61 |
| 206994_at | **CST1*** | **9.98** | 4.90E-06 | 0.014874475 | 10.38 | 67.81 | 676.94 | 65.22 | 6.53 |
| 210107_at | **CLCA1*** | **9.77** | 1.96E-07 | 0.001785296 | 29.61 | 95.06 | 928.81 | 31.37 | 3.21 |
| 208555_x_ at | **CST2*** | **9.13** | 9.04E-07 | 0.004119975 | 5.13 | 14.75 | 134.71 | 26.26 | 2.88 |
| 210809 _s_ at | **POSTN*** | **7.70** | 3.72E-12 | 2.03E-07 | 260.24 | 334.28 | 2572.46 | 9.88 | 1.28 |
| 204919_at at | **PRR4*** | **6.09** | 5.73E-06 | 0.01649484 | 37.33 | 97.20 | 592.05 | 15.86 | 2.60 |
| 207741_x_ at | **TPSD1** | **4.76** | 1.54E-06 | 0.005250514 | 9.86 | 18.81 | 89.64 | 9.09 | 1.91 |
| 204614 _at | **SERPINB2*** | **4.52** | 4.30E-07 | 0.002615287: | 97.43 | 212.63 | 960.75 | 9.86 | 2.18 |
| 201884_ at | **CEACAM5 *** | **3.48** | 4.30E-07 | 0.002615287 | 426.04 | 525.17 | 1830.00 | 4.30 | 1.23 |
| 210037_s_ at | | **3.30** | 2.51E-05: | 0.045024394 | 6.39 | 6.54 | 21.60 | 3.38 | 1.02 |
| 216485_s_ at | **TPSG1** | **3.23** | 7.81E-06 | 0.0203328 | 10.04 | 17.84 | 57.65 | 5.74 | 1.78 |
| 216474_x_ at | **TPSD1** | **3.06** | 160E-07 | 0.00174608 | 46.63 | 77.82 | 238.00 | 5.10 | 1.67 |
| 205683_x_ at | **TPSD1** | **2.97** | 2.72E-08 | 0.00049597 | 53.99 | 76.74 | 227.95 | 4.22 | 1.42 |
| 225316_ at | **MFSD2** | **2.96** | 2.18E-05 | 0.042590277 | 29.03 | 26.00 | 76.95 | 2.65 | 0.90 |
| 205624_at | **CPA3** | **2.94** | 3.55E-07 | 0.002615287 | 99.69 | 166.29 | 489.17 | 4.91 | 1.67 |
| 206637_at | **GPR105** | **2.88** | 6.27E-07 | 0.003117623 | 40.90 | 65.93 | 189.66 | 4.64 | 1.61 |

(continued)

| Probe | Gene Name | Fold change, High vs. Low | p-value, Low High vs. Low | q-value, High vs. Low | Healthy Mean | IL-4/13 signature Low mean | IL-4/13 signature High mean | Fold change, High vs. Control | Fold change, Low vs. Control |
|---|---|---|---|---|---|---|---|---|---|
| 232306_at | **CDH26** | **2.85** | 1.29E-06 | 0.00470447 | 223.92 | 326.79 | 932.02 | 4.16 | 1.46 |
| 207134_x_ at | **TPSD1** | **2.66** | 6.27E-07 | 0.003117623 | 50.28 | 86.08 | 228.78 | 4.55 | 1 .71 |
| 210084_x_ at | **TPSD1** | **2.56** | 6.70E-06 | 0.018307462 | 48.91 | 77.59 | 198.54 | 4.06 | 1.59 |
| 200696_s_ at | **GSN** | **2.50** | 2.88E-05 | 0.045024394 | 246.87 | 224.72 | 562.74 | 2.28 | 0.91 |
| 226751_ at | **C2ORF32** | **2.50** | 9.09E-06 | 0.021609818 | 35.39 | 32.96 | 82.47 | 2.33 | 0.93 |
| 238429_at | **TRACH2000196** | **2.39** | 2.88E-05 | 0.045024394 | 36.79 | 37.68 | 89.91 | 2.44 | 1.02 |
| 218976_at | **DNAJC12** | **2.32** | 2.18E-05 | 0.042590277 | 48.54 | 38.92 | 90.11 | 1.86 | 0.80 |
| 217023_x_ at | **TPSD1** | **2.31** | 1.29E-06 | 0.00470447 | 53.13 | 67.76 | 156.32 | 2.94 | 1.28 |
| 215382_x_ at | **TPSD1** | **2.30** | 1.08E-06 | 0.004549197 | 38.96 | 52.95 | 121.86 | 3.13 | 1.36 |
| 210258_at | **RGS13** | **2.25** | 2.88E-05 | 0.045024394 | 8.75 | 7.56 | 17.04 | 1.95 | 0.86 |
| 205857 _at | **SLC18A2** | **2.23** | 1.05E-07 | 0.001435039 | 84.08 | 100.96 | 225.07 | 2.68 | 1.20 |
| 214539_at | **SERPINB10** | **2.15** | 1.06E-05 | 0.024063758 | 42.37 | 40.04 | 86.16 | 2.03 | 0.95 |
| 243582_at | **SH3RF2** | **2.00** | 1.89E-05 | 0.039805857 | 82.64 | 85.89 | 171.80 | 2.08 | 1.04 |
| 207496_at | **FCER1B** | **1.92** | 2.51E-05 | 0.045024394 | 37.55 | 37.03 | 71.28 | 1.90 | 0.99 |
| 232231_at | **RUNX2** | **1.77** | 2.88E-05 | 0.045024394 | 288. 42 | 299.21. | 529.59 | 1.84 | 1.04 |
| 238669_at | **PTGS1** | **1.73** | 4.18E-06 | 0.013429003 | 82.69 | 88.43 | 152.98 | 1.85 | 1.07 |
| 207328 _at | **ALOX15** | **1.72** | 1.64E-05 | 0.03586964 | 812.57 | 895.94 | 1538.53 | 1.89 | 1.10 |

SerpinB2 is a member of a large family of serine protease inhibitors encoded in a gene cluster on chromosome 18q21 (Fig. 2A, top; screen capture from UCSC Genome Browser at http://genome.ucsc.edu). Expression levels of serpins B2 [8], B3, and B4 are induced in airway epithelial cells upon stimulation by recombinant IL-4 and IL-13 [7, 15].

Cystatins (CST) 1 and 2 are members of a large family of cysteine protease inhibitors encoded in a gene cluster on chromosome 20p11 (Fig. 2A, middle; screen capture from UCSC Genome Browser at http://genome.ucsc.edu). Several cystatins are expressed in bronchial epithelium [16]; CST4 has been identified at elevated levels in bronchoalveolar lavage fluid (BAL) of asthmatics [17]; serum CST3 is elevated in asthmatics relative to healthy controls and its levels are decreased by ICS treatment [18]. As serpin and CST gene families are each colocalized on the chromosome, we explored whether any additional members of the serpin and cystatin gene families are co-regulated with those already identified. We performed unsupervised clustering of the microarray data, restricted to serpin and cystatin gene families. We found that serpins B2, B4, and B10; and cystatins 1, 2, and 4 were significantly co-regulated, with the highest expression levels occurring in asthmatics positive for the "IL-4/13 signature" (Fig. 2B).

PRR4 is a member of a large family of proteins encoded in a gene cluster on chromosome 12p13 (Fig. 2A, bottom; screen capture from UCSC Genome Browser at http://genome.ucsc.edu). These proline-rich proteins are found in mucosal secretions including saliva and tears. Related, but non-orthologous proteins SPRR1a, 2a, and 2b have been identified in bronchial epithelium in a mouse model of asthma and are induced by IL-13 [19, 20], Proline-rich proteins from the PRR/PRB family have been identified in bronchial secretions [21] and their expression has been documented in bronchial epithelium [16]. Of the PRR/PRB family, PRR4 and PRB4 were significantly upregulated in asthmatics with high expression of the IL-4/13 gene signature (Fig. 2C, left and middle).

CCL26 (Eotaxin-3) is an IL-4 and IL-13 inducible chemokine in asthmatic airway epithelium.

CEACAM5 encodes a cell-surface glycoprotein found in many epithelial tissues and elevated serum. CEACAM5 (carcinoembryonic antigen; CEA) is a well-documented systemic biomarker of epithelial malignancies and metastatic disease. Elevated CEA levels have been reported in a subset of asthmatics, with particularly high serum levels observed in asthmatics with mucoid impaction [22]. CEACAM5 is significantly upregulated in IL-4/13 signature high asthmatic airway epithelium compared to IL-4/13 signature low and healthy control airway epithelium (Fig. 2C, right), which suggests that serum CEA levels may be used to distinguish between these two asthmatic sub-phenotypes.

Inducible nitric oxide synthase (iNOS) is associated with airway inflammation and is induced by IL-13 in human primary bronchial epithelial cell cultures [23]. The measurement of exhaled nitric oxide (eNO), a product of iNOS enzymatic activity, is commonly used in the diagnosis and monitoring of asthma.

Example 4 - Mast Cells

Although the airway brushings used in this study comprised predominantly epithelial and goblet cells, there were small but significant percentages of infiltrating leukocytes in many of the samples. Genes whose expression is specific to mast cells, including tryptases (TPSD1, TPSG1), caboxypeptidase A3 (CPA3), and FcepsilonRIbeta, were significantly correlated with the IL-4/13 gene signature (Table 2 and Table 4, mast cell genes marked with double astericks in Table 4). Given the significant role of tissue-resident mast cells in allergic disease and the recent observation that the presence of IL-13 expressing mast cells in asthmatic endobronchial biopsy specimens is positively correlated with detectable levels of IL-13 in sputum [6], the high correlation between mast cell-specific genes and the IL-4/13 signature suggests that: 1) mast cells may be a significant source of IL-13 in the airway epithelium and 2) mast cell infiltration into airway epithelium may be a unique feature of the IL-4/13 signature high subset of asthmatics.

Example 5 - Combinations that Predict IL-4/13 Signature

Expression levels of individual genes in the IL-4/13 signature may predict the IL-4/13 signature status of individual subjects with variable accuracy; however combinations of these genes may be used to assign individual subjects to the IL-4/13 signature high or low category with increased sensitivity and specificity.

Example 6 - Steroid Effect

The standard of care for bronchial asthma that is not well-controlled on symptomatic therapy (i.e. beta-adrenergic agonists) is inhaled corticosteroids (ICS). In mild-to-moderate asthmatics with elevated levels of IL-13 in the airway [6] and in eosinophilic esophagitis patients with elevated expression levels of IL-13 in esophageal tissue [11], ICS treatment substantially reduces the level of IL-13 and IL-13-induced genes in the affected tissues. In airway epithelium of asthmatics after one week of ICS treatment and in cultured bronchial epithelial cells, we have shown that corticosteroid treatment substantially reduces IL-13-induced expression levels of periostin, serpinB2, and CLCA1 [8]. Further examination of the genes listed in Table 2 revealed that, in the 19 subjects in our study who received one week of ICS treatment prior to a second bronchoscopy, the vast majority of IL-4/13 signature genes was significantly downregulated by ICS treatment in asthmatic bronchial airway epithelium (periostin shown as an example, Fig. 3A). This downregulation could be the result of ICS-mediated reduction of IL-13 levels, ICS-mediated reduction of target gene expression, or a combination of the two. However, two genes in the IL-4/13 signature, PRR4 (Fig. 3B) and RUNX2 (Fig. 3C), were not substantially downregulated in individual subjects after one week of ICS treatment. This suggests that PRR4 and RUNX2 may be steroid-insensitive markers of the IL-4/13 signature in asthmatic airway epithelium. Another possibility is that PRR4 and RUNX2 are only indirectly regulated by IL-4 and/or IL-13; for example, as PRR4 is found in many secretions, it may be a goblet cell-specific gene. As goblet cell differentiation from epithelial cells is induced by IL-13, ICS-mediated inhibition of IL-13 and IL-13 dependent processes may not substantially impact on goblet cell number after only 7 days of treatment, but after longer-term ICS treatment, goblet cell numbers (and hence PRR4 expression in endobronchial brushings) may be expected to decrease. In severe asthmatics who are refractory to ICS treatment, a similar fraction of subjects (approximately 40%) was found to have detectable sputum IL-13 levels to that seen in mild, ICS-naive asthmatics [6], which is consistent with the fraction of subjects with the IL-4/13 signature observed in this study. This observation suggests that, although the IL-4/13 signature is significantly downregulated by ICS treatment in the mild-moderate, ICS-responsive asthmatics examined in the present study, it may still be present in severe steroid-resistant asthmatics.

Example 7 - Relationship of IL-4/13 signature to clinical features and other biomarkers

• Demographics

Eosinophilic asthma, as defined by elevated levels of airway eosinophils, is associated with atopy and occurs with approximately equal prevalence between males and females, while the non-eosinophilic phenotype, as defined by a relative absence of eosinophils in the airway and associated with a lack of atopy, shows a significant female predominance [1]. Of the subjects classified according to the airway epithelial IL-4/13 gene signature, 10/21 (48%) IL-4/13 signature high subjects were female while 15/21 (71%) IL-4/13 signature low subjects were female (Table 3). There was no significant skewing by self-reported ethnicity between the IL-4/13 signature low and high groups.

Gender distribution of IL-4/13 signature, N (%)

| Category | F | M |
|---|---|---|
| LOW | 15/(71) | 6(29) |
| HIGH | 10(48) | 11(52) |
| CONTROL | 15(54) | 13(46) |

• $FEV_i$ and Methacholine responsiveness

While the gender skewing between the IL-4/13 low and high groups suggest that the observed gene expression patterns in asthmatic airway epithelium reflect stable underlying phenotypes, it is possible that the observed gene expression patterns merely reflect disease severity or activity at the time of bronchoscopy. To determine whether the IL-4/13 signature was correlated to asthma severity, we compared forced expiratory volume in one second ($FEV_1$, as a percentage of predicted from patient weight, measured at a screening visit one week prior to bronchoscopy) between the groups and found that, while both the IL-4/13 signature high and low groups had significantly lower $FEV_1$ than healthy controls, there was no statistically significant difference between the groups *(see* Fig. 5A), although there were more subjects that might be classified as "moderate" (i.e. $FEV_i$ 60-80% predicted) in the IL-4/13 signature high group than in the low group. The minimal concentration of methacholine in mg/ml required to induce a decrease in $FEV_1$ of 20% ($PC_{20}$, measured at a screening visit one week prior to bronchoscopy) is a measure of bronchial hyperresponsiveness. This is a measure of bronchial hyper-reactivity (BHR). Both the IL-4/13 signature high and low groups had significantly lower $PC_{20}$ values than healthy controls; while there was a trend toward lower $PC_{20}$ values in the IL-4/13 signature high group than in the low group, this difference did not reach statistical significance *(see* Fig. 5C).

• IgE and Eosinophils (peripheral and airway)

To determine whether the IL-4/13 signature status of an individual subject could be predicted by standard measures of atopy, we examined levels of serum IgE (international units per milliliter; 1 IU = 2.4 ng), peripheral blood eosinophil counts (absolute number of eosinophils x 10^9 per liter of blood), and eosinophil percentages in bronchoalveolar lavage fluid (BAL) (percentage of eosinophils relative to the total number of non-squamous cells in bronchoalveolar lavage fluid) using standard clinical laboratory tests, obtained at the time of bronchoscopy. When subjects were stratified for IL-4/13 signature status, there were significant differences in serum IgE *(see* Fig. 6B), peripheral blood eosinophil counts *(see* Fig. 6C), and BAL eosinophil percentage *(see* Fig. 6D), with significantly higher values for each analyte observed in the IL-4/13 signature high group relative to the low group. Taken individually, neither IgE level nor peripheral blood eosinophil count predicts the airway epithelial IL-4/13 signature status of any individual subject with simultaneously high sensitivity and specificity. However, among individual asthmatics, IgE level and peripheral blood eosinophil counts are weakly but significantly correlated (rho=0.44, p=$3.4x10^{-3}$). When considered as a composite, empirically derived cutoff values of both 100 IU/ml IgE and $0.14x10^9$/L eosinophils predict the airway epithelial IL-4/13 signature status of individual subjects with high sensitivity and specificity (Fig. 4; 18/21 correct for both low and high IL-4/13 signature; sensitivity = 86%, specificity = 86%).

**Table 4.** IL-4/13 gene signature genes and exemplary probes.

| Gene | Example Probes |
| --- | --- |
| POSTN | 1555778_a_at:<br>AAAGAATCTGACATCATGACAACAAATGGTGTAATTCATGTTGTAGATAAACTCCTCTAT<br>CCAGCAGACACACCTGTTGGAAATGATCAACTGCTGGAAATACTTAATAAATTAATCAAA<br>TACATCCAAATTAAGTTTGTTCGTGGTAGCACCTTCAAAGAAATCCCCGTGACTGTCTAT<br>AGACCCACACTAACAAAAGTCAAAATTGAAGGTGAACCTGAATTCAGACTGATTAAAGAA<br>GGTGAAACAATAACTGAAGTGATCCATGGAGAGCCAATTATTAAAAAATACACCAAAATC<br>ATTGATGGAGTGCCTGTGGAAATAACTGAAAAAGAGACACGAGAAGAACGAATCATTACA<br>GGTCCTGAAATAAAATACACTAGGATTTCTACTGGAGGTGGAGAAACAGAAGAAACTCTG<br>AAGAAATTGTTACAAGAAGAAGACACACCCGTGAGGAAGTTGCAAGCCAACAAAAAAGTT<br>CAANGGATCTAGAAGACGATTAAGGGAAGGTCGTTCTCAGTGAAAATCCA<br>[SEQ ID NO: 31]<br><br>210809_s_at:<br>AAATTGTGGAGTTAGCCTCCTGTGGAGTTAGCCTCCTGTGGTAAAGGAATTGAAGAAAAT<br>ATAACACCTTACACCCTTTTTCATCTTGACATTAAAAGTTCTGGCTAACTTTGGAATCCA<br>TTAGAGAAAAATCCTTGTCACCAGATTCATTACAATTCAAATCGAAGAGTTGTGAACTGT<br>TATCCCATTGAAAAGACCGAGCCTTGTATGTATGTTATGGATACATAAAATGCACGCAAG<br>CCATTATCTCTCCATGGGAAGCTAAGTTATAAAAATAGGTGCTTGGTGTACAAAACTTTT<br>TATATCAAAAGGCTTTGCACATTTCTATATGAGTGGGTTTACTGGTAAATTATGTTATTT<br>TTTACAACTAATTTTGTACTCTCAGAATGTTTGTCATATGCTTCTTGCAATGC [SEQ ID NO:32] |
| CST1 | 206994_at:<br>GCGAGTACAACAAGGCCACCGAAGATGAGTACTACAGACGCCCGCTGCAGGTGCTGCGAG<br>CCAGGGAGCAGACCTTTGGGGGGGTGAATTACTTCTTCGACGTAGAGGTGGGCCGCACCA<br>TATGTACCAAGTCCCAGCCCAACTTGGACACCTGTGCCTTCCATGAACAGCCAGAACTGC<br>AGAAGAAACAGTTATGCTCTTTCGAGATCTACGAAGTTCCCTGGGAGGACAGAATGTCCC<br>TGGTGAATTCCAGGTGTCAAGAAGCCTAGGGGTCTGTGCCAGGCCAGTCACACCGACCAC<br>CACCCACTCCCACCCCCTGTAGTGCTCCCACCCCTGGACTGGTGGCCCCCACCCTGCGGG<br>AGGCCTCCCCATGTGCCTGTGCCAAGAGACAGACAGAGAAGGCTGCAGGAGTCCTTTGTT<br>GCTCAGCAGGGCGCTCTGCCCTCCCTCCTTCCTTCTTGCTTCTAATAGACCTGGTACATG<br>GTACACACACCCC<br>[SEQ ID NO:33] |

(continued)

| Gene | Example Probes |
| --- | --- |
| | 206224_at:<br>GGAGGATAGGATAATCCCGGGTGGCATCTATAACGCAGACCTCAATGATGAGTGGGTACA<br>GCGTGCCCTTCACTTCGCCATCAGCGAGTATAACAAGGCCACCAAAGATGACTACTACAG<br>ACGTCCGCTGCGGGTACTAAGAGCCAGGCAACAGACCGTTGGGGGGGTGAATTACTTCTT<br>CGACGTAGAGGTGGGCCGAACCATATGTACCAAGTCCCAGCCCAACTTGGACACCTGTGC<br>CTTCCATGAACAGCCAGAACTGCAGAAGAAACAGTTGTGCTCTTTCGAGATCTACGAAGT<br>TCCCTGGGAGAACAGAAGGTCCCTGGTGAAATCCAGGTGTCAAGAATCCTAGGGATCTGT<br>GCCAG<br>[SEQ ID NO:34] |
| CCL26 | 223710_at:<br>GAGAAGGGCCTGATTTGCAGCATCATGATGGGCCTCTCCTTGGCCTCTGCTGTGCTCCTG<br>GCCTCCCTCCTGAGTCTCCACCTTGGAACTGCCACACGTGGGAGTGACATATCCAAGACC<br>TGCTGCTTCCAATACAGCCACAAGCCCCTTCCCTGGACCTGGGTGCGAAGCTATGAATTC<br>ACCAGTAACAGCTGCTCCCAGCGGGCTGTGATATTCACTACCAAAAGAGGCAAGAAAGTC<br>TGTACCCATCCAAGGAAAAAATGGGTGCAAAAATACATTTCTTTACTGAAAACTCCGAAA<br>CAATTGTGACTCAGCTGAATTTTCATCCGAGGACGCTTGGACCCCGCTCTTGGCTCTGCA<br>GCCCTCTGGGGAGCCTGCGGAATCTTTTCTGAAGGCTACATGGACCCGCT<br>[SEQ ID NO:35] |
| CLCA1 | 210107_at:<br>GGCCAAATCACCGACCTGAAGGCGGAAATTCACGGGGGCAGTCTCATTAATCTGACTTGG<br>ACAGCTCCTGGGGATGATTATGACCATGGAACAGCTCACAAGTATATCATTCGAATAAGT<br>ACAAGTATTCTTGATCTCAGAGACAAGTTCAATGAATCTCTTCAAGTGAATACTACTGCT<br>CTCATCCCAAAGGAAGCCAACTCTGAGGAAGTCTTTTTGTTTAAACCAGAAAACATTACT<br>TTTGAAAATGGCACAGATCTTTTCATTGCTATTCAGGCTGTTGATAAGGTCGATCTGAAA<br>TCAGAAATATCCAACATTGCACGAGTATCTTTGTTTATTCCTCCACAGACTCCGCCAGAG<br>ACACCTAGTCCTGATGAAACGTCTGCTCCTTGTCCTAATATTCATATCAACAGCACCATT<br>CCTGGCATTCACATTTTAAAAATTATGTGGAAGTGGATAGGAGAACTGCAGCTGTCAATA<br>GCCTAGGGC<br>[SEQ ID NO:36] |
| CST2 | 208555_x_at: |

(continued)

| Gene | Example Probes |
|---|---|
| | GAGCCCCCAGGAGGAGGACAGGATAATCGAGGGTGGCATCTATGATGCAGACCTCAATGA TGAGCGGGTACAGCGTGCCCTTCACTTTGTCATCAGCGAGTATAACAAGGCCACTGAAGA TGAGTACTACAGACGCCTGCTGCGGGTGCTACGAGCCAGGGAGCAGATCGTGGGCGGGGT GAATTACTTCTTCGACATAGAGGTGGGCCGAACCATATGTACCAAGTCCCAGCCCAACTT GGACACCTGTGCCTTCCATGAACAGCCAGAACTGCAGAAGAAACAGTTGTGCTCTTTCCA GATCTACGAAGTTCCCTGGGAGGA<br>[SEQ ID NO:37] |
| PRR4 | 204919_at:<br>AAGACTTTACTTTCACCATACCAGATGTAGAGGACTCAAGTCAGAGACCAGATCAGGGAC CCCAGAGACCTCCTCCTGAAGGACTCCTACCTAGACCCCCTGGTGATAGTGGTAACCAAG ATGATGGTCCTCAGCAGAGACCACCAAAACCAGGAGGCCATCACCGCCATCCTCCCCCAC CTCCTTTTCAAAATCAGCAACGACCACCCCAACGAGGACACCGTCAACTCTCTCTACCCC GATTTCCTTCTGTCAGCCTGCAGGAAGCATCATCATTCTTCCGGAGGGACAGACCAGCAA GACATCCCCA<br>[SEQ ID NO:38] |
| SERPINB2 | Serpin peptidase inhibitor, clade B (ovalbumin), member 2<br><br>204614_at:<br>TTCCTCACCCTAAAACTAAGCGTGCTGCTTCTGCAAAAGATTTTTGTAGATGAGCTGTGT GCCTCAGAATTGCTATTTCAAATTGCCAAAAATTTAGAGATGTTTTCTACATATTTCTGC TCTTCTGAACAACTTCTGCTACCCACTAAATAAAAACACAGAAATAATTAGACAATTGTC TATTATAACATGACAACCCTATTAATCATTTGGTCTTCTAAAATGGGATCATGCCCATTT AGATTTTCCTTACTATCAGTTTATTTTTATAACATTAACTTTTACTTTGTTATTTATTAT TTTATATAATGGTGAGTTTTTAAATTATTGCTCACTGCCTATTTAATGTAGCTAATAAAG TTATAGAAGCAGATGATCTGTTAATTTCCTATCTAATAAATGCCTTTAATTGTTCTCATA ATGAAGAATAAGTAGGTACCCTCCATGCCCTTCTGTAATAAATAT<br>[SEQ ID NO:39] |
| CEACAM5 | 201884_at: |

(continued)

| Gene | Example Probes |
|---|---|
| | AGAAGACTCTGACCTGTACTCTTGAATACAAGTTTCTGATACCACTGCACTGTCTGAGAA TTTCCAAAACTTTAATGAACTAACTGACAGCTTCATGAAACTGTCCACCAAGATCAAGCA GAGAAAATAATTAATTTCATGGGACTAAATGAACTAATGAGGATTGCTGATTCTTTAAAT GTCTTGTTTCCCAGATTTCAGGAAACTTTTTTTCTTTTAAGCTATCCACTCTTACAGCAA TTTGATAAAATATACTTTTGTGAACAAAAATTGAGACATTTACATTTTCTCCCTATGTGG TCGCTCCAGACTTGGGAAACTAT<br>[SEQ ID NO:40] |
| iNOS | Inducible nitric oxide synthase<br><br>210037_s_at:<br>TCATCGGGCCTGGCACAGGCATCGCGCCCTTCCGCAGTTTCTGGCAGCAACGGCTCCATG ACTCCCAGCACAAGGGAGTGCGGGGAGGCCGCATGACCTTGGTGTTTGGGTGCCGCCGCC CAGATGAGGACCACATCTACCAGGAGGAGATGCTGGAGATGGCCCAGAAGGGGGTGCTGC ATGCGGTGCACACAGCCTATTCCCGCCTGCCTGGCAAGCCCAAGGTCTATGTTCAGGACA TCCTGCGGCAGCAGCTGGCCAGCGAGGTGCTCCGTGTGCTCCACAAGGAGCCAGGCCACC TCTATGTTTGCGGGGATGTGCGCATGGCCCGGGACGTGGCCCACACCCTGAAGCAGCTGG TGGCTGCCAAGCTGAAATTGAATGAGGAGCAGGTCGAGGACTATTTCTTTCAGCTCAAGA GCCAGAAGCGCTATCACGAAGATATCTTTGGTGCTGTATTTCCTTACGAGGCGAAGAAGG ACAGGGTGGCGGTGCAGCCC<br>[SEQ ID NO:41] |
| SERPINB4 | 210413_x_at:<br>GTCGATTTACACTTACCTCGGTTCAAAATGGAAGAGAGCTATGACCTCAAGGACACGTTG AGAACCATGGGAATGGTGAATATCTTCAATGGGGATGCAGACCTCTCAGGCATGACCTGG AGCCACGGTCTCTCAGTATCTAAAGTCCTACACAAGGCCTTTGTGGAGGTCACTGAGGAG GGAGTGGAAGCTGCAGCTGCCACCGCTGTAGTAGTAGTCGAATTATCATCTCCTTCAACT AATGAAGAGTTCTGTTGTAATCACCCTTTCCTATTCTTCATAAGGCAAAATAAGACCAAC AGCATCCTCTTCTATGGCAGATTCTCATCCCCATAGATGCAATTAGTCTGTCACTCCATT TAG<br>[SEQ ID NO:42]<br><br>211906_s_at: |

(continued)

| Gene | Example Probes |
|---|---|
| | GATACGACACTGGTTCTTGTGAACGCAATCTATTTCAAAGGGCAGTGGGAGAATAAATTT<br>AAAAAAGAAAACACTAAAGAGGAAAAATTTTGGCCAAACAAGGATGTACAGGCCAAGGTC<br>CTGGAAATACCATACAAAGGCAAAGATCTAAGCATGATTGTGCTGCTGCCAAATGAAATC<br>GATGGTCTGCAGAAGCTTGAAGAGAAACTCACTGCTGAGAAATTGATGGAATGGACAAGT<br>TTGCAGAATATGAGAGAGACATGTGTCGATTTACACTTACCTCGGTTCAAAATGGAAGAG<br>AGCTATGACCTCAAGGACACGTTGAGAACCATGGGAATGGTGAATATCTTCAATGGGGAT<br>GCAGACCTCTCAGGCATGACCTGGAGCCACGGTCTCTCAGTATCTAAAGTCCTACACAAG<br>GCCTTTGTGGAGGTCACTGAGGAGGGAGTGGAAGCTGCAGCTGCCACCGCTGTAGTAGTA<br>GTCGAATTATCATCTCCTTCAACTAATG<br>[SEQ ID NO:43] |
| CST4 | Cystatin-4<br><br>206994_at:<br>GCGAGTACAACAAGGCCACCGAAGATGAGTACTACAGACGCCCGCTGCAGGTGCTGCGAG<br>CCAGGGAGCAGACCTTTGGGGGGGTGAATTACTTCTTCGACGTAGAGGTGGGCCGCACCA<br>TATGTACCAAGTCCCAGCCCAACTTGGACACCTGTGCCTTCCATGAACAGCCAGAACTGC<br>AGAAGAAACAGTTATGCTCTTTCGAGATCTACGAAGTTCCCTGGGAGGACAGAATGTCCC<br>TGGTGAATTCCAGGTGTCAAGAAGCCTAGGGGTCTGTGCCAGGCCAGTCACACCGACCAC<br>CACCCACTCCCACCCCCTGTAGTGCTCCCACCCCTGGACTGGTGGCCCCCACCCTGCGGG<br>AGGCCTCCCCATGTGCCTGTGCCAAGAGACAGACAGAGAAGGCTGCAGGAGTCCTTTGTT<br>GCTCAGCAGGGCGCTCTGCCCTCCCTCCTTCCTTCTTGCTTCTAATAGACCTGGTACATG<br>GTACACACACCCC<br>[SEQ ID NO:44] |
| PRB4 | proline-rich protein BstNI subfamily 4 precursor<br><br>216881_x_at: |

(continued)

| Gene | Example Probes |
|---|---|
| | CCACCTCCTCCAGGAAAGCCAGAAAGACCACCCCCACAAGGAGGTAACCAGTCCCAAGGT<br>CCCCCACCTCATCCAGGAAAGCCAGAAGGACCACCCCCACAGGAAGGAAACAAGTCCCGA<br>AGTGCCCGATCTCCTCCAGGAAAGCCACAAGGACCACCCCAACAAGAAGGCAACAAGCCT<br>CAAGGTCCCCCACCTCCTGGAAAGCCACAAGGCCCACCCCCAGCAGGAGGCAATCCCCAG<br>CAGCCTCAGGCACCTCCTGCTGGAAAGCCCCAGGGGCCACCTCCACCTCCTCAAGGGGGC<br>AGGCCACCCAGACCTGCCCAGGGACAACAGCCTCCCCAGTAATCTAGGATTCAATGACAG<br>GAAGTGAATAAGAAGATATCAGTGAATTCAAATAATTCAATTGCTACAAATGCCGTGACA<br>TTGGAACAAGGTCATCATAGCTCTAAC<br>[SEQ ID NO:45] |
| TPSD1** | 207741_x_at:<br>TGACGCAAAATACCACCTTGGCGCCTACACGGGAGACGACGTCCGCATCATCCGTGACGA<br>CATGCTGTGTGCCGGGAACAGCCAGAGGGACTCCTGCAAGGGCGACTCTGGAGGGCCCCT<br>GGTGTGCAAGGTGAATGGCACCTGGCTACAGGCGGGCGTGGTCAGCTGGGACGAGGGCTG<br>TGCCCAGCCCAACCGGCCTGGCATCTACACCCGTGTCACCTACTACTTGGACTGGATCCA<br>CCACTATGTCCCCAAAAAGCCGTGAGTCAGGCCTGGGTGTGCCACCTGGGTCACTGGAGG<br>ACCA<br>[SEQ ID NO:46]<br><br>Affy 216474_x_at<br>CCGCCATTTCCTCTGAAGCAGGTGAAGGTCCCCATAATGGAAAACCACATTTGTGACGCA<br>AAATACCACCTTGGCGCCTACACGGGAGACGACGTCCGCATCGTCCGTGACGACATGCTG<br>TGTGCCGGGAACACCCGGAGGGACTCATGCCAGGGCGACTCCGGAGGGCCCCTGGTGTGC<br>AAGGTGAATGGCACCTGGCTGCAGGCGGGCGTGGTCAGCTGGGGCGAGGGCTGTGCCCAG<br>CCCAACCGGCCTGGCATCTACACCCGTGTCACCTACTACTTGGACTGGATCCACCACTAT<br>GTCCCCAAAAAGCCGTGAGTCAGGCCTGGGTTGGCCACCTGGGTCACTGGAGGACCAA<br>[SEQ ID NO:47]<br><br>205683_x_at: |

(continued)

| Gene | Example Probes |
|---|---|
| | TGACGCAAAATACCACCTTGGCGCCTACACGGGAGACGACGTCCGCATCGTCCGTGACGA<br>CATGCTGTGTGCCGGGAACACCCGGAGGGACTCATGCCAGGGCGACTCCGGAGGGCCCCT<br>GGTGTGCAAGGTGAATGGCACCTGGCTGCAGGCGGGCGTGGTCAGCTGGGGCGAGGGCTG<br>TGCCCAGCCCAACCGGCCTGGCATCTACACCCGTGTCACCTACTACTTGGACTGGATCCA<br>CCACTATGTCCCCAAAAAGCCGTGAGTCAGGCCTGGGTTGGCCACCTGGGTCACTGGAGG<br>ACCAACCCCTGCTGTCCAAAACACCACTGCTTCCTACCCAGGTGGCGACTGCCCCCCACA<br>CCTTCCCTGCCCCGTCCTGAGTGCCCCTTCCTGTCCTAAGCCCCCTGCTCTCTTCTGAGC<br>CCCTTCCCCTGTCCTGAGGACCCTTCCCTATCCTGAGCCCCCTTCCCTGTCCTAAGCCTG<br>ACGCCTGCACCGGGCCCTCCAGCCCTCCCCTGCCCAGATAGCTGGTGGTGGGCGCTAATC<br>CT<br>[SEQ ID NO:48]<br><br>207134_x at:<br>TGACGCAAAATACCACCTTGGCGCCTACACGGGAGACGACGTCCGCATCGTCCGTGACGA<br>CATGCTGTGTGCCGGGAACACCCGGAGGGACTCATGCCAGGGCGACTCCGGAGGGCCCCT<br>GGTGTGCAAGGTGAATGGCACCTGGCTGCAGGCGGGCGTGGTCAGCTGGGGCGAGGGCTG<br>TGCCCAGCCCAACCGGCCTGGCATCTACACCCGTGTCACCTACTACTTGGACTGGATCCA<br>CCACTATGTCCCCAAAAAGCCGTGAGTCAGGCCTGGGTTGGCCACCTGGGTCACTGGAGG<br>ACCAACCCCTGCTGTCCAAAACACCACTGCTTCCTACCCAGGTGGCGACTGCCCCCCACA<br>CCTTCCCTGCCCCGTCCTGAGTGCCCCTTCCTGTCCTAAGCCCCCTGCTCTCTTCTGAGC<br>CCCTTCCCCTGTCCTGAGGACCCTTCCCCATCCTGAGCCCCCTTCCCTGTCCTAAGCCTG<br>ACGCCTGCACCGGGCCCTCCGGCCCTCCCCTGCCCAGGCAGCTGGTGGTGGGCGCT<br>[SEQ ID NO:49]<br><br>210084_ x_ at: |

(continued)

| Gene | Example Probes |
|---|---|
| | CCGGTCAGCAGGATCATCGTGCACCCACAGTTCTACATCATCCAGACTGGAGCGGATATC<br>GCCCTGCTGGAGCTGGAGGAGCCCGTGAACATCTCCAGCCGCGTCCACACGGTCATGCTG<br>CCCCCTGCCTCGGAGACCTTCCCCCCGGGGATGCCGTGCTGGGTCACTGGCTGGGGCGAT<br>GTGGACAATGATGAGCCCCTCCCACCGCCATTTCCCCTGAAGCAGGTGAAGGTCCCCATA<br>ATGGAAAACCACATTTGTGACGCAAAATACCACCTTGGCGCCTACACGGGAGACGACGTC<br>CGCATCATCCGTGACGACATGCTGTGTGCCGGGAACACCCGGAGGGACTCATGCCAGGGC<br>GACTCTGGAGGGCCCCTGGTGTGCAAGGTGAATGGCACCTGGCTACAGGCGGGCGTGGTC<br>AGCTGGGACGAGGGCTGTGCCCAGCCCAACCGGCCTGGCATCTACACCCGTGTCACCTAC<br>TACTTGGACTGGATCCACCACTATGTCCCCAAAAAGCCGTGAGTCAGGCCTGGGGTGT<br>[SEQ ID NO:50]<br><br>217023_x_at:<br>CCGGTCAGCAGGATCATCGTGCACCCACAGTTCTACACCGCCCAGATCGGAGCGGACATC<br>GCCCTGCTGGAGCTGGAGGAGCCGGTGAACGTCTCCAGCCACGTCCACACGGTCACCCTG<br>CCCCCTGCCTCAGAGACCTTCCCCCCGGGGATGCCGTGCTGGGTCACTGGCTGGGGCGAT<br>GTGGACAATGATGAGCGCCTCCCACCGCCATTTCCTCTGAAGCAGGTGAAGGTCCCCATA<br>ATGGAAAACCACATTTGTGACGCAAAATACCACCTTGGCGCCTACACGGGAGACGACGTC<br>CGCATCGTCCGTGACGACATGCTGTGTGCCGGGAACACCCGGAGGGACTCATGCCAGGTG<br>GCGACT<br>[SEQ ID NO:51]<br>215382_ x_ at:<br>CCGGTCAGCAGGATCATCGTGCACCCACAGTTCTACATCATCCAGACTGGAGCGGATATC<br>GCCCTGCTGGAGCTGGAGGAGCCCGTGAACATCTCCAGCCGCGTCCACACGGTCATGCTG<br>CCCCCTGCCTCGGAGACCTTCCCCCCGGGNNTGCCGTGCTGGGTCACTGGCTGGGGCGAT<br>GTGGACAATGATGAGCCCCTCCCACCGCCATTTCCCCTGAAGCAGGTGAAGGTCCCCATA<br>ATGGAAAACCACATTTGTGACGCAAAATACCACCTTGGCGCCTACACGGGAGACGACGTC<br>CGCATCATCCGTGACGACATGCTGTGTGCCGGGAACACCCGGAGNGNNTCATGCCAGGGC<br>GACTCNGGAGGGCCCCTGGTGTGCAAGGTGAATGGCACCTGGCTNCAGGCGGGCGTGGTC<br>AGCTGGGNCGAGGGCTGTGCCCAGCCCAACCGGCCTGGCATCTACACCCGTGTCACCTAC<br>TACTTGGACTGGATCC<br>[SEQ ID NO:52] |
| TPSG1** | 216485_s_at: |

(continued)

| Gene | Example Probes |
|---|---|
| | GTCGTCACGGACGATGCGGACGTCGTCTCCCGTGTAGGCGCCAAGGTGGTATTTTGCGTC<br>ACAAATGTGGTTTTCCATTATGGGGACCTTCACCTGCTTCAGAGGAAATGGCGGTGGGAG<br>GCGCTCATCATTGTCCACATCGCCCCAGCCAGTGACCCAGCACGGCATCCCCGGGGGGAA<br>GGTCTCTGAGGCAGGGGGCAGGGTGACCGTGTGGACGTGGCTGGAGACGTTCACCGGCTC<br>CTCCAGCTCCAGCAGGGCGATGTCCGCTCCGATCTGGGCGGTGTAGAACTGTGGGTGCAC<br>GATGATCCTGCTGACCGGCAGCAGCTGGTCCTGGTAGTAGAGGTGCTGCTCCCGCAGTTG<br>CACCGGTCCCACGCAGTGCGCTGCGGTCAGCACCCACTGGGGGTGGAT<br>[SEQ ID NO:53]<br><br>220339_s_at:<br>GGTGAAAGTCTCCGTGGTGGACACAGAGACCTGCCGCCGGGACTATCCCGGCCCCGGGGG<br>CAGCATCCTTCAGCCCGACATGCTGTGTGCCCGGGGCCCCGGGGATGCCTGCCAGGACGA<br>CTCCGGGGGGCCTCTGGTCTGCCAGGTGAACGGTGCCTGGGTGCAGGCTGGCATTGTGAG<br>CTGGGGTGAGGGCTGCGGCCGCCCCAACAGGCCGGGAGTCTACACTCGTGTCCCTGCCTA<br>CGTGAACTGGATCCGCCGCCACATCACAGCATCAGGGGGCTCAGAGTCTGGGTACCCCAG<br>GCTCCCCCTCCTGGCTGGCTTATTCCTCCCCGGCCTCTTCCTTCTGCTAGTCTCCTGTGT<br>CCTGCTGGCCAAGTGCCTGCTGCACCCATCTGCGGATGGTACTCCCTTCCCCGCCCCTGA<br>CTGATGGCAGGAATCCAAGTGCATTTCTTAAATAAGTTACTATTTATTCCGCTCCGCCCC<br>CTCCCTCTCCCTTGAGAAGCTGAGTCTTCTGCATCAGATT<br>[SEQ ID NO:54]<br>213536_s_at:<br>TGCCACAAGGTCGCTGCTTATGAGGGCGCAAACTTCTTGGCTTGTGCTCGGACCCTTTTC<br>TCGTACTCCACTCTGTTTTGGCAGTAAATCGTGTAGGCCTCTGCTTGAGCTGGGTCTTGG<br>ATATTTGGTTCATTTAGAAGTTCCTGTATTCCTAATAGGATCTGTTTGATTGTGATGGCT<br>GGCCTCCAGTCCTTGTCCTCCTCTAAGATGGACAGGCACACTGTCCCCGAAGGGTACACA<br>TTCGGGTGAAATAATGGTGGTTCGAATTTACATTTTGGTGGCGAAGATGGATAATCATCT<br>TTGAAAAGCATCCGTAGTTTAAACAAGCCTCCTTCCCACGGAGTCCCTTTCTTTCCTGGA<br>ATGGCGCACTCCCAGTTCATGAGGTTCATCGTGCCATCGGGATTTTTTGTTGGGACAGCC<br>ACGAAACCAAATGGGTGGTCTTTCCTCCATGCTTTCCTCTCCTGGGCGAGTCTGCTGAGG<br>GCGATCCCCGACATGTTCAAAGTCCCTC<br>[SEQ ID NO:55]<br>214067_at: |

(continued)

| Gene | Example Probes |
|---|---|
| | AGAGACTTTCAGGGCATACGTGGGGGCCTTGGCCTTCCTCACTCGCTCGATGGCCTCAGT<br>GTGCTCCTCAAGGCTGGTGCCAAACACCTGCTGGAGATAGCTGAGCAGGGCCTCCTCGTC<br>GTCCACCTGGTCAGGGCCCATGGTACCCGCGCGGTAAAGCACCGTGTACAGGGCCTCCTC<br>GTAGAGCATCTCCACCTCCTCTGGGGCCAGGGCTCTCAGGCCGAGGCTGGGATCCACAGG<br>CTCCGGGGGTGCTGGCGAGCCACTGCGCAGGGGGACCTCGAGGCACGGCAAGCCCTGTCT<br>GCCTTCCCCCTTCTTCAGCATGAGGCGCATGTGGGCAAAGAACTCCACGCCATCCCCGGG<br>TTTCCAGGCCCCCGTGGCAGGCTCCTGCGGGTCGGCGCTGGCACTCCCTGGGTCCTGCTC<br>AGTCCTGCGGCGGAAGGACGGGCACACCTGCACCTGCCTGAGCACGCTGCTCTTAATGTC<br>CAGCAAGGTCGACATGGCGGGTGACCGTGG<br>[SEQ ID NO:56] |
| MFSD2 | Major facilitator superfamily domain-containing protein 2<br><br>225316_at:<br>TGCTGCTCTTCAAAATGTACCCCATTGATGAGGAGAGGCGGCGGCAGAATAAGAAGGCCC<br>TGCAGGCACTGAGGGACGAGGCCAGCAGCTCTGGCTGCTCAGAAACAGACTCCACAGAGC<br>TGGCTAGCATCCTCTAGGGCCCGCCACGTTGCCCGAAGCCACCATGCAGAAGGCCACAGA<br>AGGGATCAGGACCTGTCTGCCGGCTTGCTGAGCAGCTGGACTGCAGGTGCTAGGAAGGGA<br>ACTGAAGACTCAAGGAGGTGGCCCAGGACACTTGCTGTGCTCACTGTGGGGCCGGCTGCT<br>CTGTGGCCTCCTGCCTCCCCTCTGCCTGCCTGTGGGGCCAAGCCCTGGGGCTGCCACTGT<br>GAATATGCCAAGGACTGATCGGGCCTAGCCCGGAACACTAATGTAGA<br>[SEQ ID NO:57] |
| CPA3** | Carboxypeptidase A3<br><br>205624_at |

(continued)

| Gene | Example Probes |
|---|---|
| | TATGAAACCCGCTACATCTATGGCCCAATAGAATCAACAATTTACCCGATATCAGGTTCT<br>TCTTTAGACTGGGCTTATGACCTGGGCATCAAACACACATTTGCCTTTGAGCTCCGAGAT<br>AAAGGCAAATTTGGTTTTCTCCTTCCAGAATCCCGGATAAAGCCAACGTGCAGAGAGACC<br>ATGCTAGCTGTCAAATTTATTGCCAAGTATATCCTCAAGCATACTTCCTAAAGAACTGCC<br>CTCTGTTTGGAATAAGCCAATTAATCCTTTTTTGTGCCTTTCATCAGAAAGTCAATCTTC<br>AGTTATCCCCAAATGCAGCTTCTATTTCACCTGAATCCTTCTCTTGCTCATTTAAGTCCC<br>ATGTTACTGCTGTTTGCTTTTACTTACTTTCAGTAGCACCATAACGAAGTAGCTTTAAGT<br>GAAACCTTTTAACTACCTTTCTTTGCTCCAAGTGAAGTTTGGACCCAGCAGAAAGCATTA<br>TTTTGAAAGGTGATATACAGTGGGGCACAGAAAACAAATGAAAACCCTCAGTTTCTCACA<br>GATTTTCACCATGTGGCTTCATCAA<br>[SEQ ID NO:58] |
| GPR105*** | G-protein coupled receptor 105<br><br>206637_at:<br>TGAGCCTGGGGTTCTGGTGTTAGAATATTTTTAAGTAGGCTTTACTGAGAGAAACTAAAT<br>ATTGGCATACGTTATCAGCAACTTCCCCTGTTCAATAGTATGGGAAAAATAAGATGACTG<br>GGAAAAAGACACACCCACACCGTAGAACATATATTAATCTACTGGCGAATGGGAAAGGAG<br>ACCATTTTCTTAGAAAGCAAATAAACTTGATTTTTTTAAATCTAAAATTTACATTAATGA<br>GTGCAAAATAACACATAAAATGAAAATTCACACATCACATTTTTCTGGAAAACAGACGGA<br>TTTTACTTCTGGAGACATGGCATACGGTTACTGACTTATGAGCTACCAAAACTAAATTCT<br>TTCTCTGCTATTAACTGGCTAGAAGACATTCATCTATTTTTCAAATGTTCTTTCAAAACA<br>TTTTTATAAGTAATGTTTGTATCTATTTCATGCTTTACT<br>[SEQ ID NO:59] |
| CDH26 | Cadherin-like protein 26 [Precursor]<br><br>232306_at:<br>GGGAATCACTATTCAGGGATTTTTCCCCTTTGCTCTTCTTTTCCCTCCTTAAAAGAAAAA |

(continued)

| Gene | Example Probes |
| --- | --- |
| | TTACCTTCTAGTCCTAGGATGAGGACACACTATTAGTTTGAATTAAATGCTTTGATATTC<br>TCAGATCAGCCATCTTGAACCAAAGCAAAACCACAAGTTACACTTTCTTAAAATTTGATT<br>TGTCATATTTTCTAGAGAAACTTGAATTTAATTGTGTTATTCTTAGCTTCCACTGGCAGC<br>CTAGCTTTGAGGGTAAATGAAAATATAACCCATAGATTACCCAGCCACTTGGGAACAGCA<br>GGTAATACTGAAGAAAAATAAAAATAGATTTTGAAAACGTTANNNANANNNNTATGATTA<br>TGATTCTGTTCCATTTAAGGGAAAACTTAGGTAAATAGAGAAATTTTTTCTATAACATTG<br>TGTAGTCAGT<br>[SEQ ID NO:60] |
| GSN | Gelsolin [Precursor]<br><br>200696_s_at:<br>TGCTTCTGGACACCTGGGACCAGGTCTTTGTCTGGGTTGGAAAGGATTCTCAAGAAGAAG<br>AAAAGACAGAAGCCTTGACTTCTGCTAAGCGGTACATCGAGACGGACCCAGCCAATCGGG<br>ATCGGCGGACGCCCATCACCGTGGTGAAGCAAGGCTTTGAGCCTCCCTCCTTTGTGGGCT<br>GGTTCCTTGGCTGGGATGATGATTACTGGTCTGTGGACCCCTTGGACAGGGCCATGGCTG<br>AGCTGGCTGCCTGAGGAGGGGCAGGGCCCACCCATGTCACCGGTCAGTGCCTTTTGGAAC<br>TGTCCTTCCCTCAAAGAGGCCTTAGAGCGAGCAGAGCAGCTCTGCTATGAGTGTGTGT<br>[SEQ ID NO:61] |
| C2ORF32 | 226751_at:<br>ACTTTTGACCACTTGTGACTGGAGTTCAGTGGCCCTGGCAGGCTTGTCCTGCTCTTGACC<br>ATTCCACTGACTAACTTTGGTGTTTNGTTTCCAAGTTAAGTGATTCCTCCTTTTTTTNGT<br>TCAATGTTAAATTTAAAAATAACAATGTGTATGGGTCCTCCCATGTGTAATATGGTAACA<br>TGTAACTTGCAGTGTTTGCCAGCTTTCAAAGCAGGCTTTGTGAAAATGTAATACAAACAG<br>CAGTGAATGGGACTCAAATGTTGTGCTTCCTATAAACAGCTCCGCTCTTTCAGGGAAGGA<br>TGGTAACAAACTAGAAGGACAAATATGTACGTATTTATAACGTATTAAAACTCTTTTAAG<br>TAGCTTAAGGTATTGTGCAATGGCCTAGCCTAGTAGAAATGGGGGAAAAGCATTGCTGTG<br>GACCATTGTTAAAGTGACAGGAGTTGTAGGGTTACCCCTTTGACAAGCTTCCATAGTCTT<br>CAGACACGCACATTGATGGCATCCCT<br>[SEQ ID NO:62] |

(continued)

| Gene | Example Probes |
|---|---|
| TRACH2000196 (TMEM71) | 238429_at:<br>CTAACTAATACCAACCTGACAACTTGAATAACAATAAATGCAATTTGTACATAAAATATN<br>ATGCTGCAAAAGTTNGTCATTCACCTCAGTGGAGTGACTTGATATTAGGTGGTNACCGTA<br>GATGATGGTTNATATGANAANTGGACAGGAAAGAAGCANTTTCTGAAAGTTATANTCTTT<br>TGAACCACGTTCTAAACCAAGTNTTTNATCTTCTTGGGGCTCGTAATTACCTTTCACTTT<br>AATGTCACTTAAAGATATAACACAGAAAAATGCCTTGAGGGCAAAATATAGGCAAAACAC<br>CAATGCGCTTTCAAATGCATGAAAATGGTGCAGTTGTACCCTTGAGCCTTGACTCAAGGG<br>CTGTAGATGTTCCCTTTCCACCCCCCACACTTGGTGCGTGTTCACAAAGCAAATATGGCC<br>TGTAATTCAAATTTGTTCTATGTGATACTCTCTGAGTAAAAACTCATACATGCAGAAAAT<br>TGTCTTTGCTCGAAAT<br>[SEQ ID NO:63] |
| DNAJC12 | DnaJ homolog subfamily C member 12<br><br>218976_at:<br>CCCAAGCCCCTAGAGAAGTCAGTCTCCCCGCAAAATTCAGATTCTTCAGGTTTTGCAGAT<br>GTGAATGGTTGGCACCTTCGTTTCCGCTGGTCCAAGGATGCTCCCTCAGAACTCCTGAGG<br>AAGTTCAGAAACTATGAAATATGAAATATCTCTGCTTCAAAAAATGAGGAAGAGCAAGAC<br>TGTCCCCTATGCTGCCAACATGCAGTCTTTGTTTATGTCTTAAAAATGTCATGTTTATGT<br>CATGTCTGTGAATTGCTGAGTACTAATTGATTCCTCCATCCTTGAATCAGTTCTCATAAT<br>GCTTTTTAAATAAGAAAAATTCAGAAGATGAATTTCTTCCAATATTTGAATAAATTAAAG<br>CTCTTAGATACAGAGTAGATTGTATTATATGCTTTTTCCTATTAATACTACTTATAGAAA<br>TCCATTAAAAAGCAATCTCTGTACAGTGTATTTAAATATTTCATTGACATACTGTGATCT<br>CTATTAGTGATGGATGTACAAAAAATGTTTTCTTACCCTTGACTTACAATGAAATGTGAA<br>ATTACTTGTCTGAACCCCGT<br>[SEQ ID NO:64] |
| RGS13** | Regulator of G-protein signaling 13<br><br>210258_at: |

(continued)

| Gene | Example Probes |
|---|---|
| | ACAGCAAGCCTATGTAGTTCAATTAATATATAAGGAAAAGGAAGGTCTTTCTTCATGATA<br>CAAGCATTATAAAGTTTTTACTGTAGTAGTCAATTAATGGATATTTCCTTGTTAATAAAA<br>TTTTGTGTCATAATTTACAAATTAGTTCTTTAAAAATTGTTGTTATATGAATTGTGTTTC<br>TAGCATGAATGTTCTATAGAGTACTCTAAATAACTTGAATTTATAGACAAATGCTACTCA<br>CAGTACAATCAATTGTATTATACCATGAGAAAATCAAAAAGGTGTTCTTCAGAGACATTT<br>TATCTATAAAATTTTCCTACTATTATGTTCATTAACAAACTTCTTTATCACATGTATCTT<br>CTACGTGTAAAACATTTCTGATGATTTTTTAACAAAAAATATATGAATTTCTTCATTTGC<br>TCTTGCATCTACATTGCTATAANGGATATAAAATGTGGTTTCTATATTTTGAGATGTTTT<br>TTCCTTACAATGTGAACTCATCGTGATCTTGG<br>[SEQ ID NO:65] |
| SLC18A2** | Solute carrier family 18 member 2<br><br>205857_at:<br>CTGCTACTTTGGAAGATGGCTCTGGAGGAAACTCTCATATGGCTAAAAAGGCAGGCTAGT<br>TTCTTACTTCTACAGGGGTAGAGCCTTAAAAAAGAACGTGCTACAAATTGGTTNTCTTNN<br>AGGGTTNCNGGTTCTCCCTGCCCCCAATNCCNATATACTTTANTGCNNTTTTATTTTTGC<br>CTTTACGGNCTCTGTGTCTTTCTGCAAGAAGGCCTGGCAAAGGTATGCCTGCTGTTGGTC<br>CCNTCGGGATAAGATAAAATATAAATAAAACCTTCAGAACTGTTTTGGAGCAAAAGATAG<br>CTTGTACTTGGGGAAAAAAATTCTAAGTTCTTTTATATGACTAATATTCTTGGTTAGCAA<br>GACTGGAAAGAGGTGTTTTTTTAAAATGTACATACCAGAACAAAGAACATACAGCTCTCT<br>GAACATTTATTTTTTGAACAGAGGTGGTTTTTATGTTTGGACCTGGTAATACAGATACAA<br>AAACTTTAATGAGGTAGCAATGAATATTCAACTGTTTGACTGCTAAGTGTATCTGTCCAT<br>ATTTTAGCAAG<br>[SEQ ID NO:66] |
| SERPINB10 | Serpin peptidase inhibitor clade B (Ovalbumin) member 10<br><br>214539_at: |

(continued)

| Gene | Example Probes |
|---|---|
| | TACTACAAAAGCCGTGACCTCAGCCTGCTTATACTACTGCCAGAAGACATTAATGGGCTG<br>GAACAGCTGGAAAAGGCCATCACCTATGAGAAGCTGAATGAGTGGACCAGTGCAGACATG<br>ATGGAGTTGTATGAAGTGCAGCTACACCTTCCCAAGTTCAAGCTGGAAGACAGTTATGAT<br>CTCAAGTCAACCCTGAGCAGTATGGGGATGAGTGATGCCTTCAGCCAAAGCAAAGCTGAT<br>TTCTCAGGAATGTCTTCAGCAAGAAACCTATTTTTGTCCAATGTTTTCCATAAGGCTTTT<br>GTGGAAATAAATGAACAAGGTACTGAAGCTGCAGCTGGCAGTGGGAGTGAGATAGATATA<br>CGAANTAGAGTCCCATCCATTGAATTCAATGCAAATCACCCATTCCTCTTCTTCATCAGG<br>CACAATAANAACCAACACCATTCTTTTTATGGAAGATTATGCTCCCCCTAATC<br>[SEQ ID NO:67] |
| SH3RF2 | SH3 domain-containing RING finger protein 2<br><br>243582_at:<br>GATTCTGTGGTAGACTCAGTGCTTTCAGAGTCCAGAGCTTGACTTGGGTTAGTGGCCTTA<br>ATGAAGTGCTAAATTTGCTCTTTACCGCGAGACTGATCAGAAGAAGCAAAAGGGGAAAGG<br>GGGCTAGAGGTCCACTCGCACCTTTTACATCAGACAAGAGGAGGACTGTGCCAGAAATCT<br>GTGCATGAAACACCATCTGCTCTTCATGCAGGGAGGGGTCAACCGTGTGAACGTGCAGAG<br>ATTACTCGAGCCTTCTTTGCCAAAAATATGCATTCTTCCCAGCTGTA<br>[SEQ ID NO:68] |
| FCER1B** | FcepsilonRIbeta<br><br>207496_at:<br>TAATCACATCACTTCCATGGCATGGATGTTCACATACAGACTCTTAACCCTGGTTTACCA<br>GGACCTCTAGGAGTGGATCCAATCTATATCTTTACAGTTGTATAGTATATGATATCTCTT<br>TTATTTCACTCAATTTATATTTTCATCATTGACTACATATTTCTTATACACAACACACAA<br>TTTATGAATTTTTTCTCAAGATCATTCTGAGAGTTGCCCCACCCTACCTGCCTTTTATAG<br>TACGCCCACCTCAGGCAGACACAGAGCACAATGCTGGGGTTCTCTTCACACTATCACTGC<br>CCCAAATTGTCTTTCTAAATTTCAACTTCAATGTCATCTTCTCCATGAAGACCACTGAAT<br>GAACACCTTTTCATCCAGCCTTAATTTCTTGCTCCATAACTACTCTATCCCACGATGCAG<br>TATTGTATCATTAATTATTAGTGTGCTTGTGACCTCCTTATGTATTCTCAATTACCTGTA<br>TTTGTGCAATAAATTGGAATAATGTAACTTGATTTCTTATCTGTGTTTGTGTTGGCATGC<br>AAGAT |

(continued)

| Gene | Example Probes |
|---|---|
| | [SEQ ID NO:69] |
| RUNX2 | Runt-related transcription factor 2<br><br>232231 at:<br>AAGACACTTCTTCCAAACCTTGAATTTGTTGTTTTTAGAAAACGAATGCATTTAAAAATA<br>TTTTCTATGTGAGAATTTTTTAGATGTGTGTTTACTTCATGTTTACAAATAACTGTTTGC<br>TTTTTAATGCAGTACTTTGAAATATATCAGCCAAAACCATAACTTACAATAATTTCTTAG<br>GTATTCTGAATAAAATTCCATTTCTTTTGGATATGCTTTACCATTCTTAGGTTTCTGTGG<br>AACAAAAATATTTGTAGCATTTTGTGTAAATACAAGCTTTCATTTTTATTTTTTCCAATT<br>GCTATTGCCCAAGAATTGCTTTCCATGCACATATTGTAAAAATTCCGCTTTGTGCCACAG<br>GTCATGATTGTGGATGAGTTTACTCTTAACTTCAAAGGGACTATTTGTATTGTATGTTGC [SEQ ID NO:70] |
| PTGS1 | Prostaglandin-endoperoxide synthase 1<br>238669_at:<br>AGTATTGACAACTGCACATGAAAGTTTTGCAAAGGGAAACAGGCTAAATGCACCAAGAAA<br>GCTTCTTCAGAGTGAAGAATCTTAATGCTTGTAATTTAAACATTTGTTCCTGGAGTTTTG<br>ATTTGGTGGATGTGATGGTTGGTTTTATTTGTCAGTTTGGTTGGGCTATAGCACACAGTT<br>ATTTAATCAAACAGTAATCTAGGTGTGGCTGTGAAGGTATTTTGTAGATGTGATTAACAT<br>CTACAATCAGTTGACTTTAAGTGAAAGAGATTACTTAAATAATTTGGGTGAGCTGCACCT<br>GATTAGTTGAAAGGCCTCAAGAACAAACACTGCAGTTTCCTGGAAAAGAAGAAACTTTGC<br>CTCAAGACTATAGCCATCGACTCCTGCCTGAGTTTCCAGCCTGCTAGTCTGCCCTATGGA<br>TTTGAAGTTTGCCAACCCCAACAATTGTGTGAATTAATTTCTAAAAATAAAGCTATATAC<br>AGCCANNNNNNNNNTATTTGTGGGGGATTTGTTTCAGGATCTCTACAGATACCAA<br>[SEQ ID NO:71] |
| ALOX15*** | Arachidonate 15-lipoxygenase<br><br>207328_at: |

(continued)

| Gene | Example Probes |
|---|---|
| | CCCTAGAGGGGCACCTTTTCATGGTCTCTGCACCCAGTGAACACATTTTACTCTAGAGGC<br>ATCACCTGGGACCTTACTCCTCTTTCCTTCCTTCCTCCTTTCCTATCTTCCTTCCTCTCT<br>CTCTTCCTCTTTCTTCATTCAGATCTATATGGCAAATAGCCACAATTATATAAATCATTT<br>CAAGACTAGAATAGGGGGATATAATACATATTACTCCACACCTTTTATGAATCAAATATG<br>ATTTTTTTGTTGTTGTTAAGACAGAGTCTCACTTTGACACCCAGGCTGGAGTGCAGTGGT<br>GCCATCACCACGGCTCACTGCAGCCTCAGCGTCCTGGGCTCAAATGATCCTCCCACCTCA<br>GCCTCCTGAGTAGCTGGGACTACAGGCTCATGCCATCATGCCCAGCTAATATTTTTTTAT<br>TTTCGTGGAGACGGGGCCTCACTATGTTGCCTAGGCTGGAAATAGGATTTTGAACCCA [SEQ ID NO:72] |
| ** Mast cell-specific genes<br>*** Eosinophil-specific genes | |

Example 8 - Relationship of "IL-13 high" and "IL-13 low" subphenotypes of asthma to clinical features

The asthmatic subjects were further analyzed with respect to additional demographic characteristics and clinical features as those described in Example 7. The results are shown in Table 5 and Figs. 5 and 6. Although subjects with "IL-13 high" asthma subphenotype could not be distinguished from subjects with "IL-13 low" asthma subphenotype based on demographic characteristics, lung function , or bronchodilator responsiveness (delta FEV1 with albuterol) (Table 5, Figures 5A-B), these groups differed significantly with respect to degree of airway hyper-responsiveness (AHR, $PC_{20}$ to methacholine, defined as the minimal concentration of methacholine required to induce a 20% decrease in expiratory airflow, Figure 5C). This difference in AHR was apparent despite inclusion criteria that required all asthmatics to have significant AHR (all asthmatics < 8 mg/ml, all healthy controls > 20 mg/ml).

**Table 5. Subject characteristics by asthma phenotype**

| | | Asthma | | |
|---|---|---|---|---|
| | Healthy Control | IL13 Signature Low | IL-13 Signature High | p-value low vs. high |
| Sample size | 28 | 20 | 22 | - |
| Age | 36±9 | 36±11 | 37±12 | 0.98 |
| Gender, M:F (% F) | 12:16 (56) | 6:14(70) | 11:11 (50) | 0.19 |
| Ethnicity | | | | |
| Caucasian | 20 | 9 | 9 | 0.98 |
| African-American | 0 | 4 | 4 | |
| Hispanic | 3 | 5 | 6 | |
| Asian/Pacific Islander | 5 | 2 | 3 | |
| $FEV_1$, % predicted | 107(13) | 89(10) | 85 (13) | 0.85 |
| $\Delta FEV_1$ with albuterol (% of baseline) | 2.7±3.4% | 9.7±7.4% | 12.5±9.8 | 0.51 |
| Methacholine $PC_{20}$ | 64 (22-64) | 0.93 (0.06-7.3) | 0.27 (0.05-1.9) | <0.001 |
| IgE, IU/ml | 27 (3-287) N=26 | 125 (19-1194) | 244 (32-2627) | 0.031 |
| Blood eosinophils, x $10^9$/L | 0.10±0.07 | 0.23±0.21 | 0.37±0.22 | 0.027 |
| BAL eosinophil % | 0.26±0.29 N=22 | 0.42±0.46 N=16 | 1.9±1.9 N=20 | 0.001 |
| RBM thickness, μm | 4.34±1.11 N=22 | 4.67±0.99 N=19 | 5.91±1.72 N=19 | 0.014 |
| $\Delta FEV_1$ with fluticasone at 4 weeks, L | N/A | 0.03±0.12 N=6 | 0.35±0.2 N=10 | 0.004 |
| $\Delta FEV_1$ with fluticasone 8 weeks, L | N/A | 0.04±0.12 N=6 | 0.25±0.23 N=10 | 0.05 |

For normally distributed data, values are presented as mean±standard deviation and student's t-test performed; for non-normally distributed data, values are presented as median (range) and wilcoxon rank sum test performed. In case of missing data, number of subjects for whom data exist noted. P-values relative to healthy control also depicted in Figs. 5 and 6. $PC_{20}$ denotes the provocative concentration required to cause a 20% decline in $FEV_1$; BAL, bronchoalveolar lavage; RBM, reticular basement membrane.

To determine whether the IL-13 subphenotype of an individual subject was correlated with measures of allergic inflammation, we examined the results of skin prick tests (SPT) to a panel of 12 aeroallergens (Table 6), levels of serum IgE, peripheral blood eosinophil counts, and eosinophil percentages in bronchoalveolar lavage fluid (BAL). The results are shown in Figures 6A-D and 7A-B. Both IL-13 high and low asthma subphenotypes had increased SPT sensitivity to aeroallergens as compared to healthy controls (Fig. 6A), although the IL-13 low asthma subphenotype tended to have fewer positive skin tests than the IL-13 high asthma subphenotype and to be sensitized less frequently to aeroallergens such as dog and house dust mite (Fig. 7A). Subjects with IL-13 high asthma subphenotype had higher serum IgE levels and higher peripheral blood eosinophil counts than subjects with IL-13 low asthma subphenotype, although IL-13 low asthma subphenotype differed from healthy controls with respect to these features of allergic inflammation (Figs. 6B-C). In addition, subjects with IL-13 high asthma subphenotype had increased eosinophil numbers in the lung as assessed by BAL (Fig. 6D), whereas IL-13 low asthmatics did not differ from healthy controls in BAL eosinophil percentage. These data demonstrate enrichment for AHR, IgE levels, and eosinophilic inflammation in subjects with the IL-13 high asthma subphenotype, but SPT sensitivity to aeroallergens was not restricted to this subgroup. Thus, it is likely that alternate non-Th2 mechanisms for sensitization to aeroallergens operate in subjects with the IL-13 low asthma subphenotype.

**Table 6.** Allergen skin prick test panel

| Allergen | | |
|---|---|---|
| D. farinae | Cladosporium herbarum | West Oak mix |
| D. pteronyssius | Cat | Grass mix/Bermuda/Johnson |
| American Cockroach | Dog | Histamine [10mg/ml] (positive control) |
| Alternaria tenuis | Plantain-Sorrel mix | 50% Glycerin (negative control) |
| Aspergillus mix | Short Ragweed | |

To determine whether the subphenotype of IL-13 high asthma is durable or a transient manifestation of Th2-driven inflammation due to recent exposure to allergen, we measured pathological changes in bronchial biopsies from the same subjects. We and others have previously demonstrated that asthma is associated with pathological changes known as airway remodeling and which reflect either longstanding inflammation or the effects of injury and repair over time [28, 29]. Two specific remodeling outcomes in asthma are airway fibrosis, manifest as thickening of the sub-epithelial reticular basement membrane (RBM) [30, 31] and increased mucin stores in the airway epithelium [32]. We found that RBM thickness was greater in subjects with IL-13 high asthma subphenotype than in IL-13 low asthma subphenotype or healthy controls and that RBM thickness was normal in the IL-13 low subphenotype of asthma (Fig. 6E). In addition, although we observed a trend toward increased epithelial mucin stores in both subphenotypes of subjects with asthma, this increase was significant only in subjects with IL-13 high asthma subphenotype (Fig. 8A). Although these differences in total mucin stores were modest, qPCR revealed a striking difference in the expression levels of the major gel-forming mucins in airway epithelial cells in IL-13 high asthma subphenotype as compared to both IL-13 low asthma subphenotype and healthy controls (Figs. 8B-D). Specifically, IL-13 high asthma subphenotype was distinguished from IL-13 low asthma subphenotype and healthy controls by induction of MUC5AC and MUC2 expression and repression of MUC5B expression. This alteration in the expression of specific mucin genes in IL-13 high asthma subphenotype is most evident in the ratio of MUC5AC to MUC5B expression (Fig. 6F). Without being bound by theory, we speculate that concomitant induction and repression of specific gel-forming mucins may explain the relatively modest increase in epithelial mucin stores in IL-13 high asthma subphenotype compared to IL-13 low asthma subphenotype and healthy controls. Taken together, these findings indicate that IL-13 high asthma subphenotype is associated with remodeling changes in the airway that identify this subphenotype as durable over time. These results also demonstrate the importance of the IL-13 pathway to airway remodeling in human subjects.

Alveolar macrophages may modulate allergic airway inflammation in asthma as a source of IL-13 [54] and leukotrienes or eicosanoid lipids [55, 56] or through "alternative activation" under the influence of IL-13 [57]. To determine whether alveolar macrophages from subjects with "IL-13 high" asthma manifest any of these findings, we measured the expression of relevant genes using qPCR in 14 subjects with asthma and 15 healthy controls (Table 7). We found no evidence for induction of Th2 cytokines or of alternative activation markers in asthma generally or in the "IL-13 high" subgroup specifically. Levels of expression of IL-13 were below the limit of detection (cycle threshold >40) in 26 of the 29 subjects, and IL-4 was below the limit of detection in 20 of the 29 subjects (no differences between the three groups for either cytokine, all p>0.35). All other genes were within the limit of detection across samples. In these analyses we found increased expression of 15-lipoxygenase in "IL-13 high" asthma (Figure 10, Table 8), consistent with prior findings of increased 15-lipoxygenase products in the airways in severe eosinophilic asthma [56]. We also found an increase in expression of TNF$\alpha$ that was limited to the "IL-13 high" subgroup (Figure 10, Table 8).

Only a subset of asthmatics manifests improvement in lung function when treated with inhaled corticosteroids (ICS) [33]. To identify gene expression markers of corticosteroid responsiveness, we measured $FEV_1$ in a subset of our subjects with asthma during an 8-week randomized controlled trial of inhaled fluticasone or placebo as previously reported[8]. When we re-analyzed that data while stratifying subjects by IL-13 subphenotype, we found that improvements in $FEV_1$ were limited to those with the IL-13 high subphenotype. Specifically, the subjects with the IL-13 high asthma subphenotype who were treated with inhaled fluticasone had significant improvements in $FEV_1$ at both 4 and 8 weeks as compared to subjects treated with placebo, whereas subjects with IL-13 low asthma subphenotype did not (Fig. 9A). These improvements in $FEV_1$ in the IL-13 high group were lost after a one week run out period off drug. There was no significant change in $FEV_1$ in response to placebo at any timepoint in either group (data not shown, N=5 "IL-13 high," N=6 "IL-13 low"). As described previously [8], we performed a second bronchoscopy one week after the initiation of treatment and analyzed gene expression in bronchial epithelium by microarray as at baseline. In re-analyses of these data, while stratifying subjects by IL-13 subphenotype, subjects with IL-13 high asthma at baseline continued to exhibit a strong IL-13 subphenotype after one week of placebo treatment demonstrating the short-term stability of this subphenotype in the absence of therapy. However, after one week of fluticasone treatment, subjects with IL-13 high asthma clustered with subjects who were IL-13 low at baseline, regardless of treatment (Fig. 9B). Thus, the phenotypic classification of asthma based on the IL-13 signature described herein predicts response to ICS. These data suggest that the global benefit of ICS treatment for asthma is accounted for by the IL-13 high subphenotype.

Our results provide new insights into molecular mechanisms that underlie clinical heterogeneity in asthma. Basic research previously established IL-13 and related Th2 cytokines as central regulators of allergic inflammation and many of the pathophysiologic changes associated with asthma [35, 36]. Here, using gene expression profiling, we have identified an "IL-13 high" subphenotype in patients with asthma. Using rigorous clinical criteria and methacholine challenge testing, we found that that this subphenotype comprises only ~50% of patients who are diagnosed with asthma. This "IL-13 high" subphenotype also displayed increased levels of IL-5 expression and showed certain distinguishing clinical characteristics including enhanced airway hyper-responsiveness, increased serum IgE levels and eosinophilic inflammation, subepithelial fibrosis, and altered expression of gel-forming mucins compared to an "IL-13 low" subphenotype and healthy controls.

Our work challenges certain current concepts of asthma pathogenesis by showing that a gene signature for IL-13 driven inflammation in airway epithelial cells is prominent in only half of asthmatics; non-IL-13 driven mechanisms must therefore operate in the remaining half. The findings discussed herein lead us to propose that asthma can be divided into various molecular subphenotypes such as "IL-13 high" and "IL-13 low" subphenotypes referred to herein. We validated the IL-13 high/IL-13 low classification scheme through confirmatory analyses of gene expression in bronchial biopsies, analysis of reproducibility on repeat examination, and comprehensive characterization of the distinct clinical, inflammatory, pathological and treatment-related characteristics of these two molecular subphenotypes of asthma. These findings provide a mechanistic framework for the emerging clinical observation that asthma is a complex and heterogeneous disease [58].

Molecular phenotyping of asthma based on Th2 inflammation has important therapeutic implications. First, airway obstruction in the "IL-13 high" subphenotype improves with inhaled steroids whereas the "IL-13 low" subphenotype shows little to no improvement. The Th2 markers that we have identified can be used to guide the development of clinical tests for steroid-responsiveness by providing surrogate markers of a steroid-responsive phenotype. Second, blockade of IL-13 and related Th2 cytokines is under active clinical development as a therapeutic strategy in asthma [34]. Our data suggest that clinical response to these therapies may be limited to the specific subphenotype of patients with "IL-13 high" asthma. Thus, markers of this molecular phenotype have direct application in clinical trials.

Prior studies using induced sputum analyses suggested that "eosinophilic asthma" is a distinct cellular phenotype of asthma, but molecular mechanisms underlying this cellular phenotype have been undefined. Our data suggest that IL-13 driven inflammation is a molecular mechanism underlying "eosinophilic asthma" [37] because of the airway eosinophilia that we demonstrated in "IL-13 high asthma." In addition, we demonstrated that both "eosinophilic asthma" and "IL-13 high" asthma are characterized by subepithelial fibrosis [38, 39], ALOX15 production by alveolar macrophages [55] and lung function responses to inhaled corticosteroids [40, 41]. In addition to these recognized features of eosinophilic asthma, we have identified further clinical features of "IL-13 high" asthma, including altered airway mucin gene expression and induction of TNF$\alpha$, a mediator which is not considered a Th2-cytokine but which has been previously associated with severe asthma [59]. We speculate that these features will also be found in eosinophilic asthma. In addition, it is likely that IL-5 is a major contributor to the airway and systemic eosinophilia we observe in "IL-13 high" asthma, because we found that IL-5 expression is significantly co-regulated with IL-13 expression (Fig. IE). IL-5 is a major stimulus of eosinophil differentiation, recruitment, activation, and survival [60], but IL-13 can strongly induce the expression of eosinophil chemoattractants such as CCL11, CCL22, and CCL26 in the airway [61] and may thus work cooperatively with IL-5 to promote eosinophil infiltration, activation, and survival in the airways. Residual IL-13 activity may therefore explain the incomplete tissue depletion of eosinophils observed in clinical trials of IL-5 blockade in asthma [62, 63].

In addition, these data reveal that a significant percentage of patients with asthma have an "IL-13 low" phenotype which manifests such clinical features of asthma as airway obstruction, airway hyper-responsiveness and bronchodilator reversibility despite a paucity of Th2-driven inflammation. The causes of "IL-13 low" asthma remain obscure, but possibilities include neutrophilic inflammation [37], IL-17 driven inflammation [42], intrinsic defects in barrier function [43] and chronic sub-clinical infection by atypical intracellular bacteria [44].

**Table 7.** Genes used in alveolar macrophage qPCR

| Symbol | Name | Category | Entrez Gene ID |
|---|---|---|---|
| IL13 | interleukin 13 | Th2 cytokine | 3596 |

(continued)

| Symbol | Name | Category | Entrez Gene ID |
|---|---|---|---|
| IL4 | interleukin 4 | Th2 cytokine | 3565 |
| ARG1 | arginase, liver | Alternative activation marker | 383 |
| MRC1 | mannose receptor, C type1 | Alternative activation marker | 4360 |
| MRC2 | mannose receptor, C type2 | Alternative activation marker | 9902 |
| IL1RN | interleukin 1 receptor antagonist | Alternative activation marker | 3557 |
| CCL17 | T cell-directed CC chemokine | Alternative activation marker | 6361 |
| CCL22 | macrophage derived chemokine | Alternative activation marker | 6367 |
| TNFα | tumor necrosis factor | Classical activation marker | 7124 |
| IL1β | interleukin 1, beta | Classical activation marker | 3553 |
| CCL20 | macrophage inflammatory protein 3 alpha | Classical activation marker | 6364 |
| ALOX15 | arachidonate 15-lipoxygenase | Leukotriene pathway | 246 |
| ALOX5 | arachidonate 5-lipoxygenase | Leukotriene pathway | 240 |
| ALOX5AP | arachidonate 5-lipoxygenase-activating protein | Leukotriene pathway | 241 |
| LTA4H | leukotriene A4 hydrolase | Leukotriene pathway | 4048 |
| LTC4S | leukotriene C4 synthase | Leukotriene pathway | 4056 |

**Table 8.** Alveolar macrophage gene expression by qPCR

| | Normalized Gene Copy Number | | | P-values | | |
|---|---|---|---|---|---|---|
| Gene | Control N=15 | IL-13 Low N=5 | IL-13 High N=9 | IL-13 Low vs. control | IL-13 High vs. control | IL-13 High vs IL-13 Low |
| IL13 | --- | --- | --- | --- | --- | --- |
| IL4 | --- | --- | --- | --- | --- | --- |
| ARG1 | 16,707± 49,889 | 13,188± 29,285 | 177±349 | 0.99 | 0.68 | 0.91 |
| MRC1 | 4,729,405± 2,343,659 | 4,281,358± 2,235,805 | 5,575,399± 2,211,337 | 0.98 | 0.77 | 0.69 |
| MRC2 | 323,199± 949,034 | 318,115± 704,525 | 1,627±929 | 1.00 | 0.68 | 0.84 |
| IL1RN | 1,217,545± 2,179,904 | 1,629,394± 2,679,369 | 477,775± 147,251 | 0.97 | 0.75 | 0.64 |
| CCL17 | 200±457 | 421±867 | 42±44 | 0.76 | 0.82 | 0.42 |
| CCL22 | 61,812± 163,171 | 53,105± 113,545 | 4,306±5,750 | 0.99 | 0.65 | 0.88 |
| TNFα | 75,044± 41,433 | 75,941± 43,938 | 130,385± 47,351 | 1.00 | **0.017*** | 0.10 |
| IL1β | 102,121± 37,416 | 107,456± 20,675 | 111,181± 25,317 | 0.98 | 0.88 | 0.99 |
| CCL20 | 16,033± 9,224 | 16,826± 7,375 | 16,231± 5,003 | 0.99 | 1.00 | 0.99 |
| ALOX15 | 18,741± 19,420 | 24,167± 19,036 | 142,494± 188,198 | 1.00 | **0.03*** | 0.16 |
| ALOX5 | 10,655,887± 2,754,206 | 1,1308,968± 2,851,849 | 11,033,153± 1,397,415 | 0.94 | 0.98 | 0.99 |
| ALOX5AP | 13,940,937± 3,209,466 | 12,710,464± 2,864,216 | 12,877,643± 2,812,301 | 0.83 | 0.80 | 1.00 |
| LTA4H | 8,532,533± 1,944,551 | 8,455,408± 1,191,877 | 7,859,076± 1,647,800 | 1.00 | 0.75 | 0.91 |
| LTC4S | 4,959±3,748 | 5,445±3,189 | 9,086±4,988 | 0.99 | 0.07 | 0.33 |

Levels of expression of IL-13 were below the limit of detection (cycle threshold >40) in 26 of the 29 subjects, and IL-4 was below the limit of detection in 20 of the 29 subjects (no differences between the three groups for either cytokine, all p>0.35). All other genes were within the limit of detection across samples.

Example 9 - Relationship of "IL-13 high" and "IL-13 low" subphenotypes of asthma to serum protein biomarkers

Further microarray analysis led us to identify from the set of genes and probes listed in Table 4, a set of 35 probes representing 28 genes whose expression was co-regulated with periostin in individual subjects below a threshold false discovery rate (FDR) q-value of 0.05. These genes and probes and associated data are presented in Table 9. Hierarchical cluster analysis of all subjects, including healthy controls and asthmatics, based on expression levels of those probes confirmed and further defined the two major clusters described above of (1) a cluster with high expression levels of periostin and co-regulated genes and (2) a cluster with low expression levels of periostin and co-regulated genes (Figure 11). Mast cell genes include RGS13, TPSG1, TPSAB1, FCER1B, CPA3 and SLC18A2. Eosinophil genes include include P2RY14 and ALOX15.

The cluster with high expression of periostin and co-regulated genes comprised 23 asthmatic subjects and 1 healthy control (Figure 11, cluster 1, indicated in red) whereas the cluster with low expression of periostin and co-regulated genes comprised the remaining 19 asthmatics interspersed with 26 of the healthy controls (Figure 11, cluster 2, indicated in green). In Example 8, we described clustering of subjects in this dataset based on the microarray-determined expression levels of three of these probes: 210809_s_at (periostin), 210107_at (CLCA1), and 204614_at (serpinB2). The three-probe signature described in Example 8 correlates well with this full 35-probe signature, differing for seven asthmatics and one healthy control (discrepant calls indicated in Figure 11 with *).

Table 9. IL-13 gene signature genes and exemplary probes.

**Microarray signal intensity**

| Probe | Gene Name | fold high vs. low | IL13low vs high Pval | IL13low vs high qval | IL13low vs. HC Pval | IL13low vs. HC qval | Healthy Mean | IL13 Low mean | IL13 high Mean | fold high vs. HC | fold low vs. HC |
|---|---|---|---|---|---|---|---|---|---|---|---|
| 206224_at | **CST1*** | **15.46** | ***2.33E-06*** | ***0.004*** | 0.87 | 0.95 | 8.76 | 17.86 | 276.18 | 31.54 | 2.04 |
| 208555_x_at | **CST2*** | **12.69** | ***3.02E-07*** | ***0.001*** | 0.86 | 0.95 | 5.13 | 8.95 | 113.65 | 22.16 | 1.75 |
| 206994_at | **CST4*** | **10.87** | ***1.29E-06*** | ***0.002*** | 0.71 | 0.94 | 10.38 | 48.27 | 524.78 | 50.56 | 4.65 |
| 210107_at | **CLCA1*** | **9.05** | ***2.28E-07*** | ***0.001*** | 0.71 | 0.94 | 29.61 | 74.20 | 671.32 | 22.67 | 2.51 |
| 223710_at | **CCL26*** | **8.83** | ***4.66E-05*** | ***0.030*** | 0.74 | 0.95 | 6.33 | 3.79 | 33.50 | 5.29 | 0.60 |
| 1555778_a_at | **POSTN*** | **7.21** | ***9.58E-09*** | ***4.90E-05*** | 0.88 | 0.95 | 14.93 | 17.89 | 129.07 | 8.65 | 1.20 |
| 210809_s_at | **POSTN*** | **5.31** | ***3.90E-13*** | ***1.93E-08*** | 0.60 | 0.94 | 260.24 | 372.07 | 1976.14 | 7.59 | 1.43 |
| 204919_at | **PRR4*** | **5.14** | ***1.75E-06*** | ***0.003*** | 0.49 | 0.94 | 37.33 | 97.62 | 502.21 | 13.45 | 2.62 |
| 207741_x_at | **TPSD1**** | **4.40** | ***1.90E-05*** | ***0.02*** | 0.69 | 0.94 | 9.86 | 14.84 | 65.30 | 6.62 | 1.51 |
| 204614_at | **SERPINB2*** | **3.72** | ***2.84E-07*** | ***0.001*** | 0.35 | 0.92 | 97.43 | 196.39 | 729.95 | 7.49 | 2.02 |
| 216485_s_at | **TPSG1**** | **3.65** | ***8.94E-09*** | ***4.90E-05*** | 0.61 | 0.94 | 10.04 | 13.18 | 48.06 | 4.79 | 1.31 |
| 210037_s_at | **INOS*** | **3.43** | ***2.53E-07*** | ***0.001*** | 0.91 | 0.95 | 6.39 | 6.07 | 20.86 | 3.26 | 0.95 |
| 210258_at | **RGS13**** | **3.19** | ***1.88E-07*** | ***0.001*** | 0.08 | 0.87 | 8.75 | 4.97 | 15.84 | 1.81 | 0.57 |
| 201884_at | **CEACAM5** | **2.89** | ***1.30E-08*** | ***5.82E-05*** | 0.54 | 0.94 | 426.04 | 535.38 | 1548.02 | 3.63 | 1.26 |
| 216474_x_at | **TPSD1**** | **2.88** | ***7.64E-10*** | ***1.26E-05*** | 0.29 | 0.91 | 46.63 | 69.06 | 199.21 | 4.27 | 1.48 |
| 206637_at | **GPR105***** | **2.75** | ***3.19E-08*** | ***0.0001*** | 0.45 | 0.94 | 40.90 | 53.89 | 148.29 | 3.63 | 1.32 |
| 205624_at | **CPA3**** | **2.58** | ***3.91E-09*** | ***2.76E-05*** | 0.25 | 0.90 | 99.69 | 145.25 | 374.66 | 3.76 | 1.46 |
| 226751_at | **C2ORF32**[+] | **2.55** | ***4.58E-08*** | ***0.0002*** | 0.52 | 0.94 | 35.39 | 29.78 | 76.07 | 2.15 | 0.84 |
| 205683_x_at | **TPSD1**** | **2.54** | ***3.57E-10*** | ***8.82E-06*** | 0.26 | 0.90 | 53.99 | 74.71 | 189.54 | 3.51 | 1.38 |
| 210084_x_at | TPSD1** | **2.45** | ***2.01E-09*** | ***1.99E-05*** | 0.23 | 0.90 | 48.91 | 69.02 | 169.00 | 3.46 | 1.41 |
| 225316_at | MFSD2 | **2.44** | ***2.54E-06*** | ***0.004*** | 0.88 | 0.95 | 29.03 | 27.72 | 67.73 | 2.33 | 0.95 |
| 207134_x_at | TPSD1** | **2.37** | ***9.91E-09*** | ***4.90E-05*** | 0.12 | 0.88 | 50.28 | 80.13 | 189.76 | 3.77 | 1.59 |
| 232306_at | CDH26 | **2.31** | ***3.31E-07*** | ***0.001*** | 0.23 | 0.90 | 223.92 | 330.01 | 763.78 | 3.41 | 1.47 |
| 215382_x_at | TPSD1** | **2.22** | ***1.89E-07*** | ***0.001*** | 0.40 | 0.93 | 38.96 | 48.85 | 108.32 | 2.78 | 1.25 |
| 200696_s_at | GSN | **2.08** | ***3.45E-06*** | ***0.005*** | 0.95 | 0.95 | 246.87 | 243.40 | 506.71 | 2.05 | 0.99 |
| 217023_x_at | TPSD1** | **2.02** | ***8.11E-08*** | ***0.0003*** | 0.24 | 0.90 | 53.13 | 68.70 | 138.67 | 2.61 | 1.29 |
| 205857_at | SLC18A2** | **1.88** | ***1.29E-06*** | ***0.002*** | 0.39 | 0.93 | 84.08 | 100.24 | 188.75 | 2.24 | 1.19 |
| 214539_at | SERPINB10 | **1.88** | ***1.20E-05*** | ***0.012*** | 0.54 | 0.94 | 42.37 | 37.53 | 70.62 | 1.67 | 0.89 |
| 207496_at | FCER1B** | **1.82** | ***4.18E-06*** | ***0.005*** | 0.52 | 0.94 | 37.55 | 33.51 | 61.04 | 1.63 | 0.89 |
| 238429_at | TMEM71 | **1.82** | ***3.45E-05*** | ***0.024*** | 0.51 | 0.94 | 36.79 | 42.73 | 77.72 | 2.11 | 1.16 |

| Probe | Gene Name | fold high vs. low | IL13low vs high Pval | IL13low vs high qval | IL13low vs. HC Pval | IL13low vs. HC qval | Healthy Mean | IL13 Low mean | IL13 high Mean | fold high vs. HC | fold low vs. HC |
|---|---|---|---|---|---|---|---|---|---|---|---|
| 243582_at | SH3RF2 | **1.80** | ***1.48E-07*** | ***0.0005*** | 0.36 | 0.92 | 82.64 | 96.39 | 173.86 | 2.10 | 1.17 |
| 218976_at | DNAJC12 | **1.79** | ***9.51E-05*** | ***0.044*** | 0.59 | 0.94 | 48.54 | 43.54 | 77.72 | 1.60 | 0.90 |
| 238669_at | PTGS1 | **1.56** | ***2.04E-06*** | ***0.003*** | 0.70 | 0.94 | 82.69 | 86.79 | 135.37 | 1.64 | 1.05 |
| 207328_at | ALOX15*** | **1.55** | ***7.87E-06*** | ***0.009*** | 0.36 | 0.92 | 812.57 | 919.86 | 1424.90 | 1.75 | 1.13 |
| 232231_at | RUNX2 | **1.50** | ***4.99E-05*** | ***0.032*** | 0.28 | 0.91 | 288.42 | 335.71 | 502.48 | 1.74 | 1.16 |

Probes are ranked in order of fold change in “IL-13 high” vs. “IL-13 low” asthmatics (third column from left); probes with a 2.5 fold or greater enrichment in “IL-13 high” asthma are shown with bolded gene names.
Probes corresponding to periostin (POSTN) and CEACAM5 are shaded.
Non mast cell genes > 3-fold upregulated in “IL-13 high” vs. “IL-13 low” asthma are indicated with a single asterisk (*).
Mast cell-specific genes are indicated with a double asterisk (**) (+ Note that based on clustering pattern, C2ORF32 signal is likely mast cell-derived).
Eosinophil-specific genes are indicated with a triple asterisk (***).

Using the three-gene (periostin, CLCA1, and serpinB2) IL-13 signature, we showed in Example 8 that systemic markers of allergic inflammation including serum IgE and peripheral blood eosinophil levels were significantly elevated in "IL-13 high" subphenotype asthmatics relative to "IL-13 low" subphenotype asthmatics. However, there was significant overlap between the asthmatic groups for each of these metrics taken individually. In addition, neither serum IgE or peripheral blood eosinophil levels alone constitutes a non-invasive metric for predicting the airway IL-13 signature and associated "IL-13 high" or "IL-13 low" asthma subphenotype with simultaneous high sensitivity and specificity.

To determine whether the intersection of IgE and peripheral blood eosinophil levels could predict patterns of airway inflammation with greater accuracy than either metric alone, we evaluated serum IgE and peripheral blood eosinophil counts together versus airway IL-13 signature status. We found that, across the 42 asthmatics, serum IgE and peripheral blood eosinophil counts were correlated, albeit weakly (Figure 4; data shown for the IL-4/13 signature; similar results were obtained for the IL-13 signature [*see* Table 10]). For the IL-13 signature, all of the "IL-13 high" asthmatics had eosinophil counts greater than 0.14 x $10^9$/L, but many of the "IL-13 low" asthmatics had lower eosinophil counts. All but two of the "IL-13 high" asthmatics had serum IgE levels greater than 100 IU/ml, but many "IL-13 low" asthmatics did not. The two metrics of (1) serum IgE ≥ 100 IU/ml and (2) eosinophil counts ≥ 0.14 x $10^9$/L combined yielded improved sensitivity and specificity for the IL-13 signature in the airway (Table 10). Thus, a composite of two commonly used peripheral blood metrics of allergic inflammation may be an effective noninvasive biomarker for airway IL-13 driven inflammation.

**Table 10.** Sensitivity, specificity, positive and negative predictive values of IgE and peripheral blood eosinophil metrics for the IL-13 signature.

| **Positive criteria: serum IgE > 100 IU/ml** | | **IL-13 signature status** | | Sensitivity: | 21/23 = 0.91 |
|---|---|---|---|---|---|
| | | High | Low | Specificity: | 9/19 = 0.47 |
| Test Result | + | 21 | 10 | PPV: | 21/31 = 0.68 |
| | - | 2 | 9 | NPV: | 9/11 = 0.82 |
| **Positive criteria: eosinophils ≥ 0.14x$10^9$/L** | | **IL-13 signature status** | | Sensitivity: | 23/23 = 1 |
| | | High | Low | Specificity: | 8/19 = 0.42 |
| Test Result | + | 23 | 11 | PPV: | 23/34 = 0.68 |
| | - | 0 | 8 | NPV: | 8/8 = 1 |
| **Positive criteria: IgE > 100 IU/ml AND eosinophils ≥ 0.14x$10^9$/L** | | **IL-13 signature status** | | Sensitivity: | 21/23 = 0.91 |
| | | High | Low | Specificity: | 14/19 = 0.74 |
| Test Result | + | 21 | 5 | PPV: | 21/26 = 0.81 |
| | - | 2 | 14 | NPV: | 14/16 = 0.88 |

To identify additional systemic (noninvasive) candidate biomarkers of the bronchial epithelial IL-13 signature, we examined the signature for genes encoding extracellular or secreted proteins that might be detectable in peripheral blood. Three candidates of particular interest were CCL26, periostin, and CEACAM5. As CCL26 has been previously described as a Th2 cytokine-induced chemokine in bronchial epithelium [71], we focused on the characterization of periostin and CEACAM5, which have not previously been described as serum biomarkers of Th2 inflammation. CEACAM5 encodes carcinoembryonic antigen (CEA), which is a frequently used prognostic serum biomarker in epithelial-derived cancers. Periostin has also been described in a limited number of studies as a serum biomarker for certain cancers and, intriguingly, was detectable at a level in the range of 10s-100s of ng/ml serum in most subjects, attractive characteristics for a serum marker to be readily detected by immunoassays.

As shown in Figure 12A-B, Periostin and CEACAM5 are each good individual representatives of the IL-13 signature, exhibiting significantly higher expression in "IL-13 high" asthmatics than in "IL-13 low" asthmatics or healthy controls. There was a strong correlation between microarray expression levels of periostin and CEACAM5 in individual asthmatics (Figure 12C). To confirm these gene expression patterns and determine whether periostin and CEACAM5 expression could be used in an algorithm to distinguish "IL-13 high" asthmatics from "IL-13 low" asthmatics and healthy controls, we analyzed expression levels of the two genes by qPCR in the same bronchial epithelial brushing samples used for microarray analysis. There was a high degree of concordance between microarray and qPCR values in individual subjects (not shown). We used ordinal logistic regression analysis to generate a predictive model for the microarray-derived 35-probe IL-13 status using qPCR values for periostin and CEACAM5. The model's predictive value was highly significant (p<0.0001) and periostin and CEACAM5 parameter estimates each had a significant effect in the model (p<0.02 for CEACAM5; p<0.0001 for periostin). Receiver operating characteristic (ROC) curve analysis demonstrated perfect predictivity for healthy control and very high sensitivity and specificity for "IL-13 high" and "IL-13 low" asthma (Figure 12D). Taken together, these data show that bronchial epithelial expression levels of periostin and CEACAM5 are good surrogates for the overall IL-13 signature.

To determine whether elevated levels of soluble periostin and CEA proteins were detectable in peripheral blood, we examined periostin and CEA in sera from 100 asthmatics and 48 healthy controls using immunoassays. In addition, we measured IgE and YKL-40, a serum marker previously described to be elevated in some asthmatics [72], in these same sera. We observed significantly elevated levels of IgE, periostin, CEA, and YKL-40 in asthmatics relative to healthy controls (Figure 13A-D). However, in all cases, there was substantial overlap in serum levels of each biomarker between groups. As shown in Example 8, inhaled corticosteroid (ICS) treatment reduces the bronchial epithelial expression of periostin in asthmatics that have elevated periostin at baseline (*see also* [8]). Of the 100 asthmatics whose serum we examined, 51 were taking inhaled corticosteroids (ICS) and 49 were not. When comparing asthmatics not on ICS and asthmatics on ICS, ICS-treated subjects had significantly lower median serum levels of IgE and CEA, and showed a trend for lower periostin levels, while YKL-40 levels were unchanged (Figure 13E-H). Nevertheless, asthmatics on ICS had higher median serum levels of IgE, periostin, and CEA than healthy controls (Table 13). As shown in Figure 4 and Table 10, 21/23 asthmatics positive for the bronchial epithelial IL-13 signature ("IL-13 high") had serum IgE levels greater than 100 IU/ml, although a proportion of "IL-13 low" asthmatics also had elevated IgE. We found that serum periostin levels trended higher and CEA levels were significantly higher in asthmatics with IgE ≥ 100 IU/ml (N=68) than in asthmatics with IgE < 100 IU/ml (N=32; Figure 13I-J). However, serum YKL-40 levels were significantly lower in the high IgE group (Figure 13K). As airway expression levels of periostin and CEACAM5 were highly correlated in "IL-13 high" asthmatics, we examined the correlation between serum periostin and CEA across all asthmatics (Figure 13L). We found that serum periostin and CEA levels were significantly correlated with each other across the asthmatic population, and within asthmatics not on ICS or asthmatics with IgE ≥ 100 IU/ml but not in healthy controls, asthmatics on ICS, or asthmatics with IgE < 100 IU/ml (Table 11). Taken together, these data suggest that periostin and CEA may be serum biomarkers of a bronchial epithelial IL-13 induced gene signature in asthmatics.

**Table 11.** Correlations between serum biomarkers.

| Variable | by Variable | Spearman ρ | P-value |
|---|---|---|---|
| **All subjects (Controls, N=48; Asthmatics, N=100)** | | | |
| YKL40 (ng/ml) | IgE (IU/ml) | 0.0140 | 0.8661 |
| CEA (ng/ml) | IgE (IU/ml) | 0.4040 | ***<.0001*** |
| CEA (ng/ml) | YKL40 (ng/ml) | 0.2935 | ***0.0003*** |
| Periostin (ng/ml) | IgE (IU/ml) | 0.2259 | ***0.0058*** |
| Periostin (ng/ml) | YKL40 (ng/ml) | 0.1253 | 0.1291 |
| Periostin (ng/ml) | CEA (ng/ml) | 0.3556 | ***<.0001*** |
| **Healthy Controls (N=48)** | | | |
| YKL40 (ng/ml) | IgE (IU/ml) | 0.0420 | 0.7768 |
| CEA (ng/ml) | IgE (IU/ml) | -0.0996 | 0.5007 |
| CEA (ng/ml) | YKL40 (ng/ml) | 0.1914 | 0.1926 |
| Periostin (ng/ml) | IgE (IU/ml) | -0.2451 | 0.0931 |
| Periostin (ng/ml) | YKL40 (ng/ml) | 0.2246 | 0.1249 |
| Periostin (ng/ml) | CEA (ng/ml) | 0.4495 | ***0.0014*** |
| **All Asthmatics (N=100)** | | | |
| YKL40 (ng/ml) | IgE (IU/ml) | -0.2144 | ***0.0322*** |
| CEA (ng/ml) | IgE (IU/ml) | 0.3579 | ***0.0003*** |
| CEA (ng/ml) | YKL40 (ng/ml) | 0.0890 | 0.3787 |
| Periostin (ng/ml) | IgE (IU/ml) | 0.3262 | ***0.0009*** |
| Periostin (ng/ml) | YKL40 (ng/ml) | 0.0108 | 0.9152 |

(continued)

| | | | |
|---|---|---|---|
| **All Asthmatics (N=100)** | | | |
| Periostin (ng/ml) | CEA (ng/ml) | 0.3530 | ***0.0003*** |
| **Asthmatics; not on ICS (N=49)** | | | |
| YKL40 (ng/ml) | IgE (IU/ml) | -0.1198 | 0.4123 |
| CEA (ng/ml) | IgE (IU/ml) | 0.3727 | ***0.0084*** |
| CEA (ng/ml) | YKL40 (ng/ml) | 0.1111 | 0.4471 |
| Periostin (ng/ml) | IgE (IU/ml) | 0.4236 | ***0.0024*** |
| Periostin (ng/ml) | YKL40 (ng/ml) | 0.0186 | 0.8989 |
| Periostin (ng/ml) | CEA (ng/ml) | 0.4033 | ***0.0041*** |
| **Asthmatics; on ICS (N=51)** | | | |
| YKL40 (ng/ml) | IgE (IU/ml) | -0.2553 | 0.0706 |
| CEA (ng/ml) | IgE (IU/ml) | 0.2251 | 0.1123 |
| CEA (ng/ml) | YKL40 (ng/ml) | 0.1035 | 0.4699 |
| Periostin (ng/ml) | IgE (IU/ml) | 0.1974 | 0.1650 |
| Periostin (ng/ml) | YKL40 (ng/ml) | 0.0783 | 0.5849 |
| Periostin (ng/ml) | CEA (ng/ml) | 0.2197 | 0.1213 |
| **Asthmatics; IgE < 100 IU/ml (N=32)** | | | |
| CEA (ng/ml) | YKL40 (ng/ml) | 0.4003 | ***0.0232*** |
| Periostin (ng/ml) | YKL40 (ng/ml) | 0.3513 | ***0.0487*** |
| **Variable** | **by Variable** | **Spearman ρ** | **P-value** |
| **All subjects (Controls, N=48; Asthmatics, N=100)** | | | |
| Periostin (ng/ml) | I CEA (ng/ml) | 0.1968 | 0.2802 |
| **Asthmatics; IgE ≥ 100 IU/ml (N=68)** | | | |
| CEA (ng/ml) | YKL40 (ng/ml) | 0.0370 | 0.7647 |
| Periostin (ng/ml) | YKL40 (ng/ml) | -0.1264 | 0.3043 |
| Periostin (ng/ml) | CEA (ng/ml) | 0.4145 | ***0.0004*** |
| Spearman's rank order correlation, ρ, is indicated with associated p-values for the correlations. Highly significant p-values (<0.05) are indicated in ***bold italics.*** | | | |

Within the IL-13 signature, we observed several functional groups of multiple genes, including genes encoding protease inhibitors and genes expressed in mast cells and eosinophils, which may represent infiltration into and/or anatomic localization of those cells to bronchial epithelium. Greater than 90% of cells in each bronchial brushing sample were bronchial epithelial cells or goblet cells (mean 97%, median 98%, minimum 91%), but very small numbers of infiltrating "contaminant" cells with cell-specific gene expression patterns were detectable in the microarrays. Mast cell specific genes included tryptases (TPSAB1 [TPSD1] and TPSG1), CPA3, FCER1B, RGS13, and SLC18A2 [73, 74]. Also clustering tightly with mast cell genes was CNRIP1 (C2ORF32), a cannabinoid receptor-interacting GTPase. Given the well-established role of cannabinomimetics in the regulation of mast cell function [75], it is likely that CNRIP1 represents a mast cell-specific gene as well. Given the significant role of tissue-resident mast cells in allergic disease and the recent observation that the presence of IL-13 expressing mast cells in asthmatic endobronchial biopsy specimens is positively correlated with detectable levels of IL-13 in sputum [6], the high correlation between mast cell-specific genes and the IL-13 signature suggests that: 1) mast cells may be a significant source of IL-13 in the airway epithelium and 2) mast cell infiltration into airway epithelium may be a unique feature of the "IL-13 high" subset of asthmatics. Eosinophil specific genes include P2RY14 (GPR105) and ALOX15, although in Example 8 we described ALOX15 expression in alveolar macrophages from asthmatics.

Multiple probes corresponding to serine and cysteine protease inhibitors were present in the IL-13 signature, including Serpins B2 and B10, and cystatins (CST) 1, 2, and 4. SerpinB2 is a member of a large family of serine protease inhibitors encoded in a gene cluster on chromosome 18q21. Expression levels of Serpins B2 [8], B3, and B4 are induced in airway epithelial cells upon stimulation by recombinant IL-4 and IL-13 [7, 15]. Cystatins (CST) 1, 2, and 4 are members of a large family of cysteine protease inhibitors encoded in a gene cluster on chromosome 20p11. Several cystatins are expressed in bronchial epithelium [16]; CST4 has been identified at elevated levels in bronchoalveolar lavage fluid (BAL) of asthmatics [17]; serum CST3 is elevated in asthmatics relative to healthy controls and and its levels are decreased by ICS treatment [18]. As serpin and CST gene families are each colocalized on the chromosome, we explored whether any additional members of the serpin and cystatin gene families are co-regulated with those already identified. We performed hierarchical clustering of the microarray data across all subjects, restricted to serpin and cystatin gene families. We found that, out of over 40 protease inhibitor genes represented on the array, only serpins B2, B4, and B10; and cystatins 1, 2, and 4 were significantly co-regulated, with the highest expression levels occurring in asthmatics having the "IL-13 high" signature (Figure 2B and Table 12). As many aeroallergens possess protease activity and protease-activated receptors (PARs) are associated with the activation of allergic inflammatory cascades [76], the upregulation of protease inhibitors by Th2 cytokines may represent a compensatory response to protease-containing aeroallergens.

**Table 12:** Probe IDs of Serpin and CST genes used for clustering in Figure 2B. Probes are listed in order (top to bottom, left to right) found on heatmap at left of Figure 2B. Probes clustering with IL-13 signature genes are indicated in **bold.**

| Probe ID | Gene Name | Probe ID | Gene Name | Probe ID | Gene Name |
|---|---|---|---|---|---|
| 205075_at | SERPINF2 | 236599_at | SERPINE2 | 200986_at | SERPING1 |
| 206595_at | CST6 | 233968_at | CST11 | 1555551_at | SERPINB5 |
| 206325_at | SERPINA6 | 1554616_at | SERPINB8 | 233797_s_at | CST11 |
| 206421_s_at | SERPINB7 | 213874_at | SERPINA4 | 210140_at | CST7 |
| 227369_at | SERBP1 | 220627_at | CST8 | 209720_s_at | SERPINB3 |
| 206034_at | SERPINB8 | 1568765_at | SERPINE1 | 209719_x_at | SERPINB3 |
| 202376_at | SERPINA3 | 206386_at | SERPINA7 | 210413_x_at | SERPINB4 |
| 207636_at | SERPINI2 | 202627_s_at | SERPINE1 | 208531_at | SERPINA2 |
| 1552544_at | SERPINA12 | 1554491_a_at | SERPINC1 | 209723_at | SERPINB9 |
| 231248_at | CST6 | 210076_x_at | SERBP1 | 212190_at | SERPINE2 |
| 1553057_at | SERPINB12 | 217725_x_at | SERBP1 | 211361_s_at | SERPINB13 |
| 240177_at | CST3 | 217724_at | SERBP1 | 217272_s_at | SERPINB13 |
| 202628_s_at | SERPINE1 | 236449_at | CSTB | 204855_at | SERPINB5 |
| 216258_s_at | SERPINB13 | 207714_s_at | SERPINH1 | 209725_at | UTP20 |
| 210049_at | SERPINC1 | 202283_at | SERPINF1 | **214539_at** | **SERPINB10** |
| 220626_at | SERPINA10 | 211474_s_at | SERPINB6 | **204614_at** | **SERPINB2** |
| 209443_at | SERPINA5 | 209669_s_at | SERBP1 | **208555_x_at** | **CST2** |
| 209722_s_at | SERPINB9 | 1556950_s_at | SERPINB6 | **206224_at** | **CST1** |
| 202834_at | SERPINA8 | 228129_at | SERBP 1 | **206994_at** | **CST4** |
| 205352_at | SERPINI1 | 201201_at | CSTB | **211906_s_at** | **SERPINB4** |
| 211362_s_at | SERPINB13 | 213572_s_at | SERPINB1 | 230318_at | SERPINA1 |
| 205576_at | SERPIND1 | 212268_at | SERPINB1 | 201360_at | CST3 |
| 1554386_at | CST9 | 1552463_at | SERPINB11 | 210466_s_at | SERBP1 |
| 242814_at | SERPINB9 | 202833_s_at | SERPINA1 | 204971_at | CSTA |
| 239213_at | SERPINB1 | 211429_s_at | SERPINA1 | | |
| 230829_at | CST9L | | | | |

The mouse orthologue of CLCA1, mCLCA3 (also known as gob-5) has been previously identified as a gene associated with goblet cell metaplasia of airway epithelium and mucus production; both are induced by Th2 cytokines including IL-9 and IL-13 [12-14]. PRR4 is a member of a large gene family encoded in a cluster on chromosome 12p13. These genes encode proline-rich proteins, which are found in mucosal secretions including saliva and tears. Related, but non-orthologous proteins SPRR1a, 2a, and 2b have been identified in bronchial epithelium in a mouse model of asthma and are induced by IL-13 [19, 20]. Proline-rich proteins from the PRR/PRB family have been identified in bronchial secretions [21] and their expression has been documented in bronchial epithelium [16]. CCL26 (Eotaxin-3) is a well-documented IL-4 and IL-13 inducible eosinophil attracting chemokine in asthmatic airway epithelium [71]. CDH26 is a cadherin-like molecule of unknown function that has recently been identified in a microarray analysis of eosinophilic esophagitis [11]. That study identified several additional genes overlapping with our bronchial epithelial IL-13 signature including periostin, SerpinB4, and CCL26 [11]. As CDH26 is coregulated with eotaxins and overexpressed in diseases characterized by eosinophilic inflammation, it is tempting to speculate that CDH26 plays a role in eosinophil infiltration into mucosal tissues. Inducible nitric oxide synthase (iNOS) is associated with airway inflammation and is induced by IL-13 in human primary bronchial epithelial cell cultures [23]. The measurement of exhaled nitric oxide (eNO) is commonly used in the diagnosis and monitoring of asthma. Considered together, many of the genes described here as components of the IL-13 signature are highly consistent with in vitro and animal models of Th2 inflammation and play plausible roles in Th2-driven pathology in human asthma.

**Table 13.** Levels of serum biomarkers.

| | **Healthy Control (N=48)** | **Asthma (N=100)** | **P-value** |
|---|---|---|---|
| **IgE (IU/ml)** | 63 (0-590) | 234 (1-2098) | ***<0.0001*** |
| **Periostin (ng/ml)** | 38 (0-139) | 52 (0-117) | ***0.03*** |
| **CEA (ng/ml)** | <0.2 (<0.2-5.5) | 2 (<0.2-21*) | ***<0.0001*** |
| **YKL-40 (ng/ml)** | 48 (18-265) | 64 (19-494) | ***0.0004*** |
| **Effect of inhaled corticosteroid treatment on serum biomarkers in asthmatics** | | | |
| | **No ICS (N=49)** | **ICS (N=51)** | **P-value** |
| **IgE (IU/ml)** | 322 (8-1395) | 132 (1-2098) | ***0.011*** |
| **Periostin (ng/ml)** | 54 (0-110) | 48 (0-117) | 0.07 |
| **CEA (ng/ml)** | 2.5 (<0.2-7.5) | 1.9 (<0.2-21*) | *0.041* |
| **YKL-40 (ng/ml)** | 62 (19-353) | 72 (24-494) | 0.30 |
| **Levels of serum biomarkers in asthmatics by IgE level category** | | | |
| | **IgE < 100 IU/ml (N=32)** | **IgE ≥ 100 IU/ml (N=68)** | **P-value** |
| **CEA (ng/ml)** | 1.6 (<0.2-7.5) | 2.5 (<0.2-21*) | ***0.031*** |
| **Periostin (ng/ml)** | 49 (0-117) | 57 (0-112) | 0.20 |
| **YKL-40 (ng/ml)** | 83 (19-494) | 61 (23-290) | ***0.01*** |
| Values shown as median (range)<br>p-values are Wilcoxon rank rank sum<br>*99/100 asthmatics had CEA values ≤ 7.5 ng/ml | | | |

CEACAM5 encodes a cell-surface glycoprotein found in many epithelial tissues and elevated serum CEACAM5 (carcinoembryonic antigen; CEA) is a well-documented systemic biomarker of epithelial malignancies and metastatic disease. Elevated CEA levels have been reported in a subset of asthmatics, with particularly high serum levels observed in asthmatics with mucoid impaction [75]. Intriguingly, while the upper limit of normal for serum CEA is in the 2.5-3 ng/ml range, the lower limit for suspicion of malignancy is 10 ng/ml. In our analyses, we find that over 95% of healthy controls had CEA levels below 3 ng/ml while 1/3 of asthmatics had CEA levels between 3 and 7.5 ng/ml, and of these, the vast majority had serum IgE levels above 100 IU/ml. This suggests that a robust window of detection for CEA may be present in asthmatics with Th2-driven airway inflammation. Periostin has been described as an IL-4 and IL-13 inducible gene in asthmatic airways [7-9, 77] as a gene upregulated in epithelial-derived cancers that may be associated with invasiveness and extracellular matrix change [64-67], and whose serum protein levels are detectable and elevated in some cancers [68-70]. As it may play a role in eosinophilic tissue infiltration in eosinophilic esophagitis [11, 77], periostin could be an important factor in, and biomarker of, eosinophilic diseases such as Th2-driven asthma.

The standard of care for bronchial asthma that is not well-controlled on symptomatic therapy (i.e. β-agonists) is inhaled corticosteroids (ICS). In mild-to-moderate asthmatics with elevated levels of IL-13 in the airway [6] and eosinophilic esophagitis patients with elevated expression levels of IL-13 in esophageal tissue [11], ICS treatment substantially reduces the level of IL-13 and IL-13-induced genes in the affected tissues. In airway epithelium of asthmatics after one week of ICS treatment and in cultured bronchial epithelial cells, we have shown that corticosteroid treatment substantially reduces IL-13-induced expression levels of periostin, serpinB2, and CLCA1 [8]. Further examination of the genes listed in Table 9 revealed that, in the 19 subjects in our study who received one week of ICS treatment prior to a second bronchoscopy, the vast majority of IL-13 signature genes was significantly downregulated by ICS treatment in asthmatic bronchial airway epithelium. This downregulation could be the result of ICS-mediated reduction of IL-13 levels, ICS-mediated reduction of target gene expression, or a combination of the two. In severe asthmatics who are refractory to ICS treatment, a similar fraction of subjects (approximately 40%) was found to have detectable sputum IL-13 levels to that seen in mild, ICS-naïve asthmatics [6], which is comparable to the fraction of subjects with the IL-13 signature observed in this study. This observation suggests that, although the IL-13 signature is significantly downregulated by ICS treatment in the mild-moderate, ICS-responsive asthmatics examined in the present study, it may still be present in severe steroid-resistant asthmatics. Similar observations have been reported for eosinophilic inflammation in bronchial biopsies [78] and persistence of IL-4 and IL-5 expressing cells in BAL [79] of steroid-refractory asthmatics. There is currently a large number of biological therapeutics in clinical development directed against IL-13 or related factors in Th2 inflammation [50, 80], including, without limitation, those described herein. Our findings suggest that only a fraction of steroid-naive mild-to-moderate asthmatics may have activity of this pathway, and given its susceptibility to ICS treatment, it is likely that a smaller fraction of moderate-to-severe, steroid-refractory asthmatics has activity of this pathway. Therefore, biomarkers that identify asthmatics likely to have IL-13 driven inflammation in their airways may aid in the identification and selection of subjects most likely to respond to these experimental targeted therapies.

## Claims

1. A method of diagnosing an asthma subtype in a sample from an asthma patient comprising measuring the gene expression of POSTN, wherein elevated expression levels of POSTN, in comparison to healthy controls, are indicative of the IL-13-high asthma subtype.

2. The method according to claim 1, wherein the asthma patient additionally demonstrates elevated expression levels of any one gene or a combination of genes selected from the group consisting of CST1, CST2, CCL26, CLCA1, PRR4, PRB4, SERPINB2, CEACAM5, iNOS, SERPINB4, CST4, and SERPINB10.

3. The method according to claim 1, or 2, wherein the asthma patient further demonstrates elevated expression levels of any one gene or a combination of genes selected from the group consisting of PRB4, TPSD1, TPSG1, MFSD2, CPA3, GPR105, CDH26, GSN, C2ORF32, TRACH2000196 (TMEM71), DNAJC12, RGS13, SLC18A2, SH3RF2, FCER1B, RUNX2, PTGS1, and ALOX15.

4. The method according to any one of claims 1 to 3, wherein the gene expression is measured by assaying for protein or mRNA levels.

5. The method according to claim 4, wherein the mRNA levels are measured by using a PCR method or a microarray chip.

6. The method according to claim 5, wherein the PCR method is qPCR.

7. The method according to claim 5, wherein the mRNA levels of the POSTN gene, when 2.5-fold greater relative to the mRNA levels of a control gene, are indicative of the asthma subtype.

8. The method according to claim 4, wherein serum protein levels are measured in the sample.

## Patentansprüche

1. Verfahren zum Diagnostizieren eines Asthma-Subtyps in einer Probe eines Asthmapatienten, umfassend das Messen der Genexpression von POSTN, wobei erhöhte Expressionsspiegel von POSTN, im Vergleich zu gesunden Kontrollen, für den IL-13-hohen Asthma-Subtyp hinweisend sind.

2. Verfahren nach Anspruch 1, wobei der Asthmapatient zusätzlich erhöhte Expressionsspiegel eines beliebigen Gens oder einer Kombination an Genen, ausgewählt aus der Gruppe bestehend aus CST1, CST2, CCL26, CLCA1, PRR4, PRB4, SERPINB2, CEACAM5, iNOS, SERPINB4, CST4 und SERPINB10, aufweist.

3. Verfahren nach Anspruch 1 oder 2, wobei der Asthmapatient ferner erhöhte Expressionsspiegel eines beliebigen Gens oder einer Kombination an Genen, ausgewählt aus der Gruppe bestehend aus PRB4, TPSD1, TPSG1, MFSD2, CPA3, GPR105, CDH26, GSN, C2ORF32, TRACH2000196 (TMEM71), DNAJC12, RGS13, SLC18A2, SH3RF2, FCER1B, RUNX2, PTGS1 und ALOX15, aufweist.

4. Verfahren nach einem der Ansprüche 1 bis 3, wobei die Genexpression mittels Testen auf Protein- oder mRNA-Spiegel gemessen wird.

5. Verfahren nach Anspruch 4, wobei die mRNA-Spiegel mittels Verwendung eines PCR-Verfahrens oder eines Mikroarraychips gemessen werden.

6. Verfahren nach Anspruch 5, wobei das PCR-Verfahren qPCR ist.

**7.** Verfahren nach Anspruch 5, wobei die mRNA-Spiegel des POSTN-Gens, wenn 2,5-fach höher, relativ zu den mRNA-Spiegel eines Kontrollgens, für den Asthma-Subtyp hinweisend sind.

**8.** Verfahren nach Anspruch 4, wobei Serumproteinspiegel in der Probe gemessen werden.

## Revendications

**1.** Procédé de diagnostic d'un sous-type d'asthme dans un échantillon d'un patient asthmatique comprenant la mesure de l'expression génique du POSTN, dans lequel les niveaux d'expression élevés du POSTN, en comparaison avec les contrôles sains, sont indicatifs du sous-type d'asthme haut en IL-13.

**2.** Procédé selon la revendication 1, dans lequel le patient asthmatique manifeste en outre des niveaux d'expression élevés de l'un quelconque ou d'une combinaison de gènes sélectionnés dans le groupe constitué par CST1, CST2, CCL26, CLCA1, PRR4, PRB4, SERPINB2, CEACAM5, iNOS, SERPINB4, CST4, et SERPINB10.

**3.** Procédé selon la revendication 1 ou 2, dans lequel le patient asthmatique manifeste en outre des niveaux d'expression élevés de l'un quelconque ou d'une combinaison de gènes sélectionnés dans le groupe constitué par PRB4, TPSD1, TPSG1, MFSD2, CPA3, GPR105, CDH26, GSN, C2ORF32, TRACH2000196 (TMEM71), DNAJC12, RGS13, SLC18A2, SH3RF2, FCER1B, RUNX2, PTGS1, et ALOX15.

**4.** Procédé selon l'une quelconque des revendications 1 à 3, dans lequel l'expression génique est mesurée en analysant les niveaux de protéine ou d'ARNm.

**5.** Procédé selon la revendication 4, dans lequel les niveaux d'ARNm sont mesurés en utilisant une méthode de PCR ou une puce de microréseau.

**6.** Procédé selon la revendication 5, dans lequel la méthode de PCR est la PCR quantitative.

**7.** Procédé selon la revendication 5, dans lequel les niveaux d'ARNm du gène POSTN, quand 2,5 fois plus élevé par rapport aux niveaux d'ARNm d'un gène de contrôle, sont indicatifs du sous-type d'asthme.

**8.** Procédé selon la revendication 4, dans lequel les niveaux protéine sériquessont mesurés dans l'échantillon.

FIG. 1A

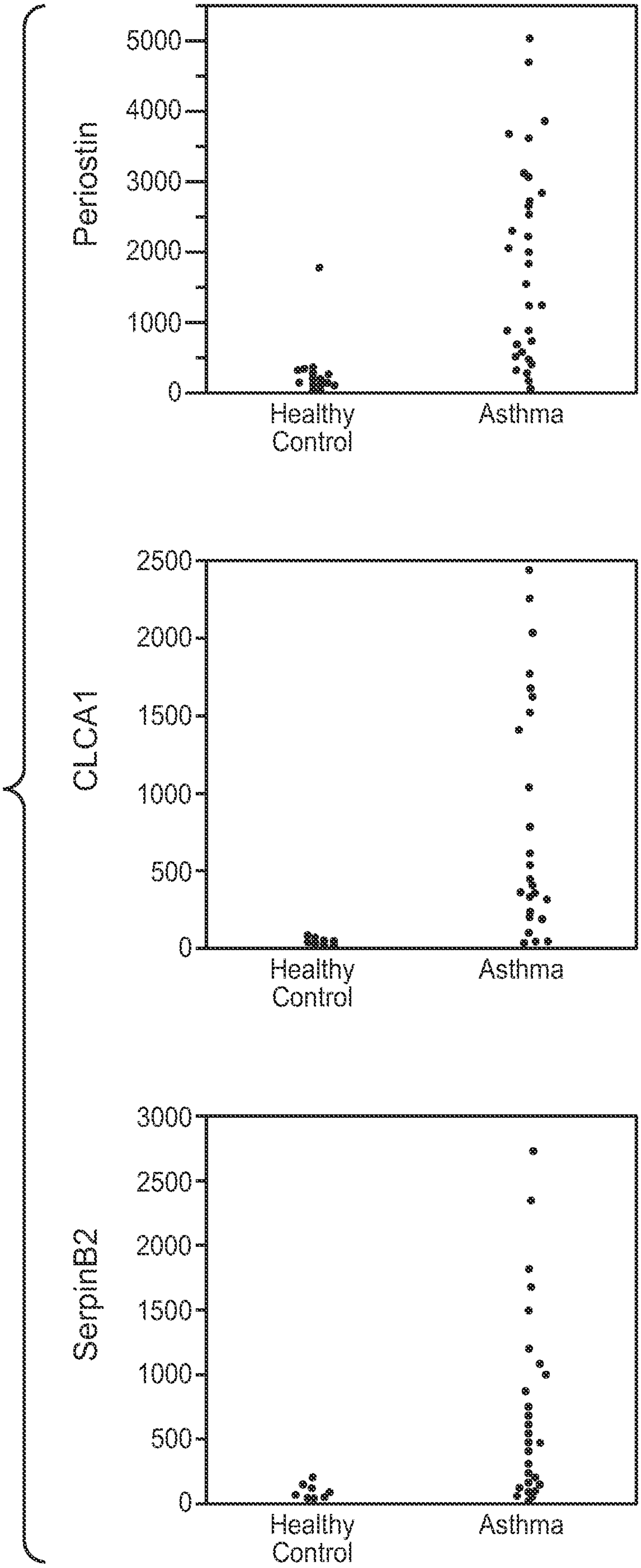
Periostin
5000
4000
3000
2000
1000
0
Healthy Control
Asthma
CLCA1
2500
2000
1500
1000
500
0
Healthy Control
Asthma
SerpinB2
3000
2500
2000
1500
1000
500
0
Healthy Control
Asthma

FIG. 1B

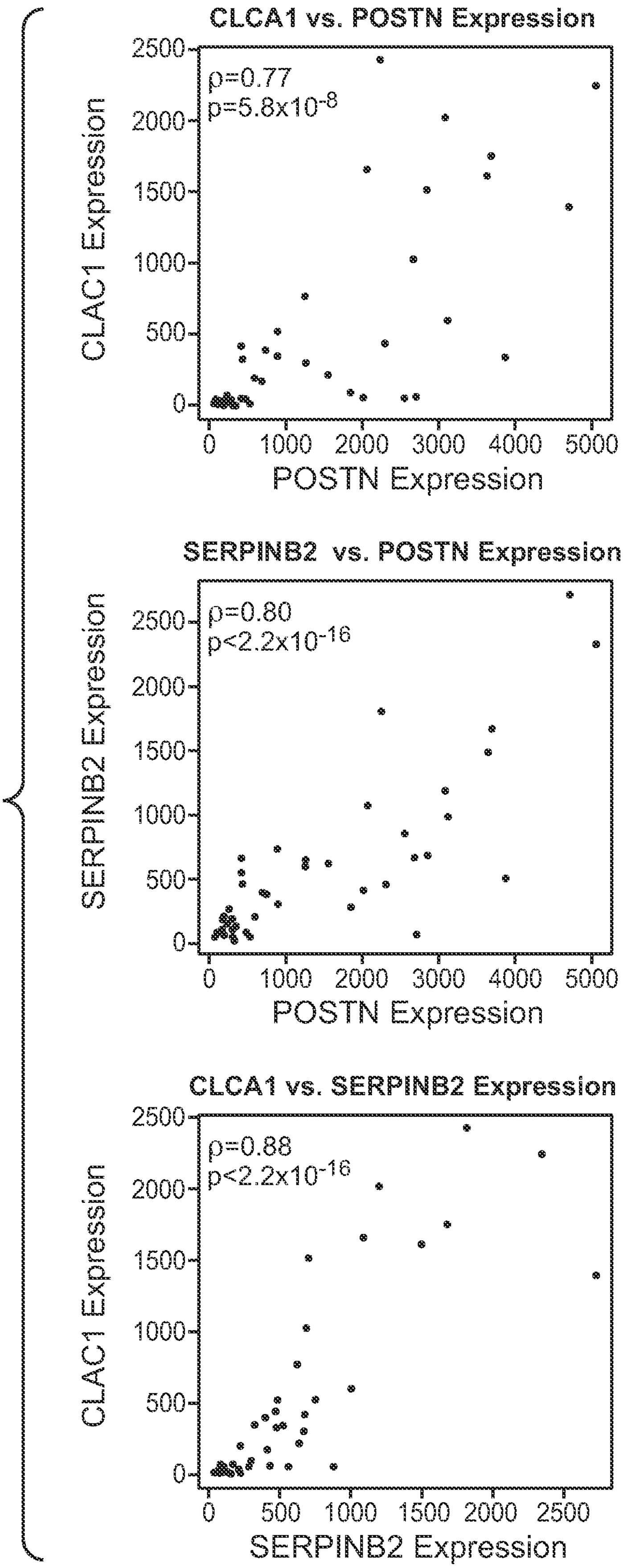
CLCA1 vs. POSTN Expression
ρ=0.77
p=5.8x10-8
CLAC1 Expression
POSTN Expression
0
500
1000
1500
2000
2500
0
1000
2000
3000
4000
5000
SERPINB2 vs. POSTN Expression
ρ=0.80
p<2.2x10-16
SERPINB2 Expression
POSTN Expression
CLCA1 vs. SERPINB2 Expression
ρ=0.88
p<2.2x10-16
CLAC1 Expression
SERPINB2 Expression

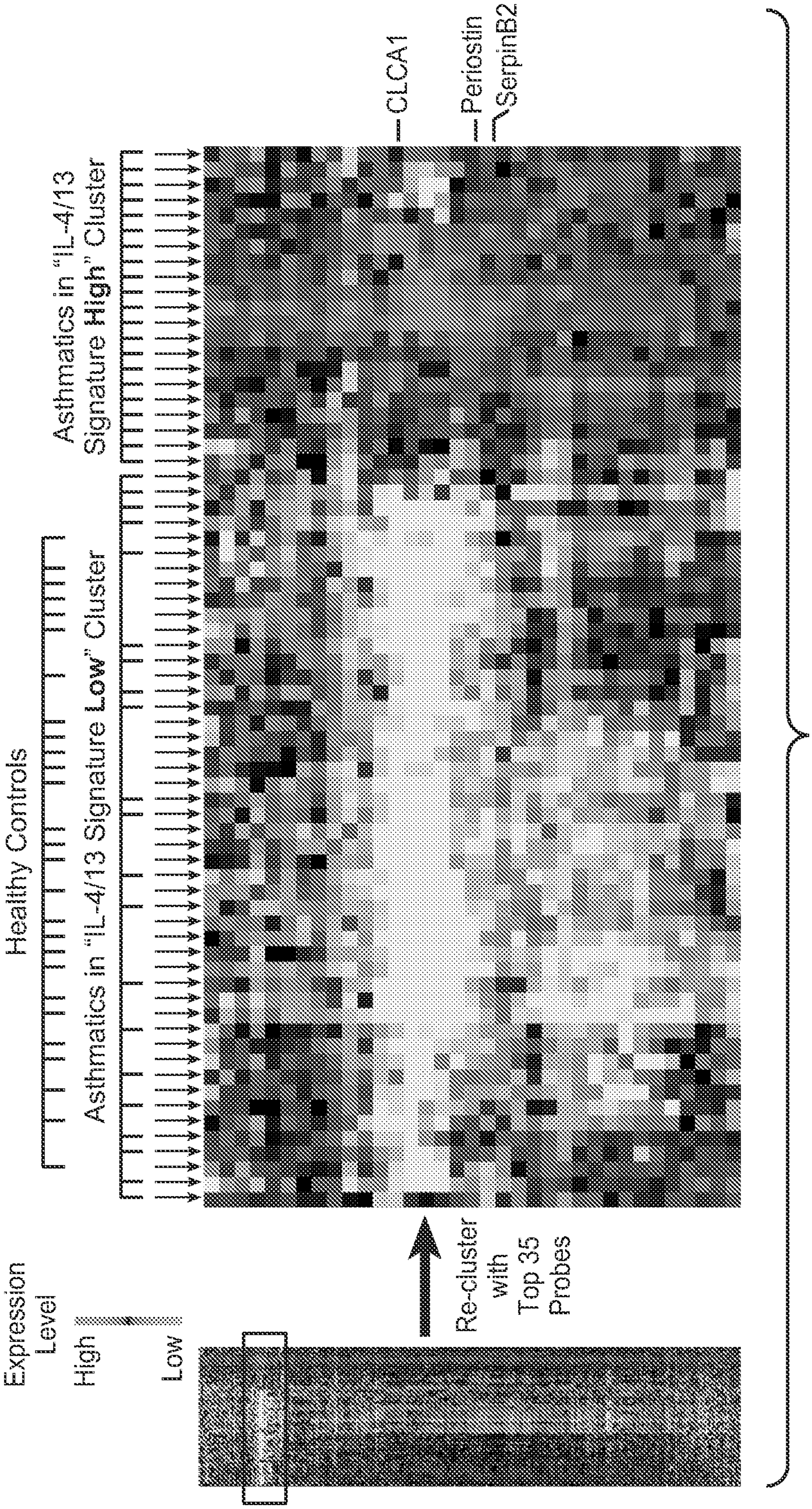
Expression Level
High
Low
Re-cluster with Top 35 Probes
Healthy Controls
Asthmatics in "IL-4/13 Signature Low" Cluster
Asthmatics in "IL-4/13 Signature High" Cluster
CLCA1
Periostin
SerpinB2

FIG. 1C

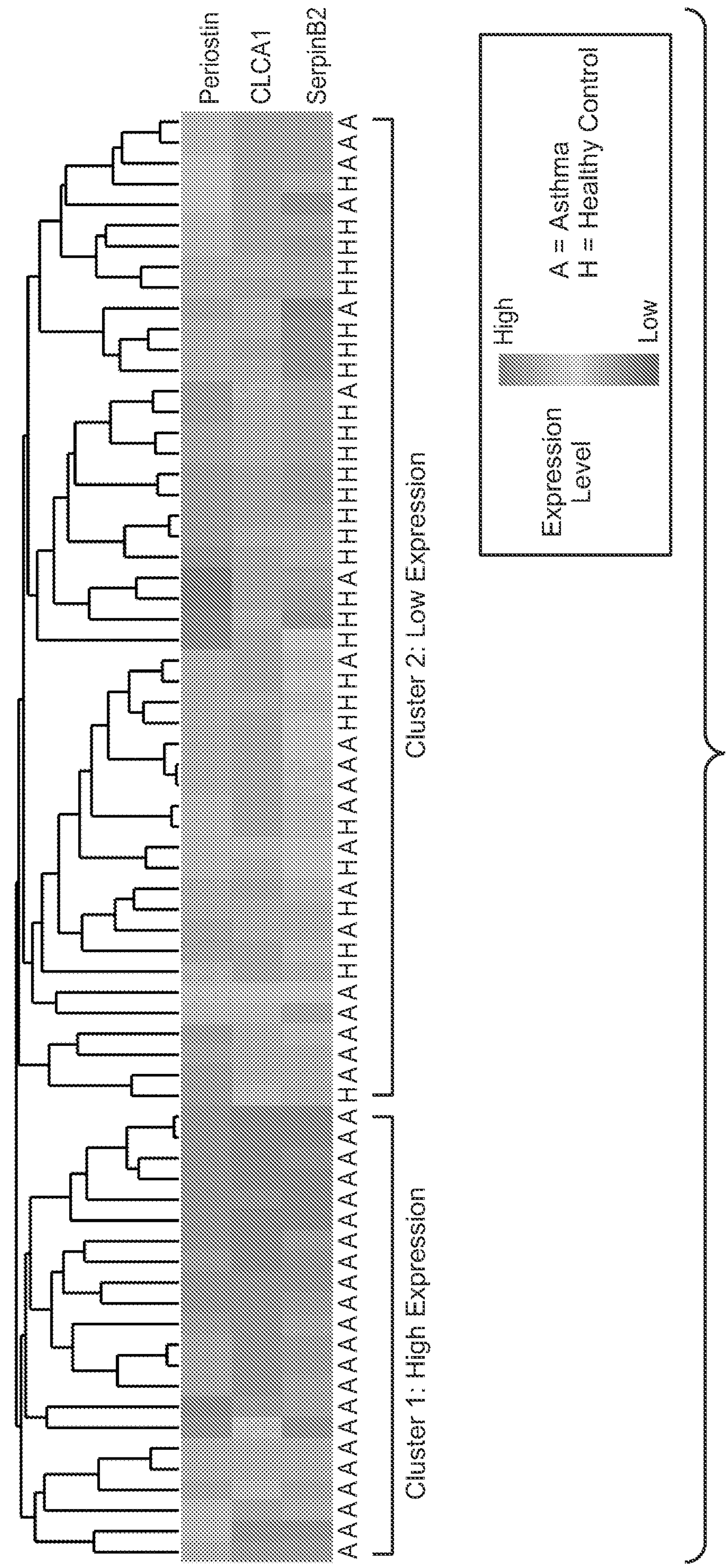
Periostin
CLCA1
SerpinB2
Cluster 1: High Expression
Cluster 2: Low Expression
Expression Level
High
Low
A = Asthma
H = Healthy Control

FIG. 1D

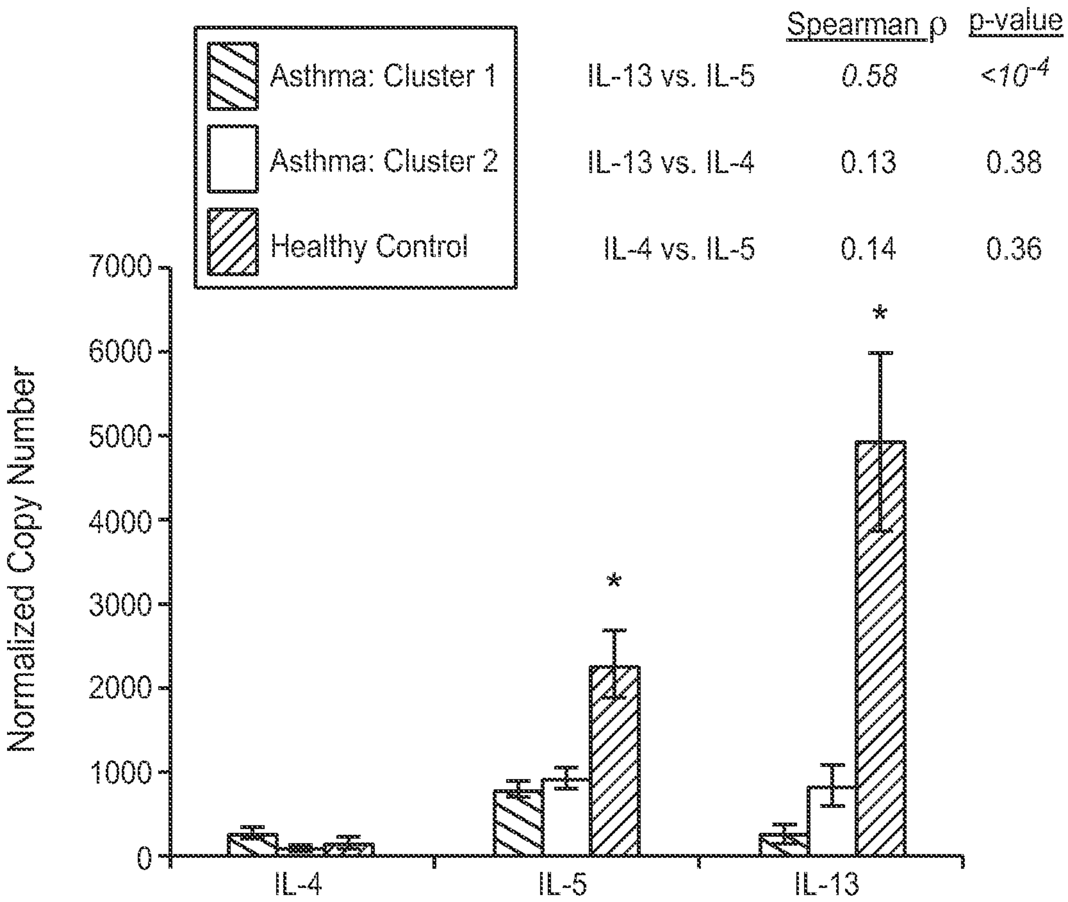
Asthma: Cluster 1
Asthma: Cluster 2
Healthy Control
Spearman ρ
p-value
IL-13 vs. IL-5
0.58
<10-4
IL-13 vs. IL-4
0.13
0.38
IL-4 vs. IL-5
0.14
0.36
Normalized Copy Number
7000
6000
5000
4000
3000
2000
1000
0
*
*
IL-4
IL-5
IL-13

FIG. 1E

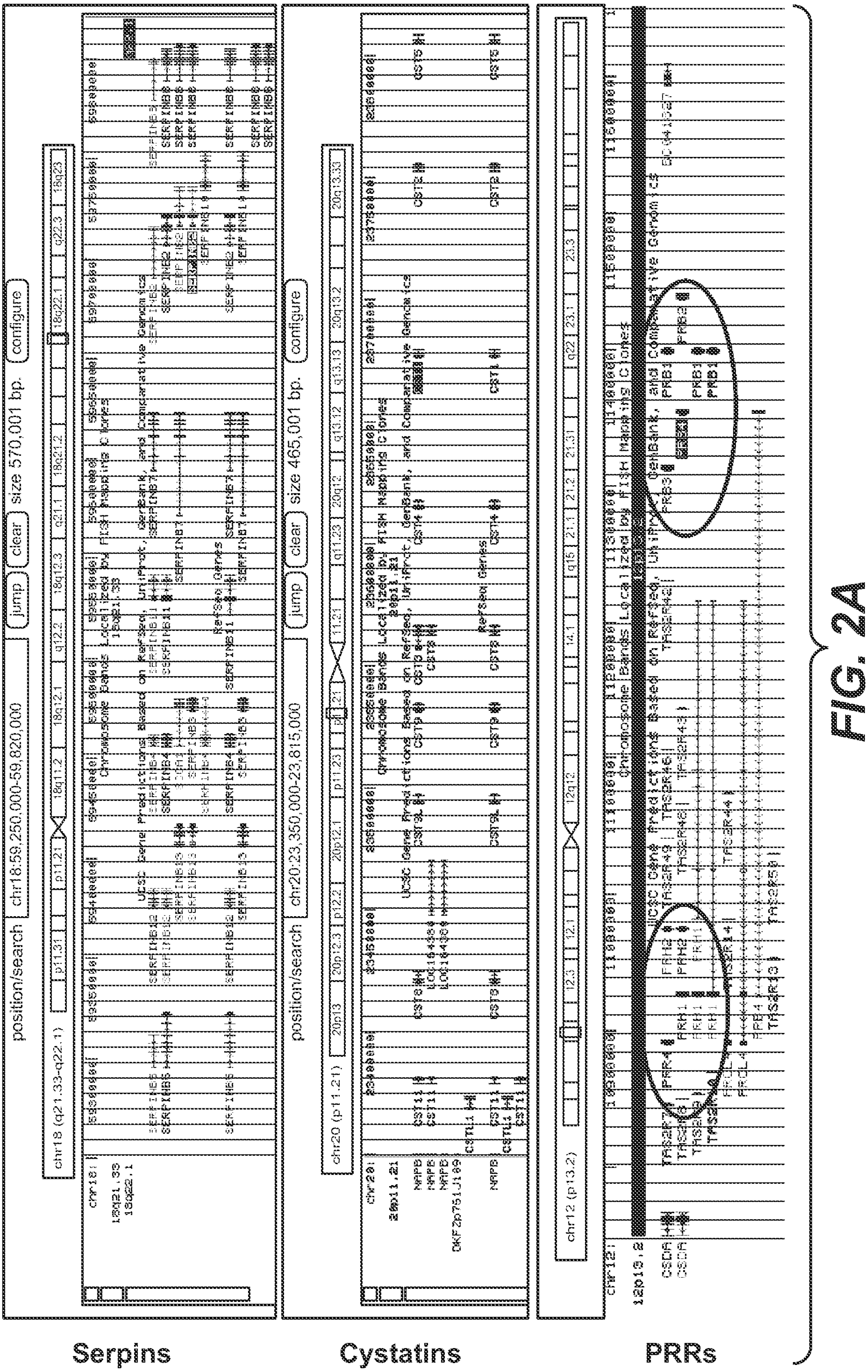


FIG. 2A

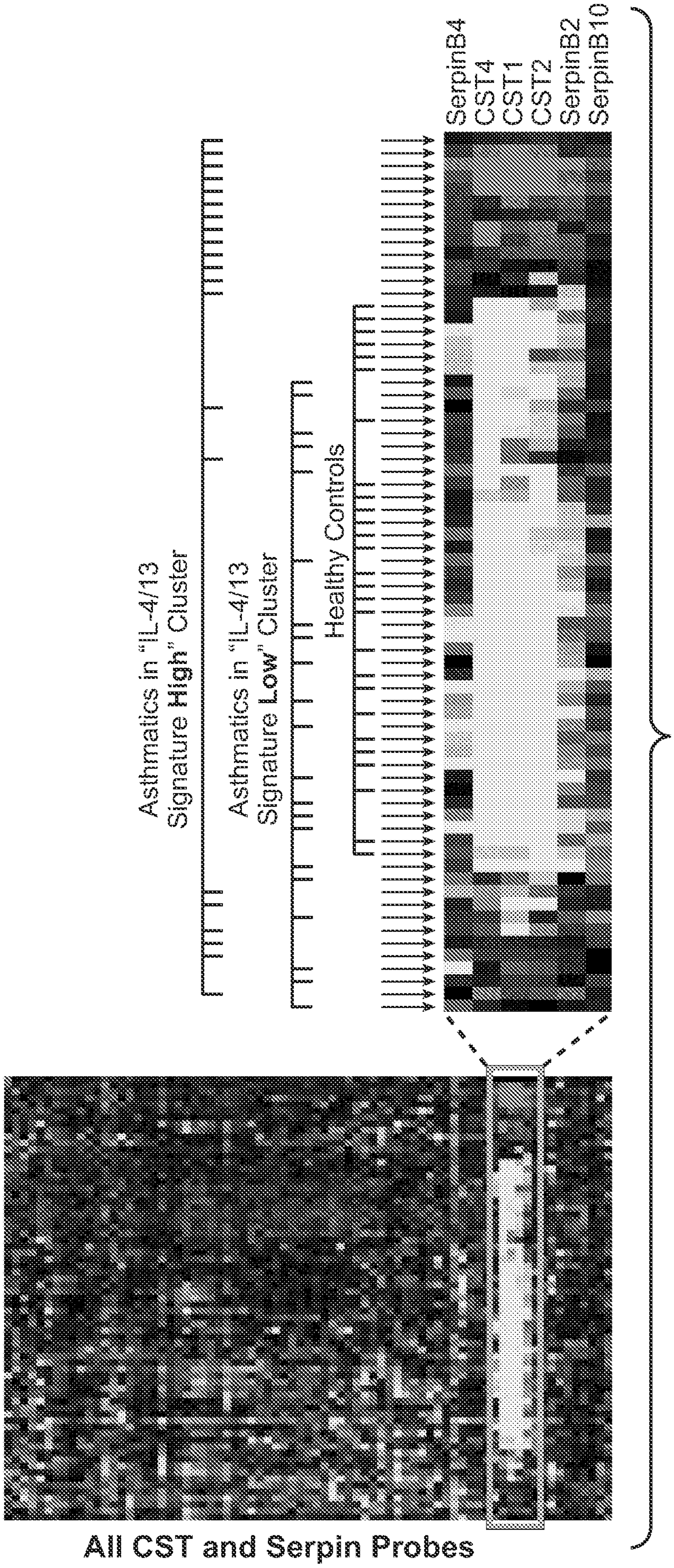
Asthmatics in "IL-4/13 Signature High" Cluster
Asthmatics in "IL-4/13 Signature Low" Cluster
Healthy Controls
SerpinB4
CST4
CST1
CST2
SerpinB2
SerpinB10
All CST and Serpin Probes

FIG. 2B

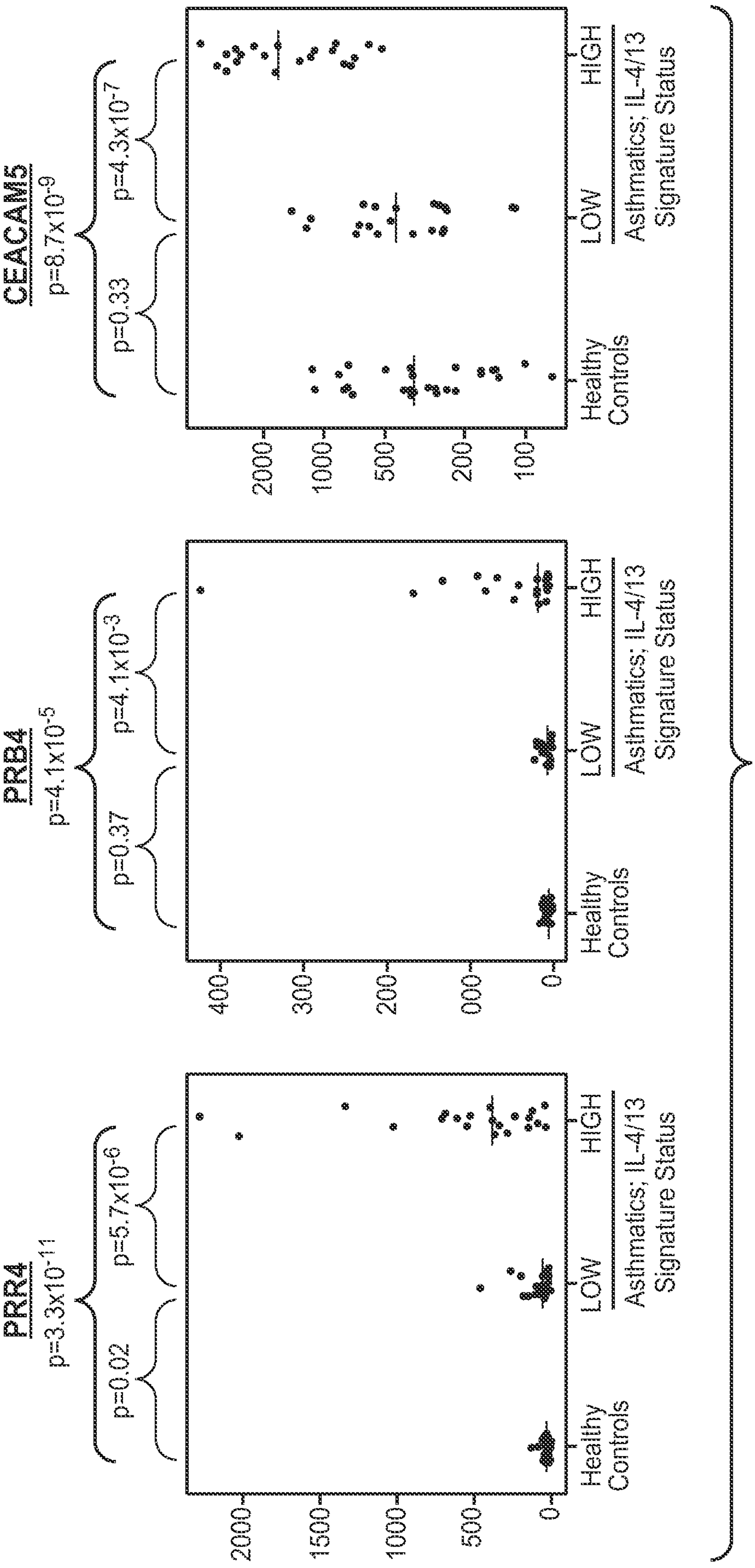
PRR4
p=3.3x10-11
p=0.02
p=5.7x10-6
2000
1500
1000
500
0
Healthy Controls
LOW
HIGH
Asthmatics; IL-4/13 Signature Status
PRB4
p=4.1x10-5
p=0.37
p=4.1x10-3
400
300
200
000
0
Healthy Controls
LOW
HIGH
Asthmatics; IL-4/13 Signature Status
CEACAM5
p=8.7x10-9
p=0.33
p=4.3x10-7
2000
1000
500
200
100
Healthy Controls
LOW
HIGH
Asthmatics; IL-4/13 Signature Status

FIG. 2C

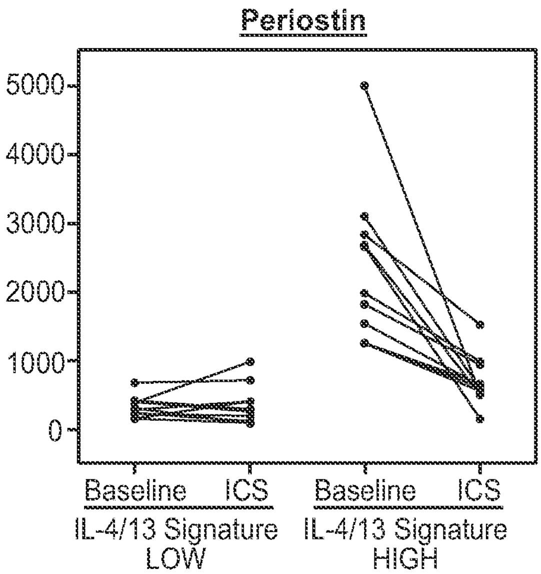


FIG. 3A

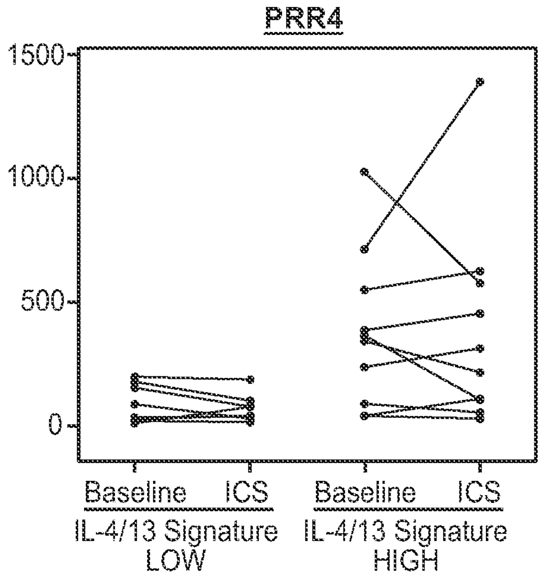


FIG. 3B

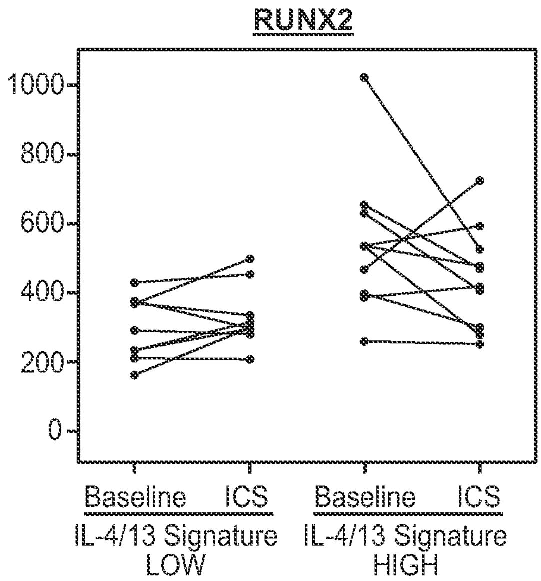


FIG. 3C

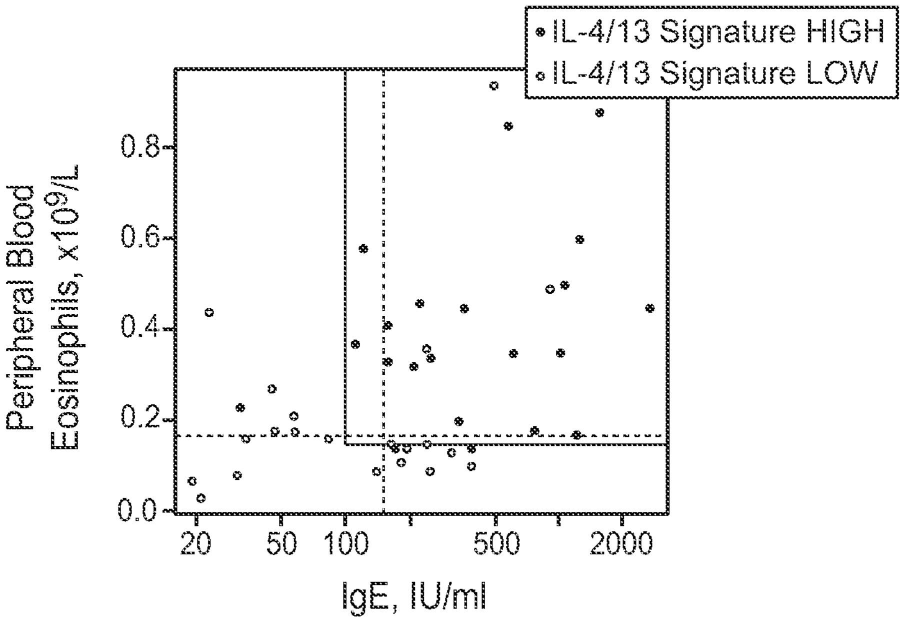
IL-4/13 Signature HIGH
IL-4/13 Signature LOW
Peripheral Blood Eosinophils, x10^9/L
0.0
0.2
0.4
0.6
0.8
20
50
100
500
2000
IgE, IU/ml

*FIG. 4*

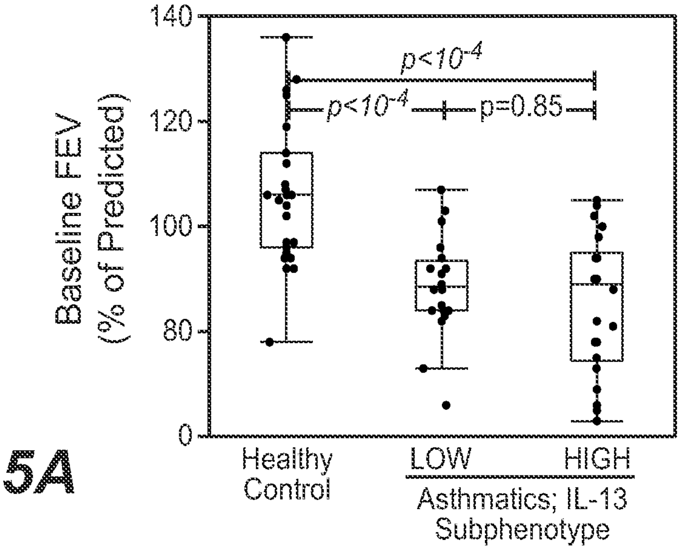


FIG. 5A

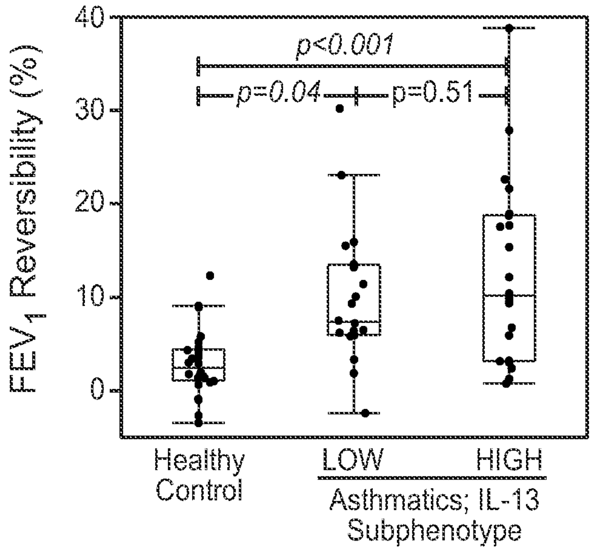


FIG. 5B

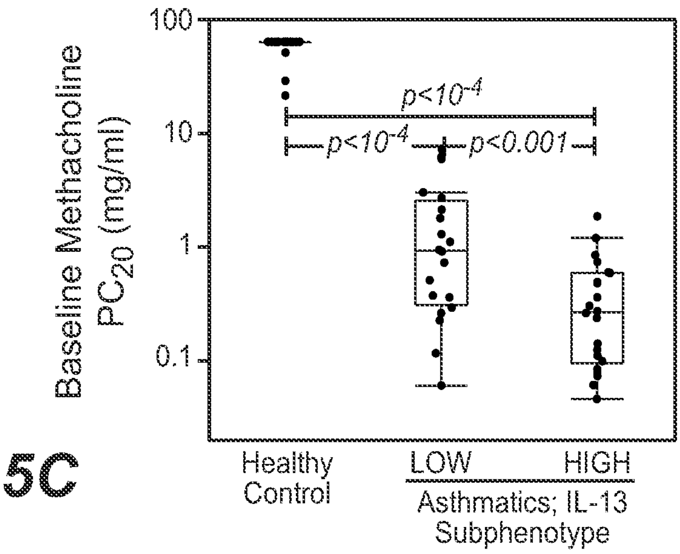


FIG. 5C

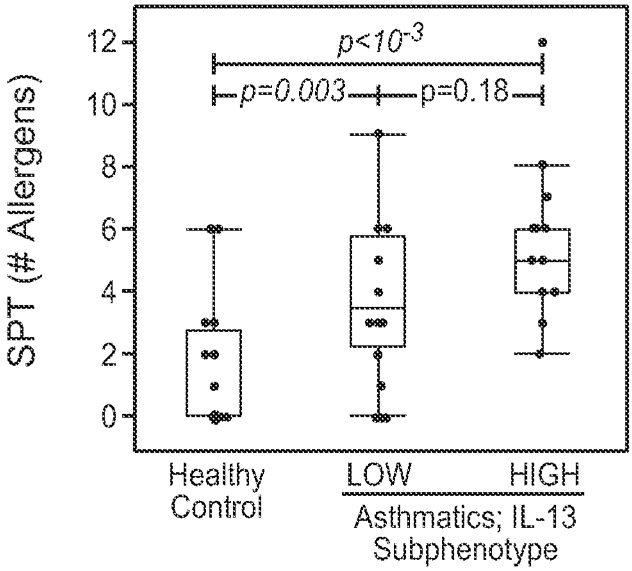
SPT (# Allergens)
12
10
8
6
4
2
0
p<10-3
p=0.003
p=0.18
Healthy Control
LOW
HIGH
Asthmatics; IL-13 Subphenotype

FIG. 6A

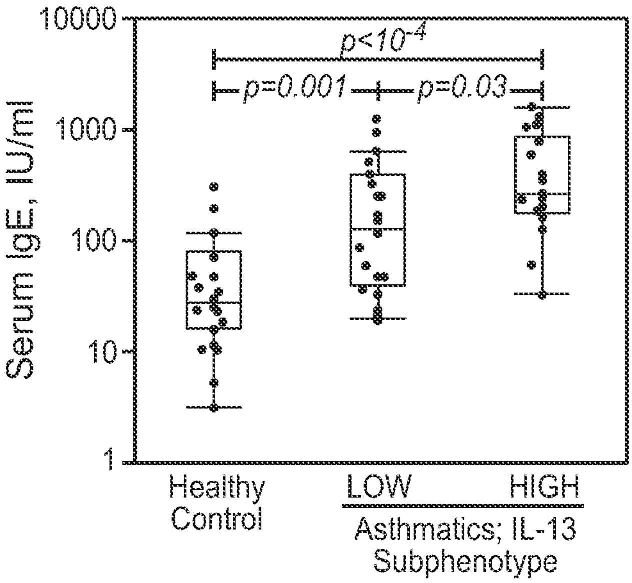
Serum IgE, IU/ml
10000
1000
100
10
1
p<10-4
p=0.001
p=0.03
Healthy Control
LOW
HIGH
Asthmatics; IL-13 Subphenotype

FIG. 6B

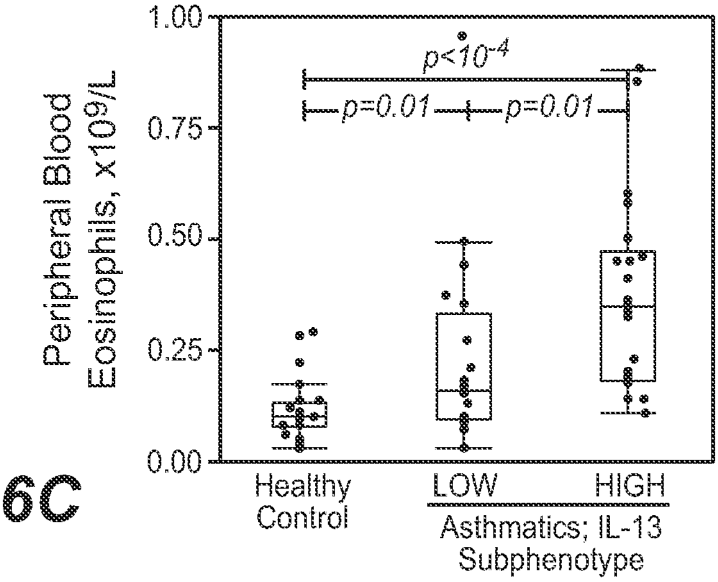
Peripheral Blood Eosinophils, x10^9/L
1.00
0.75
0.50
0.25
0.00
p<10-4
p=0.01
p=0.01
Healthy Control
LOW
HIGH
Asthmatics; IL-13 Subphenotype

FIG. 6C

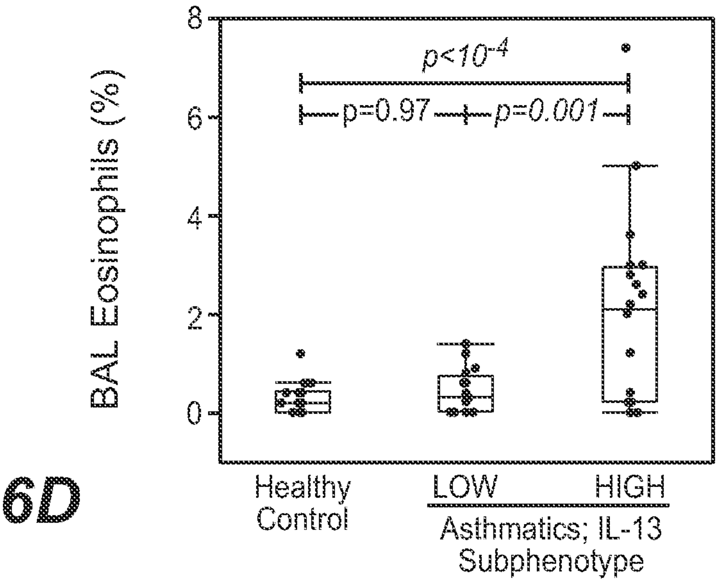
BAL Eosinophils (%)
8
6
4
2
0
p<10-4
p=0.97
p=0.001
Healthy Control
LOW
HIGH
Asthmatics; IL-13 Subphenotype

FIG. 6D

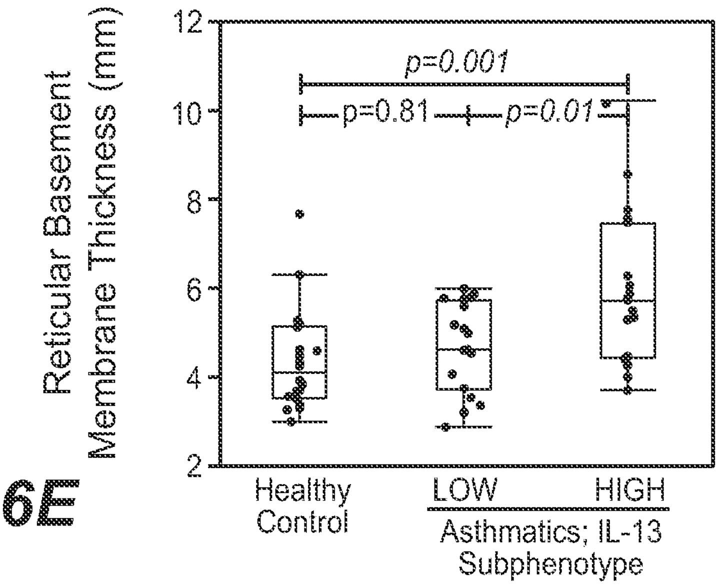
Reticular Basement Membrane Thickness (mm)
12
10
8
6
4
2
p=0.001
p=0.81
p=0.01
Healthy Control
LOW
HIGH
Asthmatics; IL-13 Subphenotype

FIG. 6E

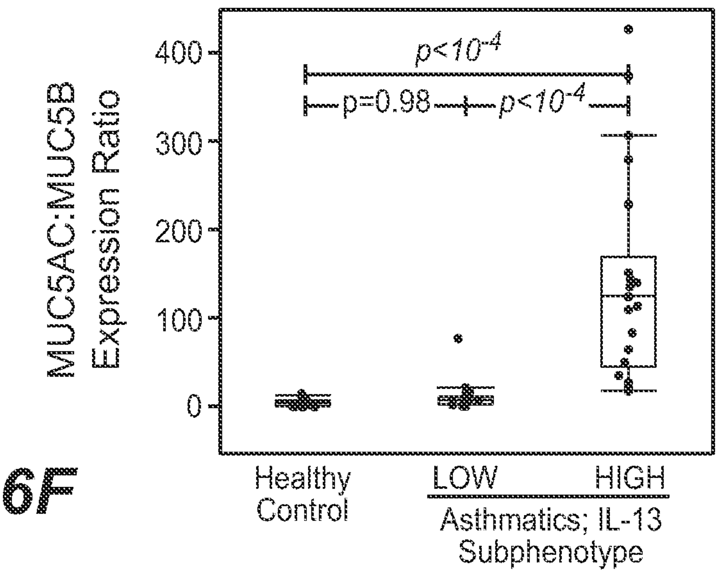
MUC5AC:MUC5B Expression Ratio
400
300
200
100
0
p<10-4
p=0.98
p<10-4
Healthy Control
LOW
HIGH
Asthmatics; IL-13 Subphenotype

FIG. 6F

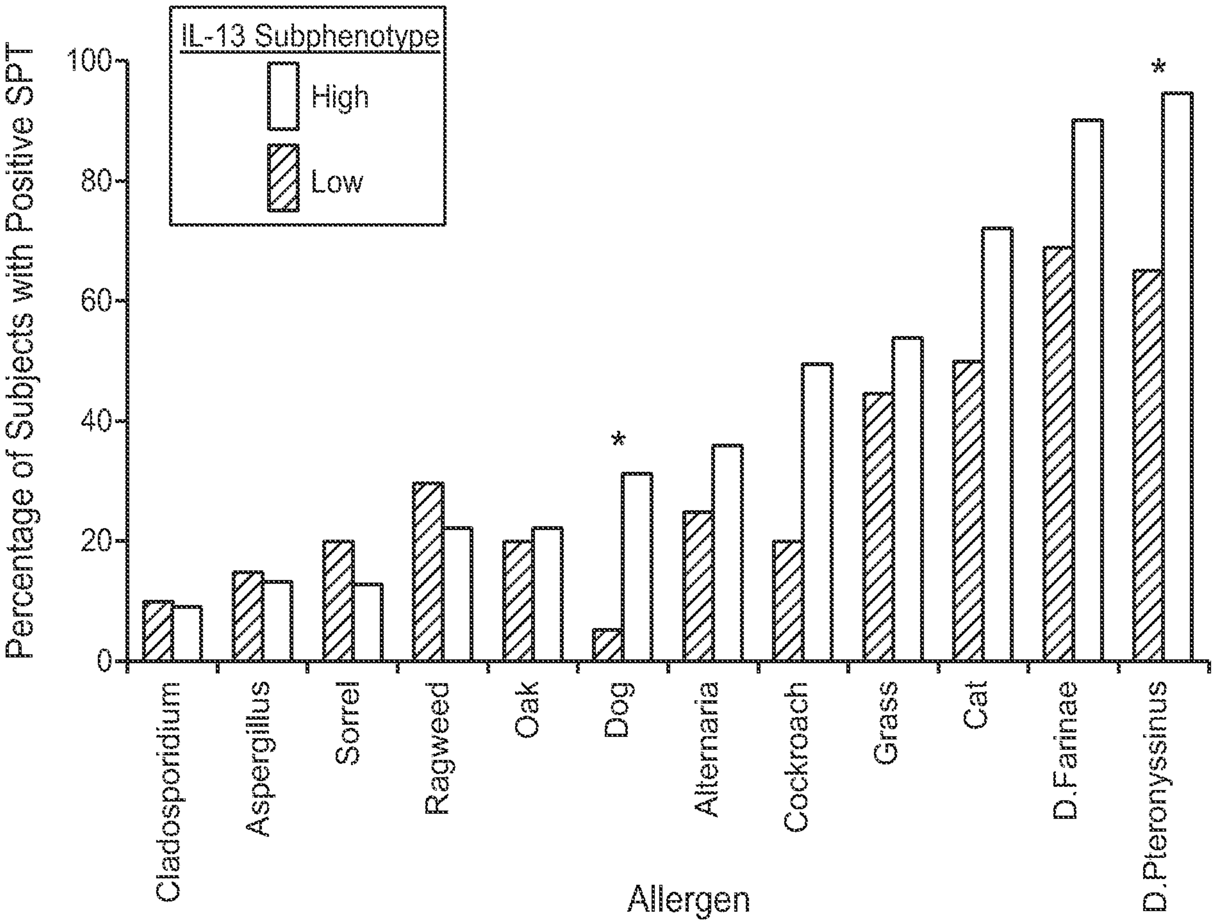
IL-13 Subphenotype
High
Low
Percentage of Subjects with Positive SPT
0
20
40
60
80
100
*
*
Cladosporidium
Aspergillus
Sorrel
Ragweed
Oak
Dog
Alternaria
Cockroach
Grass
Cat
D.Farinae
D.Pteronyssinus
Allergen

*FIG. 7A*

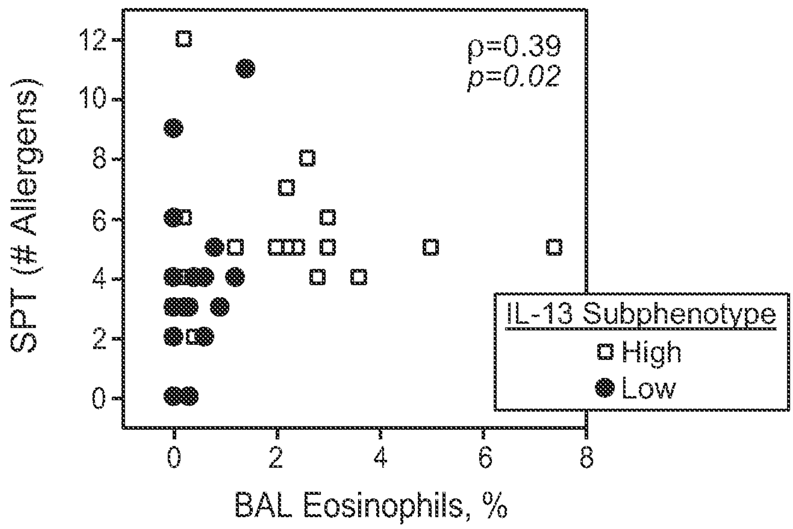


FIG. 7B

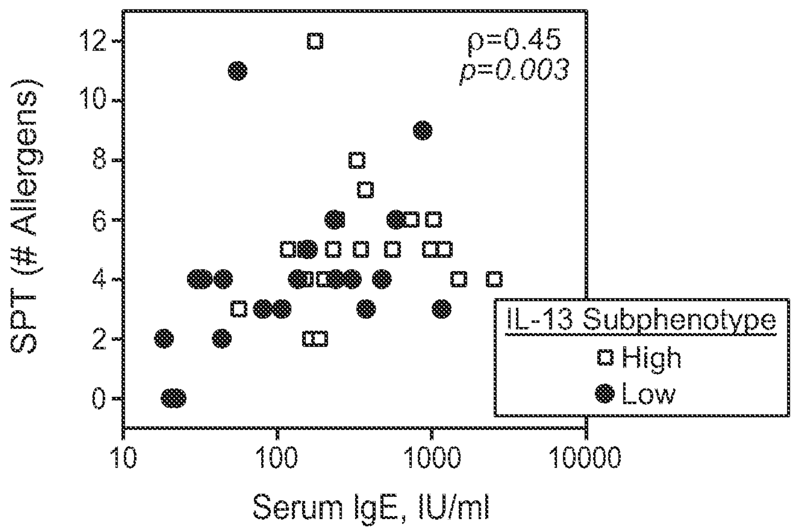


FIG. 7C

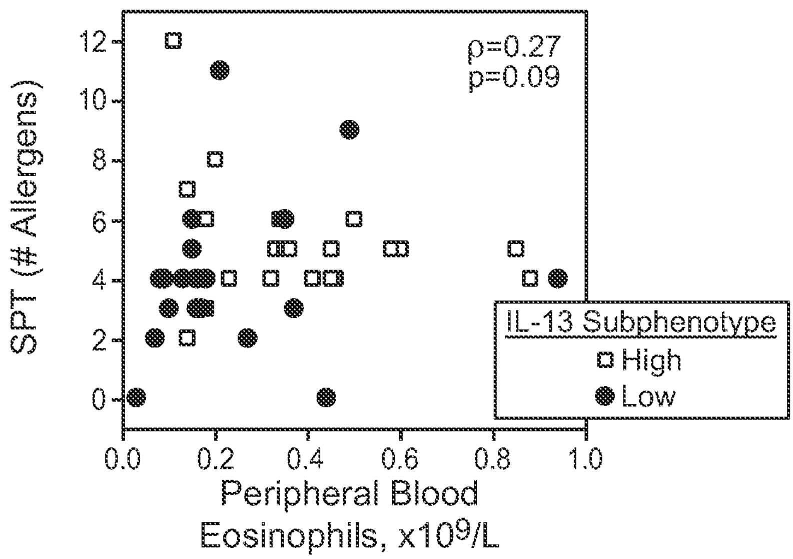


FIG. 7D

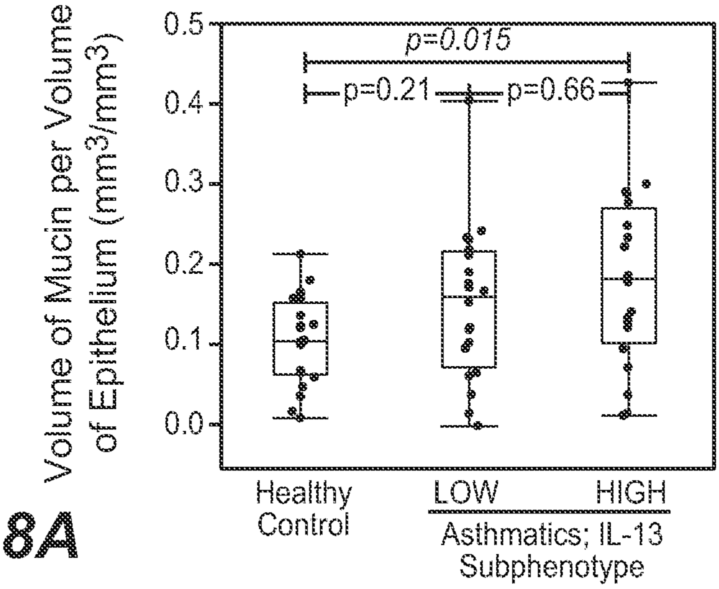
Volume of Mucin per Volume of Epithelium (mm3/mm3)
0.5
0.4
0.3
0.2
0.1
0.0
p=0.015
p=0.21
p=0.66
Healthy Control
LOW
HIGH
Asthmatics; IL-13 Subphenotype

FIG. 8A

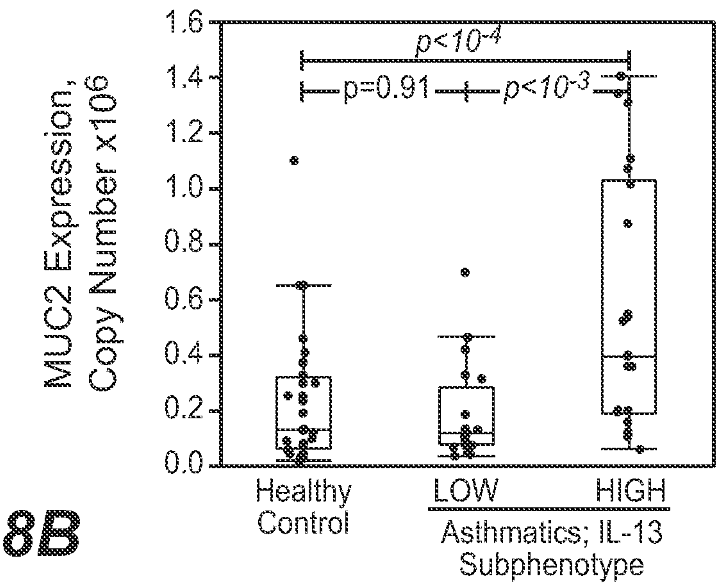
MUC2 Expression, Copy Number x106
1.6
1.4
1.2
1.0
0.8
0.6
0.4
0.2
0.0
p<10-4
p=0.91
p<10-3
Healthy Control
LOW
HIGH
Asthmatics; IL-13 Subphenotype

FIG. 8B

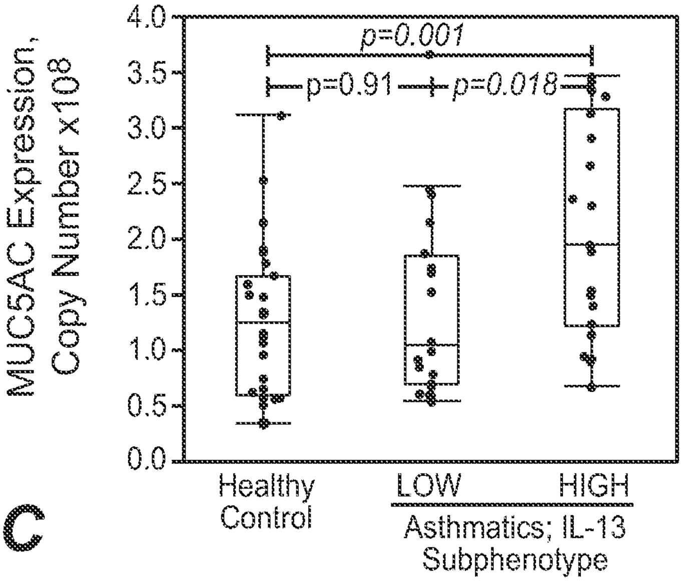
MUC5AC Expression, Copy Number x108
4.0
3.5
3.0
2.5
2.0
1.5
1.0
0.5
0.0
p=0.001
p=0.91
p=0.018
Healthy Control
LOW
HIGH
Asthmatics; IL-13 Subphenotype

FIG. 8C

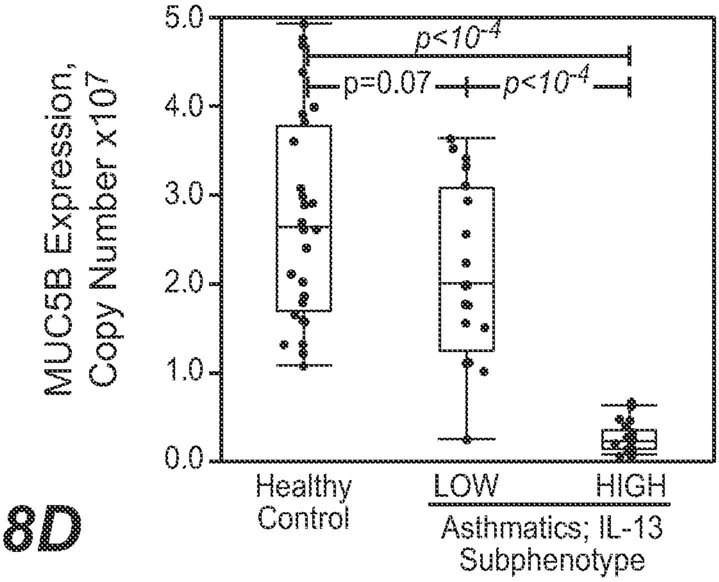
MUC5B Expression, Copy Number x107
5.0
4.0
3.0
2.0
1.0
0.0
p<10-4
p=0.07
p<10-4
Healthy Control
LOW
HIGH
Asthmatics; IL-13 Subphenotype

FIG. 8D

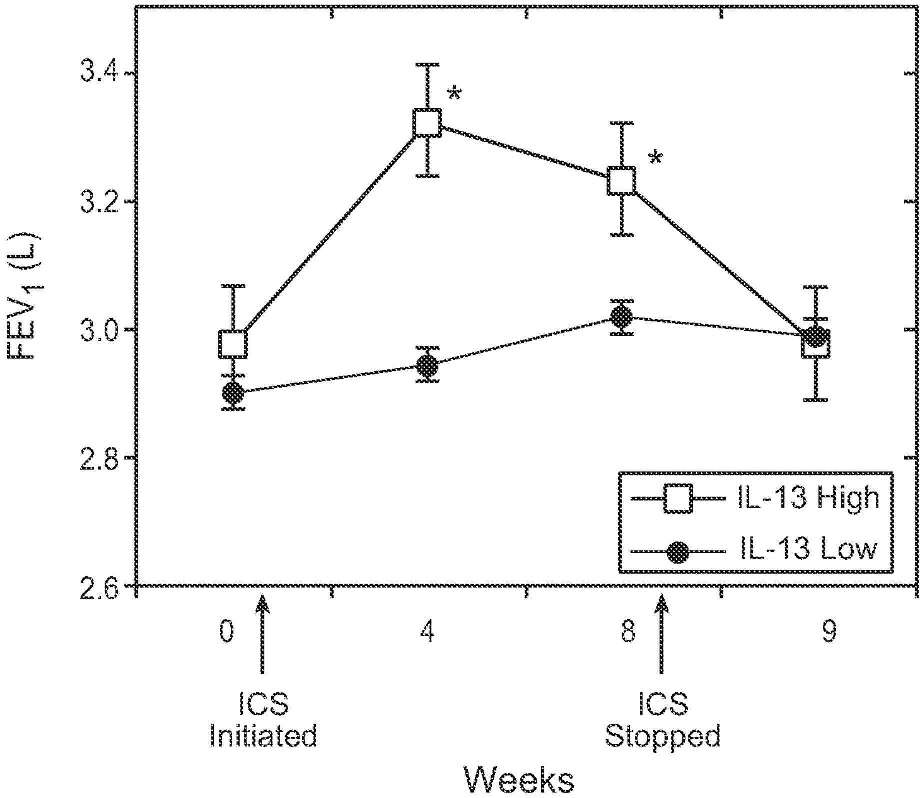
3.4
3.2
3.0
2.8
2.6
FEV$_1$ (L)
*
*
IL-13 High
IL-13 Low
0
4
8
9
ICS
Initiated
ICS
Stopped
Weeks

*FIG. 9A*

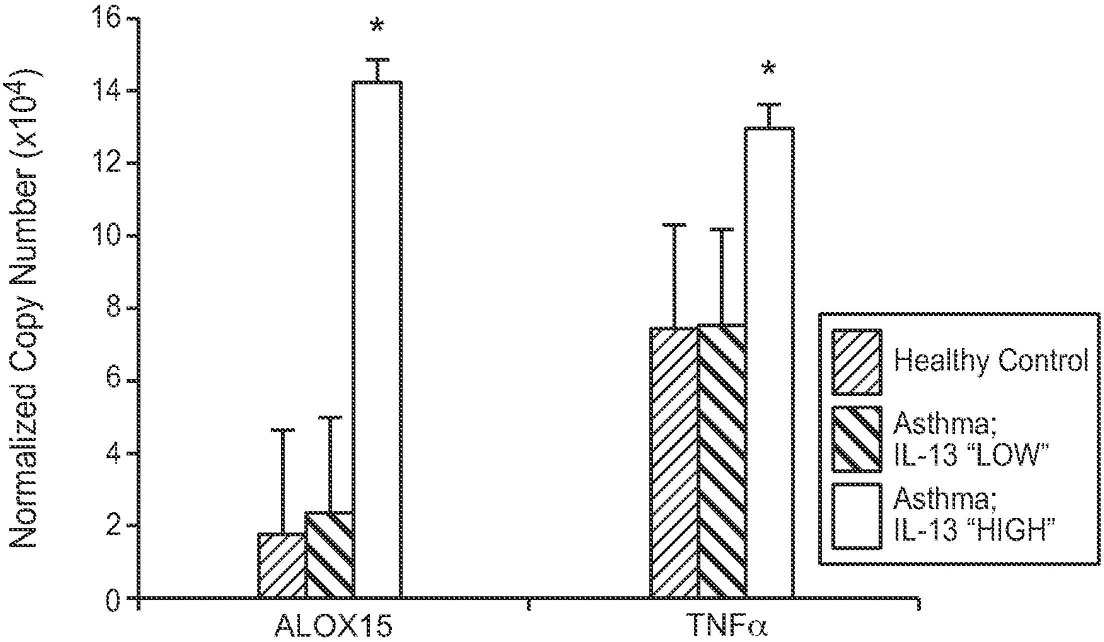
16
14
12
10
8
6
4
2
0
Normalized Copy Number (x10$^4$)
*
*
Healthy Control
Asthma;
IL-13 "LOW"
Asthma;
IL-13 "HIGH"
ALOX15
TNFα

*FIG. 10*

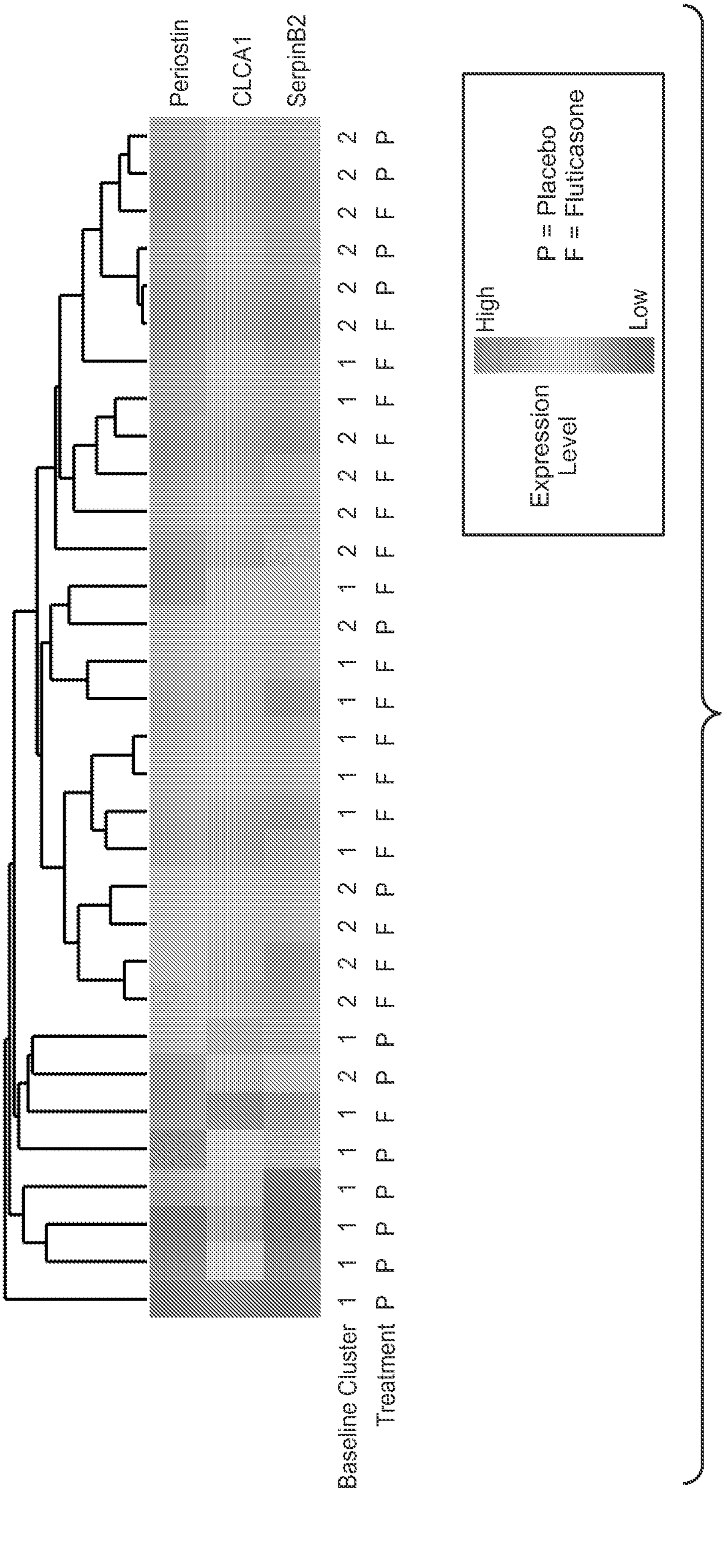
Periostin
CLCA1
SerpinB2
Baseline Cluster 1 1 1 1 1 1 2 1 2 2 2 2 1 1 1 1 1 1 2 1 2 2 2 2 1 1 2 2 2 2 2 2
Treatment P P P P P F P P F F F P F F F F F F P F F F F F F F F P P F P P
Expression Level
High
Low
P = Placebo
F = Fluticasone

FIG. 9B

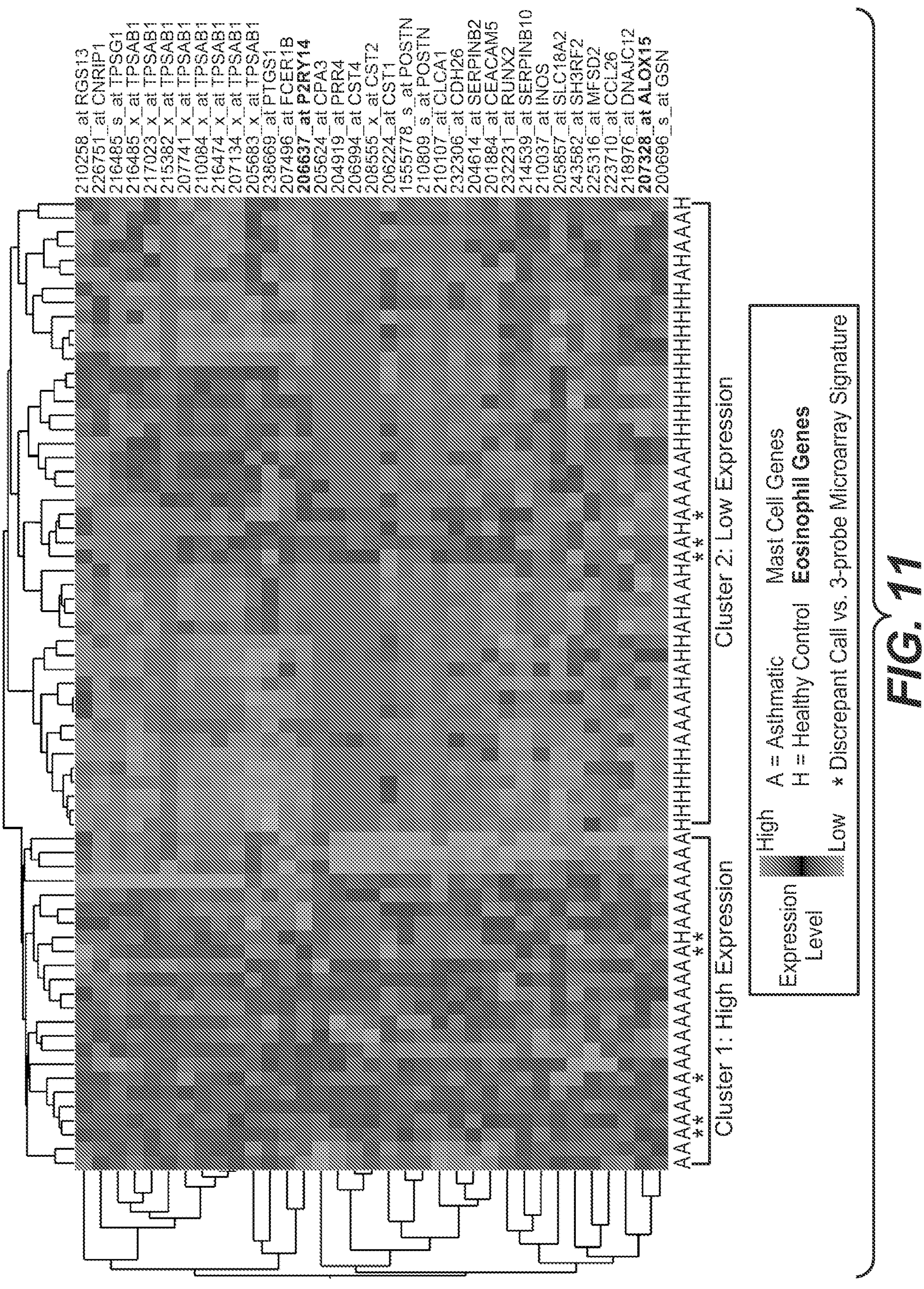
210258_at RGS13
226751_at CNRIP1
216485_s_at TPSG1
216485_x_at TPSAB1
217023_x_at TPSAB1
215382_x_at TPSAB1
207741_x_at TPSAB1
210084_x_at TPSAB1
216474_x_at TPSAB1
207134_x_at TPSAB1
205683_x_at TPSAB1
238669_at PTGS1
207496_at FCER1B
206637_at P2RY14
205624_at CPA3
204919_at PRR4
206994_at CST4
208555_x_at CST2
206224_at CST1
1555778_s_at POSTN
210809_s_at POSTN
210107_at CLCA1
232306_at CDH26
204614_at SERPINB2
201884_at CEACAM5
232231_at RUNX2
214539_at SERPINB10
210037_at INOS
205857_at SLC18A2
243582_at SH3RF2
225316_at MFSD2
223710_at CCL26
218976_at DNAJC12
207328_at ALOX15
200696_s_at GSN
Cluster 1: High Expression
Cluster 2: Low Expression
Expression Level
High
Low
A = Asthmatic
H = Healthy Control
Mast Cell Genes
Eosinophil Genes
* Discrepant Call vs. 3-probe Microarray Signature

*FIG. 11*

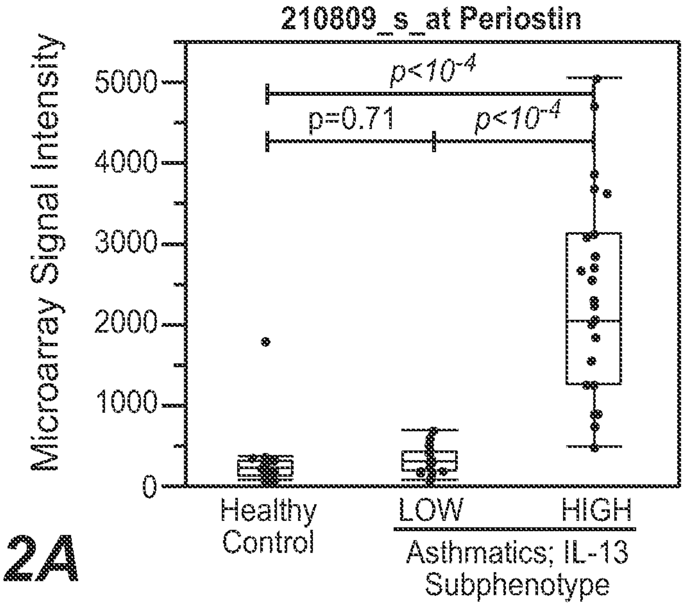


FIG. 12A

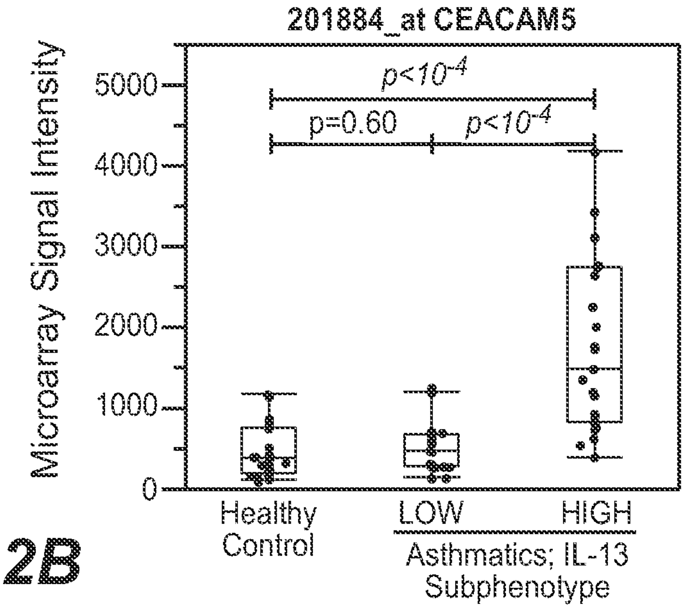


FIG. 12B

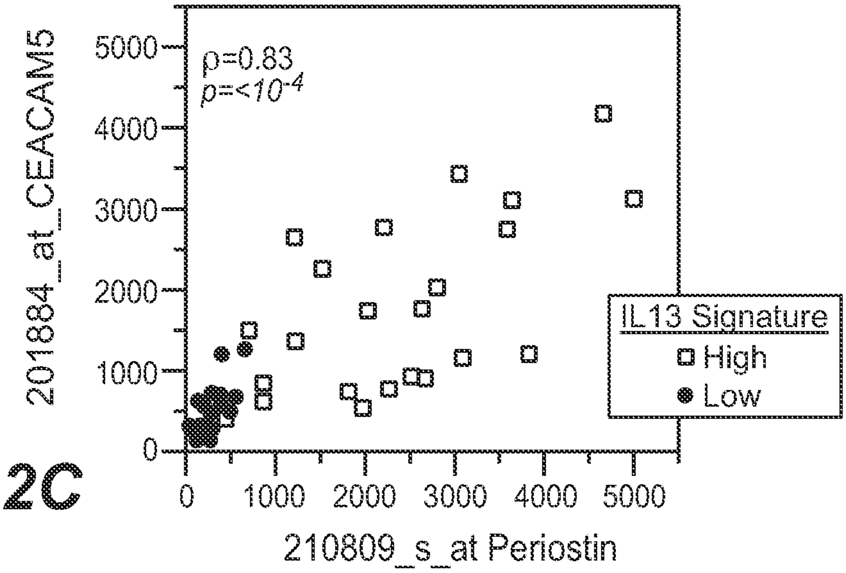


FIG. 12C

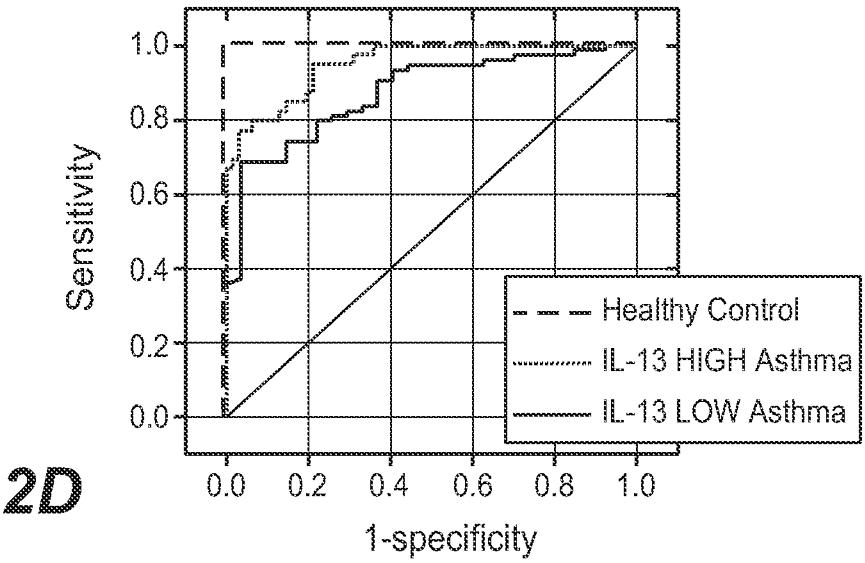


FIG. 12D

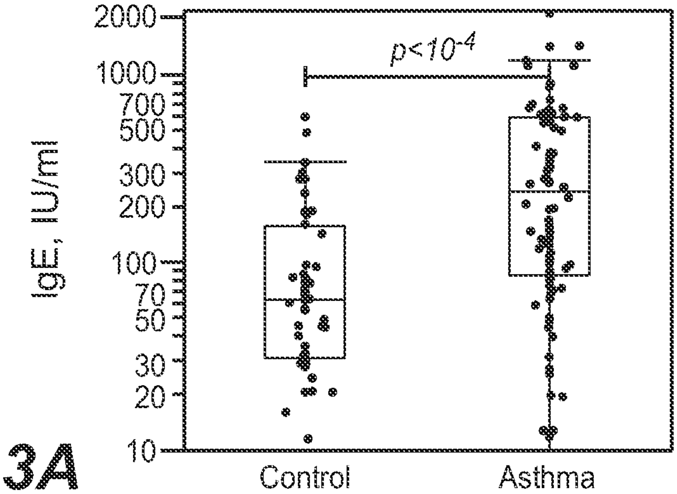


FIG. 13A

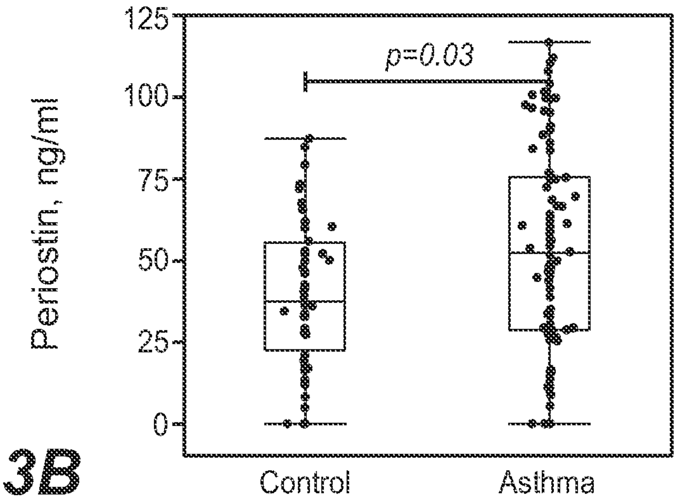


FIG. 13B

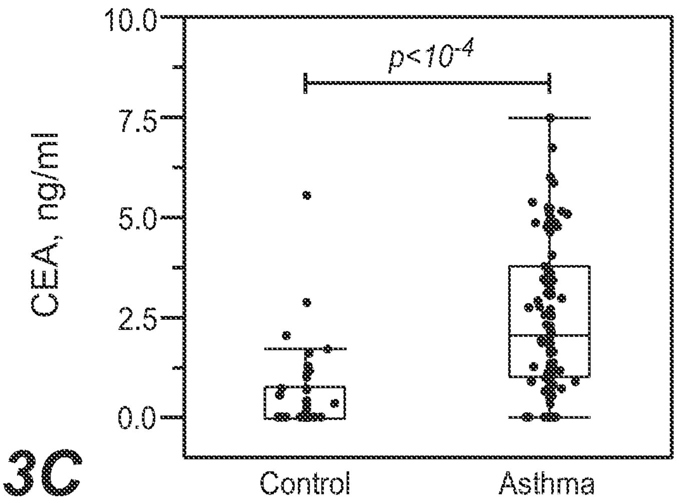


FIG. 13C

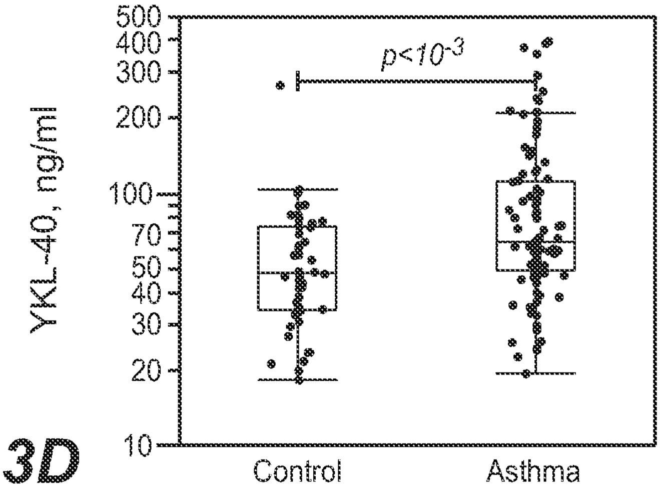


FIG. 13D

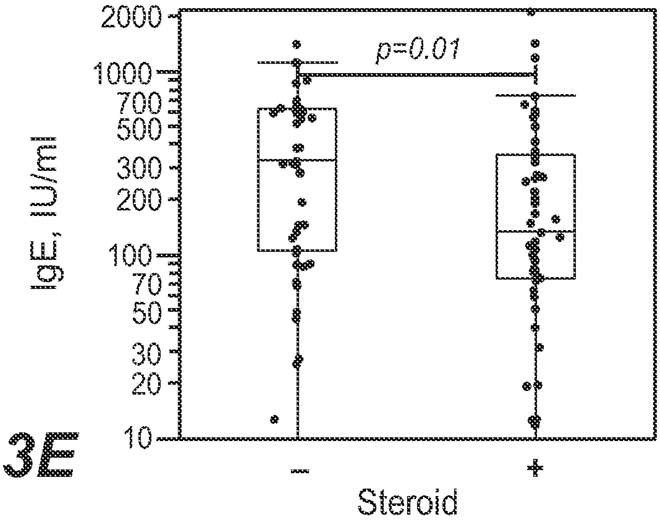


FIG. 13E

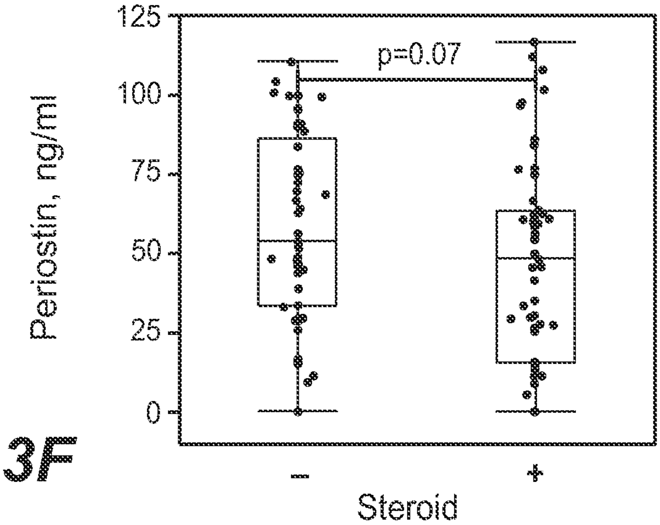


FIG. 13F

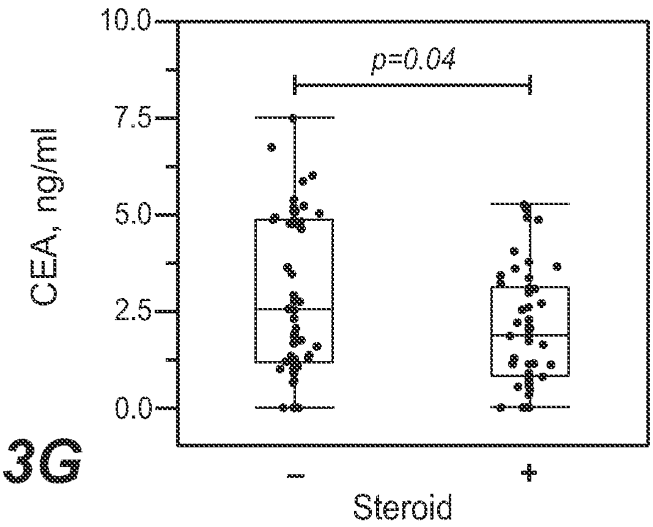


FIG. 13G

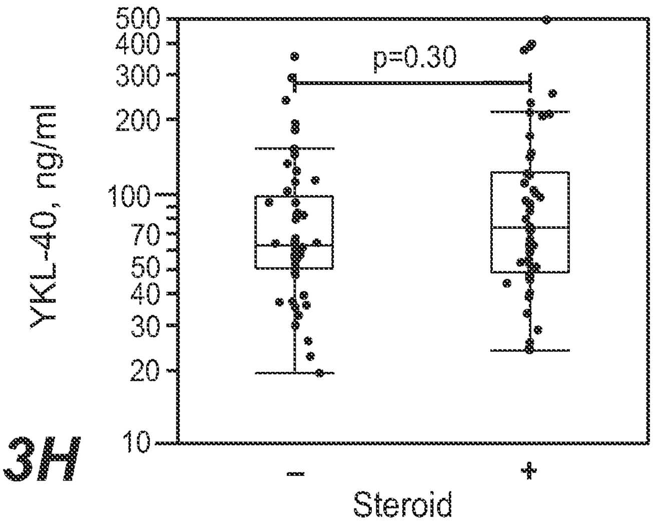


FIG. 13H

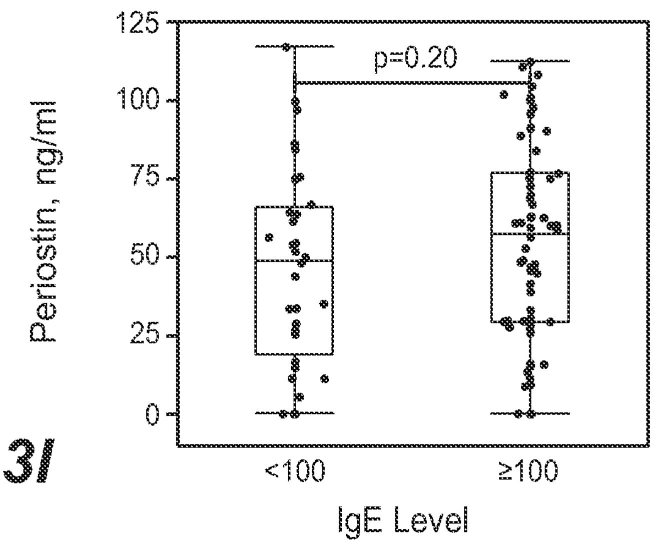


FIG. 13I

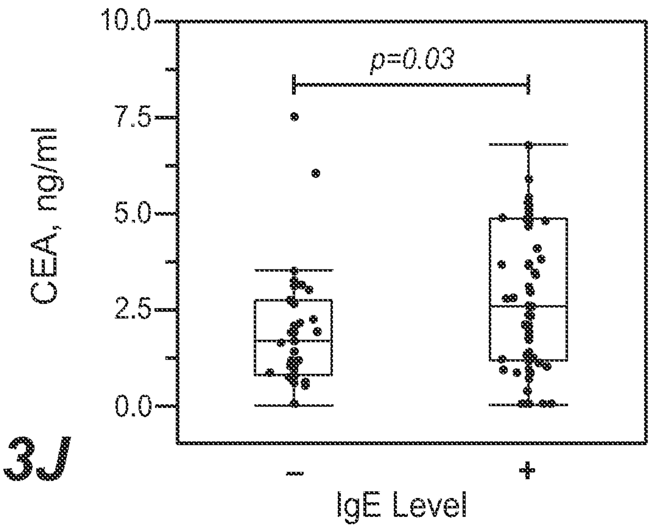


FIG. 13J

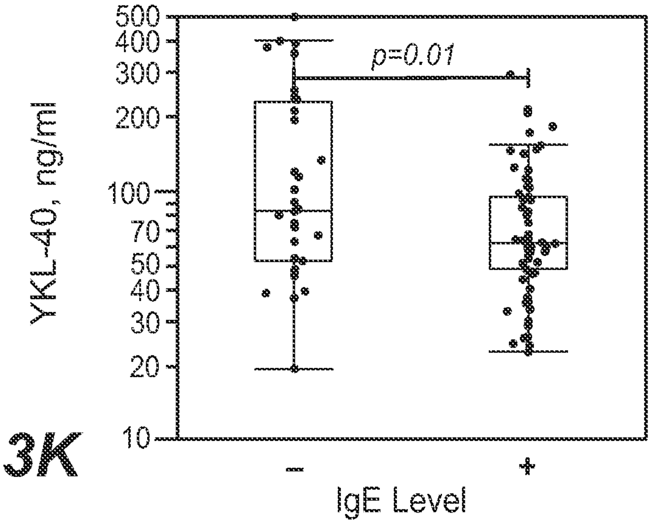


FIG. 13K

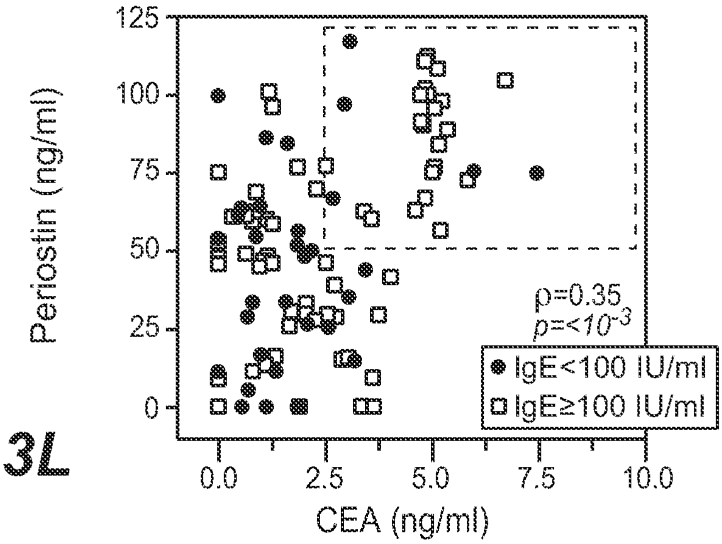


FIG. 13L

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005062972 A
- WO 2008083695 A
- US 20080267959 A
- US 20080044420 A
- US 20080248048 A
- WO 2008116149 A
- US 4816567 A
- WO 9824893 A
- WO 9634096 A
- WO 9633735 A
- WO 9110741 A
- US 5545806 A
- US 5569825 A
- US 5591669 A
- US 5545807 A
- WO 9717852 A
- US 5625126 A
- US 5633425 A
- US 5661016 A
- WO 9201047 A
- EP 0598877 A
- US 5413923 A
- US 5814318 A
- US 5885793 A
- US 5916771 A
- US 5939598 A
- US 60640323 B
- WO 61205392 A
- WO 61128383 A
- WO 61041480 A
- WO 61072572 A


### Non-patent literature cited in the description


- **SAMBROOK et al.** Molecular Cloning: A Laboratory Manual. Cold Spring Harbor Laboratory Press, 1989
- **KABAT et al.** Sequences of Proteins of Immunological Interest. Public Health Service, National Institutes of Health, 1991
- **CHOTHIA ; LESK.** *J. Mol. Biol.,* 1987, vol. 196, 901-917
- **KOHLER et al.** *Nature,* 1975, vol. 256, 495
- **HARLOW et al.** Antibodies: A Laboratory Manual. Cold Spring Harbor Laboratory Press, 1988
- **HAMMERLING et al.** Monoclonal Antibodies and T-Cell Hybridomas. Elsevier, 1981, 563-681
- **CLACKSON et al.** *Nature,* 1991, vol. 352, 624-628
- **MARKS et al.** *J. Mol. Biol,* 1991, vol. 222, 581-597
- **SIDHU et al.** *J. Mol. Biol.,* 2004, vol. 338 (2), 299-310
- **LEE et al.** *J.Mol.Biol.,* 2004, vol. 340 (5), 1073-1093
- **FELLOUSE.** *Proc. Nat. Acad. Sci. USA,* 2004, vol. 101 (34), 12467-12472
- **LEE et al.** *J. Immunol. Methods,* 2004, vol. 284 (1-2), 119-132
- **JAKOBOVITS et al.** *Proc. Natl. Acad. Sci. USA,* 1993, vol. 90, 2551
- **JAKOBOVITS et al.** *Nature,* 1993, vol. 362, 255-258
- **BRUGGEMANN et al.** *Year in Immuno.,* 1993, vol. 7, 33
- **MARKS et al.** *Bio/Technology,* 1992, vol. 10, 779-783
- **LONBERG et al.** *Nature,* 1994, vol. 368, 856-859
- **MORRISON.** *Nature,* 1994, vol. 368, 812-813
- **FISHWILD et al.** *Nature Biotechnology,* 1996, vol. 14, 845-851
- **NEUBERGER.** *Nature Biotechnology,* 1996, vol. 14, 826
- **LONBERG ; HUSZAR.** *Intern. Rev. Immunol.,* 1995, vol. 13, 65-93
- **MORRISON et al.** *Proc. Natl. Acad. Sci. USA,* 1984, vol. 81, 6851-6855
- **JONES et al.** *Nature,* 1986, vol. 321, 522-525
- **REICHMANN et al.** *Nature,* 1988, vol. 332, 323-329
- **PRESTA.** *Curr. Op. Struct. Biol.,* 1992, vol. 2, 593-596
- **HOOGENBOOM ; WINTER.** *J. Mol. Biol.,* 1991, vol. 227, 381
- **MARKS et al.** *J. Mol. Biol.,* 1991, vol. 222, 581
- **COLE et al.** Monoclonal Antibodies and Cancer Therapy. Alan R. Liss, 1985, 77
- **BOERNER et al.** *J. Immunol.,* 1991, vol. 147 (1), 86-95
- **LONBERG ; HUSZAR.** *Int. Rev. Immunol.,* 1995, vol. 13, 65-93

- **KABAT et al.** Sequences of Immunological Interest. Public Health Service, National Institutes of Health, 1991
- **AUSUBEL et al.** Current Protocols in Molecular Biology. Wiley Interscience Publishers, 1995
- **SAMBROOK et al.** Molecular Cloning: A Laboratory Manual. Cold Spring Harbor Press, 1989
- **HALDAR, P. ; I.D. PAVORD.** Noneosinophilic asthma: a distinct clinical and pathologic phenotype. *J Allergy Clin Immunol,* 2007, vol. 119 (5), 1043-52
- **HERSHEY, G.K.** IL-13 receptors and signaling pathways: an evolving web. *J Allergy Clin Immunol,* 2003, vol. 111 (4), 677-90
- **BERRY, M.A. et al.** Sputum and bronchial submucosal IL-13 expression in asthma and eosinophilic bronchitis. *J Allergy Clin Immunol,* 2004, vol. 114 (5), 1106-9
- **HUMBERT, M. et al.** Elevated expression of messenger ribonucleic acid encoding IL-13 in the bronchial mucosa of atopic and nonatopic subjects with asthma. *J Allergy Clin Immunol,* 1997, vol. 99 (5), 657-65
- **TRUYEN, E. et al.** Evaluation of airway inflammation by quantitative Th1/Th2 cytokine mRNA measurement in sputum of asthma patients. *Thorax,* 2006, vol. 61 (3), 202-8
- **SAHA, S.K. et al.** Increased sputum and bronchial biopsy IL-13 expression in severe asthma. *J Allergy Clin Immunol,* 2008, vol. 121 (3), 685-91
- **YUYAMA, N. et al.** Analysis of novel disease-related genes in bronchial asthma. *Cytokine,* 2002, vol. 19 (6), 287-96
- **WOODRUFF, P.G. et al.** Genome-wide profiling identifies epithelial cell genes associated with asthma and with treatment response to corticosteroids. *Proc Natl Acad Sci U S A,* 2007, vol. 104 (40), 15858-63
- **TAKAYAMA, G. et al.** Periostin: a novel component of subepithelial fibrosis of bronchial asthma downstream of IL-4 and IL-13 signals. *J Allergy Clin Immunol,* 2006, vol. 118 (1), 98-104
- **HAYASHI, N. et al.** T helper 1 cells stimulated with ovalbumin and IL-18 induce airway hyperresponsiveness and lung fibrosis by IFN-gamma and IL-13 production. *Proc Natl Acad Sci USA,* 2007, vol. 104 (37), 14765-70
- **BLANCHARD, C. et al.** IL-13 involvement in eosinophilic esophagitis: transcriptome analysis and reversibility with glucocorticoids. *J Allergy Clin Immunol,* 2007, vol. 120 (6), 1292-300
- **NAKANISHI, A. et al.** Role of gob-5 in mucus overproduction and airway hyperresponsiveness in asthma. *Proc Natl Acad Sci USA,* 2001, vol. 98 (9), 5175-80
- **ZHOU, Y. et al.** Characterization of a calcium-activated chloride channel as a shared target of Th2 cytokine pathways and its potential involvement in asthma. *Am J Respir Cell Mol Biol,* 2001, vol. 25 (4), 486-91
- **KUPERMAN, D.A. et al.** Dissecting asthma using focused transgenic modeling and functional genomics. *J Allergy Clin Immunol,* 2005, vol. 116 (2), 305-11
- **RAY, R. et al.** Uteroglobin suppresses SCCA gene expression associated with allergic asthma. *J Biol Chem,* 2005, vol. 280 (11), 9761-4
- **SABATINI, L.M. et al.** Tissue distribution of RNAs for cystatins, histatins, statherin, and proline-rich salivary proteins in humans and macaques. *J Dent Res,* 1989, vol. 68 (7), 1138-45
- **LINDAHL, M. ; B. STAHLBOM ; C. TAGESSON.** Newly identified proteins in human nasal and bronchoalveolar lavage fluids: potential biomedical and clinical applications. *Electrophoresis,* 1999, vol. 20 (18), 3670-6
- **CIMERMAN, N. et al.** Serum cystatin C, a potent inhibitor of cysteine proteinases, is elevated in asthmatic patients. *Clin Chim Acta,* 2000, vol. 300 (1-2), 83-95
- **FINKELMAN, F.D. et al.** Suppressive effect of IL-4 on IL-13-induced genes in mouse lung. *J Immunol,* 2005, vol. 174 (8), 4630-8
- **ZIMMERMANN, N. et al.** Expression and regulation of small proline-rich protein 2 in allergic inflammation. *Am J Respir Cell Mol Biol,* 2005, vol. 32 (5), 428-35
- **WARNER, T.F. ; E.A. AZEN.** Proline-rich proteins are present in serous cells of submucosal glands in the respiratory tract. *Am Rev Respir Dis,* 1984, vol. 130 (1), 115-8
- **MAEDA, Y. et al.** *Concentrations of carcinoembryonic antigen in serum and bronchoalveolar lavage fluid of asthmatic patients with mucoid impaction]. Nihon Kokyuki Gakkai Zasshi,* 2004, vol. 42 (12), 988-93
- **SURESH, V. ; J.D. MIH ; S.C. GEORGE.** Measurement of IL-13-induced iNOS-derived gas phase nitric oxide in human bronchial epithelial cells. *Am J Respir Cell Mol Biol,* 2007, vol. 37 (1), 97-104
- **STOREY, J.D. ; R. TIBSHIRANI.** Statistical significance for genomewide studies. *Proc Natl Acad Sci USA,* 2003, vol. 100 (16), 9440-5
- **EISEN, M.B. et al.** Cluster analysis and display of genome-wide expression patterns. *Proc Natl Acad Sci USA,* 1998, vol. 95 (25), 14863-8
- **SALDANHA, A.J.** Java Treeview--extensible visualization of microarray data. *Bioinformatics,* 2004, vol. 20 (17), 3246-8
- **KELLY-WELCH, A. E. ; HANSON, E. M. ; BOOTHBY, M. R. ; KEEGAN, A. D.** Interleukin-4 and interleukin-13 signaling connections maps. *Science,* 2003, vol. 300, 1527-8

- **FIXMAN, E. D. ; STEWART, A. ; MARTIN, J. G.** Basic mechanisms of development of airway structural changes in asthma. *Eur Respir J,* 2007, vol. 29, 379-89
- **HOLGATE, S. T.** Pathogenesis of asthma. *Clin Exp Allergy,* 2008, vol. 38, 872-97
- **ROCHE, W. R. ; BEASLEY, R. ; WILLIAMS, J. H. ; HOLGATE, S. T.** Subepithelial fibrosis in the bronchi of asthmatics. *Lancet,* 1989, vol. 1, 520-4
- **FERRANDO, R. E. ; NYENGAARD, J. R. ; HAYS, S. R. ; FAHY, J. V. ; WOODRUFF, P. G.** Applying stereology to measure thickness of the basement membrane zone in bronchial biopsy specimens. *J Allergy Clin Immunol,* 2003, vol. 112, 1243-5
- **ORDONEZ, C. L. et al.** Mild and moderate asthma is associated with airway goblet cell hyperplasia and abnormalities in mucin gene expression. *Am J Respir Crit Care Med,* 2001, vol. 163, 517-23
- **MARTIN, R. J. et al.** The Predicting Response to Inhaled Corticosteroid Efficacy (PRICE) trial. *J Allergy Clin Immunol,* 2007, vol. 119, 73-80
- **WENZEL, S. ; WILBRAHAM, D. ; FULLER, R. ; GETZ, E. B. ; LONGPHRE, M.** Effect of an interleukin-4 variant on late phase asthmatic response to allergen challenge in asthmatic patients: results of two phase 2a studies. *Lancet,* 2007, vol. 370, 1422-31
- **GRUNIG, G. et al.** Requirement for IL-13 independently of IL-4 in experimental asthma. *Science,* 1998, vol. 282, 2261-3
- **WILLS-KARP, M. et al.** Interleukin-13: central mediator of allergic asthma. *Science,* 1998, vol. 282, 2258-61
- **SIMPSON, J. L. ; SCOTT, R. ; BOYLE, M. J. ; GIBSON, P. G.** Inflammatory subtypes in asthma: assessment and identification using induced sputum. *Respirology,* 2006, vol. 11, 54-61
- **WENZEL, S. E. et al.** Evidence that severe asthma can be divided pathologically into two inflammatory subtypes with distinct physiologic and clinical characteristics. *Am J Respir Crit Care Med,* 1999, vol. 160, 1001-8
- **WENZEL, S. E.** Asthma: defining of the persistent adult phenotypes. *Lancet,* 2006, vol. 368, 804-13
- **BERRY, M. et al.** Pathological features and inhaled corticosteroid response of eosinophilic and non-eosinophilic asthma. *Thorax,* 2007, vol. 62, 1043-9
- **PAVORD, I. D. ; BRIGHTLING, C. E. ; WOLTMANN, G. ; WARDLAW, A. J.** Non-eosinophilic corticosteroid unresponsive asthma. *Lancet,* 1999, vol. 353, 2213-4
- **MCKINLEY, L. et al.** TH17 cells mediate steroid-resistant airway inflammation and airway hyperresponsiveness in mice. *J Immunol,* 2008, vol. 181, 4089-97
- **HOLGATE, S. T.** Epithelium dysfunction in asthma. *J Allergy Clin Immunol,* vol. 120, 1233-44
- **MARTIN, R. J. ; KRAFT, M. ; CHU, H. W. ; BERNS, E. A. ; CASSELL, G. H.** A link between chronic asthma and chronic infection. *J Allergy Clin Immunol,* 2001, vol. 107, 595-601
- **DOLGANOV, G. M. et al.** A novel method of gene transcript profiling in airway biopsy homogenates reveals increased expression of a Na+-K+-Cl- cotransporter (NKCC1) in asthmatic subjects. *Genome Res,* 2001, vol. 11, 1473-83
- **INNES, A. L. et al.** Epithelial mucin stores are increased in the large airways of smokers with airflow obstruction. *Chest,* 2006, vol. 130, 1102-8
- **GENTLEMAN, R. C. et al.** Bioconductor: open software development for computational biology and bioinformatics. *Genome Biol,* 2004, vol. 5, R80
- **DOUWES J ; GIBSON P ; PEKKANEN J ; PEARCE N.** Non-eosinophilic asthma: importance and possible mechanisms. *Thorax,* 2002, vol. 57 (7), 643-8
- **GALLI SJ ; TSAI M ; PILIPONSKY AM.** The development of allergic inflammation. *Nature,* 2008, vol. 454 (7203), 445-54
- **BARNES PJ.** The cytokine network in asthma and chronic obstructive pulmonary disease. *J Clin Invest,* 2008, vol. 118 (11), 3546-56
- **WOODRUFF PG ; KOTH LL ; YANG YH et al.** A distinctive alveolar macrophage activation state induced by cigarette smoking. *Am J Respir Crit Care Med,* 2005, vol. 172 (11), 1383-92
- **WEIBEL ER ; HSIA CC ; OCHS M.** How much is there really? Why stereology is essential in lung morphometry. *J Appl Physiol,* 2007, vol. 102 (1), 459-67
- **SEIBOLD MA ; DONNELLY S ; SOLON M et al.** Chitotriosidase is the primary active chitinase in the human lung and is modulated by genotype and smoking habit. *J Allergy Clin Immunol,* 2008, vol. 122 (5), 944-50 e3
- **KIM EY ; BATTAILE JT ; PATEL AC et al.** Persistent activation of an innate immune response translates respiratory viral infection into chronic lung disease. *Nat Med,* 2008, vol. 14 (6), 633-40
- **PETERS-GOLDEN M.** The alveolar macrophage: the forgotten cell in asthma. *Am J Respir Cell Mol Biol,* 2004, vol. 31 (1), 3-7
- **CHU HW ; BALZAR S ; WESTCOTT JY et al.** Expression and activation of 15-lipoxygenase pathway in severe asthma: relationship to eosinophilic phenotype and collagen deposition. *Clin Exp Allergy,* 2002, vol. 32 (11), 1558-65
- **GORDON S.** Alternative activation of macrophages. *Nat Rev Immunol,* 2003, vol. 3 (1), 23-35
- **ANDERSON GP.** Endotyping asthma: new insights into key pathogenic mechanisms in a complex, heterogeneous disease. *Lancet,* 2008, vol. 372 (9643), 1107-19

- **BERRY MA ; HARGADON B ; SHELLEY M et al.** Evidence of a role of tumor necrosis factor alpha in refractory asthma. *N Engl J Med,* 2006, vol. 354 (7), 697-708
- **TAKATSU K ; NAKAJIMA H.** IL-5 and eosinophilia. *Curr Opin Immunol,* 2008, vol. 20 (3), 288-94
- **ZIMMERMANN N ; HERSHEY GK ; FOSTER PS ; ROTHENBERG ME.** Chemokines in asthma: cooperative interaction between chemokines and IL-13. *J Allergy Clin Immunol,* 2003, vol. 111 (2), 227-42
- **FLOOD-PAGE PT ; MENZIES-GOW AN ; KAY AB ; ROBINSON DS.** Eosinophil's role remains uncertain as anti-interleukin-5 only partially depletes numbers in asthmatic airway. *Am J Respir Crit Care Med,* 2003, vol. 167 (2), 199-204
- **FLOOD-PAGE P ; SWENSON C ; FAIFERMAN I et al.** A study to evaluate safety and efficacy of mepolizumab in patients with moderate persistent asthma. *Am J Respir Crit Care Med,* 2007, vol. 176 (11), 1062-71
- **BARIL, P. et al.** Periostin promotes invasiveness and resistance of pancreatic cancer cells to hypoxia-induced cell death: role of the beta4 integrin and the PI3k pathway. *Oncogene,* 2007, vol. 26 (14), 2082-94
- **KUDO, Y. et al.** Periostin promotes invasion and anchorage-independent growth in the metastatic process of head and neck cancer. *Cancer Res,* 2006, vol. 66 (14), 6928-35
- **PUGLISI, F. et al.** Expression of periostin in human breast cancer. *J Clin Pathol,* 2008, vol. 61 (4), 494-8
- **SIRIWARDENA, B.S. et al.** Periostin is frequently overexpressed and enhances invasion and angiogenesis in oral cancer. *Br J Cancer,* 2006, vol. 95 (10), 1396-403
- **SASAKI, H. et al.** Serum level of the periostin, a homologue of an insect cell adhesion molecule, as a prognostic marker in nonsmall cell lung carcinomas. *Cancer,* 2001, vol. 92 (4), 843-8
- **SASAKI, H. et al.** Expression of Periostin, homologous with an insect cell adhesion molecule, as a prognostic marker in non-small cell lung cancers. *Jpn J Cancer Res,* 2001, vol. 92 (8), 869-73
- **SASAKI, H. et al.** Elevated serum periostin levels in patients with bone metastases from breast but not lung cancer. *Breast Cancer Res Treat,* 2003, vol. 77 (3), 245-52
- **KOMIYA, A. et al.** Concerted expression of eotaxin-1, eotaxin-2, and eotaxin-3 in human bronchial epithelial cells. *Cell Immunol,* 2003, vol. 225 (2), 91-100
- **CHUPP, G.L. et al.** A chitinase-like protein in the lung and circulation of patients with severe asthma. *N Engl J Med,* 2007, vol. 357 (20), 2016-27
- **LIU, S.M. et al.** Immune cell transcriptome datasets reveal novel leukocyte subset-specific genes and genes associated with allergic processes. *J Allergy Clin Immunol,* 2006, vol. 118 (2), 496-503
- **NAKAJIMA, T. et al.** Identification of granulocyte subtype-selective receptors and ion channels by using a high-density oligonucleotide probe array. *J Allergy Clin Immunol,* 2004, vol. 113 (3), 528-35
- **DE FILIPPIS, D. ; A. D'AMICO ; T. IUVONE.** Cannabinomimetic control of mast cell mediator release: new perspective in chronic inflammation. *J Neuroendocrinol,* 2008, vol. 20 (1), 20-5
- **REED, C.E. ; H. KITA.** The role of protease activation of inflammation in allergic respiratory diseases. *J Allergy Clin Immunol,* 2004, vol. 114 (5), 997-1008
- **BLANCHARD, C. et al.** Periostin facilitates eosinophil tissue infiltration in allergic lung and esophageal responses. *Mucosal Immunol,* 2008, vol. 1 (4), 289-96
- **WENZEL, S.E. et al.** Bronchoscopic evaluation of severe asthma. Persistent inflammation associated with high dose glucocorticoids. *Am J Respir Crit Care Med,* 1997, vol. 156 (3), 737-43
- **LEUNG, D.Y. et al.** Dysregulation of interleukin 4, interleukin 5, and interferon gamma gene expression in steroid-resistant asthma. *J Exp Med,* 1995, vol. 181 (1), 33-40
- **WALSH, G.M.** Emerging drugs for asthma. *Expert Opin Emerg Drugs,* 2008, vol. 13 (4), 643-53
- **BALLESTA, A.M. et al.** Carcinoembryonic antigen in staging and follow-up of patients with solid tumors. *Tumour Biol,* 1995, vol. 16 (1), 32-41

SEQUENCE LISTING

<110> GENENTECH, INC.
THE REGENTS OF THE UNIVERSITY OF CALIFORNIA
ARRON, Joseph R.
FAHY, John V.
MODREK, Barmak
WOODRUFF, Prescott

<120> COMPOSITIONS AND METHODS FOR TREATING AND DIAGNOSING ASTHMA

<130> P4190R1

<140>
<141>

<150> 61/205,392
<151> 2009-01-16

<150> 61/128,383
<151> 2008-05-20

<150> 61/041,480
<151> 2008-04-01

<150> 61/072,572
<151> 2008-03-31

<160> 261

<170> Patent In version 3.5

<210> 1
<211> 836
<212> PRT
<213> Homo sapiens

<400> 1

```
Met Ile Pro Phe Leu Pro Met Phe Ser Leu Leu Leu Leu Leu Ile Val
1               5                   10                  15

Asn Pro Ile Asn Ala Asn Asn His Tyr Asp Lys Ile Leu Ala His Ser
            20                  25                  30

Arg Ile Arg Gly Arg Asp Gln Gly Pro Asn Val Cys Ala Leu Gln Gln
        35                  40                  45

Ile Leu Gly Thr Lys Lys Lys Tyr Phe Ser Thr Cys Lys Asn Trp Tyr
    50                  55                  60

Lys Lys Ser Ile Cys Gly Gln Lys Thr Thr Val Leu Tyr Glu Cys Cys
65                  70                  75                  80

Pro Gly Tyr Met Arg Met Glu Gly Met Lys Gly Cys Pro Ala Val Leu
                85                  90                  95
```

```
Pro Ile Asp His Val Tyr Gly Thr Leu Gly Ile Val Gly Ala Thr Thr
            100                 105                 110

Thr Gln Arg Tyr Ser Asp Ala Ser Lys Leu Arg Glu Glu Ile Glu Gly
        115                 120                 125

Lys Gly Ser Phe Thr Tyr Phe Ala Pro Ser Asn Glu Ala Trp Asp Asn
    130                 135                 140

Leu Asp Ser Asp Ile Arg Arg Gly Leu Glu Ser Asn Val Asn Val Glu
145                 150                 155                 160

Leu Leu Asn Ala Leu His Ser His Met Ile Asn Lys Arg Met Leu Thr
                165                 170                 175

Lys Asp Leu Lys Asn Gly Met Ile Ile Pro Ser Met Tyr Asn Asn Leu
            180                 185                 190

Gly Leu Phe Ile Asn His Tyr Pro Asn Gly Val Val Thr Val Asn Cys
        195                 200                 205

Ala Arg Ile Ile His Gly Asn Gln Ile Ala Thr Asn Gly Val Val His
    210                 215                 220

Val Ile Asp Arg Val Leu Thr Gln Ile Gly Thr Ser Ile Gln Asp Phe
225                 230                 235                 240

Ile Glu Ala Glu Asp Asp Leu Ser Ser Phe Arg Ala Ala Ala Ile Thr
                245                 250                 255

Ser Asp Ile Leu Glu Ala Leu Gly Arg Asp Gly His Phe Thr Leu Phe
            260                 265                 270

Ala Pro Thr Asn Glu Ala Phe Glu Lys Leu Pro Arg Gly Val Leu Glu
        275                 280                 285

Arg Ile Met Gly Asp Lys Val Ala Ser Glu Ala Leu Met Lys Tyr His
    290                 295                 300

Ile Leu Asn Thr Leu Gln Cys Ser Glu Ser Ile Met Gly Gly Ala Val
305                 310                 315                 320

Phe Glu Thr Leu Glu Gly Asn Thr Ile Glu Ile Gly Cys Asp Gly Asp
                325                 330                 335
```

```
Ser Ile Thr Val Asn Gly Ile Lys Met Val Asn Lys Lys Asp Ile Val
            340                 345                 350

Thr Asn Asn Gly Val Ile His Leu Ile Asp Gln Val Leu Ile Pro Asp
        355                 360                 365

Ser Ala Lys Gln Val Ile Glu Leu Ala Gly Lys Gln Gln Thr Thr Phe
    370                 375                 380

Thr Asp Leu Val Ala Gln Leu Gly Leu Ala Ser Ala Leu Arg Pro Asp
385                 390                 395                 400

Gly Glu Tyr Thr Leu Leu Ala Pro Val Asn Asn Ala Phe Ser Asp Asp
                405                 410                 415

Thr Leu Ser Met Asp Gln Arg Leu Leu Lys Leu Ile Leu Gln Asn His
            420                 425                 430

Ile Leu Lys Val Lys Val Gly Leu Asn Glu Leu Tyr Asn Gly Gln Ile
        435                 440                 445

Leu Glu Thr Ile Gly Gly Lys Gln Leu Arg Val Phe Val Tyr Arg Thr
    450                 455                 460

Ala Val Cys Ile Glu Asn Ser Cys Met Glu Lys Gly Ser Lys Gln Gly
465                 470                 475                 480

Arg Asn Gly Ala Ile His Ile Phe Arg Glu Ile Ile Lys Pro Ala Glu
                485                 490                 495

Lys Ser Leu His Glu Lys Leu Lys Gln Asp Lys Arg Phe Ser Thr Phe
            500                 505                 510

Leu Ser Leu Leu Glu Ala Ala Asp Leu Lys Glu Leu Leu Thr Gln Pro
        515                 520                 525

Gly Asp Trp Thr Leu Phe Val Pro Thr Asn Asp Ala Phe Lys Gly Met
    530                 535                 540

Thr Ser Glu Glu Lys Glu Ile Leu Ile Arg Asp Lys Asn Ala Leu Gln
545                 550                 555                 560

Asn Ile Ile Leu Tyr His Leu Thr Pro Gly Val Phe Ile Gly Lys Gly
                565                 570                 575

Phe Glu Pro Gly Val Thr Asn Ile Leu Lys Thr Thr Gln Gly Ser Lys
```

```
            580                 585                 590

Ile Phe Leu Lys Glu Val Asn Asp Thr Leu Leu Val Asn Glu Leu Lys
        595                 600                 605

Ser Lys Glu Ser Asp Ile Met Thr Thr Asn Gly Val Ile His Val Val
    610                 615                 620

Asp Lys Leu Leu Tyr Pro Ala Asp Thr Pro Val Gly Asn Asp Gln Leu
625                 630                 635                 640

Leu Glu Ile Leu Asn Lys Leu Ile Lys Tyr Ile Gln Ile Lys Phe Val
                645                 650                 655

Arg Gly Ser Thr Phe Lys Glu Ile Pro Val Thr Val Tyr Thr Thr Lys
            660                 665                 670

Ile Ile Thr Lys Val Val Glu Pro Lys Ile Lys Val Ile Glu Gly Ser
        675                 680                 685

Leu Gln Pro Ile Ile Lys Thr Glu Gly Pro Thr Leu Thr Lys Val Lys
    690                 695                 700

Ile Glu Gly Glu Pro Glu Phe Arg Leu Ile Lys Glu Gly Glu Thr Ile
705                 710                 715                 720

Thr Glu Val Ile His Gly Glu Pro Ile Ile Lys Lys Tyr Thr Lys Ile
                725                 730                 735

Ile Asp Gly Val Pro Val Glu Ile Thr Glu Lys Glu Thr Arg Glu Glu
            740                 745                 750

Arg Ile Ile Thr Gly Pro Glu Ile Lys Tyr Thr Arg Ile Ser Thr Gly
        755                 760                 765

Gly Gly Glu Thr Glu Glu Thr Leu Lys Lys Leu Leu Gln Glu Glu Val
    770                 775                 780

Thr Lys Val Thr Lys Phe Ile Glu Gly Gly Asp Gly His Leu Phe Glu
785                 790                 795                 800

Asp Glu Glu Ile Lys Arg Leu Leu Gln Gly Asp Thr Pro Val Arg Lys
                805                 810                 815

Leu Gln Ala Asn Lys Lys Val Gln Gly Ser Arg Arg Arg Leu Arg Glu
            820                 825                 830
```

```
Gly Arg Ser Gln
                835
```

<210> 2
<211> 141
<212> PRT
<213> Homo sapiens

<400> 2

```
Met Ala Gln His Leu Ser Thr Leu Leu Leu Leu Leu Ala Thr Leu Ala
1               5                   10                  15

Val Ala Leu Ala Trp Ser Pro Lys Glu Glu Asp Arg Ile Ile Pro Gly
            20                  25                  30

Gly Ile Tyr Asn Ala Asp Leu Asn Asp Glu Trp Val Gln Arg Ala Leu
        35                  40                  45

His Phe Ala Ile Ser Glu Tyr Asn Lys Ala Thr Lys Asp Asp Tyr Tyr
    50                  55                  60

Arg Arg Pro Leu Arg Val Leu Arg Ala Arg Gln Gln Thr Val Gly Gly
65                  70                  75                  80

Val Asn Tyr Phe Phe Asp Val Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Glu Ile Tyr Glu Val Pro Trp Glu
        115                 120                 125

Asn Arg Arg Ser Leu Val Lys Ser Arg Cys Gln Glu Ser
    130                 135                 140
```

<210> 3
<211> 94
<212> PRT
<213> Homo sapiens

<400> 3

```
Met Met Gly Leu Ser Leu Ala Ser Ala Val Leu Leu Ala Ser Leu Leu
1               5                   10                  15

Ser Leu His Leu Gly Thr Ala Thr Arg Gly Ser Asp Ile Ser Lys Thr
            20                  25                  30
```

```
Cys Cys Phe Gln Tyr Ser His Lys Pro Leu Pro Trp Thr Trp Val Arg
        35                  40                  45

Ser Tyr Glu Phe Thr Ser Asn Ser Cys Ser Gln Arg Ala Val Ile Phe
    50                  55                  60

Thr Thr Lys Arg Gly Lys Lys Val Cys Thr His Pro Arg Lys Lys Trp
65                  70                  75                  80

Val Gln Lys Tyr Ile Ser Leu Leu Lys Thr Pro Lys Gln Leu
                85                  90
```

<210> 4
<211> 914
<212> PRT
<213> Homo sapiens

<400> 4

```
Met Gly Pro Phe Lys Ser Ser Val Phe Ile Leu Ile Leu His Leu Leu
1               5                   10                  15

Glu Gly Ala Leu Ser Asn Ser Leu Ile Gln Leu Asn Asn Asn Gly Tyr
            20                  25                  30

Glu Gly Ile Val Val Ala Ile Asp Pro Asn Val Pro Glu Asp Glu Thr
        35                  40                  45

Leu Ile Gln Gln Ile Lys Asp Met Val Thr Gln Ala Ser Leu Tyr Leu
    50                  55                  60

Phe Glu Ala Thr Gly Lys Arg Phe Tyr Phe Lys Asn Val Ala Ile Leu
65                  70                  75                  80

Ile Pro Glu Thr Trp Lys Thr Lys Ala Asp Tyr Val Arg Pro Lys Leu
                85                  90                  95

Glu Thr Tyr Lys Asn Ala Asp Val Leu Val Ala Glu Ser Thr Pro Pro
            100                 105                 110

Gly Asn Asp Glu Pro Tyr Thr Glu Gln Met Gly Asn Cys Gly Glu Lys
        115                 120                 125

Gly Glu Arg Ile His Leu Thr Pro Asp Phe Ile Ala Gly Lys Lys Leu
    130                 135                 140

Ala Glu Tyr Gly Pro Gln Gly Lys Ala Phe Val His Glu Trp Ala His
145                 150                 155                 160
```

```
Leu Arg Trp Gly Val Phe Asp Glu Tyr Asn Asn Asp Glu Lys Phe Tyr
                165                 170                 175

Leu Ser Asn Gly Arg Ile Gln Ala Val Arg Cys Ser Ala Gly Ile Thr
            180                 185                 190

Gly Thr Asn Val Val Lys Lys Cys Gln Gly Gly Ser Cys Tyr Thr Lys
        195                 200                 205

Arg Cys Thr Phe Asn Lys Val Thr Gly Leu Tyr Glu Lys Gly Cys Glu
    210                 215                 220

Phe Val Leu Gln Ser Arg Gln Thr Glu Lys Ala Ser Ile Met Phe Ala
225                 230                 235                 240

Gln His Val Asp Ser Ile Val Glu Phe Cys Thr Glu Gln Asn His Asn
                245                 250                 255

Lys Glu Ala Pro Asn Lys Gln Asn Gln Lys Cys Asn Leu Arg Ser Thr
            260                 265                 270

Trp Glu Val Ile Arg Asp Ser Glu Asp Phe Lys Lys Thr Thr Pro Met
        275                 280                 285

Thr Thr Gln Pro Pro Asn Pro Thr Phe Ser Leu Leu Gln Ile Gly Gln
    290                 295                 300

Arg Ile Val Cys Leu Val Leu Asp Lys Ser Gly Ser Met Ala Thr Gly
305                 310                 315                 320

Asn Arg Leu Asn Arg Leu Asn Gln Ala Gly Gln Leu Phe Leu Leu Gln
                325                 330                 335

Thr Val Glu Leu Gly Ser Trp Val Gly Met Val Thr Phe Asp Ser Ala
            340                 345                 350

Ala His Val Gln Ser Glu Leu Ile Gln Ile Asn Ser Gly Ser Asp Arg
        355                 360                 365

Asp Thr Leu Ala Lys Arg Leu Pro Ala Ala Ala Ser Gly Gly Thr Ser
    370                 375                 380

Ile Cys Ser Gly Leu Arg Ser Ala Phe Thr Val Ile Arg Lys Lys Tyr
385                 390                 395                 400
```

```
Pro Thr Asp Gly Ser Glu Ile Val Leu Leu Thr Asp Gly Glu Asp Asn
                405                 410                 415

Thr Ile Ser Gly Cys Phe Asn Glu Val Lys Gln Ser Gly Ala Ile Ile
            420                 425                 430

His Thr Val Ala Leu Gly Pro Ser Ala Ala Gln Glu Leu Glu Glu Leu
        435                 440                 445

Ser Lys Met Thr Gly Gly Leu Gln Thr Tyr Ala Ser Asp Gln Val Gln
    450                 455                 460

Asn Asn Gly Leu Ile Asp Ala Phe Gly Ala Leu Ser Ser Gly Asn Gly
465                 470                 475                 480

Ala Val Ser Gln Arg Ser Ile Gln Leu Glu Ser Lys Gly Leu Thr Leu
                485                 490                 495

Gln Asn Ser Gln Trp Met Asn Gly Thr Val Ile Val Asp Ser Thr Val
            500                 505                 510

Gly Lys Asp Thr Leu Phe Leu Ile Thr Trp Thr Thr Gln Pro Pro Gln
        515                 520                 525

Ile Leu Leu Trp Asp Pro Ser Gly Gln Lys Gln Gly Gly Phe Val Val
    530                 535                 540

Asp Lys Asn Thr Lys Met Ala Tyr Leu Gln Ile Pro Gly Ile Ala Lys
545                 550                 555                 560

Val Gly Thr Trp Lys Tyr Ser Leu Gln Ala Ser Ser Gln Thr Leu Thr
                565                 570                 575

Leu Thr Val Thr Ser Arg Ala Ser Asn Ala Thr Leu Pro Pro Ile Thr
            580                 585                 590

Val Thr Ser Lys Thr Asn Lys Asp Thr Ser Lys Phe Pro Ser Pro Leu
        595                 600                 605

Val Val Tyr Ala Asn Ile Arg Gln Gly Ala Ser Pro Ile Leu Arg Ala
    610                 615                 620

Ser Val Thr Ala Leu Ile Glu Ser Val Asn Gly Lys Thr Val Thr Leu
625                 630                 635                 640
```

```
Glu Leu Leu Asp Asn Gly Ala Gly Ala Asp Ala Thr Lys Asp Asp Gly
                645                 650                 655

Val Tyr Ser Arg Tyr Phe Thr Thr Tyr Asp Thr Asn Gly Arg Tyr Ser
            660                 665                 670

Val Lys Val Arg Ala Leu Gly Gly Val Asn Ala Ala Arg Arg Arg Val
        675                 680                 685

Ile Pro Gln Gln Ser Gly Ala Leu Tyr Ile Pro Gly Trp Ile Glu Asn
    690                 695                 700

Asp Glu Ile Gln Trp Asn Pro Pro Arg Pro Glu Ile Asn Lys Asp Asp
705                 710                 715                 720

Val Gln His Lys Gln Val Cys Phe Ser Arg Thr Ser Ser Gly Gly Ser
                725                 730                 735

Phe Val Ala Ser Asp Val Pro Asn Ala Pro Ile Pro Asp Leu Phe Pro
            740                 745                 750

Pro Gly Gln Ile Thr Asp Leu Lys Ala Glu Ile His Gly Gly Ser Leu
        755                 760                 765

Ile Asn Leu Thr Trp Thr Ala Pro Gly Asp Asp Tyr Asp His Gly Thr
    770                 775                 780

Ala His Lys Tyr Ile Ile Arg Ile Ser Thr Ser Ile Leu Asp Leu Arg
785                 790                 795                 800

Asp Lys Phe Asn Glu Ser Leu Gln Val Asn Thr Thr Ala Leu Ile Pro
                805                 810                 815

Lys Glu Ala Asn Ser Glu Glu Val Phe Leu Phe Lys Pro Glu Asn Ile
            820                 825                 830

Thr Phe Glu Asn Gly Thr Asp Leu Phe Ile Ala Ile Gln Ala Val Asp
        835                 840                 845

Lys Val Asp Leu Lys Ser Glu Ile Ser Asn Ile Ala Arg Val Ser Leu
    850                 855                 860

Phe Ile Pro Pro Gln Thr Pro Pro Glu Thr Pro Ser Pro Asp Glu Thr
865                 870                 875                 880

Ser Ala Pro Cys Pro Asn Ile His Ile Asn Ser Thr Ile Pro Gly Ile
```

```
                885                 890                 895

His Ile Leu Lys Ile Met Trp Lys Trp Ile Gly Glu Leu Gln Leu Ser
            900                 905                 910

Ile Ala
```

<210> 5
<211> 141
<212> PRT
<213> Homo sapiens

<400> 5

```
Met Ala Trp Pro Leu Cys Thr Leu Leu Leu Leu Leu Ala Thr Gln Ala
1               5                   10                  15

Val Ala Leu Ala Trp Ser Pro Gln Glu Glu Asp Arg Ile Ile Glu Gly
            20                  25                  30

Gly Ile Tyr Asp Ala Asp Leu Asn Asp Glu Arg Val Gln Arg Ala Leu
        35                  40                  45

His Phe Val Ile Ser Glu Tyr Asn Lys Ala Thr Glu Asp Glu Tyr Tyr
    50                  55                  60

Arg Arg Leu Leu Arg Val Leu Arg Ala Arg Glu Gln Ile Val Gly Gly
65                  70                  75                  80

Val Asn Tyr Phe Phe Asp Ile Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Gln Ile Tyr Glu Val Pro Trp Glu
        115                 120                 125

Asp Arg Met Ser Leu Val Asn Ser Arg Cys Gln Glu Ala
    130                 135                 140
```

<210> 6
<211> 134
<212> PRT
<213> Homo sapiens

<400> 6

```
Met Leu Leu Val Leu Leu Ser Val Val Leu Leu Ala Leu Ser Ser Ala
1               5                   10                  15

Gln Ser Thr Asp Asn Asp Val Asn Tyr Glu Asp Phe Thr Phe Thr Ile
            20                  25                  30

Pro Asp Val Glu Asp Ser Ser Gln Arg Pro Asp Gln Gly Pro Gln Arg
        35                  40                  45

Pro Pro Pro Glu Gly Leu Leu Pro Arg Pro Pro Gly Asp Ser Gly Asn
    50                  55                  60

Gln Asp Asp Gly Pro Gln Gln Arg Pro Pro Lys Pro Gly Gly His His
65                  70                  75                  80

Arg His Pro Pro Pro Pro Pro Phe Gln Asn Gln Gln Arg Pro Pro Arg
                85                  90                  95

Arg Gly His Arg Gln Leu Ser Leu Pro Arg Phe Pro Ser Val Ser Leu
            100                 105                 110

Gln Glu Ala Ser Ser Phe Phe Arg Arg Asp Arg Pro Ala Arg His Pro
        115                 120                 125

Gln Glu Gln Pro Leu Trp
    130
```

<210> 7
<211> 415
<212> PRT
<213> Homo sapiens

<400> 7

```
Met Glu Asp Leu Cys Val Ala Asn Thr Leu Phe Ala Leu Asn Leu Phe
1               5                   10                  15

Lys His Leu Ala Lys Ala Ser Pro Thr Gln Asn Leu Phe Leu Ser Pro
            20                  25                  30

Trp Ser Ile Ser Ser Thr Met Ala Met Val Tyr Met Gly Ser Arg Gly
        35                  40                  45

Ser Thr Glu Asp Gln Met Ala Lys Val Leu Gln Phe Asn Glu Val Gly
    50                  55                  60

Ala Asn Ala Val Thr Pro Met Thr Pro Glu Asn Phe Thr Ser Cys Gly
65                  70                  75                  80

Phe Met Gln Gln Ile Gln Lys Gly Ser Tyr Pro Asp Ala Ile Leu Gln
```

85 90 95

Ala Gln Ala Ala Asp Lys Ile His Ser Ser Phe Arg Ser Leu Ser Ser
100 105 110

Ala Ile Asn Ala Ser Thr Gly Asn Tyr Leu Leu Glu Ser Val Asn Lys
115 120 125

Leu Phe Gly Glu Lys Ser Ala Ser Phe Arg Glu Glu Tyr Ile Arg Leu
130 135 140

Cys Gln Lys Tyr Tyr Ser Ser Glu Pro Gln Ala Val Asp Phe Leu Glu
145 150 155 160

Cys Ala Glu Glu Ala Arg Lys Lys Ile Asn Ser Trp Val Lys Thr Gln
165 170 175

Thr Lys Gly Lys Ile Pro Asn Leu Leu Pro Glu Gly Ser Val Asp Gly
180 185 190

Asp Thr Arg Met Val Leu Val Asn Ala Val Tyr Phe Lys Gly Lys Trp
195 200 205

Lys Thr Pro Phe Glu Lys Lys Leu Asn Gly Leu Tyr Pro Phe Arg Val
210 215 220

Asn Ser Ala Gln Arg Thr Pro Val Gln Met Met Tyr Leu Arg Glu Lys
225 230 235 240

Leu Asn Ile Gly Tyr Ile Glu Asp Leu Lys Ala Gln Ile Leu Glu Leu
245 250 255

Pro Tyr Ala Gly Asp Val Ser Met Phe Leu Leu Leu Pro Asp Glu Ile
260 265 270

Ala Asp Val Ser Thr Gly Leu Glu Leu Leu Glu Ser Glu Ile Thr Tyr
275 280 285

Asp Lys Leu Asn Lys Trp Thr Ser Lys Asp Lys Met Ala Glu Asp Glu
290 295 300

Val Glu Val Tyr Ile Pro Gln Phe Lys Leu Glu Glu His Tyr Glu Leu
305 310 315 320

Arg Ser Ile Leu Arg Ser Met Gly Met Glu Asp Ala Phe Asn Lys Gly
325 330 335

```
Arg Ala Asn Phe Ser Gly Met Ser Glu Arg Asn Asp Leu Phe Leu Ser
            340                 345                 350

Glu Val Phe His Gln Ala Met Val Asp Val Asn Glu Glu Gly Thr Glu
        355                 360                 365

Ala Ala Ala Gly Thr Gly Gly Val Met Thr Gly Arg Thr Gly His Gly
    370                 375                 380

Gly Pro Gln Phe Val Ala Asp His Pro Phe Leu Phe Leu Ile Met His
385                 390                 395                 400

Lys Ile Thr Asn Cys Ile Leu Phe Phe Gly Arg Phe Ser Ser Pro
                405                 410                 415
```

<210> 8
<211> 702
<212> PRT
<213> Homo sapiens

<400> 8

```
Met Glu Ser Pro Ser Ala Pro Pro His Arg Trp Cys Ile Pro Trp Gln
1               5                   10                  15

Arg Leu Leu Leu Thr Ala Ser Leu Leu Thr Phe Trp Asn Pro Pro Thr
            20                  25                  30

Thr Ala Lys Leu Thr Ile Glu Ser Thr Pro Phe Asn Val Ala Glu Gly
        35                  40                  45

Lys Glu Val Leu Leu Leu Val His Asn Leu Pro Gln His Leu Phe Gly
    50                  55                  60

Tyr Ser Trp Tyr Lys Gly Glu Arg Val Asp Gly Asn Arg Gln Ile Ile
65                  70                  75                  80

Gly Tyr Val Ile Gly Thr Gln Gln Ala Thr Pro Gly Pro Ala Tyr Ser
                85                  90                  95

Gly Arg Glu Ile Ile Tyr Pro Asn Ala Ser Leu Leu Ile Gln Asn Ile
            100                 105                 110

Ile Gln Asn Asp Thr Gly Phe Tyr Thr Leu His Val Ile Lys Ser Asp
        115                 120                 125

Leu Val Asn Glu Glu Ala Thr Gly Gln Phe Arg Val Tyr Pro Glu Leu
```

```
    130                 135                 140

Pro Lys Pro Ser Ile Ser Ser Asn Asn Ser Lys Pro Val Glu Asp Lys
145                 150                 155                 160

Asp Ala Val Ala Phe Thr Cys Glu Pro Glu Thr Gln Asp Ala Thr Tyr
                165                 170                 175

Leu Trp Trp Val Asn Asn Gln Ser Leu Pro Val Ser Pro Arg Leu Gln
            180                 185                 190

Leu Ser Asn Gly Asn Arg Thr Leu Thr Leu Phe Asn Val Thr Arg Asn
        195                 200                 205

Asp Thr Ala Ser Tyr Lys Cys Glu Thr Gln Asn Pro Val Ser Ala Arg
    210                 215                 220

Arg Ser Asp Ser Val Ile Leu Asn Val Leu Tyr Gly Pro Asp Ala Pro
225                 230                 235                 240

Thr Ile Ser Pro Leu Asn Thr Ser Tyr Arg Ser Gly Glu Asn Leu Asn
                245                 250                 255

Leu Ser Cys His Ala Ala Ser Asn Pro Pro Ala Gln Tyr Ser Trp Phe
            260                 265                 270

Val Asn Gly Thr Phe Gln Gln Ser Thr Gln Glu Leu Phe Ile Pro Asn
        275                 280                 285

Ile Thr Val Asn Asn Ser Gly Ser Tyr Thr Cys Gln Ala His Asn Ser
    290                 295                 300

Asp Thr Gly Leu Asn Arg Thr Thr Val Thr Thr Ile Thr Val Tyr Ala
305                 310                 315                 320

Glu Pro Pro Lys Pro Phe Ile Thr Ser Asn Asn Ser Asn Pro Val Glu
                325                 330                 335

Asp Glu Asp Ala Val Ala Leu Thr Cys Glu Pro Glu Ile Gln Asn Thr
            340                 345                 350

Thr Tyr Leu Trp Trp Val Asn Asn Gln Ser Leu Pro Val Ser Pro Arg
        355                 360                 365

Leu Gln Leu Ser Asn Asp Asn Arg Thr Leu Thr Leu Leu Ser Val Thr
    370                 375                 380
```

```
Arg Asn Asp Val Gly Pro Tyr Glu Cys Gly Ile Gln Asn Glu Leu Ser
385                 390                 395                 400

Val Asp His Ser Asp Pro Val Ile Leu Asn Val Leu Tyr Gly Pro Asp
                405                 410                 415

Asp Pro Thr Ile Ser Pro Ser Tyr Thr Tyr Tyr Arg Pro Gly Val Asn
            420                 425                 430

Leu Ser Leu Ser Cys His Ala Ala Ser Asn Pro Pro Ala Gln Tyr Ser
        435                 440                 445

Trp Leu Ile Asp Gly Asn Ile Gln Gln His Thr Gln Glu Leu Phe Ile
    450                 455                 460

Ser Asn Ile Thr Glu Lys Asn Ser Gly Leu Tyr Thr Cys Gln Ala Asn
465                 470                 475                 480

Asn Ser Ala Ser Gly His Ser Arg Thr Thr Val Lys Thr Ile Thr Val
                485                 490                 495

Ser Ala Glu Leu Pro Lys Pro Ser Ile Ser Ser Asn Asn Ser Lys Pro
            500                 505                 510

Val Glu Asp Lys Asp Ala Val Ala Phe Thr Cys Glu Pro Glu Ala Gln
        515                 520                 525

Asn Thr Thr Tyr Leu Trp Trp Val Asn Gly Gln Ser Leu Pro Val Ser
    530                 535                 540

Pro Arg Leu Gln Leu Ser Asn Gly Asn Arg Thr Leu Thr Leu Phe Asn
545                 550                 555                 560

Val Thr Arg Asn Asp Ala Arg Ala Tyr Val Cys Gly Ile Gln Asn Ser
                565                 570                 575

Val Ser Ala Asn Arg Ser Asp Pro Val Thr Leu Asp Val Leu Tyr Gly
            580                 585                 590

Pro Asp Thr Pro Ile Ile Ser Pro Pro Asp Ser Ser Tyr Leu Ser Gly
        595                 600                 605

Ala Asn Leu Asn Leu Ser Cys His Ser Ala Ser Asn Pro Ser Pro Gln
    610                 615                 620
```

```
Tyr Ser Trp Arg Ile Asn Gly Ile Pro Gln Gln His Thr Gln Val Leu
625                 630                 635                 640

Phe Ile Ala Lys Ile Thr Pro Asn Asn Asn Gly Thr Tyr Ala Cys Phe
                645                 650                 655

Val Ser Asn Leu Ala Thr Gly Arg Asn Asn Ser Ile Val Lys Ser Ile
            660                 665                 670

Thr Val Ser Ala Ser Gly Thr Ser Pro Gly Leu Ser Ala Gly Ala Thr
        675                 680                 685

Val Gly Ile Met Ile Gly Val Leu Val Gly Val Ala Leu Ile
    690                 695                 700
```

<210> 9
<211> 1153
<212> PRT
<213> Homo sapiens

<400> 9

```
Met Ala Cys Pro Trp Lys Phe Leu Phe Lys Thr Lys Phe His Gln Tyr
1               5                   10                  15

Ala Met Asn Gly Glu Lys Asp Ile Asn Asn Asn Val Glu Lys Ala Pro
            20                  25                  30

Cys Ala Thr Ser Ser Pro Val Thr Gln Asp Asp Leu Gln Tyr His Asn
        35                  40                  45

Leu Ser Lys Gln Gln Asn Glu Ser Pro Gln Pro Leu Val Glu Thr Gly
    50                  55                  60

Lys Lys Ser Pro Glu Ser Leu Val Lys Leu Asp Ala Thr Pro Leu Ser
65                  70                  75                  80

Ser Pro Arg His Val Arg Ile Lys Asn Trp Gly Ser Gly Met Thr Phe
                85                  90                  95

Gln Asp Thr Leu His His Lys Ala Lys Gly Ile Leu Thr Cys Arg Ser
            100                 105                 110

Lys Ser Cys Leu Gly Ser Ile Met Thr Pro Lys Ser Leu Thr Arg Gly
        115                 120                 125

Pro Arg Asp Lys Pro Thr Pro Pro Asp Glu Leu Leu Pro Gln Ala Ile
    130                 135                 140
```

```
Glu Phe Val Asn Gln Tyr Tyr Gly Ser Phe Lys Glu Ala Lys Ile Glu
145                 150                 155                 160

Glu His Leu Ala Arg Val Glu Ala Val Thr Lys Glu Ile Glu Thr Thr
                165                 170                 175

Gly Thr Tyr Gln Leu Thr Gly Asp Glu Leu Ile Phe Ala Thr Lys Gln
            180                 185                 190

Ala Trp Arg Asn Ala Pro Arg Cys Ile Gly Arg Ile Gln Trp Ser Asn
        195                 200                 205

Leu Gln Val Phe Asp Ala Arg Ser Cys Ser Thr Ala Arg Glu Met Phe
    210                 215                 220

Glu His Ile Cys Arg His Val Arg Tyr Ser Thr Asn Asn Gly Asn Ile
225                 230                 235                 240

Arg Ser Ala Ile Thr Val Phe Pro Gln Arg Ser Asp Gly Lys His Asp
                245                 250                 255

Phe Arg Val Trp Asn Ala Gln Leu Ile Arg Tyr Ala Gly Tyr Gln Met
            260                 265                 270

Pro Asp Gly Ser Ile Arg Gly Asp Pro Ala Asn Val Glu Phe Thr Gln
        275                 280                 285

Leu Cys Ile Asp Leu Gly Trp Lys Pro Lys Tyr Gly Arg Phe Asp Val
    290                 295                 300

Val Pro Leu Val Leu Gln Ala Asn Gly Arg Asp Pro Glu Leu Phe Glu
305                 310                 315                 320

Ile Pro Pro Asp Leu Val Leu Glu Val Ala Met Glu His Pro Lys Tyr
                325                 330                 335

Glu Trp Phe Arg Glu Leu Glu Leu Lys Trp Tyr Ala Leu Pro Ala Val
            340                 345                 350

Ala Asn Met Leu Leu Glu Val Gly Gly Leu Glu Phe Pro Gly Cys Pro
        355                 360                 365

Phe Asn Gly Trp Tyr Met Gly Thr Glu Ile Gly Val Arg Asp Phe Cys
    370                 375                 380
```

```
Asp Val Gln Arg Tyr Asn Ile Leu Glu Glu Val Gly Arg Arg Met Gly
385                 390                 395                 400

Leu Glu Thr His Lys Leu Ala Ser Leu Trp Lys Asp Gln Ala Val Val
                405                 410                 415

Glu Ile Asn Ile Ala Val Leu His Ser Phe Gln Lys Gln Asn Val Thr
            420                 425                 430

Ile Met Asp His His Ser Ala Ala Glu Ser Phe Met Lys Tyr Met Gln
        435                 440                 445

Asn Glu Tyr Arg Ser Arg Gly Gly Cys Pro Ala Asp Trp Ile Trp Leu
    450                 455                 460

Val Pro Pro Met Ser Gly Ser Ile Thr Pro Val Phe His Gln Glu Met
465                 470                 475                 480

Leu Asn Tyr Val Leu Ser Pro Phe Tyr Tyr Tyr Gln Val Glu Ala Trp
                485                 490                 495

Lys Thr His Val Trp Gln Asp Glu Lys Arg Arg Pro Lys Arg Arg Glu
            500                 505                 510

Ile Pro Leu Lys Val Leu Val Lys Ala Val Leu Phe Ala Cys Met Leu
        515                 520                 525

Met Arg Lys Thr Met Ala Ser Arg Val Arg Val Thr Ile Leu Phe Ala
    530                 535                 540

Thr Glu Thr Gly Lys Ser Glu Ala Leu Ala Trp Asp Leu Gly Ala Leu
545                 550                 555                 560

Phe Ser Cys Ala Phe Asn Pro Lys Val Val Cys Met Asp Lys Tyr Arg
                565                 570                 575

Leu Ser Cys Leu Glu Glu Glu Arg Leu Leu Leu Val Val Thr Ser Thr
            580                 585                 590

Phe Gly Asn Gly Asp Cys Pro Gly Asn Gly Glu Lys Leu Lys Lys Ser
        595                 600                 605

Leu Phe Met Leu Lys Glu Leu Asn Asn Lys Phe Arg Tyr Ala Val Phe
    610                 615                 620
```

```
Gly Leu Gly Ser Ser Met Tyr Pro Arg Phe Cys Ala Phe Ala His Asp
625                 630                 635                 640

Ile Asp Gln Lys Leu Ser His Leu Gly Ala Ser Gln Leu Thr Pro Met
                645                 650                 655

Gly Glu Gly Asp Glu Leu Ser Gly Gln Glu Asp Ala Phe Arg Ser Trp
            660                 665                 670

Ala Val Gln Thr Phe Lys Ala Ala Cys Glu Thr Phe Asp Val Arg Gly
        675                 680                 685

Lys Gln His Ile Gln Ile Pro Lys Leu Tyr Thr Ser Asn Val Thr Trp
    690                 695                 700

Asp Pro His His Tyr Arg Leu Val Gln Asp Ser Gln Pro Leu Asp Leu
705                 710                 715                 720

Ser Lys Ala Leu Ser Ser Met His Ala Lys Asn Val Phe Thr Met Arg
                725                 730                 735

Leu Lys Ser Arg Gln Asn Leu Gln Ser Pro Thr Ser Ser Arg Ala Thr
            740                 745                 750

Ile Leu Val Glu Leu Ser Cys Glu Asp Gly Gln Gly Leu Asn Tyr Leu
        755                 760                 765

Pro Gly Glu His Leu Gly Val Cys Pro Gly Asn Gln Pro Ala Leu Val
    770                 775                 780

Gln Gly Ile Leu Glu Arg Val Val Asp Gly Pro Thr Pro His Gln Thr
785                 790                 795                 800

Val Arg Leu Glu Ala Leu Asp Glu Ser Gly Ser Tyr Trp Val Ser Asp
                805                 810                 815

Lys Arg Leu Pro Pro Cys Ser Leu Ser Gln Ala Leu Thr Tyr Phe Leu
            820                 825                 830

Asp Ile Thr Thr Pro Pro Thr Gln Leu Leu Leu Gln Lys Leu Ala Gln
        835                 840                 845

Val Ala Thr Glu Glu Pro Glu Arg Gln Arg Leu Glu Ala Leu Cys Gln
    850                 855                 860

Pro Ser Glu Tyr Ser Lys Trp Lys Phe Thr Asn Ser Pro Thr Phe Leu
```

865 870 875 880

Glu Val Leu Glu Glu Phe Pro Ser Leu Arg Val Ser Ala Gly Phe Leu
885 890 895

Leu Ser Gln Leu Pro Ile Leu Lys Pro Arg Phe Tyr Ser Ile Ser Ser
900 905 910

Ser Arg Asp His Thr Pro Thr Glu Ile His Leu Thr Val Ala Val Val
915 920 925

Thr Tyr His Thr Arg Asp Gly Gln Gly Pro Leu His His Gly Val Cys
930 935 940

Ser Thr Trp Leu Asn Ser Leu Lys Pro Gln Asp Pro Val Pro Cys Phe
945 950 955 960

Val Arg Asn Ala Ser Gly Phe His Leu Pro Glu Asp Pro Ser His Pro
965 970 975

Cys Ile Leu Ile Gly Pro Gly Thr Gly Ile Ala Pro Phe Arg Ser Phe
980 985 990

Trp Gln Gln Arg Leu His Asp Ser Gln His Lys Gly Val Arg Gly Gly
995 1000 1005

Arg Met Thr Leu Val Phe Gly Cys Arg Arg Pro Asp Glu Asp His
1010 1015 1020

Ile Tyr Gln Glu Glu Met Leu Glu Met Ala Gln Lys Gly Val Leu
1025 1030 1035

His Ala Val His Thr Ala Tyr Ser Arg Leu Pro Gly Lys Pro Lys
1040 1045 1050

Val Tyr Val Gln Asp Ile Leu Arg Gln Gln Leu Ala Ser Glu Val
1055 1060 1065

Leu Arg Val Leu His Lys Glu Pro Gly His Leu Tyr Val Cys Gly
1070 1075 1080

Asp Val Arg Met Ala Arg Asp Val Ala His Thr Leu Lys Gln Leu
1085 1090 1095

Val Ala Ala Lys Leu Lys Leu Asn Glu Glu Gln Val Glu Asp Tyr
1100 1105 1110

```
Phe Phe  Gln Leu Lys Ser Gln  Lys Arg Tyr His Glu  Asp Ile Phe
    1115                 1120                 1125

Gly Ala  Val Phe Pro Tyr Glu  Ala Lys Lys Asp Arg  Val Ala Val
    1130                 1135                 1140

Gln Pro  Ser Ser Leu Glu Met  Ser Ala Leu
    1145                 1150
```

<210> 10
<211> 390
<212> PRT
<213> Homo sapiens

<400> 10

```
Met Asn Ser Leu Ser Glu Ala Asn Thr Lys Phe Met Phe Asp Leu Phe
1               5                   10                  15

Gln Gln Phe Arg Lys Ser Lys Glu Asn Asn Ile Phe Tyr Ser Pro Ile
            20                  25                  30

Ser Ile Thr Ser Ala Leu Gly Met Val Leu Leu Gly Ala Lys Asp Asn
        35                  40                  45

Thr Ala Gln Gln Ile Ser Lys Val Leu His Phe Asp Gln Val Thr Glu
    50                  55                  60

Asn Thr Thr Glu Lys Ala Ala Thr Tyr His Val Asp Arg Ser Gly Asn
65                  70                  75                  80

Val His His Gln Phe Gln Lys Leu Leu Thr Glu Phe Asn Lys Ser Thr
                85                  90                  95

Asp Ala Tyr Glu Leu Lys Ile Ala Asn Lys Leu Phe Gly Glu Lys Thr
            100                 105                 110

Tyr Gln Phe Leu Gln Glu Tyr Leu Asp Ala Ile Lys Lys Phe Tyr Gln
        115                 120                 125

Thr Ser Val Glu Ser Thr Asp Phe Ala Asn Ala Pro Glu Glu Ser Arg
    130                 135                 140

Lys Lys Ile Asn Ser Trp Val Glu Ser Gln Thr Asn Glu Lys Ile Lys
145                 150                 155                 160

Asn Leu Phe Pro Asp Gly Thr Ile Gly Asn Asp Thr Thr Leu Val Leu
```

165 170 175

Val Asn Ala Ile Tyr Phe Lys Gly Gln Trp Glu Asn Lys Phe Lys Lys
180 185 190

Glu Asn Thr Lys Glu Glu Lys Phe Trp Pro Asn Lys Asn Thr Tyr Lys
195 200 205

Ser Val Gln Met Met Arg Gln Tyr Asn Ser Phe Asn Phe Ala Leu Leu
210 215 220

Glu Asp Val Gln Ala Lys Val Leu Glu Ile Pro Tyr Lys Gly Lys Asp
225 230 235 240

Leu Ser Met Ile Val Leu Leu Pro Asn Glu Ile Asp Gly Leu Gln Lys
245 250 255

Leu Glu Glu Lys Leu Thr Ala Glu Lys Leu Met Glu Trp Thr Ser Leu
260 265 270

Gln Asn Met Arg Glu Thr Cys Val Asp Leu His Leu Pro Arg Phe Lys
275 280 285

Met Glu Glu Ser Tyr Asp Leu Lys Asp Thr Leu Arg Thr Met Gly Met
290 295 300

Val Asn Ile Phe Asn Gly Asp Ala Asp Leu Ser Gly Met Thr Trp Ser
305 310 315 320

His Gly Leu Ser Val Ser Lys Val Leu His Lys Ala Phe Val Glu Val
325 330 335

Thr Glu Glu Gly Val Glu Ala Ala Ala Ala Thr Ala Val Val Val Val
340 345 350

Glu Leu Ser Ser Pro Ser Thr Asn Glu Glu Phe Cys Cys Asn His Pro
355 360 365

Phe Leu Phe Phe Ile Arg Gln Asn Lys Thr Asn Ser Ile Leu Phe Tyr
370 375 380

Gly Arg Phe Ser Ser Pro
385 390

<210> 11
<211> 141

<212> PRT
<213> Homo sapiens

<400> 11

```
Met Ala Arg Pro Leu Cys Thr Leu Leu Leu Leu Met Ala Thr Leu Ala
1               5                   10                  15

Gly Ala Leu Ala Ser Ser Ser Lys Glu Glu Asn Arg Ile Ile Pro Gly
            20                  25                  30

Gly Ile Tyr Asp Ala Asp Leu Asn Asp Glu Trp Val Gln Arg Ala Leu
        35                  40                  45

His Phe Ala Ile Ser Glu Tyr Asn Lys Ala Thr Glu Asp Glu Tyr Tyr
    50                  55                  60

Arg Arg Pro Leu Gln Val Leu Arg Ala Arg Glu Gln Thr Phe Gly Gly
65                  70                  75                  80

Val Asn Tyr Phe Phe Asp Val Glu Val Gly Arg Thr Ile Cys Thr Lys
                85                  90                  95

Ser Gln Pro Asn Leu Asp Thr Cys Ala Phe His Glu Gln Pro Glu Leu
            100                 105                 110

Gln Lys Lys Gln Leu Cys Ser Phe Glu Ile Tyr Glu Val Pro Trp Glu
        115                 120                 125

Asp Arg Met Ser Leu Val Asn Ser Arg Cys Gln Glu Ala
    130                 135                 140
```

<210> 12
<211> 393
<212> PRT
<213> Homo sapiens

<400> 12

```
Met Leu Leu Ile Leu Leu Ser Val Ala Leu Leu Ala Leu Ser Ser Ala
1               5                   10                  15

Gln Asn Leu Asn Glu Asp Val Ser Gln Glu Glu Ser Pro Ser Leu Ile
            20                  25                  30

Ala Gly Lys Pro Gln Gly Pro Pro Pro Gln Gly Gly Asn Gln Pro Gln
        35                  40                  45

Gly Pro Pro Pro Pro Pro Gly Lys Pro Gln Gly Pro Pro Pro Gln Gly
    50                  55                  60
```

```
Gly Asn Lys Pro Gln Gly Pro Pro Pro Pro Gly Lys Pro Gln Gly Pro
65                  70                  75                  80

Pro Pro Gln Gly Asp Lys Ser Arg Ser Pro Arg Ser Pro Pro Gly Lys
                85                  90                  95

Pro Gln Gly Pro Pro Pro Gln Gly Gly Asn Gln Pro Gln Gly Pro Pro
            100                 105                 110

Pro Pro Pro Gly Lys Pro Gln Gly Pro Pro Pro Gln Gly Gly Asn Lys
        115                 120                 125

Pro Gln Gly Pro Pro Pro Pro Gly Lys Pro Gln Gly Pro Pro Pro Gln
    130                 135                 140

Gly Asp Asn Lys Ser Arg Ser Ser Arg Ser Pro Pro Gly Lys Pro Gln
145                 150                 155                 160

Gly Pro Pro Pro Gln Gly Gly Asn Gln Pro Gln Gly Pro Pro Pro Pro
                165                 170                 175

Pro Gly Lys Pro Gln Gly Pro Pro Pro Gln Gly Gly Asn Lys Pro Gln
            180                 185                 190

Gly Pro Pro Pro Pro Gly Lys Pro Gln Gly Pro Pro Pro Gln Gly Asp
        195                 200                 205

Lys Ser Arg Ser Pro Arg Ser Pro Pro Gly Lys Pro Gln Gly Pro Pro
    210                 215                 220

Pro Gln Gly Gly Asn Gln Pro Gln Gly Pro Pro Pro Pro Pro Gly Lys
225                 230                 235                 240

Pro Gln Gly Pro Pro Pro Gln Gly Gly Asn Arg Pro Gln Gly Pro Pro
                245                 250                 255

Pro Pro Gly Lys Pro Gln Gly Pro Pro Pro Gln Gly Asp Lys Ser Arg
            260                 265                 270

Ser Ser Gln Ser Pro Pro Gly Lys Pro Gln Gly Pro Pro Pro Gln Gly
        275                 280                 285

Gly Asn Gln Pro Gln Gly Pro Pro Pro Pro Pro Gly Lys Pro Gln Gly
    290                 295                 300
```

```
Pro Pro Pro Gln Gly Gly Asn Lys Pro Gln Gly Pro Pro Pro Pro Gly
305                 310                 315                 320

Lys Pro Gln Gly Pro Pro Ala Gln Gly Gly Ser Lys Ser Gln Ser Ala
                325                 330                 335

Arg Ser Pro Pro Gly Lys Pro Gln Gly Pro Pro Gln Gln Glu Gly Asn
            340                 345                 350

Asn Pro Gln Gly Pro Pro Pro Pro Ala Gly Gly Asn Pro Gln Gln Pro
        355                 360                 365

Gln Ala Pro Pro Ala Gly Gln Pro Gln Gly Pro Pro Arg Pro Pro Gln
    370                 375                 380

Gly Gly Arg Pro Ser Arg Pro Pro Gln
385                 390
```

<210> 13
<211> 235
<212> PRT
<213> Homo sapiens

<400> 13

```
Met Leu Ser Leu Leu Leu Leu Ala Leu Pro Val Leu Ala Ser Pro Ala
1               5                   10                  15

Tyr Val Ala Pro Ala Pro Gly Gln Ala Leu Gln Gln Thr Gly Ile Val
            20                  25                  30

Gly Gly Gln Glu Ala Pro Arg Ser Lys Trp Pro Trp Gln Val Ser Leu
        35                  40                  45

Arg Val Arg Gly Pro Tyr Trp Met His Phe Cys Gly Gly Ser Leu Ile
    50                  55                  60

His Pro Gln Trp Val Leu Thr Ala Ala His Cys Val Glu Pro Asp Ile
65                  70                  75                  80

Lys Asp Leu Ala Ala Leu Arg Val Gln Leu Arg Glu Gln His Leu Tyr
                85                  90                  95

Tyr Gln Asp Gln Leu Leu Pro Val Ser Arg Ile Ile Val His Pro Gln
            100                 105                 110

Phe Tyr Ile Ile Gln Thr Gly Ala Asp Ile Ala Leu Leu Glu Leu Glu
        115                 120                 125

Glu Pro Val Asn Ile Ser Ser His Ile His Thr Val Thr Leu Pro Pro
    130                 135                 140

Ala Ser Glu Thr Phe Pro Pro Gly Met Pro Cys Trp Val Thr Gly Trp
145                 150                 155                 160

Gly Asp Val Asp Asn Asn Val His Leu Pro Pro Pro Tyr Pro Leu Lys
                165                 170                 175

Glu Val Glu Val Pro Val Val Glu Asn His Leu Cys Asn Ala Glu Tyr
            180                 185                 190

His Thr Gly Leu His Thr Gly His Ser Phe Gln Ile Val Arg Asp Asp
        195                 200                 205

Met Leu Cys Ala Gly Ser Glu Asn His Asp Ser Cys Gln Gly Asp Ser
    210                 215                 220

Gly Gly Pro Leu Val Cys Lys Val Asn Gly Thr
225                 230                 235
```

<210> 14

<211> 321
<212> PRT
<213> Homo sapiens

<400> 14

```
Met Ala Leu Gly Ala Cys Gly Leu Leu Leu Leu Leu Ala Val Pro Gly
1               5                   10                  15

Val Ser Leu Arg Thr Leu Gln Pro Gly Cys Gly Arg Pro Gln Val Ser
            20                  25                  30

Asp Ala Gly Gly Arg Ile Val Gly Gly His Ala Ala Pro Ala Gly Ala
        35                  40                  45

Trp Pro Trp Gln Ala Ser Leu Arg Leu Arg Arg Met His Val Cys Gly
    50                  55                  60

Gly Ser Leu Leu Ser Pro Gln Trp Val Leu Thr Ala Ala His Cys Phe
65                  70                  75                  80

Ser Gly Ser Leu Asn Ser Ser Asp Tyr Gln Val His Leu Gly Glu Leu
                85                  90                  95

Glu Ile Thr Leu Ser Pro His Phe Ser Thr Val Arg Gln Ile Ile Leu
            100                 105                 110
```

```
His Ser Ser Pro Ser Gly Gln Pro Gly Thr Ser Gly Asp Ile Ala Leu
        115                 120                 125

Val Glu Leu Ser Val Pro Val Thr Leu Ser Ser Arg Ile Leu Pro Val
    130                 135                 140

Cys Leu Pro Glu Ala Ser Asp Asp Phe Cys Pro Gly Ile Arg Cys Trp
145                 150                 155                 160

Val Thr Gly Trp Gly Tyr Thr Arg Glu Gly Glu Pro Leu Pro Pro Pro
                165                 170                 175

Tyr Ser Leu Arg Glu Val Lys Val Ser Val Val Asp Thr Glu Thr Cys
            180                 185                 190

Arg Arg Asp Tyr Pro Gly Pro Gly Gly Ser Ile Leu Gln Pro Asp Met
        195                 200                 205

Leu Cys Ala Arg Gly Pro Gly Asp Ala Cys Gln Asp Asp Ser Gly Gly
    210                 215                 220

Pro Leu Val Cys Gln Val Asn Gly Ala Trp Val Gln Ala Gly Ile Val
225                 230                 235                 240

Ser Trp Gly Glu Gly Cys Gly Arg Pro Asn Arg Pro Gly Val Tyr Thr
                245                 250                 255

Arg Val Pro Ala Tyr Val Asn Trp Ile Arg Arg His Ile Thr Ala Ser
            260                 265                 270

Gly Gly Ser Glu Ser Gly Tyr Pro Arg Leu Pro Leu Leu Ala Gly Leu
        275                 280                 285

Phe Leu Pro Gly Leu Phe Leu Leu Leu Val Ser Cys Val Leu Leu Ala
    290                 295                 300

Lys Cys Leu Leu His Pro Ser Ala Asp Gly Thr Pro Phe Pro Ala Pro
305                 310                 315                 320

Asp
```

<210> 15
<211> 540
<212> PRT
<213> Homo sapiens

<400> 15

```
Met Ala Lys Gly Glu Gly Ala Glu Ser Gly Ser Ala Ala Gly Leu Leu
1               5                   10                  15

Pro Thr Ser Ile Leu Gln Ser Thr Glu Arg Pro Ala Gln Val Lys Lys
            20                  25                  30

Glu Pro Lys Lys Lys Lys Gln Gln Leu Ser Val Cys Asn Lys Leu Cys
        35                  40                  45

Tyr Ala Leu Gly Gly Ala Pro Tyr Gln Val Thr Gly Cys Ala Leu Gly
    50                  55                  60

Phe Phe Leu Gln Ile Tyr Leu Leu Asp Val Ala Gln Lys Asp Glu Glu
65                  70                  75                  80

Val Val Phe Cys Phe Ser Ser Phe Gln Val Gly Pro Phe Ser Ala Ser
                85                  90                  95

Ile Ile Leu Phe Val Gly Arg Ala Trp Asp Ala Ile Thr Asp Pro Leu
            100                 105                 110

Val Gly Leu Cys Ile Ser Lys Ser Pro Trp Thr Cys Leu Gly Arg Leu
        115                 120                 125

Met Pro Trp Ile Ile Phe Ser Thr Pro Leu Ala Val Ile Ala Tyr Phe
    130                 135                 140

Leu Ile Trp Phe Val Pro Asp Phe Pro His Gly Gln Thr Tyr Trp Tyr
145                 150                 155                 160

Leu Leu Phe Tyr Cys Leu Phe Glu Thr Met Val Thr Cys Phe His Val
                165                 170                 175

Pro Tyr Ser Ala Leu Thr Met Phe Ile Ser Thr Glu Gln Thr Glu Arg
            180                 185                 190

Asp Ser Ala Thr Ala Tyr Arg Met Thr Val Glu Val Leu Gly Thr Val
        195                 200                 205

Leu Gly Thr Ala Ile Gln Gly Gln Ile Val Gly Gln Ala Asp Thr Pro
    210                 215                 220

Cys Phe Gln Asp Leu Asn Ser Ser Thr Val Ala Ser Gln Ser Ala Asn
225                 230                 235                 240
```

```
His Thr His Gly Thr Thr Ser His Arg Glu Thr Gln Lys Ala Tyr Leu
                245                 250                 255

Leu Ala Ala Gly Val Ile Val Cys Ile Tyr Ile Ile Cys Ala Val Ile
            260                 265                 270

Leu Ile Leu Gly Val Arg Glu Gln Arg Glu Pro Tyr Glu Ala Gln Gln
        275                 280                 285

Ser Glu Pro Ile Ala Tyr Phe Arg Gly Leu Arg Leu Val Met Ser His
    290                 295                 300

Gly Pro Tyr Ile Lys Leu Ile Thr Gly Phe Leu Phe Thr Ser Leu Ala
305                 310                 315                 320

Phe Met Leu Val Glu Gly Asn Phe Val Leu Phe Cys Thr Tyr Thr Leu
                325                 330                 335

Gly Phe Arg Asn Glu Phe Gln Asn Leu Leu Leu Ala Ile Met Leu Ser
            340                 345                 350

Ala Thr Leu Thr Ile Pro Ile Trp Gln Trp Phe Leu Thr Arg Phe Gly
        355                 360                 365

Lys Lys Thr Ala Val Tyr Val Gly Ile Ser Ser Ala Val Pro Phe Leu
    370                 375                 380

Ile Leu Val Ala Leu Met Glu Ser Asn Leu Ile Ile Thr Tyr Ala Val
385                 390                 395                 400

Ala Val Ala Ala Gly Ile Ser Val Ala Ala Ala Phe Leu Leu Pro Trp
                405                 410                 415

Ser Met Leu Pro Asp Val Ile Asp Asp Phe His Leu Lys Gln Pro His
            420                 425                 430

Phe His Gly Thr Glu Pro Ile Phe Phe Ser Phe Tyr Val Phe Phe Thr
        435                 440                 445

Lys Phe Ala Ser Gly Val Ser Leu Gly Ile Ser Thr Leu Ser Leu Asp
    450                 455                 460

Phe Ala Gly Tyr Gln Thr Arg Gly Cys Ser Gln Pro Glu Arg Val Lys
465                 470                 475                 480
```

```
Phe Thr Leu Asn Met Leu Val Thr Met Ala Pro Ile Val Leu Ile Leu
                485                 490                 495

Leu Gly Leu Leu Leu Phe Lys Met Tyr Pro Ile Asp Glu Glu Arg Arg
            500                 505                 510

Arg Gln Asn Lys Lys Ala Leu Gln Ala Leu Arg Asp Glu Ala Ser Ser
        515                 520                 525

Ser Gly Cys Ser Glu Thr Asp Ser Thr Glu Leu Ala
    530                 535                 540
```

<210> 16
<211> 417
<212> PRT
<213> Homo sapiens

<400> 16

```
Met Arg Leu Ile Leu Pro Val Gly Leu Ile Ala Thr Thr Leu Ala Ile
1               5                   10                  15

Ala Pro Val Arg Phe Asp Arg Glu Lys Val Phe Arg Val Lys Pro Gln
            20                  25                  30

Asp Glu Lys Gln Ala Asp Ile Ile Lys Asp Leu Ala Lys Thr Asn Glu
        35                  40                  45

Leu Asp Phe Trp Tyr Pro Gly Ala Thr His His Val Ala Ala Asn Met
    50                  55                  60

Met Val Asp Phe Arg Val Ser Glu Lys Glu Ser Gln Ala Ile Gln Ser
65                  70                  75                  80

Ala Leu Asp Gln Asn Lys Met His Tyr Glu Ile Leu Ile His Asp Leu
                85                  90                  95

Gln Glu Glu Ile Glu Lys Gln Phe Asp Val Lys Glu Asp Ile Pro Gly
            100                 105                 110

Arg His Ser Tyr Ala Lys Tyr Asn Asn Trp Glu Lys Ile Val Ala Trp
        115                 120                 125

Thr Glu Lys Met Met Asp Lys Tyr Pro Glu Met Val Ser Arg Ile Lys
    130                 135                 140

Ile Gly Ser Thr Val Glu Asp Asn Pro Leu Tyr Val Leu Lys Ile Gly
145                 150                 155                 160
```

```
Glu Lys Asn Glu Arg Arg Lys Ala Ile Phe Met Asp Cys Gly Ile His
                165                 170                 175

Ala Arg Glu Trp Val Ser Pro Ala Phe Cys Gln Trp Phe Val Tyr Gln
            180                 185                 190

Ala Thr Lys Thr Tyr Gly Arg Asn Lys Ile Met Thr Lys Leu Leu Asp
        195                 200                 205

Arg Met Asn Phe Tyr Ile Leu Pro Val Phe Asn Val Asp Gly Tyr Ile
    210                 215                 220

Trp Ser Trp Thr Lys Asn Arg Met Trp Arg Lys Asn Arg Ser Lys Asn
225                 230                 235                 240

Gln Asn Ser Lys Cys Ile Gly Thr Asp Leu Asn Arg Asn Phe Asn Ala
                245                 250                 255

Ser Trp Asn Ser Ile Pro Asn Thr Asn Asp Pro Cys Ala Asp Asn Tyr
            260                 265                 270

Arg Gly Ser Ala Pro Glu Ser Glu Lys Glu Thr Lys Ala Val Thr Asn
        275                 280                 285

Phe Ile Arg Ser His Leu Asn Glu Ile Lys Val Tyr Ile Thr Phe His
    290                 295                 300

Ser Tyr Ser Gln Met Leu Leu Phe Pro Tyr Gly Tyr Thr Ser Lys Leu
305                 310                 315                 320

Pro Pro Asn His Glu Asp Leu Ala Lys Val Ala Lys Ile Gly Thr Asp
                325                 330                 335

Val Leu Ser Thr Arg Tyr Glu Thr Arg Tyr Ile Tyr Gly Pro Ile Glu
            340                 345                 350

Ser Thr Ile Tyr Pro Ile Ser Gly Ser Ser Leu Asp Trp Ala Tyr Asp
        355                 360                 365

Leu Gly Ile Lys His Thr Phe Ala Phe Glu Leu Arg Asp Lys Gly Lys
    370                 375                 380

Phe Gly Phe Leu Leu Pro Glu Ser Arg Ile Lys Pro Thr Cys Arg Glu
385                 390                 395                 400
```

```
Thr Met Leu Ala Val Lys Phe Ile Ala Lys Tyr Ile Leu Lys His Thr
                405                 410                 415

Ser
```

<210> 17
<211> 338
<212> PRT
<213> Homo sapiens

<400> 17

```
Met Ile Asn Ser Thr Ser Thr Gln Pro Pro Asp Glu Ser Cys Ser Gln
1               5                   10                  15

Asn Leu Leu Ile Thr Gln Gln Ile Ile Pro Val Leu Tyr Cys Met Val
            20                  25                  30

Phe Ile Ala Gly Ile Leu Leu Asn Gly Val Ser Gly Trp Ile Phe Phe
        35                  40                  45

Tyr Val Pro Ser Ser Lys Ser Phe Ile Ile Tyr Leu Lys Asn Ile Val
    50                  55                  60

Ile Ala Asp Phe Val Met Ser Leu Thr Phe Pro Phe Lys Ile Leu Gly
65                  70                  75                  80

Asp Ser Gly Leu Gly Pro Trp Gln Leu Asn Val Phe Val Cys Arg Val
                85                  90                  95

Ser Ala Val Leu Phe Tyr Val Asn Met Tyr Val Ser Ile Val Phe Phe
            100                 105                 110

Gly Leu Ile Ser Phe Asp Arg Tyr Tyr Lys Ile Val Lys Pro Leu Trp
        115                 120                 125

Thr Ser Phe Ile Gln Ser Val Ser Tyr Ser Lys Leu Leu Ser Val Ile
    130                 135                 140

Val Trp Met Leu Met Leu Leu Leu Ala Val Pro Asn Ile Ile Leu Thr
145                 150                 155                 160

Asn Gln Ser Val Arg Glu Val Thr Gln Ile Lys Cys Ile Glu Leu Lys
                165                 170                 175

Ser Glu Leu Gly Arg Lys Trp His Lys Ala Ser Asn Tyr Ile Phe Val
            180                 185                 190
```

```
Ala Ile Phe Trp Ile Val Phe Leu Leu Leu Ile Val Phe Tyr Thr Ala
        195                 200                 205

Ile Thr Lys Lys Ile Phe Lys Ser His Leu Lys Ser Ser Arg Asn Ser
    210                 215                 220

Thr Ser Val Lys Lys Lys Ser Ser Arg Asn Ile Phe Ser Ile Val Phe
225                 230                 235                 240

Val Phe Phe Val Cys Phe Val Pro Tyr His Ile Ala Arg Ile Pro Tyr
                245                 250                 255

Thr Lys Ser Gln Thr Glu Ala His Tyr Ser Cys Gln Ser Lys Glu Ile
            260                 265                 270

Leu Arg Tyr Met Lys Glu Phe Thr Leu Leu Leu Ser Ala Ala Asn Val
        275                 280                 285

Cys Leu Asp Pro Ile Ile Tyr Phe Phe Leu Cys Gln Pro Phe Arg Glu
    290                 295                 300

Ile Leu Cys Lys Lys Leu His Ile Pro Leu Lys Ala Gln Asn Asp Leu
305                 310                 315                 320

Asp Ile Ser Arg Ile Lys Arg Gly Asn Thr Thr Leu Glu Ser Thr Asp
                325                 330                 335

Thr Leu
```

<210> 18
<211> 852
<212> PRT
<213> Homo sapiens

<400> 18

```
Met Ala Met Arg Ser Gly Arg His Pro Ser Leu Leu Leu Leu Leu Val
1               5                   10                  15

Leu Leu Leu Trp Leu Leu Gln Val Ser Ile Ile Asp Ser Val Gln Gln
            20                  25                  30

Glu Thr Asp Asp Leu Thr Lys Gln Thr Lys Glu Lys Ile Tyr Gln Pro
        35                  40                  45

Leu Arg Arg Ser Lys Arg Arg Trp Val Ile Thr Thr Leu Glu Leu Glu
```

```
    50                  55                  60

Glu Glu Asp Pro Gly Pro Phe Pro Lys Leu Ile Gly Glu Leu Phe Asn
65                  70                  75                  80

Asn Met Ser Tyr Asn Met Ser Leu Met Tyr Leu Ile Ser Gly Pro Gly
                85                  90                  95

Val Asp Glu Tyr Pro Glu Ile Gly Leu Phe Ser Leu Glu Asp His Glu
            100                 105                 110

Asn Gly Arg Ile Tyr Val His Arg Pro Val Asp Arg Glu Met Thr Pro
        115                 120                 125

Ser Phe Thr Val Tyr Phe Asp Val Val Glu Arg Ser Thr Gly Lys Ile
    130                 135                 140

Val Asp Thr Ser Leu Ile Phe Asn Ile Arg Ile Ser Asp Val Asn Asp
145                 150                 155                 160

His Ala Pro Gln Phe Pro Glu Lys Glu Phe Asn Ile Thr Val Gln Glu
                165                 170                 175

Asn Gln Ser Ala Gly Gln Pro Ile Phe Gln Met Leu Ala Val Asp Leu
            180                 185                 190

Asp Glu Glu Asn Thr Pro Asn Ser Gln Val Leu Tyr Phe Leu Ile Ser
        195                 200                 205

Gln Thr Pro Leu Leu Lys Glu Ser Gly Phe Arg Val Asp Arg Leu Ser
    210                 215                 220

Gly Glu Ile Arg Leu Ser Gly Cys Leu Asp Tyr Glu Thr Ala Pro Gln
225                 230                 235                 240

Phe Thr Leu Leu Ile Arg Ala Arg Asp Cys Gly Glu Pro Ser Leu Ser
                245                 250                 255

Ser Thr Thr Thr Val His Val Asp Val Gln Glu Gly Asn Asn His Arg
            260                 265                 270

Pro Ala Phe Thr Gln Glu Asn Tyr Lys Val Gln Ile Pro Glu Gly Arg
        275                 280                 285

Ala Ser Gln Gly Val Leu Arg Leu Leu Val Gln Asp Arg Asp Ser Pro
    290                 295                 300
```

```
Phe Thr Ser Ala Trp Arg Ala Lys Phe Asn Ile Leu His Gly Asn Glu
305                 310                 315                 320

Glu Gly His Phe Asp Ile Ser Thr Asp Pro Glu Thr Asn Glu Gly Ile
                325                 330                 335

Leu Asn Val Ile Lys Pro Leu Asp Tyr Glu Thr Arg Pro Ala Gln Ser
            340                 345                 350

Leu Ile Ile Val Val Glu Asn Glu Glu Arg Leu Val Phe Cys Glu Arg
        355                 360                 365

Gly Lys Leu Gln Pro Pro Arg Lys Ala Ala Ala Ser Ala Thr Val Ser
    370                 375                 380

Val Gln Val Thr Asp Ala Asn Asp Pro Pro Ala Phe His Pro Gln Ser
385                 390                 395                 400

Phe Ile Val Asn Lys Glu Glu Gly Ala Arg Pro Gly Thr Leu Leu Gly
                405                 410                 415

Thr Phe Asn Ala Met Asp Pro Asp Ser Gln Ile Arg Tyr Glu Leu Val
            420                 425                 430

His Asp Pro Ala Asn Trp Val Ser Val Asp Lys Asn Ser Gly Val Val
        435                 440                 445

Ile Thr Val Glu Pro Ile Asp Arg Glu Ser Pro His Val Asn Asn Ser
    450                 455                 460

Phe Tyr Val Ile Ile Ile His Ala Val Asp Asp Gly Phe Pro Pro Gln
465                 470                 475                 480

Thr Ala Thr Gly Thr Leu Met Leu Phe Leu Ser Asp Ile Asn Asp Asn
                485                 490                 495

Val Pro Thr Leu Arg Pro Arg Ser Arg Tyr Met Glu Val Cys Glu Ser
            500                 505                 510

Ala Val His Glu Pro Leu His Ile Glu Ala Glu Asp Pro Asp Leu Glu
        515                 520                 525

Pro Phe Ser Asp Pro Phe Thr Phe Glu Leu Asp Asn Thr Trp Gly Asn
    530                 535                 540
```

Ala Glu Asp Thr Trp Lys Leu Gly Arg Asn Trp Gly Gln Ser Val Glu
545 550 555 560

Leu Leu Thr Leu Arg Ser Leu Pro Arg Gly Asn Tyr Leu Val Pro Leu
565 570 575

Phe Ile Gly Asp Lys Gln Gly Leu Ser Gln Lys Gln Thr Val His Val
580 585 590

Arg Ile Cys Pro Cys Ala Ser Gly Leu Thr Cys Val Glu Leu Ala Asp
595 600 605

Ala Glu Val Gly Leu His Val Gly Ala Leu Phe Pro Val Cys Ala Ala
610 615 620

Phe Val Ala Leu Ala Val Ala Leu Leu Phe Leu Leu Arg Cys Tyr Phe
625 630 635 640

Val Leu Glu Pro Lys Arg His Gly Cys Ser Val Ser Asn Asp Glu Gly
645 650 655

His Gln Thr Leu Val Met Tyr Asn Ala Glu Ser Lys Gly Thr Ser Ala
660 665 670

Gln Thr Trp Ser Asp Val Glu Gly Gln Arg Pro Ala Leu Leu Ile Cys
675 680 685

Thr Ala Ala Ala Gly Pro Thr Gln Gly Val Lys Ala Tyr Pro Asp Ala
690 695 700

Thr Met His Arg Gln Leu Leu Ala Pro Val Glu Gly Arg Met Ala Glu
705 710 715 720

Thr Leu Asn Gln Ser Lys Glu Arg Asn Arg Phe Ser Leu Ser Arg Gly
725 730 735

Cys Ile Ile Pro Gln Gly Arg Ala Thr Ala Gly Arg Gly Leu Pro Gln
740 745 750

Asp Ile Tyr Lys Glu Met Met Pro Arg Arg Leu Thr Gln Thr Gly Lys
755 760 765

Arg Lys His Gly Ala Leu Ala Arg Thr Pro Ser Phe Lys Lys Val Val
770 775 780

```
Tyr Asp His Lys Glu Asp Glu Glu Asn Lys Ala Gly Arg Lys Gln Arg
785                 790                 795                 800

Ser His Leu Phe Lys Val Met Gln Leu Arg Asn Glu Gln Gly Gly Val
                805                 810                 815

Arg Val Gln Ser Ala His Ser Pro Ser Pro Leu Asn Lys Lys Ala Cys
            820                 825                 830

Phe Pro Gly Asp Tyr Arg Gly Glu Ser Ala Gly Gly His Asn Cys Arg
        835                 840                 845

Ala Val Ser Gly
    850
```

<210> 19
<211> 782
<212> PRT
<213> Homo sapiens

<400> 19

```
Met Ala Pro His Arg Pro Ala Pro Ala Leu Leu Cys Ala Leu Ser Leu
1               5                   10                  15

Ala Leu Cys Ala Leu Ser Leu Pro Val Arg Ala Ala Thr Ala Ser Arg
            20                  25                  30

Gly Ala Ser Gln Ala Gly Ala Pro Gln Gly Arg Val Pro Glu Ala Arg
        35                  40                  45

Pro Asn Ser Met Val Val Glu His Pro Glu Phe Leu Lys Ala Gly Lys
    50                  55                  60

Glu Pro Gly Leu Gln Ile Trp Arg Val Glu Lys Phe Asp Leu Val Pro
65                  70                  75                  80

Val Pro Thr Asn Leu Tyr Gly Asp Phe Phe Thr Gly Asp Ala Tyr Val
                85                  90                  95

Ile Leu Lys Thr Val Gln Leu Arg Asn Gly Asn Leu Gln Tyr Asp Leu
            100                 105                 110

His Tyr Trp Leu Gly Asn Glu Cys Ser Gln Asp Glu Ser Gly Ala Ala
        115                 120                 125

Ala Ile Phe Thr Val Gln Leu Asp Asp Tyr Leu Asn Gly Arg Ala Val
    130                 135                 140
```

```
Gln His Arg Glu Val Gln Gly Phe Glu Ser Ala Thr Phe Leu Gly Tyr
145                 150                 155                 160

Phe Lys Ser Gly Leu Lys Tyr Lys Lys Gly Gly Val Ala Ser Gly Phe
                165                 170                 175

Lys His Val Val Pro Asn Glu Val Val Val Gln Arg Leu Phe Gln Val
            180                 185                 190

Lys Gly Arg Arg Val Val Arg Ala Thr Glu Val Pro Val Ser Trp Glu
        195                 200                 205

Ser Phe Asn Asn Gly Asp Cys Phe Ile Leu Asp Leu Gly Asn Asn Ile
    210                 215                 220

His Gln Trp Cys Gly Ser Asn Ser Asn Arg Tyr Glu Arg Leu Lys Ala
225                 230                 235                 240

Thr Gln Val Ser Lys Gly Ile Arg Asp Asn Glu Arg Ser Gly Arg Ala
                245                 250                 255

Arg Val His Val Ser Glu Glu Gly Thr Glu Pro Glu Ala Met Leu Gln
            260                 265                 270

Val Leu Gly Pro Lys Pro Ala Leu Pro Ala Gly Thr Glu Asp Thr Ala
        275                 280                 285

Lys Glu Asp Ala Ala Asn Arg Lys Leu Ala Lys Leu Tyr Lys Val Ser
    290                 295                 300

Asn Gly Ala Gly Thr Met Ser Val Ser Leu Val Ala Asp Glu Asn Pro
305                 310                 315                 320

Phe Ala Gln Gly Ala Leu Lys Ser Glu Asp Cys Phe Ile Leu Asp His
                325                 330                 335

Gly Lys Asp Gly Lys Ile Phe Val Trp Lys Gly Lys Gln Ala Asn Thr
            340                 345                 350

Glu Glu Arg Lys Ala Ala Leu Lys Thr Ala Ser Asp Phe Ile Thr Lys
        355                 360                 365

Met Asp Tyr Pro Lys Gln Thr Gln Val Ser Val Leu Pro Glu Gly Gly
    370                 375                 380
```

```
Glu Thr Pro Leu Phe Lys Gln Phe Phe Lys Asn Trp Arg Asp Pro Asp
385                 390                 395                 400

Gln Thr Asp Gly Leu Gly Leu Ser Tyr Leu Ser Ser His Ile Ala Asn
                405                 410                 415

Val Glu Arg Val Pro Phe Asp Ala Ala Thr Leu His Thr Ser Thr Ala
            420                 425                 430

Met Ala Ala Gln His Gly Met Asp Asp Asp Gly Thr Gly Gln Lys Gln
        435                 440                 445

Ile Trp Arg Ile Glu Gly Ser Asn Lys Val Pro Val Asp Pro Ala Thr
    450                 455                 460

Tyr Gly Gln Phe Tyr Gly Gly Asp Ser Tyr Ile Ile Leu Tyr Asn Tyr
465                 470                 475                 480

Arg His Gly Gly Arg Gln Gly Gln Ile Ile Tyr Asn Trp Gln Gly Ala
                485                 490                 495

Gln Ser Thr Gln Asp Glu Val Ala Ala Ser Ala Ile Leu Thr Ala Gln
            500                 505                 510

Leu Asp Glu Glu Leu Gly Gly Thr Pro Val Gln Ser Arg Val Val Gln
        515                 520                 525

Gly Lys Glu Pro Ala His Leu Met Ser Leu Phe Gly Gly Lys Pro Met
    530                 535                 540

Ile Ile Tyr Lys Gly Gly Thr Ser Arg Glu Gly Gly Gln Thr Ala Pro
545                 550                 555                 560

Ala Ser Thr Arg Leu Phe Gln Val Arg Ala Asn Ser Ala Gly Ala Thr
                565                 570                 575

Arg Ala Val Glu Val Leu Pro Lys Ala Gly Ala Leu Asn Ser Asn Asp
            580                 585                 590

Ala Phe Val Leu Lys Thr Pro Ser Ala Ala Tyr Leu Trp Val Gly Thr
        595                 600                 605

Gly Ala Ser Glu Ala Glu Lys Thr Gly Ala Gln Glu Leu Leu Arg Val
    610                 615                 620

Leu Arg Ala Gln Pro Val Gln Val Ala Glu Gly Ser Glu Pro Asp Gly
```

```
625                 630                 635                 640

Phe Trp Glu Ala Leu Gly Gly Lys Ala Ala Tyr Arg Thr Ser Pro Arg
                645                 650                 655

Leu Lys Asp Lys Lys Met Asp Ala His Pro Pro Arg Leu Phe Ala Cys
            660                 665                 670

Ser Asn Lys Ile Gly Arg Phe Val Ile Glu Glu Val Pro Gly Glu Leu
        675                 680                 685

Met Gln Glu Asp Leu Ala Thr Asp Asp Val Met Leu Leu Asp Thr Trp
    690                 695                 700

Asp Gln Val Phe Val Trp Val Gly Lys Asp Ser Gln Glu Glu Glu Lys
705                 710                 715                 720

Thr Glu Ala Leu Thr Ser Ala Lys Arg Tyr Ile Glu Thr Asp Pro Ala
                725                 730                 735

Asn Arg Asp Arg Arg Thr Pro Ile Thr Val Val Lys Gln Gly Phe Glu
            740                 745                 750

Pro Pro Ser Phe Val Gly Trp Phe Leu Gly Trp Asp Asp Asp Tyr Trp
        755                 760                 765

Ser Val Asp Pro Leu Asp Arg Ala Met Ala Glu Leu Ala Ala
    770                 775                 780
```

<210> 20
<211> 164
<212> PRT
<213> Homo sapiens

<400> 20

```
Met Gly Asp Leu Pro Gly Leu Val Arg Leu Ser Ile Ala Leu Arg Ile
1               5                   10                  15

Gln Pro Asn Asp Gly Pro Val Phe Tyr Lys Val Asp Gly Gln Arg Phe
            20                  25                  30

Gly Gln Asn Arg Thr Ile Lys Leu Leu Thr Gly Ser Ser Tyr Lys Val
        35                  40                  45

Glu Val Lys Ile Lys Pro Ser Thr Leu Gln Val Glu Asn Ile Ser Ile
    50                  55                  60
```

```
Gly Gly Val Leu Val Pro Leu Glu Leu Lys Ser Lys Glu Pro Asp Gly
65                  70                  75                  80

Asp Arg Val Val Tyr Thr Gly Thr Tyr Asp Thr Glu Gly Val Thr Pro
                85                  90                  95

Thr Lys Ser Gly Glu Arg Gln Pro Ile Gln Ile Thr Met Pro Phe Thr
            100                 105                 110

Asp Ile Gly Thr Phe Glu Thr Val Trp Gln Val Lys Phe Tyr Asn Tyr
        115                 120                 125

His Lys Arg Asp His Cys Gln Trp Gly Ser Pro Phe Ser Val Ile Glu
    130                 135                 140

Tyr Glu Cys Lys Pro Asn Glu Thr Arg Ser Leu Met Trp Val Asn Lys
145                 150                 155                 160

Glu Ser Phe Leu
```

<210> 21
<211> 295
<212> PRT
<213> Homo sapiens

<400> 21

```
Met Tyr Arg Ile Ser Gln Leu Met Ser Thr Pro Val Ala Ser Ser Ser
1               5                   10                  15

Arg Leu Glu Arg Glu Tyr Ala Gly Glu Leu Ser Pro Thr Cys Ile Phe
            20                  25                  30

Pro Ser Phe Thr Cys Asp Ser Leu Asp Gly Tyr His Ser Phe Glu Cys
        35                  40                  45

Gly Ser Ile Asp Pro Leu Thr Gly Ser His Tyr Thr Cys Arg Arg Ser
    50                  55                  60

Pro Arg Leu Leu Thr Asn Gly Tyr Tyr Ile Trp Thr Glu Asp Ser Phe
65                  70                  75                  80

Leu Cys Asp Lys Asp Gly Asn Ile Thr Leu Asn Pro Ser Gln Thr Ser
                85                  90                  95

Val Met Tyr Lys Glu Asn Leu Val Arg Ile Phe Arg Lys Lys Lys Arg
            100                 105                 110
```

```
Ile Cys His Ser Phe Ser Ser Leu Phe Asn Leu Ser Thr Ser Lys Ser
        115                 120                 125

Trp Leu His Gly Ser Ile Phe Gly Asp Ile Asn Ser Ser Pro Ser Glu
    130                 135                 140

Asp Asn Trp Leu Lys Gly Thr Arg Arg Leu Asp Thr Asp His Cys Asn
145                 150                 155                 160

Gly Asn Ala Asp Asp Leu Asp Cys Ser Ser Leu Thr Asp Asp Trp Glu
                165                 170                 175

Ser Gly Lys Met Asn Ala Glu Ser Val Ile Thr Ser Ser Ser Ser His
            180                 185                 190

Ile Ile Ser Gln Pro Pro Gly Gly Asn Ser His Ser Leu Ser Leu Gln
        195                 200                 205

Ser Gln Leu Thr Ala Ser Glu Arg Phe Gln Glu Asn Ser Ser Asp His
    210                 215                 220

Ser Glu Thr Arg Leu Leu Gln Glu Val Phe Phe Gln Ala Ile Leu Leu
225                 230                 235                 240

Ala Val Cys Leu Ile Ile Ser Ala Cys Ala Arg Trp Phe Met Gly Glu
                245                 250                 255

Ile Leu Ala Ser Val Phe Thr Cys Ser Leu Met Ile Thr Val Ala Tyr
            260                 265                 270

Val Lys Ser Leu Phe Leu Ser Leu Ala Ser Tyr Phe Lys Thr Thr Ala
        275                 280                 285

Cys Ala Arg Phe Val Lys Ile
    290                 295
```

<210> 22
<211> 198
<212> PRT
<213> Homo sapiens

<400> 22

```
Met Asp Ala Ile Leu Asn Tyr Arg Ser Glu Asp Thr Glu Asp Tyr Tyr
1               5                   10                  15

Thr Leu Leu Gly Cys Asp Glu Leu Ser Ser Val Glu Gln Ile Leu Ala
            20                  25                  30

Glu Phe Lys Val Arg Ala Leu Glu Cys His Pro Asp Lys His Pro Glu
        35                  40                  45

Asn Pro Lys Ala Val Glu Thr Phe Gln Lys Leu Gln Lys Ala Lys Glu
    50                  55                  60

Ile Leu Thr Asn Glu Glu Ser Arg Ala Arg Tyr Asp His Trp Arg Arg
65                  70                  75                  80

Ser Gln Met Ser Met Pro Phe Gln Gln Trp Glu Ala Leu Asn Asp Ser
                85                  90                  95

Val Lys Thr Ser Met His Trp Val Val Arg Gly Lys Lys Asp Leu Met
            100                 105                 110

Leu Glu Glu Ser Asp Lys Thr His Thr Thr Lys Met Glu Asn Glu Glu
        115                 120                 125

Cys Asn Glu Gln Arg Glu Arg Lys Lys Glu Glu Leu Ala Ser Thr Ala
    130                 135                 140

Glu Lys Thr Glu Gln Lys Glu Pro Lys Pro Leu Glu Lys Ser Val Ser
145                 150                 155                 160

Pro Gln Asn Ser Asp Ser Ser Gly Phe Ala Asp Val Asn Gly Trp His
                165                 170                 175

Leu Arg Phe Arg Trp Ser Lys Asp Ala Pro Ser Glu Leu Leu Arg Lys
            180                 185                 190

Phe Arg Asn Tyr Glu Ile
        195
```

<210> 23
<211> 159
<212> PRT
<213> Homo sapiens

<400> 23

```
Met Ser Arg Arg Asn Cys Trp Ile Cys Lys Met Cys Arg Asp Glu Ser
1               5                   10                  15

Lys Arg Pro Pro Ser Asn Leu Thr Leu Glu Glu Val Leu Gln Trp Ala
            20                  25                  30

Gln Ser Phe Glu Asn Leu Met Ala Thr Lys Tyr Gly Pro Val Val Tyr

        35                  40                  45

Ala Ala Tyr Leu Lys Met Glu His Ser Asp Glu Asn Ile Gln Phe Trp
    50                  55                  60

Met Ala Cys Glu Thr Tyr Lys Lys Ile Ala Ser Arg Trp Ser Arg Ile
65                  70                  75                  80

Ser Arg Ala Lys Lys Leu Tyr Lys Ile Tyr Ile Gln Pro Gln Ser Pro
                85                  90                  95

Arg Glu Ile Asn Ile Asp Ser Ser Thr Arg Glu Thr Ile Ile Arg Asn
            100                 105                 110

Ile Gln Glu Pro Thr Glu Thr Cys Phe Glu Glu Ala Gln Lys Ile Val
        115                 120                 125

Tyr Met His Met Glu Arg Asp Ser Tyr Pro Arg Phe Leu Lys Ser Glu
    130                 135                 140

Met Tyr Gln Lys Leu Leu Lys Thr Met Gln Ser Asn Asn Ser Phe
145                 150                 155
```

<210> 24
<211> 514
<212> PRT
<213> Homo sapiens

<400> 24

```
Met Ala Leu Ser Glu Leu Ala Leu Val Arg Trp Leu Gln Glu Ser Arg
1               5                   10                  15

Arg Ser Arg Lys Leu Ile Leu Phe Ile Val Phe Leu Ala Leu Leu Leu
            20                  25                  30

Asp Asn Met Leu Leu Thr Val Val Val Pro Ile Ile Pro Ser Tyr Leu
        35                  40                  45

Tyr Ser Ile Lys His Glu Lys Asn Ala Thr Glu Ile Gln Thr Ala Arg
    50                  55                  60

Pro Val His Thr Ala Ser Ile Ser Asp Ser Phe Gln Ser Ile Phe Ser
65                  70                  75                  80

Tyr Tyr Asp Asn Ser Thr Met Val Thr Gly Asn Ala Thr Arg Asp Leu
                85                  90                  95
```

```
Thr Leu His Gln Thr Ala Thr Gln His Met Val Thr Asn Ala Ser Ala
            100                 105                 110

Val Pro Ser Asp Cys Pro Ser Glu Asp Lys Asp Leu Leu Asn Glu Asn
        115                 120                 125

Val Gln Val Gly Leu Leu Phe Ala Ser Lys Ala Thr Val Gln Leu Ile
    130                 135                 140

Thr Asn Pro Phe Ile Gly Leu Leu Thr Asn Arg Ile Gly Tyr Pro Ile
145                 150                 155                 160

Pro Ile Phe Ala Gly Phe Cys Ile Met Phe Val Ser Thr Ile Met Phe
                165                 170                 175

Ala Phe Ser Ser Ser Tyr Ala Phe Leu Leu Ile Ala Arg Ser Leu Gln
            180                 185                 190

Gly Ile Gly Ser Ser Cys Ser Ser Val Ala Gly Met Gly Met Leu Ala
        195                 200                 205

Ser Val Tyr Thr Asp Asp Glu Glu Arg Gly Asn Val Met Gly Ile Ala
    210                 215                 220

Leu Gly Gly Leu Ala Met Gly Val Leu Val Gly Pro Pro Phe Gly Ser
225                 230                 235                 240

Val Leu Tyr Glu Phe Val Gly Lys Thr Ala Pro Phe Leu Val Leu Ala
                245                 250                 255

Ala Leu Val Leu Leu Asp Gly Ala Ile Gln Leu Phe Val Leu Gln Pro
            260                 265                 270

Ser Arg Val Gln Pro Glu Ser Gln Lys Gly Thr Pro Leu Thr Thr Leu
        275                 280                 285

Leu Lys Asp Pro Tyr Ile Leu Ile Ala Ala Gly Ser Ile Cys Phe Ala
    290                 295                 300

Asn Met Gly Ile Ala Met Leu Glu Pro Ala Leu Pro Ile Trp Met Met
305                 310                 315                 320

Glu Thr Met Cys Ser Arg Lys Trp Gln Leu Gly Val Ala Phe Leu Pro
                325                 330                 335

Ala Ser Ile Ser Tyr Leu Ile Gly Thr Asn Ile Phe Gly Ile Leu Ala
```

340 345 350

His Lys Met Gly Arg Trp Leu Cys Ala Leu Leu Gly Met Ile Ile Val
355 360 365

Gly Val Ser Ile Leu Cys Ile Pro Phe Ala Lys Asn Ile Tyr Gly Leu
370 375 380

Ile Ala Pro Asn Phe Gly Val Gly Phe Ala Ile Gly Met Val Asp Ser
385 390 395 400

Ser Met Met Pro Ile Met Gly Tyr Leu Val Asp Leu Arg His Val Ser
405 410 415

Val Tyr Gly Ser Val Tyr Ala Ile Ala Asp Val Ala Phe Cys Met Gly
420 425 430

Tyr Ala Ile Gly Pro Ser Ala Gly Gly Ala Ile Ala Lys Ala Ile Gly
435 440 445

Phe Pro Trp Leu Met Thr Ile Ile Gly Ile Ile Asp Ile Leu Phe Ala
450 455 460

Pro Leu Cys Phe Phe Leu Arg Ser Pro Pro Ala Lys Glu Glu Lys Met
465 470 475 480

Ala Ile Leu Met Asp His Asn Cys Pro Ile Lys Thr Lys Met Tyr Thr
485 490 495

Gln Asn Asn Ile Gln Ser Tyr Pro Ile Gly Glu Asp Glu Glu Ser Glu
500 505 510

Ser Asp

<210> 25
<211> 397
<212> PRT
<213> Homo sapiens

<400> 25

Met Asp Ser Leu Ala Thr Ser Ile Asn Gln Phe Ala Leu Glu Leu Ser
1 5 10 15

Lys Lys Leu Ala Glu Ser Ala Gln Gly Lys Asn Ile Phe Phe Ser Ser
20 25 30

```
Trp Ser Ile Ser Thr Ser Leu Thr Ile Val Tyr Leu Gly Ala Lys Gly
        35                  40                  45

Thr Thr Ala Ala Gln Met Ala Gln Val Leu Gln Phe Asn Arg Asp Gln
    50                  55                  60

Gly Val Lys Cys Asp Pro Glu Ser Glu Lys Lys Arg Lys Met Glu Phe
65                  70                  75                  80

Asn Leu Ser Asn Ser Glu Glu Ile His Ser Asp Phe Gln Thr Leu Ile
                85                  90                  95

Ser Glu Ile Leu Lys Pro Asn Asp Asp Tyr Leu Leu Lys Thr Ala Asn
            100                 105                 110

Ala Ile Tyr Gly Glu Lys Thr Tyr Ala Phe His Asn Lys Tyr Leu Glu
        115                 120                 125

Asp Met Lys Thr Tyr Phe Gly Ala Glu Pro Gln Pro Val Asn Phe Val
    130                 135                 140

Glu Ala Ser Asp Gln Ile Arg Lys Asp Ile Asn Ser Trp Val Glu Arg
145                 150                 155                 160

Gln Thr Glu Gly Lys Ile Gln Asn Leu Leu Pro Asp Asp Ser Val Asp
                165                 170                 175

Ser Thr Thr Arg Met Ile Leu Val Asn Ala Leu Tyr Phe Lys Gly Ile
            180                 185                 190

Trp Glu His Gln Phe Leu Val Gln Asn Thr Thr Glu Lys Pro Phe Arg
        195                 200                 205

Ile Asn Glu Thr Thr Ser Lys Pro Val Gln Met Met Phe Met Lys Lys
    210                 215                 220

Lys Leu His Ile Phe His Ile Glu Lys Pro Lys Ala Val Gly Leu Gln
225                 230                 235                 240

Leu Tyr Tyr Lys Ser Arg Asp Leu Ser Leu Leu Ile Leu Leu Pro Glu
                245                 250                 255

Asp Ile Asn Gly Leu Glu Gln Leu Glu Lys Ala Ile Thr Tyr Glu Lys
            260                 265                 270

Leu Asn Glu Trp Thr Ser Ala Asp Met Met Glu Leu Tyr Glu Val Gln
```

275 280 285

Leu His Leu Pro Lys Phe Lys Leu Glu Asp Ser Tyr Asp Leu Lys Ser
290 295 300

Thr Leu Ser Ser Met Gly Met Ser Asp Ala Phe Ser Gln Ser Lys Ala
305 310 315 320

Asp Phe Ser Gly Met Ser Ser Ala Arg Asn Leu Phe Leu Ser Asn Val
325 330 335

Phe His Lys Ala Phe Val Glu Ile Asn Glu Gln Gly Thr Glu Ala Ala
340 345 350

Ala Gly Ser Gly Ser Glu Ile Asp Ile Arg Ile Arg Val Pro Ser Ile
355 360 365

Glu Phe Asn Ala Asn His Pro Phe Leu Phe Phe Ile Arg His Asn Lys
370 375 380

Thr Asn Thr Ile Leu Phe Tyr Gly Arg Leu Cys Ser Pro
385 390 395

<210> 26
<211> 729
<212> PRT
<213> Homo sapiens

<400> 26

Met Asp Asp Leu Thr Leu Leu Asp Leu Leu Glu Cys Pro Val Cys Phe
1 5 10 15

Glu Lys Leu Asp Val Thr Ala Lys Val Leu Pro Cys Gln His Thr Phe
20 25 30

Cys Lys Pro Cys Leu Gln Arg Val Phe Lys Ala His Lys Glu Leu Arg
35 40 45

Cys Pro Glu Cys Arg Thr Pro Val Phe Ser Asn Ile Glu Ala Leu Pro
50 55 60

Ala Asn Leu Leu Leu Val Arg Leu Leu Asp Gly Val Arg Ser Gly Gln
65 70 75 80

Ser Ser Gly Arg Gly Gly Ser Phe Arg Arg Pro Gly Thr Met Thr Leu
85 90 95

```
Gln Asp Gly Arg Lys Ser Arg Thr Asn Pro Arg Arg Leu Gln Ala Ser
            100                 105                 110

Pro Phe Arg Leu Val Pro Asn Val Arg Ile His Met Asp Gly Val Pro
        115                 120                 125

Arg Ala Lys Ala Leu Cys Asn Tyr Arg Gly Gln Asn Pro Gly Asp Leu
    130                 135                 140

Arg Phe Asn Lys Gly Asp Ile Ile Leu Leu Arg Arg Gln Leu Asp Glu
145                 150                 155                 160

Asn Trp Tyr Gln Gly Glu Ile Asn Gly Ile Ser Gly Asn Phe Pro Ala
                165                 170                 175

Ser Ser Val Glu Val Ile Lys Gln Leu Pro Gln Pro Pro Pro Leu Cys
            180                 185                 190

Arg Ala Leu Tyr Asn Phe Asp Leu Arg Gly Lys Asp Lys Ser Glu Asn
        195                 200                 205

Gln Asp Cys Leu Thr Phe Leu Lys Asp Asp Ile Ile Thr Val Ile Ser
    210                 215                 220

Arg Val Asp Glu Asn Trp Ala Glu Gly Lys Leu Gly Asp Lys Val Gly
225                 230                 235                 240

Ile Phe Pro Ile Leu Phe Val Glu Pro Asn Leu Thr Ala Arg His Leu
                245                 250                 255

Leu Glu Lys Asn Lys Gly Arg Gln Ser Ser Cys Thr Lys Asn Leu Ser
            260                 265                 270

Leu Val Ser Ser Ser Ser Arg Gly Asn Thr Ser Thr Leu Arg Arg Gly
        275                 280                 285

Pro Gly Ser Arg Arg Lys Val Pro Gly Gln Phe Ser Ile Thr Thr Ala
    290                 295                 300

Leu Asn Thr Leu Asn Arg Met Val His Ser Pro Ser Gly Arg His Met
305                 310                 315                 320

Val Glu Ile Ser Thr Pro Val Leu Ile Ser Ser Ser Asn Pro Ser Val
                325                 330                 335

Ile Thr Gln Pro Met Glu Lys Ala Asp Val Pro Ser Ser Cys Val Gly
```

```
            340                 345                 350

Gln Val Ser Thr Tyr His Pro Ala Pro Val Ser Pro Gly His Ser Thr
        355                 360                 365

Ala Val Val Ser Leu Pro Gly Ser Gln Gln His Leu Ser Ala Asn Met
    370                 375                 380

Phe Val Ala Leu His Ser Tyr Ser Ala His Gly Pro Asp Glu Leu Asp
385                 390                 395                 400

Leu Gln Lys Gly Glu Gly Val Arg Val Leu Gly Lys Cys Gln Asp Gly
                405                 410                 415

Trp Leu Arg Gly Val Ser Leu Val Thr Gly Arg Val Gly Ile Phe Pro
            420                 425                 430

Asn Asn Tyr Val Ile Pro Ile Phe Arg Lys Thr Ser Ser Phe Pro Asp
        435                 440                 445

Ser Arg Ser Pro Gly Leu Tyr Thr Thr Trp Thr Leu Ser Thr Ser Ser
    450                 455                 460

Val Ser Ser Gln Gly Ser Ile Ser Glu Gly Asp Pro Arg Gln Ser Arg
465                 470                 475                 480

Pro Phe Lys Ser Val Phe Val Pro Thr Ala Ile Val Asn Pro Val Arg
                485                 490                 495

Ser Thr Ala Gly Pro Gly Thr Leu Gly Gln Gly Ser Leu Arg Lys Gly
            500                 505                 510

Arg Ser Ser Met Arg Lys Asn Gly Ser Leu Gln Arg Pro Leu Gln Ser
        515                 520                 525

Gly Ile Pro Thr Leu Val Val Gly Ser Leu Arg Arg Ser Pro Thr Met
    530                 535                 540

Val Leu Arg Pro Gln Gln Phe Gln Phe Tyr Gln Pro Gln Gly Ile Pro
545                 550                 555                 560

Ser Ser Pro Ser Ala Val Val Val Glu Met Gly Ser Lys Pro Ala Leu
                565                 570                 575

Thr Gly Glu Pro Ala Leu Thr Cys Ile Ser Arg Gly Ser Glu Ala Arg
            580                 585                 590
```

```
Ile His Ser Ala Ala Ser Ser Leu Ile Met Glu Asp Lys Glu Ile Pro
        595                 600                 605

Ile Lys Ser Glu Pro Leu Pro Lys Pro Pro Ala Ser Ala Pro Pro Ser
    610                 615                 620

Ile Leu Val Lys Pro Glu Asn Ser Arg Asn Gly Ile Glu Lys Gln Val
625                 630                 635                 640

Lys Thr Val Arg Phe Gln Asn Tyr Ser Pro Pro Pro Thr Lys His Tyr
                645                 650                 655

Thr Ser His Pro Thr Ser Gly Lys Pro Glu Gln Pro Ala Thr Leu Lys
            660                 665                 670

Ala Ser Gln Pro Glu Ala Ala Ser Leu Gly Pro Glu Met Thr Val Leu
        675                 680                 685

Phe Ala His Arg Ser Gly Cys His Ser Gly Gln Gln Thr Asp Leu Arg
    690                 695                 700

Arg Lys Ser Ala Leu Ala Lys Ala Thr Thr Leu Val Ser Thr Ala Ser
705                 710                 715                 720

Gly Thr Gln Thr Val Phe Pro Ser Lys
                725
```

<210> 27
<211> 240
<212> PRT
<213> Homo sapiens

<400> 27

```
Met Asp Thr Glu Ser Asn Arg Arg Ala Asn Leu Ala Leu Pro Gln Glu
1               5                   10                  15

Pro Ser Ser Val Pro Ala Phe Glu Val Leu Glu Ile Ser Pro Gln Glu
            20                  25                  30

Val Ser Ser Gly Arg Leu Leu Lys Ser Ala Ser Ser Pro Pro Leu His
        35                  40                  45

Thr Trp Leu Thr Val Leu Lys Lys Glu Gln Glu Phe Leu Gly Val Thr
    50                  55                  60

Gln Ile Leu Thr Ala Met Ile Cys Leu Cys Phe Gly Thr Val Val Cys
```

```
65                  70                  75                  80

Ser Val Leu Asp Ile Ser His Ile Glu Gly Asp Ile Phe Ser Ser Phe
                85                  90                  95

Lys Ala Gly Tyr Pro Phe Trp Gly Ala Ile Phe Phe Ser Ile Ser Gly
            100                 105                 110

Met Leu Ser Ile Ile Ser Glu Arg Arg Asn Ala Thr Tyr Leu Val Arg
        115                 120                 125

Gly Ser Leu Gly Ala Asn Thr Ala Ser Ser Ile Ala Gly Gly Thr Gly
    130                 135                 140

Ile Thr Ile Leu Ile Ile Asn Leu Lys Lys Ser Leu Ala Tyr Ile His
145                 150                 155                 160

Ile His Ser Cys Gln Lys Phe Phe Glu Thr Lys Cys Phe Met Ala Ser
                165                 170                 175

Phe Ser Thr Glu Ile Val Val Met Met Leu Phe Leu Thr Ile Leu Gly
            180                 185                 190

Leu Gly Ser Ala Val Ser Leu Thr Ile Cys Gly Ala Gly Glu Glu Leu
        195                 200                 205

Lys Gly Asn Lys Val Pro Glu Asp Arg Val Tyr Glu Glu Leu Asn Ile
    210                 215                 220

Tyr Ser Ala Thr Tyr Ser Glu Leu Glu Asp Pro Gly Glu Met Ser Pro
225                 230                 235                 240
```

<210> 28
<211> 521
<212> PRT
<213> Homo sapiens

<400> 28

```
Met Ala Ser Asn Ser Leu Phe Ser Thr Val Thr Pro Cys Gln Gln Asn
1               5                   10                  15

Phe Phe Trp Asp Pro Ser Thr Ser Arg Arg Phe Ser Pro Pro Ser Ser
            20                  25                  30

Ser Leu Gln Pro Gly Lys Met Ser Asp Val Ser Pro Val Val Ala Ala
        35                  40                  45
```

```
Gln Gln Gln Gln Gln Gln Gln Gln Gln Gln Gln Gln Gln Gln Gln Gln
    50                  55                  60

Gln Gln Gln Gln Gln Gln Gln Glu Ala Ala Ala Ala Ala Ala Ala Ala
65                  70                  75                  80

Ala Ala Ala Ala Ala Ala Ala Ala Ala Val Pro Arg Leu Arg Pro Pro
                85                  90                  95

His Asp Asn Arg Thr Met Val Glu Ile Ile Ala Asp His Pro Ala Glu
            100                 105                 110

Leu Val Arg Thr Asp Ser Pro Asn Phe Leu Cys Ser Val Leu Pro Ser
        115                 120                 125

His Trp Arg Cys Asn Lys Thr Leu Pro Val Ala Phe Lys Val Val Ala
    130                 135                 140

Leu Gly Glu Val Pro Asp Gly Thr Val Val Thr Val Met Ala Gly Asn
145                 150                 155                 160

Asp Glu Asn Tyr Ser Ala Glu Leu Arg Asn Ala Ser Ala Val Met Lys
                165                 170                 175

Asn Gln Val Ala Arg Phe Asn Asp Leu Arg Phe Val Gly Arg Ser Gly
            180                 185                 190

Arg Gly Lys Ser Phe Thr Leu Thr Ile Thr Val Phe Thr Asn Pro Pro
        195                 200                 205

Gln Val Ala Thr Tyr His Arg Ala Ile Lys Val Thr Val Asp Gly Pro
    210                 215                 220

Arg Glu Pro Arg Arg His Arg Gln Lys Leu Asp Asp Ser Lys Pro Ser
225                 230                 235                 240

Leu Phe Ser Asp Arg Leu Ser Asp Leu Gly Arg Ile Pro His Pro Ser
                245                 250                 255

Met Arg Val Gly Val Pro Pro Gln Asn Pro Arg Pro Ser Leu Asn Ser
            260                 265                 270

Ala Pro Ser Pro Phe Asn Pro Gln Gly Gln Ser Gln Ile Thr Asp Pro
        275                 280                 285

Arg Gln Ala Gln Ser Ser Pro Pro Trp Ser Tyr Asp Gln Ser Tyr Pro
```

```
290                 295                 300

Ser Tyr Leu Ser Gln Met Thr Ser Pro Ser Ile His Ser Thr Thr Pro
305                 310                 315                 320

Leu Ser Ser Thr Arg Gly Thr Gly Leu Pro Ala Ile Thr Asp Val Pro
                325                 330                 335

Arg Arg Ile Ser Asp Asp Asp Thr Ala Thr Ser Asp Phe Cys Leu Trp
            340                 345                 350

Pro Ser Thr Leu Ser Lys Lys Ser Gln Ala Gly Ala Ser Glu Leu Gly
        355                 360                 365

Pro Phe Ser Asp Pro Arg Gln Phe Pro Ser Ile Ser Ser Leu Thr Glu
    370                 375                 380

Ser Arg Phe Ser Asn Pro Arg Met His Tyr Pro Ala Thr Phe Thr Tyr
385                 390                 395                 400

Thr Pro Pro Val Thr Ser Gly Met Ser Leu Gly Met Ser Ala Thr Thr
                405                 410                 415

His Tyr His Thr Tyr Leu Pro Pro Pro Tyr Pro Gly Ser Ser Gln Ser
            420                 425                 430

Gln Ser Gly Pro Phe Gln Thr Ser Ser Thr Pro Tyr Leu Tyr Tyr Gly
        435                 440                 445

Thr Ser Ser Gly Ser Tyr Gln Phe Pro Met Val Pro Gly Gly Asp Arg
    450                 455                 460

Ser Pro Ser Arg Met Leu Pro Pro Cys Thr Thr Thr Ser Asn Gly Ser
465                 470                 475                 480

Thr Leu Leu Asn Pro Asn Leu Pro Asn Gln Asn Asp Gly Val Asp Ala
                485                 490                 495

Asp Gly Ser His Ser Ser Ser Pro Thr Val Leu Asn Ser Ser Gly Arg
            500                 505                 510

Met Asp Glu Ser Val Trp Arg Pro Tyr
        515                 520
```

<210> 29
<211> 599

<212> PRT
<213> Homo sapiens

<400> 29

```
Met Ser Arg Ser Leu Leu Leu Arg Phe Leu Leu Phe Leu Leu Leu Leu
1               5                   10                  15

Pro Pro Leu Pro Val Leu Leu Ala Asp Pro Gly Ala Pro Thr Pro Val
            20                  25                  30

Asn Pro Cys Cys Tyr Tyr Pro Cys Gln His Gln Gly Ile Cys Val Arg
        35                  40                  45

Phe Gly Leu Asp Arg Tyr Gln Cys Asp Cys Thr Arg Thr Gly Tyr Ser
    50                  55                  60

Gly Pro Asn Cys Thr Ile Pro Gly Leu Trp Thr Trp Leu Arg Asn Ser
65                  70                  75                  80

Leu Arg Pro Ser Pro Ser Phe Thr His Phe Leu Leu Thr His Gly Arg
                85                  90                  95

Trp Phe Trp Glu Phe Val Asn Ala Thr Phe Ile Arg Glu Met Leu Met
            100                 105                 110

Arg Leu Val Leu Thr Val Arg Ser Asn Leu Ile Pro Ser Pro Pro Thr
        115                 120                 125

Tyr Asn Ser Ala His Asp Tyr Ile Ser Trp Glu Ser Phe Ser Asn Val
    130                 135                 140

Ser Tyr Tyr Thr Arg Ile Leu Pro Ser Val Pro Lys Asp Cys Pro Thr
145                 150                 155                 160

Pro Met Gly Thr Lys Gly Lys Lys Gln Leu Pro Asp Ala Gln Leu Leu
                165                 170                 175

Ala Arg Arg Phe Leu Leu Arg Arg Lys Phe Ile Pro Asp Pro Gln Gly
            180                 185                 190

Thr Asn Leu Met Phe Ala Phe Phe Ala Gln His Phe Thr His Gln Phe
        195                 200                 205

Phe Lys Thr Ser Gly Lys Met Gly Pro Gly Phe Thr Lys Ala Leu Gly
    210                 215                 220

His Gly Val Asp Leu Gly His Ile Tyr Gly Asp Asn Leu Glu Arg Gln
```

225 230 235 240

Tyr Gln Leu Arg Leu Phe Lys Asp Gly Lys Leu Lys Tyr Gln Val Leu
245 250 255

Asp Gly Glu Met Tyr Pro Pro Ser Val Glu Glu Ala Pro Val Leu Met
260 265 270

His Tyr Pro Arg Gly Ile Pro Pro Gln Ser Gln Met Ala Val Gly Gln
275 280 285

Glu Val Phe Gly Leu Leu Pro Gly Leu Met Leu Tyr Ala Thr Leu Trp
290 295 300

Leu Arg Glu His Asn Arg Val Cys Asp Leu Leu Lys Ala Glu His Pro
305 310 315 320

Thr Trp Gly Asp Glu Gln Leu Phe Gln Thr Thr Arg Leu Ile Leu Ile
325 330 335

Gly Glu Thr Ile Lys Ile Val Ile Glu Glu Tyr Val Gln Gln Leu Ser
340 345 350

Gly Tyr Phe Leu Gln Leu Lys Phe Asp Pro Glu Leu Leu Phe Gly Val
355 360 365

Gln Phe Gln Tyr Arg Asn Arg Ile Ala Met Glu Phe Asn His Leu Tyr
370 375 380

His Trp His Pro Leu Met Pro Asp Ser Phe Lys Val Gly Ser Gln Glu
385 390 395 400

Tyr Ser Tyr Glu Gln Phe Leu Phe Asn Thr Ser Met Leu Val Asp Tyr
405 410 415

Gly Val Glu Ala Leu Val Asp Ala Phe Ser Arg Gln Ile Ala Gly Arg
420 425 430

Ile Gly Gly Gly Arg Asn Met Asp His His Ile Leu His Val Ala Val
435 440 445

Asp Val Ile Arg Glu Ser Arg Glu Met Arg Leu Gln Pro Phe Asn Glu
450 455 460

Tyr Arg Lys Arg Phe Gly Met Lys Pro Tyr Thr Ser Phe Gln Glu Leu
465 470 475 480

```
Val Gly Glu Lys Glu Met Ala Ala Glu Leu Glu Glu Leu Tyr Gly Asp
                485                 490                 495

Ile Asp Ala Leu Glu Phe Tyr Pro Gly Leu Leu Leu Glu Lys Cys His
            500                 505                 510

Pro Asn Ser Ile Phe Gly Glu Ser Met Ile Glu Ile Gly Ala Pro Phe
        515                 520                 525

Ser Leu Lys Gly Leu Leu Gly Asn Pro Ile Cys Ser Pro Glu Tyr Trp
    530                 535                 540

Lys Pro Ser Thr Phe Gly Gly Glu Val Gly Phe Asn Ile Val Lys Thr
545                 550                 555                 560

Ala Thr Leu Lys Lys Leu Val Cys Leu Asn Thr Lys Thr Cys Pro Tyr
                565                 570                 575

Val Ser Phe Arg Val Pro Asp Ala Ser Gln Asp Asp Gly Pro Ala Val
            580                 585                 590

Glu Arg Pro Ser Thr Glu Leu
        595
```

<210> 30
<211> 662
<212> PRT
<213> Homo sapiens

<400> 30

```
Met Gly Leu Tyr Arg Ile Arg Val Ser Thr Gly Ala Ser Leu Tyr Ala
1               5                   10                  15

Gly Ser Asn Asn Gln Val Gln Leu Trp Leu Val Gly Gln His Gly Glu
            20                  25                  30

Ala Ala Leu Gly Lys Arg Leu Trp Pro Ala Arg Gly Lys Glu Thr Glu
        35                  40                  45

Leu Lys Val Glu Val Pro Glu Tyr Leu Gly Pro Leu Leu Phe Val Lys
    50                  55                  60

Leu Arg Lys Arg His Leu Leu Lys Asp Asp Ala Trp Phe Cys Asn Trp
65                  70                  75                  80

Ile Ser Val Gln Gly Pro Gly Ala Gly Asp Glu Val Arg Phe Pro Cys
```

```
            85                  90                  95

Tyr Arg Trp Val Glu Gly Asn Gly Val Leu Ser Leu Pro Glu Gly Thr
            100                 105                 110

Gly Arg Thr Val Gly Glu Asp Pro Gln Gly Leu Phe Gln Lys His Arg
        115                 120                 125

Glu Glu Glu Leu Glu Glu Arg Arg Lys Leu Tyr Arg Trp Gly Asn Trp
    130                 135                 140

Lys Asp Gly Leu Ile Leu Asn Met Ala Gly Ala Lys Leu Tyr Asp Leu
145                 150                 155                 160

Pro Val Asp Glu Arg Phe Leu Glu Asp Lys Arg Val Asp Phe Glu Val
                165                 170                 175

Ser Leu Ala Lys Gly Leu Ala Asp Leu Ala Ile Lys Asp Ser Leu Asn
            180                 185                 190

Val Leu Thr Cys Trp Lys Asp Leu Asp Asp Phe Asn Arg Ile Phe Trp
        195                 200                 205

Cys Gly Gln Ser Lys Leu Ala Glu Arg Val Arg Asp Ser Trp Lys Glu
    210                 215                 220

Asp Ala Leu Phe Gly Tyr Gln Phe Leu Asn Gly Ala Asn Pro Val Val
225                 230                 235                 240

Leu Arg Arg Ser Ala His Leu Pro Ala Arg Leu Val Phe Pro Pro Gly
                245                 250                 255

Met Glu Glu Leu Gln Ala Gln Leu Glu Lys Glu Leu Glu Gly Gly Thr
            260                 265                 270

Leu Phe Glu Ala Asp Phe Ser Leu Leu Asp Gly Ile Lys Ala Asn Val
        275                 280                 285

Ile Leu Cys Ser Gln Gln His Leu Ala Ala Pro Leu Val Met Leu Lys
    290                 295                 300

Leu Gln Pro Asp Gly Lys Leu Leu Pro Met Val Ile Gln Leu Gln Leu
305                 310                 315                 320

Pro Arg Thr Gly Ser Pro Pro Pro Pro Leu Phe Leu Pro Thr Asp Pro
                325                 330                 335
```

```
Pro Met Ala Trp Leu Leu Ala Lys Cys Trp Val Arg Ser Ser Asp Phe
            340                 345                 350

Gln Leu His Glu Leu Gln Ser His Leu Leu Arg Gly His Leu Met Ala
        355                 360                 365

Glu Val Ile Val Val Ala Thr Met Arg Cys Leu Pro Ser Ile His Pro
    370                 375                 380

Ile Phe Lys Leu Ile Ile Pro His Leu Arg Tyr Thr Leu Glu Ile Asn
385                 390                 395                 400

Val Arg Ala Arg Thr Gly Leu Val Ser Asp Met Gly Ile Phe Asp Gln
                405                 410                 415

Ile Met Ser Thr Gly Gly Gly Gly His Val Gln Leu Leu Lys Gln Ala
            420                 425                 430

Gly Ala Phe Leu Thr Tyr Ser Ser Phe Cys Pro Pro Asp Asp Leu Ala
        435                 440                 445

Asp Arg Gly Leu Leu Gly Val Lys Ser Ser Phe Tyr Ala Gln Asp Ala
    450                 455                 460

Leu Arg Leu Trp Glu Ile Ile Tyr Arg Tyr Val Glu Gly Ile Val Ser
465                 470                 475                 480

Leu His Tyr Lys Thr Asp Val Ala Val Lys Asp Asp Pro Glu Leu Gln
                485                 490                 495

Thr Trp Cys Arg Glu Ile Thr Glu Ile Gly Leu Gln Gly Ala Gln Asp
            500                 505                 510

Arg Gly Phe Pro Val Ser Leu Gln Ala Arg Asp Gln Val Cys His Phe
        515                 520                 525

Val Thr Met Cys Ile Phe Thr Cys Thr Gly Gln His Ala Ser Val His
    530                 535                 540

Leu Gly Gln Leu Asp Trp Tyr Ser Trp Val Pro Asn Ala Pro Cys Thr
545                 550                 555                 560

Met Arg Leu Pro Pro Pro Thr Thr Lys Asp Ala Thr Leu Glu Thr Val
                565                 570                 575
```

```
Met Ala Thr Leu Pro Asn Phe His Gln Ala Ser Leu Gln Met Ser Ile
            580                 585                 590

Thr Trp Gln Leu Gly Arg Arg Gln Pro Val Met Val Ala Val Gly Gln
        595                 600                 605

His Glu Glu Glu Tyr Phe Ser Gly Pro Glu Pro Lys Ala Val Leu Lys
    610                 615                 620

Lys Phe Arg Glu Glu Leu Ala Ala Leu Asp Lys Glu Ile Glu Ile Arg
625                 630                 635                 640

Asn Ala Lys Leu Asp Met Pro Tyr Glu Tyr Leu Arg Pro Ser Val Val
                645                 650                 655

Glu Asn Ser Val Ala Ile
            660
```

<210> 31
<211> 530
<212> DNA
<213> Homo sapiens

<220>
<221> modified_base
<222> (484)..(484)
<223> a, c, g, t, unknown or other

<400> 31

```
aaagaatctg acatcatgac aacaaatggt gtaattcatg ttgtagataa actcctctat       60

ccagcagaca cacctgttgg aaatgatcaa ctgctggaaa tacttaataa attaatcaaa      120

tacatccaaa ttaagtttgt tcgtggtagc accttcaaag aaatccccgt gactgtctat      180

agacccacac taacaaaagt caaaattgaa ggtgaacctg aattcagact gattaaagaa      240

ggtgaaacaa taactgaagt gatccatgga gagccaatta ttaaaaaata caccaaaatc      300

attgatggag tgcctgtgga aataactgaa aaagagacac gagaagaacg aatcattaca      360

ggtcctgaaa taaaatacac taggatttct actggaggtg gagaaacaga agaaactctg      420

aagaaattgt tacaagaaga agacacaccc gtgaggaagt tgcaagccaa caaaaaagtt      480

caanggatct agaagacgat taagggaagg tcgttctcag tgaaaatcca                 530
```

<210> 32
<211> 413
<212> DNA
<213> Homo sapiens

<400> 32

```
aaattgtgga gttagcctcc tgtggagtta gcctcctgtg gtaaaggaat tgaagaaaat      60
ataacacctt acaccctttt tcatcttgac attaaaagtt ctggctaact ttggaatcca     120
ttagagaaaa atccttgtca ccagattcat tacaattcaa atcgaagagt tgtgaactgt     180
tatcccattg aaaagaccga gccttgtatg tatgttatgg atacataaaa tgcacgcaag     240
ccattatctc tccatgggaa gctaagttat aaaaataggt gcttggtgta caaaactttt     300
tatatcaaaa ggctttgcac atttctatat gagtgggttt actggtaaat tatgttattt     360
tttacaacta attttgtact ctcagaatgt ttgtcatatg cttcttgcaa tgc            413
```

<210> 33
<211> 493
<212> DNA
<213> Homo sapiens

<400> 33

```
gcgagtacaa caaggccacc gaagatgagt actacagacg cccgctgcag gtgctgcgag      60
ccagggagca gacctttggg ggggtgaatt acttcttcga cgtagaggtg ggccgcacca     120
tatgtaccaa gtcccagccc aacttggaca cctgtgcctt ccatgaacag ccagaactgc     180
agaagaaaca gttatgctct ttcgagatct acgaagttcc ctgggaggac agaatgtccc     240
tggtgaattc caggtgtcaa gaagcctagg ggtctgtgcc aggccagtca caccgaccac     300
cacccactcc cacccccctgt agtgctccca cccctggact ggtggccccc accctgcggg     360
aggcctcccc atgtgcctgt gccaagagac agacagagaa ggctgcagga gtcctttgtt     420
gctcagcagg gcgctctgcc ctccctcctt ccttcttgct tctaatagac ctggtacatg     480
gtacacacac ccc                                                        493
```

<210> 34
<211> 365
<212> DNA
<213> Homo sapiens

<400> 34

```
ggaggatagg ataatcccgg gtggcatcta taacgcagac ctcaatgatg agtgggtaca      60
gcgtgccctt cacttcgcca tcagcgagta taacaaggcc accaaagatg actactacag     120
acgtccgctg cgggtactaa gagccaggca acagaccgtt gggggggtga attacttctt     180
cgacgtagag gtgggccgaa ccatatgtac caagtcccag cccaacttgg acacctgtgc     240
cttccatgaa cagccagaac tgcagaagaa acagttgtgc tctttcgaga tctacgaagt     300
tccctgggag aacagaaggt ccctggtgaa atccaggtgt caagaatcct agggatctgt     360
gccag                                                                 365
```

<210> 35
<211> 410
<212> DNA
<213> Homo sapiens

<400> 35

```
gagaagggcc tgatttgcag catcatgatg ggcctctcct tggcctctgc tgtgctcctg      60
gcctccctcc tgagtctcca ccttggaact gccacacgtg ggagtgacat atccaagacc     120
tgctgcttcc aatacagcca caagcccctt ccctggacct gggtgcgaag ctatgaattc     180
accagtaaca gctgctccca gcgggctgtg atattcacta ccaaaagagg caagaaagtc     240
tgtacccatc caaggaaaaa atgggtgcaa aaatacattt ctttactgaa aactccgaaa     300
caattgtgac tcagctgaat tttcatccga ggacgcttgg accccgctct tggctctgca     360
gccctctggg gagcctgcgg aatcttttct gaaggctaca tggacccgct                410
```

<210> 36
<211> 489
<212> DNA
<213> Homo sapiens

<400> 36

```
ggccaaatca ccgacctgaa ggcggaaatt cacgggggca gtctcattaa tctgacttgg        60
acagctcctg gggatgatta tgaccatgga acagctcaca agtatatcat tcgaataagt       120
acaagtattc ttgatctcag agacaagttc aatgaatctc ttcaagtgaa tactactgct       180
ctcatcccaa aggaagccaa ctctgaggaa gtctttttgt ttaaaccaga aaacattact       240
tttgaaaatg gcacagatct tttcattgct attcaggctg ttgataaggt cgatctgaaa       300
tcagaaatat ccaacattgc acgagtatct ttgtttattc ctccacagac tccgccagag       360
acacctagtc ctgatgaaac gtctgctcct tgtcctaata ttcatatcaa cagcaccatt       420
cctggcattc acattttaaa aattatgtgg aagtggatag gagaactgca gctgtcaata       480
gcctagggc                                                               489
```

<210> 37
<211> 324
<212> DNA
<213> Homo sapiens

<400> 37

```
gagcccccag gaggaggaca ggataatcga gggtggcatc tatgatgcag acctcaatga        60
tgagcgggta cagcgtgccc ttcactttgt catcagcgag tataacaagg ccactgaaga       120
tgagtactac agacgcctgc tgcgggtgct acgagccagg gagcagatcg tgggcggggt       180
gaattacttc ttcgacatag aggtgggccg aaccatatgt accaagtccc agcccaactt       240
ggacacctgt gccttccatg aacagccaga actgcagaag aaacagttgt gctctttcca       300
gatctacgaa gttccctggg agga                                              324
```

<210> 38
<211> 310
<212> DNA
<213> Homo sapiens

<400> 38

```
aagactttac tttcaccata ccagatgtag aggactcaag tcagagacca gatcagggac        60
cccagagacc tcctcctgaa ggactcctac ctagaccccc tggtgatagt ggtaaccaag       120
atgatggtcc tcagcagaga ccaccaaaac caggaggcca tcaccgccat cctcccccac       180
ctccttttca aaatcagcaa cgaccacccc aacgaggaca ccgtcaactc tctctacccc       240
gatttccttc tgtcagcctg caggaagcat catcattctt ccggagggac agaccagcaa       300
gacatcccca                                                              310
```

<210> 39

<211> 465
<212> DNA
<213> Homo sapiens

<400> 39

```
ttcctcaccc taaaactaag cgtgctgctt ctgcaaaaga tttttgtaga tgagctgtgt      60
gcctcagaat tgctatttca aattgccaaa aatttagaga tgttttctac atatttctgc     120
tcttctgaac aacttctgct acccactaaa taaaaacaca gaaataatta gacaattgtc     180
tattataaca tgacaaccct attaatcatt tggtcttcta aaatgggatc atgcccattt     240
agattttcct tactatcagt ttatttttat aacattaact tttactttgt tatttattat     300
tttatataat ggtgagtttt taaattattg ctcactgcct atttaatgta gctaataaag     360
ttatagaagc agatgatctg ttaatttcct atctaataaa tgcctttaat tgttctcata     420
atgaagaata agtaggtacc ctccatgccc ttctgtaata aatat                     465
```

<210> 40
<211> 323
<212> DNA
<213> Homo sapiens

<400> 40

```
agaagactct gacctgtact cttgaataca agtttctgat accactgcac tgtctgagaa      60
tttccaaaac tttaatgaac taactgacag cttcatgaaa ctgtccacca agatcaagca     120
gagaaaataa ttaatttcat gggactaaat gaactaatga ggattgctga ttctttaaat     180
gtcttgtttc ccagatttca ggaaactttt tttcttttaa gctatccact cttacagcaa     240
tttgataaaa tatacttttg tgaacaaaaa ttgagacatt tacattttct ccctatgtgg     300
tcgctccaga cttgggaaac tat                                             323
```

<210> 41
<211> 500
<212> DNA
<213> Homo sapiens

<400> 41

```
tcatcgggcc tggcacaggc atcgcgccct tccgcagttt ctggcagcaa cggctccatg        60
actcccagca caagggagtg cggggaggcc gcatgacctt ggtgtttggg tgccgccgcc       120
cagatgagga ccacatctac caggaggaga tgctggagat ggcccagaag gggtgctgc        180
atgcggtgca cacagcctat tcccgcctgc ctggcaagcc caaggtctat gttcaggaca       240
tcctgcggca gcagctggcc agcgaggtgc tccgtgtgct ccacaaggag ccaggccacc       300
tctatgtttg cggggatgtg cgcatggccc gggacgtggc ccacaccctg aagcagctgg       360
tggctgccaa gctgaaattg aatgaggagc aggtcgagga ctatttcttt cagctcaaga       420
gccagaagcg ctatcacgaa gatatctttg gtgctgtatt tccttacgag gcgaagaagg       480
acagggtggc ggtgcagccc                                                   500
```

<210> 42
<211> 363
<212> DNA
<213> Homo sapiens

<400> 42

```
gtcgatttac acttacctcg gttcaaaatg gaagagagct atgacctcaa ggacacgttg        60
agaaccatgg gaatggtgaa tatcttcaat ggggatgcag acctctcagg catgacctgg       120
agccacggtc tctcagtatc taaagtccta cacaaggcct ttgtggaggt cactgaggag       180
ggagtggaag ctgcagctgc caccgctgta gtagtagtcg aattatcatc tccttcaact       240
aatgaagagt tctgttgtaa tcaccctttc ctattcttca taaggcaaaa taagaccaac       300
agcatcctct tctatggcag attctcatcc ccatagatgc aattagtctg tcactccatt       360
tag                                                                     363
```

<210> 43
<211> 508
<212> DNA
<213> Homo sapiens

<400> 43

```
gatacgacac tggttcttgt gaacgcaatc tatttcaaag ggcagtggga gaataaattt        60
aaaaaagaaa acactaaaga ggaaaaattt tggccaaaca aggatgtaca ggccaaggtc       120
```

```
ctggaaatac catacaaagg caaagatcta agcatgattg tgctgctgcc aaatgaaatc      180
gatggtctgc agaagcttga agagaaactc actgctgaga aattgatgga atggacaagt      240
ttgcagaata tgagagagac atgtgtcgat ttacacttac ctcggttcaa aatggaagag      300
agctatgacc tcaaggacac gttgagaacc atgggaatgg tgaatatctt caatggggat      360
gcagacctct caggcatgac ctggagccac ggtctctcag tatctaaagt cctacacaag      420
gcctttgtgg aggtcactga ggagggagtg gaagctgcag ctgccaccgc tgtagtagta      480
gtcgaattat catctccttc aactaatg                                         508
```

<210> 44
<211> 493
<212> DNA
<213> Homo sapiens

<400> 44

```
gcgagtacaa caaggccacc gaagatgagt actacagacg cccgctgcag gtgctgcgag       60
ccagggagca gacctttggg ggggtgaatt acttcttcga cgtagaggtg ggccgcacca      120
tatgtaccaa gtcccagccc aacttggaca cctgtgcctt ccatgaacag ccagaactgc      180
agaagaaaca gttatgctct ttcgagatct acgaagttcc ctgggaggac agaatgtccc      240
tggtgaattc caggtgtcaa gaagcctagg ggtctgtgcc aggccagtca caccgaccac      300
cacccactcc cacccccctgt agtgctccca cccctggact ggtggccccc accctgcggg      360
aggcctcccc atgtgcctgt gccaagagac agacagagaa ggctgcagga gtcctttgtt      420
gctcagcagg gcgctctgcc ctccctcctt ccttcttgct tctaatagac ctggtacatg      480
gtacacacac ccc                                                         493
```

<210> 45
<211> 447
<212> DNA
<213> Homo sapiens

<400> 45

ccacctcctc caggaaagcc agaaagacca cccccacaag gaggtaacca gtcccaaggt 60

cccccacctc atccaggaaa gccagaagga ccaccccac aggaaggaaa caagtcccga 120

agtgcccgat ctcctccagg aaagccacaa ggaccacccc aacaagaagg caacaagcct 180

caaggtcccc cacctcctgg aaagccacaa ggcccacccc cagcaggagg caatccccag 240

cagcctcagg cacctcctgc tggaaagccc caggggccac ctccacctcc tcaagggggc 300

aggccaccca gacctgccca gggacaacag cctccccagt aatctaggat tcaatgacag 360

gaagtgaata agaagatatc agtgaattca aataattcaa ttgctacaaa tgccgtgaca 420

ttggaacaag gtcatcatag ctctaac 447

<210> 46
<211> 304
<212> DNA
<213> Homo sapiens

<400> 46

tgacgcaaaa taccaccttg gcgcctacac gggagacgac gtccgcatca tccgtgacga 60

catgctgtgt gccgggaaca gccagaggga ctcctgcaag ggcgactctg gagggcccct 120

ggtgtgcaag gtgaatggca cctggctaca ggcgggcgtg gtcagctggg acgagggctg 180

tgcccagccc aaccggcctg gcatctacac ccgtgtcacc tactacttgg actggatcca 240

ccactatgtc cccaaaaagc cgtgagtcag gcctgggtgt gccacctggg tcactggagg 300

acca 304

<210> 47
<211> 358
<212> DNA
<213> Homo sapiens

<400> 47

ccgccatttc ctctgaagca ggtgaaggtc cccataatgg aaaaccacat ttgtgacgca 60

aaataccacc ttggcgccta cacgggagac gacgtccgca tcgtccgtga cgacatgctg 120

tgtgccggga acacccggag ggactcatgc cagggcgact ccggagggcc cctggtgtgc 180

aaggtgaatg gcacctggct gcaggcgggc gtggtcagct ggggcgaggg ctgtgcccag 240

cccaaccggc ctggcatcta cacccgtgtc acctactact tggactggat ccaccactat 300

gtccccaaaa agccgtgagt caggcctggg ttggccacct gggtcactgg aggaccaa 358

<210> 48
<211> 542
<212> DNA
<213> Homo sapiens

<400> 48

```
tgacgcaaaa taccaccttg gcgcctacac gggagacgac gtccgcatcg tccgtgacga      60
catgctgtgt gccgggaaca cccggaggga ctcatgccag ggcgactccg gagggcccct     120
ggtgtgcaag gtgaatggca cctggctgca ggcgggcgtg gtcagctggg gcgagggctg     180
tgcccagccc aaccggcctg gcatctacac ccgtgtcacc tactacttgg actggatcca     240
ccactatgtc cccaaaaagc cgtgagtcag gcctgggttg gccacctggg tcactggagg     300
accaacccct gctgtccaaa acaccactgc ttcctaccca ggtggcgact gccccccaca     360
ccttccctgc cccgtcctga gtgccccttc ctgtcctaag ccccctgctc tcttctgagc     420
cccttcccct gtcctgagga cccttcccta tcctgagccc ccttccctgt cctaagcctg     480
acgcctgcac cgggccctcc agccctcccc tgcccagata gctggtggtg ggcgctaatc     540
ct                                                                    542
```

<210> 49
<211> 536
<212> DNA
<213> Homo sapiens

<400> 49

```
tgacgcaaaa taccaccttg gcgcctacac gggagacgac gtccgcatcg tccgtgacga      60
catgctgtgt gccgggaaca cccggaggga ctcatgccag ggcgactccg gagggcccct     120
ggtgtgcaag gtgaatggca cctggctgca ggcgggcgtg gtcagctggg gcgagggctg     180
tgcccagccc aaccggcctg gcatctacac ccgtgtcacc tactacttgg actggatcca     240
ccactatgtc cccaaaaagc cgtgagtcag gcctgggttg gccacctggg tcactggagg     300
accaacccct gctgtccaaa acaccactgc ttcctaccca ggtggcgact gccccccaca     360
ccttccctgc cccgtcctga gtgccccttc ctgtcctaag ccccctgctc tcttctgagc     420
cccttcccct gtcctgagga cccttcccca tcctgagccc ccttccctgt cctaagcctg     480
acgcctgcac cgggccctcc ggccctcccc tgcccaggca gctggtggtg ggcgct         536
```

<210> 50
<211> 538
<212> DNA
<213> Homo sapiens

<400> 50

```
ccggtcagca ggatcatcgt gcacccacag ttctacatca tccagactgg agcggatatc     60
gccctgctgg agctggagga gcccgtgaac atctccagcc gcgtccacac ggtcatgctg    120
ccccctgcct cggagacctt ccccccgggg atgccgtgct gggtcactgg ctggggcgat    180
gtggacaatg atgagcccct cccaccgcca tttcccctga agcaggtgaa ggtccccata    240
atggaaaacc acatttgtga cgcaaaatac caccttggcg cctacacggg agacgacgtc    300
cgcatcatcc gtgacgacat gctgtgtgcc gggaacaccc ggagggactc atgccagggc    360
gactctggag ggcccctggt gtgcaaggtg aatggcacct ggctacaggc gggcgtggtc    420
agctgggacg agggctgtgc ccagcccaac cggcctggca tctacacccg tgtcacctac    480
tacttggact ggatccacca ctatgtcccc aaaaagccgt gagtcaggcc tggggtgt      538
```

<210> 51
<211> 366
<212> DNA
<213> Homo sapiens

<400> 51

```
ccggtcagca ggatcatcgt gcacccacag ttctacaccg cccagatcgg agcggacatc     60
gccctgctgg agctggagga gccggtgaac gtctccagcc acgtccacac ggtcaccctg    120
ccccctgcct cagagacctt ccccccgggg atgccgtgct gggtcactgg ctggggcgat    180
gtggacaatg atgagcgcct cccaccgcca tttcctctga agcaggtgaa ggtccccata    240
atggaaaacc acatttgtga cgcaaaatac caccttggcg cctacacggg agacgacgtc    300
cgcatcgtcc gtgacgacat gctgtgtgcc gggaacaccc ggagggactc atgccaggtg    360
gcgact                                                               366
```

<210> 52
<211> 496
<212> DNA
<213> Homo sapiens

<220>
<221> modified base
<222> (150)..(151)
<223> a, c, g, t, unknown or other

<220>
<221> modified base
<222> (345)..(345)
<223> a, c, g, t, unknown or other

<220>
<221> modified base
<222> (347)..(348)
<223> a, c, g, t, unknown or other

<220>
<221> modified base
<222> (366)..(366)
<223> a, c, g, t, unknown or other

<220>
<221> modified base
<222> (405)..(405)
<223> a, c, g, t, unknown or other

<220>
<221> modified base
<222> (428)..(428)
<223> a, c, g, t, unknown or other

<400> 52

```
ccggtcagca ggatcatcgt gcacccacag ttctacatca tccagactgg agcggatatc      60
gccctgctgg agctggagga gcccgtgaac atctccagcc gcgtccacac ggtcatgctg     120
ccccctgcct cggagacctt ccccccgggn ntgccgtgct gggtcactgg ctggggcgat     180
gtggacaatg atgagcccct cccaccgcca tttcccctga agcaggtgaa ggtccccata     240
atggaaaacc acatttgtga cgcaaaatac caccttggcg cctacacggg agacgacgtc     300
cgcatcatcc gtgacgacat gctgtgtgcc gggaacaccc ggagngnntc atgccagggc     360
gactcnggag ggcccctggt gtgcaaggtg aatggcacct ggctncaggc gggcgtggtc     420
agctgggncg agggctgtgc ccagcccaac cggcctggca tctacacccg tgtcacctac     480
tacttggact ggatcc                                                     496
```

<210> 53
<211> 408
<212> DNA
<213> Homo sapiens

<400> 53

```
gtcgtcacgg acgatgcgga cgtcgtctcc cgtgtaggcg ccaaggtggt attttgcgtc      60
acaaatgtgg ttttccatta tggggacctt cacctgcttc agaggaaatg gcggtgggag     120
gcgctcatca ttgtccacat cgccccagcc agtgacccag cacggcatcc ccgggggggaa    180
ggtctctgag gcagggggca gggtgaccgt gtggacgtgg ctggagacgt tcaccggctc     240
ctccagctcc agcagggcga tgtccgctcc gatctgggcg gtgtagaact gtgggtgcac     300
gatgatcctg ctgaccggca gcagctggtc ctggtagtag aggtgctgct cccgcagttg     360
caccggtccc acgcagtgcg ctgcggtcag cacccactgg gggtggat                  408
```

<210> 54
<211> 520

<212> DNA
<213> Homo sapiens

<400> 54

```
ggtgaaagtc tccgtggtgg acacagagac ctgccgccgg gactatcccg gccccggggg      60
cagcatcctt cagcccgaca tgctgtgtgc ccggggcccc ggggatgcct gccaggacga     120
ctccgggggg cctctggtct gccaggtgaa cggtgcctgg gtgcaggctg gcattgtgag     180
ctggggtgag ggctgcggcc gccccaacag gccgggagtc tacactcgtg tccctgccta     240
cgtgaactgg atccgccgcc acatcacagc atcagggggc tcagagtctg ggtaccccag     300
gctcccccctc ctggctggct tattcctccc cggcctcttc cttctgctag tctcctgtgt     360
cctgctggcc aagtgcctgc tgcacccatc tgcggatggt actcccttcc ccgcccctga     420
ctgatggcag gaatccaagt gcatttctta aataagttac tatttattcc gctccgcccc     480
ctccctctcc cttgagaagc tgagtcttct gcatcagatt                           520
```

<210> 55
<211> 508
<212> DNA
<213> Homo sapiens

<400> 55

```
tgccacaagg tcgctgctta tgagggcgca aacttcttgg cttgtgctcg gacccttttc      60
tcgtactcca ctctgttttg gcagtaaatc gtgtaggcct ctgcttgagc tgggtcttgg     120
atatttggtt catttagaag ttcctgtatt cctaatagga tctgtttgat tgtgatggct     180
ggcctccagt ccttgtcctc ctctaagatg gacaggcaca ctgtccccga agggtacaca     240
ttcgggtgaa ataatggtgg ttcgaattta cattttggtg gcgaagatgg ataatcatct     300
ttgaaaagca tccgtagttt aaacaagcct ccttcccacg gagtcccttt ctttcctgga     360
atggcgcact cccagttcat gaggttcatc gtgccatcgg gatttttttgt tgggacagcc     420
acgaaaccaa atgggtggtc tttcctccat gctttcctct cctgggcgag tctgctgagg     480
gcgatccccg acatgttcaa agtccctc                                        508
```

<210> 56
<211> 510
<212> DNA
<213> Homo sapiens

<400> 56

agagactttc agggcatacg tgggggcctt ggccttcctc actcgctcga tggcctcagt 60

gtgctcctca aggctggtgc caaacacctg ctggagatag ctgagcaggg cctcctcgtc 120

gtccacctgg tcagggccca tggtacccgc gcggtaaagc accgtgtaca gggcctcctc 180

gtagagcatc tccacctcct ctggggccag ggctctcagg ccgaggctgg gatccacagg 240

ctccgggggt gctggcgagc cactgcgcag ggggacctcg aggcacggca agccctgtct 300

gccttccccc ttcttcagca tgaggcgcat gtgggcaaag aactccacgc catccccggg 360

tttccaggcc cccgtggcag gctcctgcgg gtcggcgctg gcactccctg ggtcctgctc 420

agtcctgcgg cggaaggacg ggcacacctg cacctgcctg agcacgctgc tcttaatgtc 480

cagcaaggtc gacatggcgg gtgaccgtgg 510

<210> 57
<211> 407
<212> DNA
<213> Homo sapiens

<400> 57

tgctgctctt caaaatgtac cccattgatg aggagaggcg gcggcagaat aagaaggccc 60

tgcaggcact gagggacgag gccagcagct ctggctgctc agaaacagac tccacagagc 120

tggctagcat cctctagggc ccgccacgtt gcccgaagcc accatgcaga aggccacaga 180

agggatcagg acctgtctgc cggcttgctg agcagctgga ctgcaggtgc taggaaggga 240

actgaagact caaggaggtg gcccaggaca cttgctgtgc tcactgtggg gccggctgct 300

ctgtggcctc ctgcctcccc tctgcctgcc tgtggggcca agccctgggg ctgccactgt 360

gaatatgcca aggactgatc gggcctagcc cggaacacta atgtaga 407

<210> 58
<211> 565
<212> DNA
<213> Homo sapiens

<400> 58

```
tatgaaaccc gctacatcta tggcccaata gaatcaacaa tttacccgat atcaggttct      60
tctttagact gggcttatga cctgggcatc aaacacacat ttgcctttga gctccgagat     120
aaaggcaaat ttggttttct ccttccagaa tcccggataa agccaacgtg cagagagacc     180
atgctagctg tcaaatttat tgccaagtat atcctcaagc atacttccta aagaactgcc     240
ctctgtttgg aataagccaa ttaatccttt tttgtgcctt tcatcagaaa gtcaatcttc     300
agttatcccc aaatgcagct tctatttcac ctgaatcctt ctcttgctca tttaagtccc     360
atgttactgc tgtttgcttt tacttacttt cagtagcacc ataacgaagt agctttaagt     420
gaaacctttt aactaccttt ctttgctcca agtgaagttt ggacccagca gaaagcatta     480
ttttgaaagg tgatatacag tggggcacag aaaacaaatg aaaaccctca gtttctcaca     540
gattttcacc atgtggcttc atcaa                                           565
```

<210> 59
<211> 459
<212> DNA
<213> Homo sapiens

<400> 59

```
tgagcctggg gttctggtgt tagaatattt ttaagtaggc tttactgaga gaaactaaat      60
attggcatac gttatcagca acttcccctg ttcaatagta tgggaaaaat aagatgactg     120
ggaaaaagac acacccacac cgtagaacat atattaatct actggcgaat gggaaaggag     180
accattttct tagaaagcaa ataaacttga ttttttaaa tctaaaattt acattaatga     240
gtgcaaaata acacataaaa tgaaaattca cacatcacat ttttctggaa aacagacgga     300
ttttacttct ggagacatgg catacggtta ctgacttatg agctaccaaa actaaattct     360
ttctctgcta ttaactggct agaagacatt catctatttt tcaaatgttc tttcaaaaca     420
tttttataag taatgtttgt atctatttca tgctttact                            459
```

<210> 60
<211> 430
<212> DNA
<213> Homo sapiens

<220>
<221> modified base
<222> (343)..(345)
<223> a, c, g, t, unknown or other

<220>
<221> modified base
<222> (347)..(347)
<223> a, c, g, t, unknown or other

<220>
<221> modified base
<222> (349)..(352)
<223> a, c, g, t, unknown or other

<400> 60

```
gggaatcact attcagggat ttttcccctt tgctcttctt ttccctcctt aaaagaaaaa      60
ttaccttcta gtcctaggat gaggacacac tattagtttg aattaaatgc tttgatattc     120
tcagatcagc catcttgaac caaagcaaaa ccacaagtta cactttctta aaatttgatt     180
tgtcatattt tctagagaaa cttgaattta attgtgttat tcttagcttc cactggcagc     240
ctagctttga gggtaaatga aaatataacc catagattac ccagccactt gggaacagca     300
ggtaatactg aagaaaaata aaaatagatt ttgaaaacgt tannnanann nntatgatta     360
tgattctgtt ccatttaagg gaaaacttag gtaaatagag aaattttttc tataacattg     420
tgtagtcagt                                                            430
```

<210> 61
<211> 358
<212> DNA
<213> Homo sapiens

<400> 61

```
tgcttctgga cacctgggac caggtctttg tctgggttgg aaaggattct caagaagaag      60
aaaagacaga agccttgact tctgctaagc ggtacatcga gacggaccca gccaatcggg     120
atcggcggac gcccatcacc gtggtgaagc aaggctttga gcctccctcc tttgtgggct     180
ggttccttgg ctgggatgat gattactggt ctgtggaccc cttggacagg gccatggctg     240
agctggctgc ctgaggaggg gcagggccca cccatgtcac cggtcagtgc cttttggaac     300
tgtccttccc tcaaagaggc cttagagcga gcagagcagc tctgctatga gtgtgtgt       358
```

<210> 62
<211> 506
<212> DNA
<213> Homo sapiens

<220>
<221> modified_base
<222> (86)..(86)
<223> a, c, g, t, unknown or other

<220>
<221> modified_base
<222> (118)..(118)
<223> a, c, g, t, unknown or other

<400> 62

```
acttttgacc acttgtgact ggagttcagt ggccctggca ggcttgtcct gctcttgacc     60
attccactga ctaactttgg tgtttngttt ccaagttaag tgattcctcc tttttttngt    120
tcaatgttaa atttaaaaat aacaatgtgt atgggtcctc ccatgtgtaa tatggtaaca    180
tgtaacttgc agtgtttgcc agctttcaaa gcaggctttg tgaaaatgta atacaaacag    240
cagtgaatgg gactcaaatg ttgtgcttcc tataaacagc tccgctcttt cagggaagga    300
tggtaacaaa ctagaaggac aaatatgtac gtatttataa cgtattaaaa ctcttttaag    360
tagcttaagg tattgtgcaa tggcctagcc tagtagaaat gggggaaaag cattgctgtg    420
gaccattgtt aaagtgacag gagttgtagg gttacccctt tgacaagctt ccatagtctt    480
cagacacgca cattgatggc atccct                                         506
```

<210> 63
<211> 496
<212> DNA
<213> Homo sapiens

<220>
<221> modified base
<222> (60)..(60)
<223> a, c, g, t, unknown or other

<220>
<221> modified_base
<222> (75)..(75)
<223> a, c, g, t, unknown or other

<220>
<221> modified_base
<222> (114)..(114)
<223> a, c, g, t, unknown or other

<220>
<221> modified_base
<222> (131)..(131)
<223> a, c, g, t, unknown or other

<220>
<221> modified_base
<222> (138)..(138)
<223> a, c, g, t, unknown or other

<220>
<221> modified_base
<222> (141)..(141)
<223> a, c, g, t, unknown or other

<220>
<221> modified_base
<222> (159)..(159)
<223> a, c, g, t, unknown or other

<220>
<221> modified_base
<222> (175)..(175)
<223> a, c, g, t, unknown or other

<220>
<221> modified_base
<222> (203)..(203)
<223> a, c, g, t, unknown or other

<220>
<221> modified_base
<222> (207)..(207)
<223> a, c, g, t, unknown or other

<400> 63

```
ctaactaata ccaacctgac aacttgaata acaataaatg caatttgtac ataaaatatn      60
atgctgcaaa agttngtcat tcacctcagt ggagtgactt gatattaggt ggtnaccgta     120
gatgatggtt natatganaa ntggacagga aagaagcant ttctgaaagt tatantcttt     180
tgaaccacgt tctaaaccaa gtntttnatc ttcttggggc tcgtaattac ctttcacttt     240
aatgtcactt aaagatataa cacagaaaaa tgccttgagg gcaaaatata ggcaaaacac     300
caatgcgctt tcaaatgcat gaaaatggtg cagttgtacc cttgagcctt gactcaaggg     360
ctgtagatgt tccctttcca ccccccacac ttggtgcgtg ttcacaaagc aaatatggcc     420
tgtaattcaa atttgttcta tgtgatactc tctgagtaaa aactcataca tgcagaaaat     480
tgtctttgct cgaaat                                                     496
```

<210> 64
<211> 560
<212> DNA
<213> Homo sapiens

<400> 64

```
cccaagcccc tagagaagtc agtctccccg caaaattcag attcttcagg ttttgcagat      60
gtgaatggtt ggcaccttcg tttccgctgg tccaaggatg ctccctcaga actcctgagg     120
aagttcagaa actatgaaat atgaaatatc tctgcttcaa aaaatgagga agagcaagac     180
tgtcccctat gctgccaaca tgcagtcttt gtttatgtct taaaaatgtc atgtttatgt     240
catgtctgtg aattgctgag tactaattga ttcctccatc cttgaatcag ttctcataat     300
gctttttaaa taagaaaaat tcagaagatg aatttcttcc aatatttgaa taaattaaag     360
```

```
ctcttagata cagagtagat tgtattatat gctttttcct attaatacta cttatagaaa      420

tccattaaaa agcaatctct gtacagtgta tttaaatatt tcattgacat actgtgatct      480

ctattagtga tggatgtaca aaaaatgttt tcttaccctt gacttacaat gaaatgtgaa      540

attacttgtc tgaaccccgt                                                  560
```

<210> 65
<211> 512
<212> DNA
<213> Homo sapiens

<220>
<221> modified_base
<222> (443)..(443)
<223> a, c, g, t, unknown or other

<400> 65

```
acagcaagcc tatgtagttc aattaatata taaggaaaag gaaggtcttt cttcatgata       60

caagcattat aaagttttta ctgtagtagt caattaatgg atatttcctt gttaataaaa      120

ttttgtgtca taatttacaa attagttctt taaaaattgt tgttatatga attgtgtttc      180

tagcatgaat gttctataga gtactctaaa taacttgaat ttatagacaa atgctactca      240

cagtacaatc aattgtatta taccatgaga aaatcaaaaa ggtgttcttc agagacattt      300

tatctataaa attttcctac tattatgttc attaacaaac ttctttatca catgtatctt      360

ctacgtgtaa aacatttctg atgatttttt aacaaaaaat atatgaattt cttcatttgc      420

tcttgcatct acattgctat aanggatata aaatgtggtt tctatatttt gagatgtttt      480

ttccttacaa tgtgaactca tcgtgatctt gg                                    512
```

<210> 66
<211> 551
<212> DNA
<213> Homo sapiens

<220>
<221> modified_base
<222> (114)..(114)
<223> a, c, g, t, unknown or other

<220>
<221> modified_base
<222> (119)..(120)
<223> a, c, g, t, unknown or other

<220>
<221> modified_base
<222> (127)..(127)
<223> a, c, g, t, unknown or other

<220>
<221> modified_base
<222> (129)..(129)
<223> a, c, g, t, unknown or other

<220>
<221> modified_base
<222> (149)..(149)
<223> a, c, g, t, unknown or other

<220>
<221> modified_base
<222> (152)..(152)
<223> a, c, g, t, unknown or other

<220>
<221> modified_base
<222> (163)..(163)
<223> a, c, g, t, unknown or other

<220>
<221> modified_base
<222> (167)..(168)
<223> a, c, g, t, unknown or other

<220>
<221> modified_base
<222> (189)..(189)
<223> a, c, g, t, unknown or other

<220>
<221> modified_base
<222> (243)..(243)
<223> a, c, g, t, unknown or other

<400> 66

```
ctgctacttt ggaagatggc tctggaggaa actctcatat ggctaaaaag gcaggctagt      60
ttcttacttc tacaggggta gagccttaaa aaagaacgtg ctacaaattg gttntcttnn     120
agggttncng gttctccctg cccccaatnc cnatatactt tantgcnntt ttatttttgc     180
ctttacggnc tctgtgtctt tctgcaagaa ggcctggcaa aggtatgcct gctgttggtc     240
ccntcgggat aagataaaat ataaataaaa ccttcagaac tgttttggag caaaagatag     300
cttgtacttg gggaaaaaaa ttctaagttc ttttatatga ctaatattct tggttagcaa     360
gactggaaag aggtgttttt ttaaaatgta cataccagaa caaagaacat acagctctct     420
gaacatttat tttttgaaca gaggtggttt ttatgtttgg acctggtaat acagatacaa     480
aaactttaat gaggtagcaa tgaatattca actgtttgac tgctaagtgt atctgtccat     540
attttagcaa g                                                          551
```

<210> 67

<211> 474
<212> DNA
<213> Homo sapiens

<220>
<221> modified_base
<222> (365)..(365)
<223> a, c, g, t, unknown or other

<220>
<221> modified_base
<222> (429).. (429)
<223> a, c, g, t, unknown or other

<400> 67

```
tactacaaaa gccgtgacct cagcctgctt atactactgc cagaagacat taatgggctg      60
gaacagctgg aaaaggccat cacctatgag aagctgaatg agtggaccag tgcagacatg     120
atggagttgt atgaagtgca gctacacctt cccaagttca agctggaaga cagttatgat     180
ctcaagtcaa ccctgagcag tatggggatg agtgatgcct tcagccaaag caaagctgat     240
ttctcaggaa tgtcttcagc aagaaaccta tttttgtcca atgttttcca taaggctttt     300
gtggaaataa atgaacaagg tactgaagct gcagctggca gtgggagtga gatagatata     360
cgaantagag tcccatccat tgaattcaat gcaaatcacc cattcctctt cttcatcagg     420
cacaataana accaacacca ttcttttta tggaagatta tgctccccct aatc           474
```

<210> 68
<211> 287
<212> DNA
<213> Homo sapiens

<400> 68

```
gattctgtgg tagactcagt gctttcagag tccagagctt gacttgggtt agtggcctta      60
atgaagtgct aaatttgctc tttaccgcga gactgatcag aagaagcaaa aggggaaagg     120
gggctagagg tccactcgca ccttttacat cagacaagag gaggactgtg ccagaaatct     180
gtgcatgaaa caccatctgc tcttcatgca gggaggggtc aaccgtgtga acgtgcagag     240
attactcgag ccttctttgc caaaaatatg cattcttccc agctgta                   287
```

<210> 69
<211> 545
<212> DNA
<213> Homo sapiens

<400> 69

```
taatcacatc acttccatgg catggatgtt cacatacaga ctcttaaccc tggtttacca      60
ggacctctag gagtggatcc aatctatatc tttacagttg tatagtatat gatatctctt     120
ttatttcact caatttatat tttcatcatt gactacatat ttcttataca caacacacaa     180
tttatgaatt ttttctcaag atcattctga gagttgcccc accctacctg ccttttatag     240
tacgcccacc tcaggcagac acagagcaca atgctggggt tctcttcaca ctatcactgc     300
cccaaattgt ctttctaaat ttcaacttca atgtcatctt ctccatgaag accactgaat     360
gaacaccttt tcatccagcc ttaatttctt gctccataac tactctatcc cacgatgcag     420
tattgtatca ttaattatta gtgtgcttgt gacctcctta tgtattctca attacctgta     480
tttgtgcaat aaattggaat aatgtaactt gatttcttat ctgtgtttgt gttggcatgc     540
aagat                                                                 545
```

<210> 70
<211> 420
<212> DNA
<213> Homo sapiens

<400> 70

```
aagacacttc ttccaaacct tgaatttgtt gtttttagaa aacgaatgca tttaaaaata      60
ttttctatgt gagaattttt tagatgtgtg tttacttcat gtttacaaat aactgtttgc     120
tttttaatgc agtactttga aatatatcag ccaaaaccat aacttacaat aatttcttag     180
gtattctgaa taaaattcca tttcttttgg atatgcttta ccattcttag gtttctgtgg     240
aacaaaaata tttgtagcat tttgtgtaaa tacaagcttt catttttatt ttttccaatt     300
gctattgccc aagaattgct ttccatgcac atattgtaaa aattccgctt tgtgccacag     360
gtcatgattg tggatgagtt tactcttaac ttcaaaggga ctatttgtat tgtatgttgc     420
```

<210> 71
<211> 534
<212> DNA
<213> Homo sapiens

<220>
<221> modified_base
<222> (486)..(493)
<223> a, c, g, t, unknown or other

<400> 71

agtattgaca actgcacatg aaagttttgc aaagggaaac aggctaaatg caccaagaaa 60

gcttcttcag agtgaagaat cttaatgctt gtaatttaaa catttgttcc tggagttttg 120

atttggtgga tgtgatggtt ggttttattt gtcagtttgg ttgggctata gcacacagtt 180

atttaatcaa acagtaatct aggtgtggct gtgaaggtat tttgtagatg tgattaacat 240

ctacaatcag ttgactttaa gtgaaagaga ttacttaaat aatttgggtg agctgcacct 300

gattagttga aaggcctcaa gaacaaacac tgcagtttcc tggaaaagaa gaaactttgc 360

ctcaagacta tagccatcga ctcctgcctg agtttccagc ctgctagtct gccctatgga 420

tttgaagttt gccaacccca acaattgtgt gaattaattt ctaaaaataa agctatatac 480

agccannnnn nnntatttgt gggggatttg tttcaggatc tctacagata ccaa 534

<210> 72
<211> 478
<212> DNA
<213> Homo sapiens

<400> 72

ccctagaggg gcaccttttc atggtctctg cacccagtga acacatttta ctctagaggc 60

atcacctggg accttactcc tctttccttc cttcctcctt tcctatcttc cttcctctct 120

ctcttcctct ttcttcattc agatctatat ggcaaatagc cacaattata taaatcattt 180

caagactaga atagggggat ataatacata ttactccaca ccttttatga atcaaatatg 240

atttttttgt tgttgttaag acagagtctc actttgacac ccaggctgga gtgcagtggt 300

gccatcacca cggctcactg cagcctcagc gtcctgggct caaatgatcc tcccacctca 360

gcctcctgag tagctgggac tacaggctca tgccatcatg cccagctaat atttttttat 420

tttcgtggag acggggcctc actatgttgc ctaggctgga aataggattt tgaaccca 478

<210> 73
<211> 19
<212> DNA
<213> Artificial Sequence

<220>
<221> source
<223> /note="Description of Artificial Sequence: Synthetic primer"

<400> 73
ggatgctgag cggattctg 19

<210> 74
<211> 20
<212> DNA
<213> Artificial Sequence

<220>
<221> source
<223> /note="Description of Artificial Sequence: Synthetic primer"

<400> 74
ccctcgcgaa aaagtttctt 20

<210> 75
<211> 21
<212> DNA
<213> Artificial Sequence

<220>
<221> source
<223> /note="Description of Artificial Sequence: Synthetic primer"

<400> 75
aaggtctcag ctgggcagtt t 21

<210> 76
<211> 19
<212> DNA
<213> Artificial Sequence

<220>
<221> source
<223> /note="Description of Artificial Sequence: Synthetic primer"

<400> 76
aaactgggcc acctcgatt 19

<210> 77
<211> 24
<212> DNA
<213> Artificial Sequence

<220>
<221> source
<223> /note="Description of Artificial Sequence: Synthetic probe"

<400> 77
ccagcttgca tgtccgagac acca 24

<210> 78
<211> 20
<212> DNA
<213> Artificial Sequence

<220>
<221> source
<223> /note="Description of Artificial Sequence: Synthetic primer"

<400> 78
gggtctcacc tcccaactgc 20

<210> 79
<211> 22
<212> DNA

<213> Artificial Sequence

<220>
<221> source
<223> /note="Description of Artificial Sequence: Synthetic primer"

<400> 79
tgtctgttac ggtcaactcg gt 22

<210> 80
<211> 21
<212> DNA
<213> Artificial Sequence

<220>
<221> source
<223> /note="Description of Artificial Sequence: Synthetic primer"

<400> 80
gcttccccct ctgttcttcc t 21

<210> 81
<211> 23
<212> DNA
<213> Artificial Sequence

<220>
<221> source
<223> /note="Description of Artificial Sequence: Synthetic primer"

<400> 81
gctctgtgag gctgttcaaa gtt 23

<210> 82
<211> 26
<212> DNA
<213> Artificial Sequence

<220>
<221> source
<223> /note="Description of Artificial Sequence: Synthetic probe"

<400> 82
tccacggaca caagtgcgat atcacc 26

<210> 83
<211> 20
<212> DNA
<213> Artificial Sequence

<220>
<221> source
<223> /note="Description of Artificial Sequence: Synthetic primer"

<400> 83
gccatgagga tgcttctgca 20

<210> 84

<211> 26
<212> DNA
<213> Artificial Sequence

<220>
<221> source
<223> /note="Description of Artificial Sequence: Synthetic primer"

<400> 84
gaatcctcag agtctcattg gctatc 26

<210> 85
<211> 20
<212> DNA
<213> Artificial Sequence

<220>
<221> source
<223> /note="Description of Artificial Sequence: Synthetic primer"

<400> 85
agctgcctac gtgtatgcca 20

<210> 86
<211> 21
<212> DNA
<213> Artificial Sequence

<220>
<221> source
<223> /note="Description of Artificial Sequence: Synthetic primer"

<400> 86
gtgccaaggt ctctttcacc a 21

<210> 87
<211> 25
<212> DNA
<213> Artificial Sequence

<220>
<221> source
<223> /note="Description of Artificial Sequence: Synthetic probe"

<400> 87
ccccacagaa attcccacaa gtgca 25

<210> 88
<211> 26
<212> DNA
<213> Artificial Sequence

<220>
<221> source
<223> /note="Description of Artificial Sequence: Synthetic primer"

<400> 88
actcctctac ctccatcaat aactcc 26

<210> 89
<211> 23
<212> DNA
<213> Artificial Sequence

<220>
<221> source
<223> /note="Description of Artificial Sequence: Synthetic primer"

<400> 89
tggctctgca agagatgtta gct 23

<210> 90
<211> 20
<212> DNA
<213> Artificial Sequence

<220>
<221> source
<223> /note="Description of Artificial Sequence: Synthetic primer"

<400> 90
gctggctgga ttctggaaaa 20

<210> 91
<211> 22
<212> DNA
<213> Artificial Sequence

<220>
<221> source
<223> /note="Description of Artificial Sequence: Synthetic primer"

<400> 91
tggctctgca agagatgtta gc 22

<210> 92
<211> 29
<212> DNA
<213> Artificial Sequence

<220>
<221> source
<223> /note="Description of Artificial Sequence: Synthetic probe"

<400> 92
tctccaatca attctgtgtc tccacctgg 29

<210> 93
<211> 18
<212> DNA
<213> Artificial Sequence

<220>
<221> source
<223> /note="Description of Artificial Sequence: Synthetic primer"

<400> 93

tgtggcggga aagacagc 18

<210> 94
<211> 23
<212> DNA
<213> Artificial Sequence

<220>
<221> source
<223> /note="Description of Artificial Sequence: Synthetic primer"

<400> 94
ccttcctatg gcttagcttc agc 23

<210> 95
<211> 20
<212> DNA
<213> Artificial Sequence

<220>
<221> source
<223> /note="Description of Artificial Sequence: Synthetic primer"

<400> 95
cgtgttgtca ccgagaacgt 20

<210> 96
<211> 20
<212> DNA
<213> Artificial Sequence

<220>
<221> source
<223> /note="Description of Artificial Sequence: Synthetic primer"

<400> 96
atcttgatgg ccttggagca 20

<210> 97
<211> 20
<212> DNA
<213> Artificial Sequence

<220>
<221> source
<223> /note="Description of Artificial Sequence: Synthetic probe"

<400> 97
ctgcggcacc acagggacca 20

<210> 98
<211> 19
<212> DNA
<213> Artificial Sequence

<220>
<221> source
<223> /note="Description of Artificial Sequence: Synthetic primer"

<400> 98
ttgaggaccc ctgctccct 19

<210> 99
<211> 20
<212> DNA
<213> Artificial Sequence

<220>
<221> source
<223> /note="Description of Artificial Sequence: Synthetic primer"

<400> 99
aggcgtgcac ataggaggac 20

<210> 100
<211> 20
<212> DNA
<213> Artificial Sequence

<220>
<221> source
<223> /note="Description of Artificial Sequence: Synthetic primer"

<400> 100
cgatcccaac agtgccttct 20

<210> 101
<211> 18
<212> DNA
<213> Artificial Sequence

<220>
<221> source
<223> /note="Description of Artificial Sequence: Synthetic primer"

<400> 101
cctcgctccg ctcacagt 18

<210> 102
<211> 23
<212> DNA
<213> Artificial Sequence

<220>
<221> source
<223> /note="Description of Artificial Sequence: Synthetic probe"

<400> 102
caaccccaag cccttccact cga 23

<210> 103
<211> 19
<212> DNA
<213> Artificial Sequence

<220>
<221> source

<223> /note="Description of Artificial Sequence: Synthetic primer"

<400> 103
ccaaccacca aggatgcaa 19

<210> 104
<211> 18
<212> DNA
<213> Artificial Sequence

<220>
<221> source
<223> /note="Description of Artificial Sequence: Synthetic primer"

<400> 104
tctgcccagc tgccaagt 18

<210> 105
<211> 19
<212> DNA
<213> Artificial Sequence

<220>
<221> source
<223> /note="Description of Artificial Sequence: Synthetic primer"

<400> 105
ccaaccacca aggatgcaa 19

<210> 106
<211> 21
<212> DNA
<213> Artificial Sequence

<220>
<221> source
<223> /note="Description of Artificial Sequence: Synthetic primer"

<400> 106
ggagagaagc ctggtggaag t 21

<210> 107
<211> 25
<212> DNA
<213> Artificial Sequence

<220>
<221> source
<223> /note="Description of Artificial Sequence: Synthetic probe"

<400> 107
cagtgtcgcc atcactgtct ccagc 25

<210> 108
<211> 20
<212> DNA
<213> Artificial Sequence

<220>
<221> source
<223> /note="Description of Artificial Sequence: Synthetic primer"

<400> 108
acgtccacca gaccatcacc 20

<210> 109
<211> 20
<212> DNA
<213> Artificial Sequence

<220>
<221> source
<223> /note="Description of Artificial Sequence: Synthetic primer"

<400> 109
gaatctcacg tgtgccacca 20

<210> 110
<211> 19
<212> DNA
<213> Artificial Sequence

<220>
<221> source
<223> /note="Description of Artificial Sequence: Synthetic primer"

<400> 110
attgcaatgt accgccagc 19

<210> 111
<211> 20
<212> DNA
<213> Artificial Sequence

<220>
<221> source
<223> /note="Description of Artificial Sequence: Synthetic primer"

<400> 111
gaatctcacg tgtgccacca 20

<210> 112
<211> 26
<212> DNA
<213> Artificial Sequence

<220>
<221> source
<223> /note="Description of Artificial Sequence: Synthetic probe"

<400> 112
ctgctgtgca ccccattttc aagctg 26

<210> 113
<211> 22
<212> DNA

<213> Artificial Sequence

<220>
<221> source
<223> /note="Description of Artificial Sequence: Synthetic primer"

<400> 113
cataaagtgg agcacgaaag ca 22

<210> 114
<211> 25
<212> DNA
<213> Artificial Sequence

<220>
<221> source
<223> /note="Description of Artificial Sequence: Synthetic primer"

<400> 114
ggtacgcatc tacacagttc tggtt 25

<210> 115
<211> 20
<212> DNA
<213> Artificial Sequence

<220>
<221> source
<223> /note="Description of Artificial Sequence: Synthetic primer"

<400> 115
cagaatggga ggagcttcca 20

<210> 116
<211> 23
<212> DNA
<213> Artificial Sequence

<220>
<221> source
<223> /note="Description of Artificial Sequence: Synthetic primer"

<400> 116
cacagttctg gttggcagtg tag 23

<210> 117
<211> 23
<212> DNA
<213> Artificial Sequence

<220>
<221> source
<223> /note="Description of Artificial Sequence: Synthetic probe"

<400> 117
ccggaacact tgcctttgag cgg 23

<210> 118

<211> 21
<212> DNA
<213> Artificial Sequence

<220>
<221> source
<223> /note="Description of Artificial Sequence: Synthetic primer"

<400> 118
caaggtctgt gggaaaagca a 21

<210> 119
<211> 20
<212> DNA
<213> Artificial Sequence

<220>
<221> source
<223> /note="Description of Artificial Sequence: Synthetic primer"

<400> 119
tggccagaga tgcttccaat 20

<210> 120
<211> 24
<212> DNA
<213> Artificial Sequence

<220>
<221> source
<223> /note="Description of Artificial Sequence: Synthetic primer"

<400> 120
gcagaagtca agaagaacgg aaga 24

<210> 121
<211> 20
<212> DNA
<213> Artificial Sequence

<220>
<221> source
<223> /note="Description of Artificial Sequence: Synthetic primer"

<400> 121
tgcttccaat tgccaaactg 20

<210> 122
<211> 20
<212> DNA
<213> Artificial Sequence

<220>
<221> source
<223> /note="Description of Artificial Sequence: Synthetic probe"

<400> 122
tctccgccca gcaccaggct 20

<210> 123
<211> 21
<212> DNA
<213> Artificial Sequence

<220>
<221> source
<223> /note="Description of Artificial Sequence: Synthetic primer"

<400> 123
acttaaagcc cgcctgacag a 21

<210> 124
<211> 22
<212> DNA
<213> Artificial Sequence

<220>
<221> source
<223> /note="Description of Artificial Sequence: Synthetic primer"

<400> 124
gctacttctt gcccccttg aa 22

<210> 125
<211> 18
<212> DNA
<213> Artificial Sequence

<220>
<221> source
<223> /note="Description of Artificial Sequence: Synthetic primer"

<400> 125
ccacggccac atttggtt 18

<210> 126
<211> 19
<212> DNA
<213> Artificial Sequence

<220>
<221> source
<223> /note="Description of Artificial Sequence: Synthetic primer"

<400> 126
agggaagcgg ttgctcatc 19

<210> 127
<211> 28
<212> DNA
<213> Artificial Sequence

<220>
<221> source
<223> /note="Description of Artificial Sequence: Synthetic probe"

<400> 127

agaaaccctc tgtcattcgc tcccacat 28

<210> 128
<211> 23
<212> DNA
<213> Artificial Sequence

<220>
<221> source
<223> /note="Description of Artificial Sequence: Synthetic primer"

<400> 128
ctccgcagtc acctaatcac tct 23

<210> 129
<211> 25
<212> DNA
<213> Artificial Sequence

<220>
<221> source
<223> /note="Description of Artificial Sequence: Synthetic primer"

<400> 129
ggctcaatgg gtaccacatc tatct 25

<210> 130
<211> 23
<212> DNA
<213> Artificial Sequence

<220>
<221> source
<223> /note="Description of Artificial Sequence: Synthetic primer"

<400> 130
ttcctgttcc attcagagac gat 23

<210> 131
<211> 23
<212> DNA
<213> Artificial Sequence

<220>
<221> source
<223> /note="Description of Artificial Sequence: Synthetic primer"

<400> 131
agattctgaa ggcttgcatc ttg 23

<210> 132
<211> 24
<212> DNA
<213> Artificial Sequence

<220>
<221> source
<223> /note="Description of Artificial Sequence: Synthetic probe"

<400> 132
tgccgaccct ctgggagaaa atcc 24

<210> 133
<211> 22
<212> DNA
<213> Artificial Sequence

<220>
<221> source
<223> /note="Description of Artificial Sequence: Synthetic primer"

<400> 133
attcaaggat cttgctgcct tt 22

<210> 134
<211> 18
<212> DNA
<213> Artificial Sequence

<220>
<221> source
<223> /note="Description of Artificial Sequence: Synthetic primer"

<400> 134
tgcagtcacg ggatgcat 18

<210> 135
<211> 21
<212> DNA
<213> Artificial Sequence

<220>
<221> source
<223> /note="Description of Artificial Sequence: Synthetic primer"

<400> 135
caaggatctt gctgcctttg a 21

<210> 136
<211> 21
<212> DNA
<213> Artificial Sequence

<220>
<221> source
<223> /note="Description of Artificial Sequence: Synthetic primer"

<400> 136
tgcttgcttt gtgctcttgg t 21

<210> 137
<211> 27
<212> DNA
<213> Artificial Sequence

<220>
<221> source

<223> /note="Description of Artificial Sequence: Synthetic probe"

<400> 137
aaatcccatg atcaagctgt ccgaacc 27

<210> 138
<211> 19
<212> DNA
<213> Artificial Sequence

<220>
<221> source
<223> /note="Description of Artificial Sequence: Synthetic primer"

<400> 138
caccacaccg acggtacca 19

<210> 139
<211> 15
<212> DNA
<213> Artificial Sequence

<220>
<221> source
<223> /note="Description of Artificial Sequence: Synthetic primer"

<400> 139
tgcgcgccga gatca 15

<210> 140
<211> 23
<212> DNA
<213> Artificial Sequence

<220>
<221> source
<223> /note="Description of Artificial Sequence: Synthetic primer"

<400> 140
ccatgaagga cgaggtagct cta 23

<210> 141
<211> 15
<212> DNA
<213> Artificial Sequence

<220>
<221> source
<223> /note="Description of Artificial Sequence: Synthetic primer"

<400> 141
tgcgcgccga gatca 15

<210> 142
<211> 26
<212> DNA
<213> Artificial Sequence

<220>
<221> source
<223> /note="Description of Artificial Sequence: Synthetic probe"

<400> 142
cctgggagtc ctgctgcaag cctact 26

<210> 143
<211> 21
<212> DNA
<213> Artificial Sequence

<220>
<221> source
<223> /note="Description of Artificial Sequence: Synthetic primer"

<400> 143
cgctactagg caatgccaat g 21

<210> 144
<211> 23
<212> DNA
<213> Artificial Sequence

<220>
<221> source
<223> /note="Description of Artificial Sequence: Synthetic primer"

<400> 144
gcaatctgcg taccacttgt ttt 23

<210> 145
<211> 21
<212> DNA
<213> Artificial Sequence

<220>
<221> source
<223> /note="Description of Artificial Sequence: Synthetic primer"

<400> 145
cgctactagg caatgccaat g 21

<210> 146
<211> 23
<212> DNA
<213> Artificial Sequence

<220>
<221> source
<223> /note="Description of Artificial Sequence: Synthetic primer"

<400> 146
gcaatctgcg taccacttgt ttt 23

<210> 147
<211> 26
<212> DNA

<213> Artificial Sequence

<220>
<221> source
<223> /note="Description of Artificial Sequence: Synthetic probe"

<400> 147
agcaacctgt gcattcccgt tcaagt 26

<210> 148
<211> 22
<212> DNA
<213> Artificial Sequence

<220>
<221> source
<223> /note="Description of Artificial Sequence: Synthetic primer"

<400> 148
gggagcactg ctattctttc ca 22

<210> 149
<211> 24
<212> DNA
<213> Artificial Sequence

<220>
<221> source
<223> /note="Description of Artificial Sequence: Synthetic primer"

<400> 149
caaacacatt ctccatctca tcca 24

<210> 150
<211> 24
<212> DNA
<213> Artificial Sequence

<220>
<221> source
<223> /note="Description of Artificial Sequence: Synthetic primer"

<400> 150
gagcactgct attctttcca catg 24

<210> 151
<211> 24
<212> DNA
<213> Artificial Sequence

<220>
<221> source
<223> /note="Description of Artificial Sequence: Synthetic primer"

<400> 151
tctccatctc atccaggata gaca 24

<210> 152

<211> 20
<212> DNA
<213> Artificial Sequence

<220>
<221> source
<223> /note="Description of Artificial Sequence: Synthetic probe"

<400> 152
ccacccgctc tctggcagcg 20

<210> 153
<211> 20
<212> DNA
<213> Artificial Sequence

<220>
<221> source
<223> /note="Description of Artificial Sequence: Synthetic primer"

<400> 153
gcatggctcg cctacagact 20

<210> 154
<211> 23
<212> DNA
<213> Artificial Sequence

<220>
<221> source
<223> /note="Description of Artificial Sequence: Synthetic primer"

<400> 154
cagacggtaa cggacgtaat cac 23

<210> 155
<211> 18
<212> DNA
<213> Artificial Sequence

<220>
<221> source
<223> /note="Description of Artificial Sequence: Synthetic primer"

<400> 155
tggcgcttca agcaactg 18

<210> 156
<211> 22
<212> DNA
<213> Artificial Sequence

<220>
<221> source
<223> /note="Description of Artificial Sequence: Synthetic primer"

<400> 156
cagacggtaa cggacgtaat ca 22

<210> 157
<211> 21
<212> DNA
<213> Artificial Sequence

<220>
<221> source
<223> /note="Description of Artificial Sequence: Synthetic probe"

<400> 157
aggcccctac ggcgccaaca t 21

<210> 158
<211> 23
<212> DNA
<213> Artificial Sequence

<220>
<221> source
<223> /note="Description of Artificial Sequence: Synthetic primer"

<400> 158
ctggtatgag cccatctatc tgg 23

<210> 159
<211> 21
<212> DNA
<213> Artificial Sequence

<220>
<221> source
<223> /note="Description of Artificial Sequence: Synthetic primer"

<400> 159
ttggatgttc gtcctcctca c 21

<210> 160
<211> 20
<212> DNA
<213> Artificial Sequence

<220>
<221> source
<223> /note="Description of Artificial Sequence: Synthetic primer"

<400> 160
ggagaagggt gaccgactca 20

<210> 161
<211> 18
<212> DNA
<213> Artificial Sequence

<220>
<221> source
<223> /note="Description of Artificial Sequence: Synthetic primer"

<400> 161

tgcccagact cggcaaag 18

<210> 162
<211> 24
<212> DNA
<213> Artificial Sequence

<220>
<221> source
<223> /note="Description of Artificial Sequence: Synthetic probe"

<400> 162
cgctgagatc aatcggcccg acta 24

<210> 163
<211> 24
<212> DNA
<213> Artificial Sequence

<220>
<221> source
<223> /note="Description of Artificial Sequence: Synthetic primer"

<400> 163
ggctgtgaca tcaatgctat catc 24

<210> 164
<211> 26
<212> DNA
<213> Artificial Sequence

<220>
<221> source
<223> /note="Description of Artificial Sequence: Synthetic primer"

<400> 164
gtccagtgag gcacaaatta gataag 26

<210> 165
<211> 28
<212> DNA
<213> Artificial Sequence

<220>
<221> source
<223> /note="Description of Artificial Sequence: Synthetic primer"

<400> 165
tctggaatgg aattggacat agcccaag 28

<210> 166
<211> 24
<212> DNA
<213> Artificial Sequence

<220>
<221> source
<223> /note="Description of Artificial Sequence: Synthetic primer"

<400> 166
ccaaccccag caaggttctt tctg 24

<210> 167
<211> 26
<212> DNA
<213> Artificial Sequence

<220>
<221> source
<223> /note="Description of Artificial Sequence: Synthetic probe"

<400> 167
accctccatg atgtgcaagt gaaacc 26

<210> 168
<211> 23
<212> DNA
<213> Artificial Sequence

<220>
<221> source
<223> /note="Description of Artificial Sequence: Synthetic primer"

<400> 168
ggatgccatc gtttttgtaa ctg 23

<210> 169
<211> 21
<212> DNA
<213> Artificial Sequence

<220>
<221> source
<223> /note="Description of Artificial Sequence: Synthetic primer"

<400> 169
cctctcaagg ctttgcaggt a 21

<210> 170
<211> 18
<212> DNA
<213> Artificial Sequence

<220>
<221> source
<223> /note="Description of Artificial Sequence: Synthetic primer"

<400> 170
gggcagggcc atctgttc 18

<210> 171
<211> 24
<212> DNA
<213> Artificial Sequence

<220>
<221> source

<223> /note="Description of Artificial Sequence: Synthetic primer"

<400> 171
tctcaaggct ttgcaggtat ttaa 24

<210> 172
<211> 29
<212> DNA
<213> Artificial Sequence

<220>
<221> source
<223> /note="Description of Artificial Sequence: Synthetic probe"

<400> 172
accccaacaa caagagagtg aagaatgca 29

<210> 173
<211> 20
<212> DNA
<213> Artificial Sequence

<220>
<221> source
<223> /note="Description of Artificial Sequence: Synthetic primer"

<400> 173
cctcctcctt gtggctaccc 20

<210> 174
<211> 23
<212> DNA
<213> Artificial Sequence

<220>
<221> source
<223> /note="Description of Artificial Sequence: Synthetic primer"

<400> 174
caatctcttc agaagtgcaa ggg 23

<210> 175
<211> 20
<212> DNA
<213> Artificial Sequence

<220>
<221> source
<223> /note="Description of Artificial Sequence: Synthetic primer"

<400> 175
tcctcctgga ccacctcagt 20

<210> 176
<211> 22
<212> DNA
<213> Artificial Sequence

<220>
<221> source
<223> /note="Description of Artificial Sequence: Synthetic primer"

<400> 176
gaacattcct gggtctggag tg 22

<210> 177
<211> 21
<212> DNA
<213> Artificial Sequence

<220>
<221> source
<223> /note="Description of Artificial Sequence: Synthetic probe"

<400> 177
tggccagaaa cctccccgtg g 21

<210> 178
<211> 24
<212> DNA
<213> Artificial Sequence

<220>
<221> source
<223> /note="Description of Artificial Sequence: Synthetic primer"

<400> 178
gtaactgact tgaatgtcca acgc 24

<210> 179
<211> 22
<212> DNA
<213> Artificial Sequence

<220>
<221> source
<223> /note="Description of Artificial Sequence: Synthetic primer"

<400> 179
gacaaccatt actgggatgc tc 22

<210> 180
<211> 21
<212> DNA
<213> Artificial Sequence

<220>
<221> source
<223> /note="Description of Artificial Sequence: Synthetic primer"

<400> 180
ccaacgcaaa gcaatacatg a 21

<210> 181
<211> 22
<212> DNA

<213> Artificial Sequence

<220>
<221> source
<223> /note="Description of Artificial Sequence: Synthetic primer"

<400> 181
ttttcgcttc cctgttttag ct 22

<210> 182
<211> 25
<212> DNA
<213> Artificial Sequence

<220>
<221> source
<223> /note="Description of Artificial Sequence: Synthetic probe"

<400> 182
tccaagtgat ggctgaactg tcgcc 25

<210> 183
<211> 20
<212> DNA
<213> Artificial Sequence

<220>
<221> source
<223> /note="Description of Artificial Sequence: Synthetic primer"

<400> 183
gttgcctggt cctcctgact 20

<210> 184
<211> 22
<212> DNA
<213> Artificial Sequence

<220>
<221> source
<223> /note="Description of Artificial Sequence: Synthetic primer"

<400> 184
tgtccagctg atccttcatt tg 22

<210> 185
<211> 21
<212> DNA
<213> Artificial Sequence

<220>
<221> source
<223> /note="Description of Artificial Sequence: Synthetic primer"

<400> 185
tgagaacagc tgcacccact t 21

<210> 186

<211> 20
<212> DNA
<213> Artificial Sequence

<220>
<221> source
<223> /note="Description of Artificial Sequence: Synthetic primer"

<400> 186
gctgaaggca tctcggagat 20

<210> 187
<211> 25
<212> DNA
<213> Artificial Sequence

<220>
<221> source
<223> /note="Description of Artificial Sequence: Synthetic probe"

<400> 187
caggcaacct gcctaacatg cttcg 25

<210> 188
<211> 21
<212> DNA
<213> Artificial Sequence

<220>
<221> source
<223> /note="Description of Artificial Sequence: Synthetic primer"

<400> 188
actgctactg ctgctgagcc t 21

<210> 189
<211> 22
<212> DNA
<213> Artificial Sequence

<220>
<221> source
<223> /note="Description of Artificial Sequence: Synthetic primer"

<400> 189
ggtgaggtgg atcggttgta gt 22

<210> 190
<211> 20
<212> DNA
<213> Artificial Sequence

<220>
<221> source
<223> /note="Description of Artificial Sequence: Synthetic primer"

<400> 190
caatcccacg aaatccagga 20

<210> 191
<211> 22
<212> DNA
<213> Artificial Sequence

<220>
<221> source
<223> /note="Description of Artificial Sequence: Synthetic primer"

<400> 191
ttcaggttga ccatcacagt cc 22

<210> 192
<211> 28
<212> DNA
<213> Artificial Sequence

<220>
<221> source
<223> /note="Description of Artificial Sequence: Synthetic probe"

<400> 192
cccaaattct gaggacaaga acttcccc 28

<210> 193
<211> 118
<212> PRT
<213> Artificial Sequence

<220>
<221> source
<223> /note="Description of Artificial Sequence: Synthetic polypeptide"

<400> 193

```
Gln Val Thr Leu Arg Glu Ser Gly Pro Ala Leu Val Lys Pro Thr Gln
1               5                   10                  15

Thr Leu Thr Leu Thr Cys Thr Val Ser Gly Phe Ser Leu Ser Ala Tyr
            20                  25                  30

Ser Val Asn Trp Ile Arg Gln Pro Pro Gly Lys Ala Leu Glu Trp Leu
        35                  40                  45

Ala Met Ile Trp Gly Asp Gly Lys Ile Val Tyr Asn Ser Ala Leu Lys
    50                  55                  60

Ser Arg Leu Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Gly Asp Gly Tyr Tyr Pro Tyr Ala Met Asp Asn Trp Gly Gln Gly Ser
            100                 105                 110

Leu Val Thr Val Ser Ser
        115
```

<210> 194
<211> 111
<212> PRT
<213> Artificial Sequence

<220>
<221> source
<223> /note="Description of Artificial Sequence: Synthetic polypeptide"

<400> 194

```
Asp Ile Val Met Thr Gln Ser Pro Asp Ser Leu Ser Val Ser Leu Gly
1               5                   10                  15

Glu Arg Ala Thr Ile Asn Cys Arg Ala Ser Lys Ser Val Asp Ser Tyr
            20                  25                  30
```

Gly Asn Ser Phe Met His Trp Tyr Gln Gln Lys Pro Gly Gln Pro Pro
35 40 45

Lys Leu Leu Ile Tyr Leu Ala Ser Asn Leu Glu Ser Gly Val Pro Asp
50 55 60

Arg Phe Ser Gly Ser Gly Ser Gly Thr Asp Phe Thr Leu Thr Ile Ser
65 70 75 80

Ser Leu Gln Ala Glu Asp Val Ala Val Tyr Tyr Cys Gln Gln Asn Asn
85 90 95

Glu Asp Pro Arg Thr Phe Gly Gly Gly Thr Lys Val Glu Ile Lys
100 105 110

<210> 195
<211> 15
<212> PRT
<213> Artificial Sequence

<220>
<221> source
<223> /note="Description of Artificial Sequence: Synthetic peptide"

<400> 195

Arg Ala Ser Lys Ser Val Asp Ser Tyr Gly Asn Ser Phe Met His
1 5 10 15

<210> 196
<211> 7
<212> PRT
<213> Artificial Sequence

<220>
<221> source
<223> /note="Description of Artificial Sequence: Synthetic peptide"

<400> 196

Leu Ala Ser Asn Leu Glu Ser
1 5

<210> 197
<211> 9
<212> PRT
<213> Artificial Sequence

<220>
<221> source
<223> /note="Description of Artificial Sequence: Synthetic peptide"

<400> 197

Gln Gln Asn Asn Glu Asp Pro Arg Thr
1 5

<210> 198
<211> 5
<212> PRT
<213> Artificial Sequence

<220>
<221> source
<223> /note="Description of Artificial Sequence: Synthetic peptide"

<400> 198

Ala Tyr Ser Val Asn
1 5

<210> 199
<211> 16
<212> PRT
<213> Artificial Sequence

<220>
<221> source
<223> /note="Description of Artificial Sequence: Synthetic peptide"

<400> 199

Met Ile Trp Gly Asp Gly Lys Ile Val Tyr Asn Ser Ala Leu Lys Ser
1 5 10 15

<210> 200
<211> 10
<212> PRT
<213> Artificial Sequence

<220>
<221> source
<223> /note="Description of Artificial Sequence: Synthetic peptide"

<400> 200

Asp Gly Tyr Tyr Pro Tyr Ala Met Asp Asn
1 5 10

<210> 201
<211> 16
<212> PRT
<213> Artificial Sequence

<220>
<221> source
<223> /note="Description of Artificial Sequence: Synthetic peptide"

<400> 201

```
Arg Ser Ser Gln Ser Pro Val His Ser Asn Gly Asn Thr Tyr Leu His
 1               5                   10                  15
```

<210> 202
<211> 7
<212> PRT
<213> Artificial Sequence

<220>
<221> source
<223> /note="Description of Artificial Sequence: Synthetic peptide"

<400> 202

```
Lys Val Ser Asn Arg Phe Ser
1               5
```

<210> 203
<211> 9
<212> PRT
<213> Artificial Sequence

<220>
<221> source
<223> /note="Description of Artificial Sequence: Synthetic peptide"

<400> 203

```
Ser Gln Ser Thr His Ile Pro Trp Thr
1               5
```

<210> 204
<211> 5
<212> PRT
<213> Artificial Sequence

<220>
<221> source
<223> /note="Description of Artificial Sequence: Synthetic peptide"

<400> 204

```
Ser Tyr Trp Met His
1               5
```

<210> 205
<211> 17
<212> PRT
<213> Artificial Sequence

<220>
<221> source
<223> /note="Description of Artificial Sequence: Synthetic peptide"

<400> 205

Glu Ile Asp Pro Ser Asn Gly Arg Thr Asn Tyr Asn Glu Lys Phe Lys
1               5                   10                  15

Ser

<210> 206
<211> 9
<212> PRT
<213> Artificial Sequence

<220>
<221> source
<223> /note="Description of Artificial Sequence: Synthetic peptide"

<400> 206

Glu Arg Ser Pro Arg Tyr Phe Asp Val
1               5

<210> 207
<211> 16
<212> PRT
<213> Artificial Sequence

<220>
<221> source
<223> /note="Description of Artificial Sequence: Synthetic peptide"

<400> 207

Arg Ser Ser Gln Ser Ile Val His Gly Asn Gly Asn Thr Tyr Leu Glu
1               5                   10                  15

<210> 208
<211> 7
<212> PRT
<213> Artificial Sequence

<220>
<221> source
<223> /note="Description of Artificial Sequence: Synthetic peptide"

<400> 208

Arg Val Ser Asn Arg Phe Ser
1               5

<210> 209
<211> 9
<212> PRT

<213> Artificial Sequence

<220>
<221> source
<223> /note="Description of Artificial Sequence: Synthetic peptide"

<400> 209

Phe Gln Gly Ser His Val Pro Tyr Thr
1 5

<210> 210
<211> 5
<212> PRT
<213> Artificial Sequence

<220>
<221> source
<223> /note="Description of Artificial Sequence: Synthetic peptide"

<400> 210

Ser Tyr Trp Leu Asn
1 5

<210> 211
<211> 17
<212> PRT
<213> Artificial Sequence

<220>
<221> source
<223> /note="Description of Artificial Sequence: Synthetic peptide"

<400> 211

Met Ile Asp Pro Ser Asp Ser Glu Thr His Tyr Asn Gln Val Phe Lys
1 5 10 15

Asp

<210> 212
<211> 14
<212> PRT
<213> Artificial Sequence

<220>
<221> source
<223> /note="Description of Artificial Sequence: Synthetic peptide"

<400> 212

Gly Arg Gly Asn Phe Tyr Gly Gly Ser His Ala Met Glu Tyr
1 5 10

<210> 213
<211> 114
<212> PRT
<213> Artificial Sequence

<220>
<221> source
<223> /note="Description of Artificial Sequence: Synthetic E25 light chain variable sequence"

<400> 213

```
Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Val Asp Tyr Asp
            20                  25                  30

Gly Asp Ser Tyr Met Asn Trp Tyr Gln Gln Lys Pro Gly Lys Ala Pro
        35                  40                  45

Lys Leu Leu Ile Tyr Ala Ala Ser Tyr Leu Glu Ser Gly Val Pro Ser
    50                  55                  60

Arg Phe Ser Gly Ser Gly Ser Gly Thr Asp Phe Thr Leu Thr Ile Ser
65                  70                  75                  80

Ser Leu Gln Pro Glu Asp Phe Ala Thr Tyr Tyr Cys Gln Gln Ser His
                85                  90                  95

Glu Asp Pro Tyr Thr Phe Gly Gln Gly Thr Lys Val Glu Ile Lys Arg
            100                 105                 110

Thr Val
```

<210> 214
<211> 114
<212> PRT
<213> Artificial Sequence

<220>
<221> source
<223> /note="Description of Artificial Sequence: Synthetic E25 heavy chain variable sequence"

<400> 214

```
Glu Val Gln Leu Val Glu Ser Gly Gly Gly Leu Val Gln Pro Gly Gly
1               5                   10                  15

Ser Leu Arg Leu Ser Cys Ala Val Ser Gly Tyr Ser Ile Thr Ser Gly
            20                  25                  30

Tyr Ser Trp Asn Trp Ile Arg Gln Ala Pro Gly Lys Gly Leu Glu Trp
        35                  40                  45

Val Ala Ser Ile Thr Tyr Asp Gly Ser Thr Asn Tyr Asn Pro Ser Val
    50                  55                  60

Lys Gly Arg Ile Thr Ile Ser Arg Asp Asp Ser Lys Asn Thr Phe Tyr
65                  70                  75                  80

Leu Gln Met Asn Ser Leu Arg Ala Glu Asp Thr Ala Val Tyr Tyr Cys
                85                  90                  95

Ala Arg Gly Ser His Tyr Phe Gly His Trp His Phe Ala Val Trp Gly
            100                 105                 110

Gln Gly
```

<210> 215
<211> 5
<212> PRT
<213> Artificial Sequence

<220>
<221> source
<223> /note="Description of Artificial Sequence: Synthetic peptide"

<400> 215

```
Ser Tyr Thr Met His
1               5
```

<210> 216
<211> 5
<212> PRT
<213> Artificial Sequence

<220>
<221> source
<223> /note="Description of Artificial Sequence: Synthetic peptide"

<400> 216

```
Ser Tyr Ala Met Ser
1               5
```

<210> 217
<211> 5
<212> PRT
<213> Artificial Sequence

<220>
<221> source
<223> /note="Description of Artificial Sequence: Synthetic peptide"

<400> 217

Asn Phe Gly Met His
1               5

<210> 218
<211> 5
<212> PRT
<213> Artificial Sequence

<220>
<221> source
<223> /note="Description of Artificial Sequence: Synthetic peptide"

<400> 218

Asn Tyr Gly Met His
1               5

<210> 219
<211> 17
<212> PRT
<213> Artificial Sequence

<220>
<221> source
<223> /note="Description of Artificial Sequence: Synthetic peptide"

<400> 219

Ile Ile Ser Gly Ser Gly Gly Phe Thr Tyr Tyr Ala Asp Ser Val Lys
1               5                   10                  15

Gly

<210> 220
<211> 17
<212> PRT
<213> Artificial Sequence

<220>
<221> source
<223> /note="Description of Artificial Sequence: Synthetic peptide"

<400> 220

Ala Ile Trp Tyr Asp Gly His Asp Lys Tyr Tyr Ser Tyr Tyr Val Lys
1               5                   10                  15

Gly

<210> 221
<211> 17
<212> PRT
<213> Artificial Sequence

<220>
<221> source
<223> /note="Description of Artificial Sequence: Synthetic peptide"

<400> 221

Ala Ile Trp Tyr Asp Gly His Asp Lys Tyr Tyr Ala Tyr Tyr Val Lys
1               5                   10                  15

Gly

<210> 222
<211> 17
<212> PRT
<213> Artificial Sequence

<220>
<221> source
<223> /note="Description of Artificial Sequence: Synthetic peptide"

<400> 222

Val Ile Trp Tyr Asp Gly Ser Asn Lys Tyr Tyr Val Asp Ser Val Lys
1               5                   10                  15

Gly

<210> 223
<211> 17
<212> PRT
<213> Artificial Sequence

<220>
<221> source
<223> /note="Description of Artificial Sequence: Synthetic peptide"

<400> 223

Val Ile Trp Asn Asp Gly Ser Asn Lys Tyr Tyr Val Asp Ser Val Lys
1               5                   10                  15

Gly

<210> 224
<211> 11
<212> PRT
<213> Artificial Sequence

<220>
<221> source
<223> /note="Description of Artificial Sequence: Synthetic peptide"

<400> 224

```
Asp Ser Ser Ser Trp Tyr Arg Tyr Phe Asp Tyr
1               5                   10
```

<210> 225
<211> 11
<212> PRT
<213> Artificial Sequence

<220>
<221> source
<223> /note="Description of Artificial Sequence: Synthetic peptide"

<400> 225

```
Asp Arg Leu Val Ala Pro Gly Thr Phe Asp Tyr
1               5                   10
```

<210> 226
<211> 7
<212> PRT
<213> Artificial Sequence

<220>
<221> source
<223> /note="Description of Artificial Sequence: Synthetic peptide"

<400> 226

```
Lys Asn Trp Ser Phe Asp Phe
1               5
```

<210> 227
<211> 12
<212> PRT
<213> Artificial Sequence

<220>
<221> source
<223> /note="Description of Artificial Sequence: Synthetic peptide"

<400> 227

Asp Arg Met Gly Ile Tyr Tyr Tyr Gly Met Asp Val
1               5                   10

<210> 228
<211> 11
<212> PRT
<213> Artificial Sequence

<220>
<221> source
<223> /note="Description of Artificial Sequence: Synthetic peptide"

<400> 228

Arg Ala Ser Gln Gly Ile Ser Ser Trp Leu Ala
1               5                   10

<210> 229
<211> 12
<212> PRT
<213> Artificial Sequence

<220>
<221> source
<223> /note="Description of Artificial Sequence: Synthetic peptide"

<400> 229

Arg Ala Ser Gln Ser Val Ser Ser Ser Tyr Leu Ala
1               5                   10

<210> 230
<211> 12
<212> PRT
<213> Artificial Sequence

<220>
<221> source
<223> /note="Description of Artificial Sequence: Synthetic peptide"

<400> 230

Arg Ala Ser Gln Ser Val Ser Ser Asn Tyr Leu Ala
1               5                   10

<210> 231
<211> 11
<212> PRT
<213> Artificial Sequence

<220>
<221> source
<223> /note="Description of Artificial Sequence: Synthetic peptide"

<400> 231

```
Arg Ala Ser Gln Gly Val Ser Arg Tyr Leu Ala
1               5                   10
```

<210> 232
<211> 11
<212> PRT
<213> Artificial Sequence

<220>
<221> source
<223> /note="Description of Artificial Sequence: Synthetic peptide"

<400> 232

```
Arg Ala Ser Gln Ser Val Ser Ser Tyr Leu Ala
1               5                   10
```

<210> 233
<211> 7
<212> PRT
<213> Artificial Sequence

<220>
<221> source
<223> /note="Description of Artificial Sequence: Synthetic peptide"

<400> 233

```
Gly Ala Ser Ser Arg Ala Thr
1               5
```

<210> 234
<211> 7
<212> PRT
<213> Artificial Sequence

<220>
<221> source
<223> /note="Description of Artificial Sequence: Synthetic peptide"

<400> 234

```
Ala Ala Ser Ser Leu Gln Ser
1               5
```

<210> 235
<211> 7
<212> PRT
<213> Artificial Sequence

<220>
<221> source
<223> /note="Description of Artificial Sequence: Synthetic peptide"

<400> 235

Met Pro Pro Val Trp Lys Val
1 5

<210> 236
<211> 7
<212> PRT
<213> Artificial Sequence

<220>
<221> source
<223> /note="Description of Artificial Sequence: Synthetic peptide"

<400> 236

Asp Ala Ser Asn Arg Ala Thr
1 5

<210> 237
<211> 7
<212> PRT
<213> Artificial Sequence

<220>
<221> source
<223> /note="Description of Artificial Sequence: Synthetic peptide"

<400> 237

Leu His Pro Leu Cys Lys Val
1 5

<210> 238
<211> 8
<212> PRT
<213> Artificial Sequence

<220>
<221> source
<223> /note="Description of Artificial Sequence: Synthetic peptide"

<400> 238

Asn Ser Leu Ile Val Thr Leu Thr
1 5

<210> 239
<211> 9
<212> PRT
<213> Artificial Sequence

<220>
<221> source
<223> /note="Description of Artificial Sequence: Synthetic peptide"

<400> 239

Gln Gln Tyr Asn Ser Tyr Pro Tyr Thr
1 5

<210> 240
<211> 8
<212> PRT
<213> Artificial Sequence

<220>
<221> source
<223> /note="Description of Artificial Sequence: Synthetic peptide"

<400> 240

Gln Gln Tyr Gly Ser Ser Phe Thr
1 5

<210> 241
<211> 9
<212> PRT
<213> Artificial Sequence

<220>
<221> source
<223> /note="Description of Artificial Sequence: Synthetic peptide"

<400> 241

Gln Gln Arg Ser Asn Trp Gln Tyr Thr
1 5

<210> 242
<211> 7
<212> PRT
<213> Artificial Sequence

<220>
<221> source
<223> /note="Description of Artificial Sequence: Synthetic peptide"

<400> 242

Gln Gln Arg Ser Asn Trp Thr
1 5

<210> 243
<211> 8
<212> PRT
<213> Artificial Sequence

<220>
<221> source
<223> /note="Description of Artificial Sequence: Synthetic peptide"

<400> 243

Asn Ser Ile Ile Val Ser Leu Thr
1 5

<210> 244
<211> 16
<212> PRT
<213> Artificial Sequence

<220>
<221> source
<223> /note="Description of Artificial Sequence: Synthetic peptide"

<400> 244

```
Arg Ser Ser Gln Ser Leu Val His Asn Asn Ala Asn Thr Tyr Leu His
1               5                   10                  15
```

<210> 245
<211> 7
<212> PRT
<213> Artificial Sequence

<220>
<221> source
<223> /note="Description of Artificial Sequence: Synthetic peptide"

<400> 245

```
Lys Val Ser Asn Arg Phe Ser
1               5
```

<210> 246
<211> 9
<212> PRT
<213> Artificial Sequence

<220>
<221> source
<223> /note="Description of Artificial Sequence: Synthetic peptide"

<400> 246

```
Ser Gln Asn Thr Leu Val Pro Trp Thr
1               5
```

<210> 247
<211> 10
<212> PRT
<213> Artificial Sequence

<220>
<221> source
<223> /note="Description of Artificial Sequence: Synthetic peptide"

<400> 247

```
Gly Phe Thr Phe Ser Asp Tyr Gly Ile Ala
1               5                   10
```

<210> 248

<211> 18
<212> PRT
<213> Artificial Sequence

<220>
<221> source
<223> /note="Description of Artificial Sequence: Synthetic peptide"

<400> 248

```
Ala Phe Ile Ser Asp Leu Ala Tyr Thr Ile Tyr Tyr Ala Asp Thr Val
1               5                   10                  15

                            Thr Gly
```

<210> 249
<211> 10
<212> PRT
<213> Artificial Sequence

<220>
<221> source
<223> /note="Description of Artificial Sequence: Synthetic peptide"

<400> 249

```
Ala Arg Asp Asn Trp Asp Ala Met Asp Tyr
1               5                   10
```

<210> 250
<211> 117
<212> PRT
<213> Artificial Sequence

<220>
<221> source
<223> /note="Description of Artificial Sequence: Synthetic Humanized 47H4 V.5 heavy chain variable sequence"

<400> 250

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr
            20                  25                  30

Gly Ile Ala Trp Val Arg Gln Ala Pro Gly Lys Gly Leu Glu Trp Val
        35                  40                  45

Ala Phe Ile Ser Asp Leu Ala Tyr Thr Ile Tyr Tyr Ala Asp Thr Val
    50                  55                  60

Thr Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Asn Trp Asp Ala Met Asp Tyr Trp Gly Gln Gly Thr Leu
            100                 105                 110

Val Thr Val Ser Ser
        115
```

<210> 251
<211> 113
<212> PRT
<213> Artificial Sequence

<220>
<221> source
<223> /note="Description of Artificial Sequence: Synthetic Humanized 47H4 V.24-6 light chain variable sequence"

<400> 251

Asp Ile Gln Met Thr Gln Ser Pro Ser Ser Leu Ser Ala Ser Val Gly
1 5 10 15

Asp Arg Val Thr Ile Thr Cys Arg Ser Ser Gln Ser Leu Val His Asn
20 25 30

Asn Ala Asn Thr Tyr Leu His Trp Tyr Gln Gln Lys Pro Gly Lys Ala
35 40 45

Pro Lys Leu Leu Ile Tyr Lys Val Ser Asn Arg Phe Ser Gly Val Pro
50 55 60

Ser Arg Phe Ser Gly Ser Gly Ser Gly Thr Asp Phe Thr Leu Thr Ile
65 70 75 80

Ser Ser Leu Gln Pro Glu Asp Phe Ala Thr Tyr Tyr Cys Ser Gln Asn
85 90 95

Thr Leu Val Pro Trp Thr Phe Gly Gln Gly Thr Lys Val Glu Ile Lys
100 105 110

Arg

<210> 252
<211> 117
<212> PRT
<213> Artificial Sequence

<220>
<221> source
<223> /note="Description of Artificial Sequence: Synthetic Humanized 47H4 V.1,2 heavy chain variable sequence"

<400> 252

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Asp Tyr
20 25 30

```
Gly Met Ala Trp Val Arg Gln Ala Pro Gly Lys Gly Leu Glu Trp Val
        35                  40                  45

Ala Phe Ile Ser Asp Leu Ala Tyr Thr Ile Tyr Tyr Ala Asp Thr Val
    50                  55                  60

Thr Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Asn Trp Asp Ala Met Asp Tyr Trp Gly Gln Gly Thr Leu
            100                 105                 110

Val Thr Val Ser Ser
        115
```

<210> 253
<211> 113
<212> PRT
<213> Artificial Sequence

<220>
<221> source
<223> /note="Description of Artificial Sequence: Synthetic Humanized 47H4 V.1,3 light chain variable sequence"

<400> 253

```
Asp Ile Gln Met Thr Gln Ser Pro Ser Ser Leu Ser Ala Ser Val Gly
1               5                   10                  15

Asp Arg Val Thr Ile Thr Cys Arg Ser Ser Gln Ser Leu Val His Asn
            20                  25                  30

Asn Gly Asn Thr Tyr Leu His Trp Tyr Gln Gln Lys Pro Gly Lys Ala
        35                  40                  45

Pro Lys Leu Leu Ile Tyr Lys Val Ser Asn Arg Phe Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Ser Gln Asn
                85                  90                  95
```

Thr Leu Val Pro Trp Thr Phe Gly Gln Gly Thr Lys Val Glu Ile Lys
100 105 110

Arg

<210> 254
<211> 11
<212> PRT
<213> Artificial Sequence

<220>
<221> source
<223> /note="Description of Artificial Sequence: Synthetic peptide"

<400> 254

Arg Ser Ser Gln Asp Ile Ser Asn Ser Leu Asn
1 5 10

<210> 255
<211> 7
<212> PRT
<213> Artificial Sequence

<220>
<221> source
<223> /note="Description of Artificial Sequence: Synthetic peptide"

<400> 255

Ser Thr Ser Arg Leu His Ser
1 5

<210> 256
<211> 9
<212> PRT
<213> Artificial Sequence

<220>
<221> source
<223> /note="Description of Artificial Sequence: Synthetic peptide"

<400> 256

Gln Gln Gly His Thr Leu Pro Trp Thr
1 5

<210> 257
<211> 10
<212> PRT
<213> Artificial Sequence

<220>
<221> source
<223> /note="Description of Artificial Sequence: Synthetic peptide"

<400> 257

```
Gly Tyr Thr Phe Thr Asp Tyr Tyr Met Met
1               5                   10
```

<210> 258
<211> 19
<212> PRT
<213> Artificial Sequence

<220>
<221> source
<223> /note="Description of Artificial Sequence: Synthetic peptide"

<400> 258

```
Gly Asp Asn Ile Asp Pro Asn Asn Tyr Asp Thr Ser Tyr Asn Gln Lys
1               5                   10                  15

Phe Lys Gly
```

<210> 259
<211> 5
<212> PRT
<213> Artificial Sequence

<220>
<221> source
<223> /note="Description of Artificial Sequence: Synthetic peptide"

<400> 259

```
Ala Ser Lys Ala Tyr
1               5
```

<210> 260
<211> 11
<212> PRT
<213> Artificial Sequence

<220>
<221> source
<223> /note="Description of Artificial Sequence: Synthetic peptide"

<400> 260

```
Arg Ser Ser Gln Asp Ile Ser Asn Ala Leu Asn
1               5                   10
```

<210> 261
<211> 10
<212> PRT
<213> Artificial Sequence

<220>
<221> source
<223> /note="Description of Artificial Sequence: Synthetic peptide"

<400> 261

```
Gly Tyr Thr Phe Thr Asp Tyr Tyr Ile Met
1               5                   10
```